(19) Europäisches Patentamt
European Patent Office
Office européen des brevets

(11) Publication number: **0 236 618 B1**

# (12) EUROPEAN PATENT SPECIFICATION

(45) Date of publication of patent specification: **27.02.91**

(51) Int. Cl.[5]: **A01N 33/26**, C07C 243/38, C07C 255/49, C07C 255/32, C07C 271/42, C07C 281/00, C07C 305/02, C07C 317/14, C07C 323/62, C07C 327/00, C07C 331/00

(21) Application number: **86308068.5**

(22) Date of filing: **17.10.86**

The file contains technical information submitted after the application was filed and not included in this specification

## (54) Insecticidal N'-substituted-N,N'-diacylhydrazines.

(30) Priority: **21.10.85 US 789797**
**24.09.86 US 911177**

(43) Date of publication of application:
**16.09.87 Bulletin 87/38**

(45) Publication of the grant of the patent:
**27.02.91 Bulletin 91/09**

(84) Designated Contracting States:
**AT BE CH DE ES FR GB GR IT LI LU NL SE**

(56) References cited:
**DD-A- 212 889**
**DE-A- 1 668 893**
**DE-A- 2 545 788**
**DE-A- 2 708 886**
**DE-A- 2 757 584**

(73) Proprietor: **ROHM AND HAAS COMPANY**
**Independence Mall West**
**Philadelphia Pennsylvania 19105(US)**

(72) Inventor: **Hsu, Adam Chi-Tung**
**1686 Heebner Way**
**Landsdale Pennsylvania 19446(US)**
Inventor: **Aller, Harold Ernest**
**3036 Arrowhead Lane**
**Norristown Pennsylvania 19401(US)**

(74) Representative: **Angell, David Whilton et al**
**ROHM AND HAAS (UK) LTD. European Operations Patent Department Lennig House 2**
**Mason's Avenue**
**Croydon CR9 3NB(GB)**

**EP 0 236 618 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

AUSTRALIAN JOURNAL OF CHEMISTRY, vol. 25, 1972, pages 524-529, Melbourne, AU; Q.N. PORTER et al.: "Mass spectrometric studies XI. Skeletal rearrangements in acyl-hydrazines"

JOURNAL FÜR PRAKTISCHE CHEMIE, vol. 36, 1967, pages 197-201; R. BIELA et al.: "Säurezersetzung von Azoxyalkanen"

HELVETICA CHIMICA ACTA, vol. 61, no. 4, 1978, pages 1477-1507; M. MÄRKY et al.: "Zum photochemischen Verhalten von Sydnonen und 1,3,4-Oxadiazolin-2-onen"

## Description

This invention is concerned with N'-substituted-N,N'-diacylhydrazines which are useful as insecticides, compositions containing those compounds and methods of their use. The invention is also directed to certain of the disclosed hydrazines as new compounds.

The search for compounds which have a combination of excellent insecticidal activity and low undesirable toxicity is a continuing one because of factors such as the desire for compounds exhibiting greater activity, better selectivity, low undesirable environmental impact, low production cost and effectiveness against insects resistant to many known insecticides.

Compounds of the present invention are particularly suitable for controlling plant-destructive insects in crops of cultivated plants, ornamentals and forestry.

Certain hydrazine derivatives have been disclosed in the literature.

In 25 Aust. J. Chem. , 523-529 (1972), several N,N'-dibenzoylhydrazine derivatives are disclosed including N'-i -propyl-; N'-n -propyl-; N'-(2-methylpropyl)-; N'-(3-methylbutyl)-; N'-benzyl- and N'-phenyl-N,N'-dibenzoylhydrazine in which one or both nitrogen atoms are alkylated or phenylated. No biological activity is disclosed for those compounds.

In 61 Helv. Chim. Acta , 1477-1510 (1978), several N,N'-dibenzoylhydrazine and hydrazide derivatives including N'-t -butyl-N-benzoyl-N'-(4'-nitrobenzoyl)hydrazine are disclosed. No biological activity is disclosed for those compounds.

In 44 J.A.C.S. , 2556-2567 (1922), isopropylhydrazine $(CH_3)_2CH\text{-}NH\text{-}NH_2$, symmetrical diisopropyl hydrazine, dibenzoylisopropylhydrazine and certain derivatives are disclosed. No biological activity is disclosed for those compounds.

In 44 J.A.C.S. , 1557-1564 (1972), isopropyl, menthyl and bornyl semicarbazides are disclosed. No biological activity is disclosed for those compounds.

In 48 J.A.C.S. , 1030-1035 (1926), symmetrical di-methylphenylmethylhydrazine and certain related compounds including 1,2-bis-methylphenylmethyl-4-phenylsemicarbazide are disclosed. No biological activity is disclosed for those compounds.

In 27 Bull. Chem. Soc. Japan , 624-627 (1954), certain hydrazine derivatives including alpha,beta-dibenzoylphenylhydrazine are disclosed. No biological activity is disclosed for those compounds.

In J. Chem. Soc. (C) , 1531-1536 (1966), N,N'-dibenzoylphenylhydrazine and N-acetyl-N'-benzoyl-p-nitrophenylhydrazine are disclosed. No biological activity is disclosed for those compounds.

In 56B Chem. Berichte , 954-962 (1923), symmetrical di-isopropylhydrazines, symmetrical diisobutyl- and certain derivatives including N,N'-diisobutyldibenzoylhydrazine are disclosed. No biological activity is disclosed for those compounds.

In 590 Annalen der Chemie , 1-36 (1954), certain N,N'-dibenzoylhydrazine derivatives are disclosed including N'-methyl- and N'-(2-phenyl)-isopropyl-N,N'-dibenzoylhydrazine. No biological activity is disclosed for those compounds.

In J. Chem. Soc. , 4191-4198 (1952), N,N'-di-n -propylhydrazine, N-N'-dibenzoylhydrazine and bis-3,5-dinitrobenzoyl are disclosed. No biological activity is disclosed for those compounds.

In 32 Zhur. Obs. Khim. , 2806-2809 (1962), N'-2,4-methyl-2,4-pentadiene-N,N'-dibenzoylhydrazine is disclosed. No biological activity is disclosed.

In 17 Acta. Chim. Scand. , 95-102 (1963), 2-benzoylthiobenzhydrazide ($C_6H_5\text{-}CS\text{-}NHNH\text{-}CO\text{-}C_6H_5$) and certain hydrazone and hydrazine derivatives are disclosed including 1,2-dibenzoyl-benzylhydrazine. No biological activity is disclosed for those compounds.

In 25 Zhur. Obs. Khim , 1719-1723 (1955), N,N'-bis-cyclohexylhydrazine and N,N'-dibenzoylcyclohexylhydrazine are disclosed. No biological activity is disclosed for those compounds.

In J. Chem. Soc. , 4793-4800 (1964), certain dibenzoylhydrazine derivatives are disclosed including tribenzoylhydrazine and N,N'-dibenzoylcyclohexylhydrazine. No biological activity is disclosed for those compounds.

In 36 J. Prakt. Chem. , 197-201 (1967), certain dibenzoylhydrazine derivatives including N'-ethyl-; N'-n -propyl-; N'-isobutyl-; N'-neopentyl-; N'-n -heptyl-; and N'-cyclohexylmethyl-N,N'-dibenzoylhydrazines are disclosed. No Biological activity is disclosed for those compounds.

In 26 J.O.C. , 4336-4340 (1961) N'-t -butyl-N,N'-di-( t -butoxycarbonyl)hydrazide is disclosed. No biological activity is disclosed.

In 41 J.O.C. , 3763-3765 (1976), N'-t -butyl-N-(phenylmethoxycarbonyl)-N'-(chlorocarbonyl)hydrazide is disclosed. No biological activity is disclosed.

In 94 J.A.C.S. , 7406-7416 (1972) N'-t -butyl-N,N'-dimethoxycarbonylhydrazide is disclosed. No biological activity is disclosed.

In 43 J.O.C. , 808-815 (1978), N'-t -butyl-N-ethoxycarbonyl-N'-phenylaminocarbonylhydrazide and N'-t -N-ethoxycarbonyl-N'-methylaminocarbonylhydrazide are disclosed. No biological activity is disclosed for those compounds.

In 39 J. Econ. Ent. , 416-417 (1946), certain N-phenyl-N'-acylhydrazines are disclosed and evaluated for their toxicity against codling moth larvae.

The novel N'-substituted-N,N'-diacylhydrazines of the present invention differ from known compounds primarily by their N'-substituent and their N,N'-diacyl substitutents.

Compounds of the present invention are also distinguished by their excellent insecticidal activity, particularly against insects of the orders Lepidoptera and Coleoptera, and most particularly against insects of the order Lepidoptera, without material adverse impact on beneficial insects.

In accordance with the present invention, there are provided insecticidal compositions and methods of using such compositions wherein the compositions comprise an agronomically acceptable carrier and, as insecticidally active ingredient, from 0.0001% to 99% by weight of the compositions of a compound of the formula:

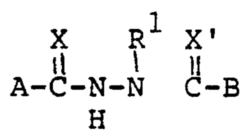

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; preferably $R^1$ has no more than 10 carbon atoms;

A and B are the same or different

unsubstituted or substituted naphthyl

where the substituents can be from one to three of the same or different halo; nitro; ($C_1$-$C_4$)alkoxy; ($C_1$-$C_4$)alkyl; or amino;

unsubstituted or substituted phenyl

where the substituents can be from one to five of the same or different halo; nitro; cyano; hydroxy; ($C_1$ to $C_6$)alkyl; halo($C_1$ to $C_6$)alkyl; cyano($C_1$ to $C_6$)alkyl; hydroxy($C_1$- to $C_6$)alkyl; ($C_1$ to $C_6$)alkoxy; halo($C_1$ to $C_6$)alkoxy; alkoxyalkyl having, independently, 1 to 6 carbon atoms in each alkyl group; alkoxyalkoxy having, independently, 1 to 6 carbon atoms in each alkyl group; -ORSR' group; -$OCO_2R$ group; alkanoyloxyalkyl having, independently, 1 to 6 carbon atoms in each alkyl group; ($C_2$-$C_6$)alkenyl,

optionally substituted with halo, cyano, ($C_1$ to $C_4$)alkyl, or ($C_1$ to $C_4$)alkoxy;

($C_2$ to $C_6$)alkenyloxy; ($C_2$ to $C_6$)alkenyl-carbonyl; ($C_2$ to $C_6$)alkenyl-oxycarbonyloxy; ($C_2$ to $C_6$)-alkynyl,

optionally substituted with halo or ($C_1$ to $C_4$)alkyl;

-$RCO_2R'$ group; -COR group; halo($C_1$ to $C_6$)alkyl-carbonyl; -$CO_2R$ group; halo ($C_1$ to $C_6$)alkoxy-carbonyl; -OCOR group; -$ORCO_2$ R' group; -NRR' group; -CONRR' group; ($C_2$ to $C_6$)alkenyl-carbonylamino; hydroxy-($C_1$ to $C_6$)alkyl-aminocarbonyl; -OCONRR' group; -NRCOR' group; -$NRCO_2R'$ group; thiocyanato; isothiocyanato; thiocyanato-($C_1$ to $C_6$)alkyl; ($C_1$ to $C_6$)alkyl-thio; -S-(O)R group; -$SO_2$ R group; -$SO_2R$ group; -$So_2NRR'$ group; -CSR group; -NRCSR' group;

unsubstituted or substituted phenyl,

where the substituents can be one to three of the same or different halo, cyano, nitro, ($C_1$ to $C_4$)-alkyl, halo($C_1$ to $C_4$)alkyl, ($C_1$ to $C_4$)alkoxy, carboxy, -$NH_2$ group, -NHZ group or -NZZ' group;

phenoxy where the phenyl ring is unsubstituted or substituted,

where the substituents can be one to three of the same or different halo, cyano, nitro, ($C_1$ to $C_4$)-alkyl, halo($C_1$ to $C_4$)alkyl, ($C_1$ to $C_4$)alkoxy, carboxy, -$NH_2$ group, -NHZ group or -NZZ' group;

benzoyl where the phenyl ring is unsubstituted or substituted,

where the substituents can be one to three of the same or different halo, cyano, nitro, ($C_1$ to $C_4$)-alkyl, halo($C_1$ to $C_4$)alkyl, ($C_1$ to $C_4$)alkoxy, carboxy, -$NH_2$ group, -NHZ group or -NZZ' group;

benzoyloxy($C_1$ to $C_6$)alkyl; phenylthio($C_1$ to $C_4$)alkyl where the phenyl ring is unsubstituted or substituted,

where the substituents can be one to three of the same or different halo, cyano, nitro ($C_1$ to $C_4$)-alkyl, halo($C_1$ to $C_4$)alkyl, ($C_1$ to $C_4$)alkoxy, carboxy, -$NH_2$ group, -NHZ group or -NZZ' group;

-CR=N-$R^2$ where $R^2$ is hydroxy, ($C_1$ to $C_4$)alkyl, ($C_1$ to $C_4$)alkoxy, amino phenylamino, -COR, or benzoyl; ($C_2$ to $C_6$)oxiranyl; pyrrolyl; acetylthiosemicarbazone; oxazolyl, optionally substituted with 1 or 2 methyl groups; or when two adjacent positions on the phenyl ring are substituted with alkoxy groups,

these groups may be joined to form, together with the carbon atoms to which they are both attached, a 5 or 6 membered dioxolano or diaxano heterocyclic ring;

where R and R' are hydrogen or ($C_1$ to $C_6$) alkyl; Z and Z' are ($C_1$ to $C_4$) alkyl; and "amino" means -NRR';

and agronomically acceptable salts thereof, provided that when X and X' are 0 and A and B are unsubstituted phenyl, $R^1$ is not isopropyl ($-CH(CH_3)_2$); 2-methylpropyl ($-CH_2CH(CH_3)_2$); 3-methylbutyl ($-CH_2CH_2CH(CH_3)_2$); neopentyl (2,2-dimethylpropyl: $-CH_2C(CH_3)_3$); or cyclohexylmethyl ($-CH_2C_6H_{11}$) and further provided that when X and X' are 0 and $R^1$ is t -butyl ($-C(CH_3)_3$) and A is unsubstituted phenyl, B is not 4-nitrophenyl.

Also in accordance with the present invention, there are provided certain novel insecticidal compounds having the formula

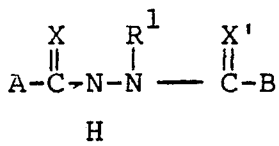

I

wherein

X and X' are the same or different; 0, S or NR; $R^1$ is unsubstituted ($c_3$-$c_{10}$) branched alkyl or a ($C_1$-$C_4$) straight chain alkyl substituted with one or two of the same or different ($C_3$-$C_6$) cycloalkyl;

preferably $R^1$ has no more than 10 carbcn atoms;

A and B are the same or different

unsubstituted naphthyl

where the substituents can be from one to three or the same or different halo; nitro; ($C_1$-$C_4$) alkoxy; ($C_1$-$C_4$) alkyl; or amino;

unsubstituted or substituted phenyl

where the substituents can be from one to five of the same or different halo, nitro; cyano; hydroxy; ($C_1$-$C_6$) alkyl; halo ($C_1$-$C_6$)-alkyl; cyano ($C_1$-$C_6$) alkyl; hydroxy ($C_1$-$C_6$) alkyl; hydroxy ($C_1$-$C_6$)-alkyl; ($C_1$-$C_6$) alkoxy; halo ($C_1$-$C_6$) alkoxy; alkoxyalkyl having independently 1 to 6 carbon atoms in each alkyl group; alkoxy-alkoxy having independently 1 to 6 carbon atoms in each alkyl group; -ORSR' group; $-OCO_2R$ group; alkanoyloxyalkyl having independently 1 to 6 carbon atoms in each alkyl group; ($C_2$-$C_6$)alkenyl,

optionally substituted with halo, cyano, ($C_1$ to $C_4$)alkyl, or ($C_1$ to $C_4$)alkoxy;

($C_2$ to $C_6$)alkenyloxy; ($C_2$-$C_6$)alkenyl-carbonyl; ($C_2$-$C_6$)alkenyl-oxycarbonyloxy; ($C_2$-$C_6$)alkynyl,

optionally substituted with halo or ($C_1$-$C_4$)alkyl;

$-RCO_2R'$ group; -COR group; halo($C_1$-$C_6$)alkyl-carbonyl; $-CO_2R$ group; halo($C_1$-$C_6$)alkoxy-carbonyl; -OCOR group; $-ORCO_2R'$ group; -NRR' group; -CONRR' group; ($C_2$-$C_6$)alkenyl-carbonylamino; hydroxy($C_1$-$C_6$)alkyl-aminocarbonyl; -OCONRR' group; -NRCOR' group; $-NRCO_2R'$ group; thiocyanato; isothiocyanato; thiocyanato($C_1$-$C_6$)alkyl; ($C_1$-$C_6$)alkyl-thio; -S(O)R group; $-SO_2R$ group; $-OSO_2R$ group; $-SO_2NRR'$ group; -CSR group; -NRCSR' group; unsubstituted or substituted phenyl,

where the substituents can be one to three of the same or different halo, cyano, nitro, ($C_1$-$C_4$)-alkyl, halo($C_1$-$C_4$)alkyl, ($C_1$-$C_4$)alkoxy, carboxy, $-NH_2$ group, NHZ group or NZZ' group;

phenoxy where the phenyl ring is unsubstituted or substituted,

where the substituents can be one to three of the same or different halo, cyano, nitro, ($C_1$-$C_4$)-alkyl, halo($C_1$-$C_4$) alkyl, ($C_1$-$C_4$)alkoxy, carboxy, $-NH_2$ group, NHZ group or NZZ' group;

benzoyl where the phenyl ring is unsubstituted or substituted,

where the substituents can be one to three of the same or different halo, cyano, nitro, ($C_1$ to $C_4$)alkyl, halo($C_1$-$C_4$)alkyl, ($C_1$-$C_4$)alkoxy, carboxy, $-NH_2$ group, NHZ group or NZZ' group;

benzoyloxy($C_1$-$C_6$)alkyl; phenylthio($C_1$-$C_6$)alkyl where the phenyl ring is unsubstituted or substituted,

where the substituents can be one to three of the same of different halo, cyano, nitro, ($C_1$-$C_4$)-alkyl, halo($C_1$-$C_4$)alkyl, ($C_1$-$C_4$)alkoxy, carboxy, -$NH_2$ group, NHZ group or NZZ' group;

-CR=N-$R^2$ group where $R^2$ is hydroxy, ($C_1$-$C_4$)alkyl, ($C_1$-$C_4$)alkoxy, amino, -NRR' group, phenylamino, -COR group, or benzoyl; ($C_2$-$C_6$)oxiranyl; pyrrolyl; oxazolyl optionally substituted with 1 or 2 methyl groups; acetylthiosemicarbazone; or, when two adjacent positions on the phenyl ring are substituted with alkoxy groups, these groups may be joined to form a 5 or 6 membered dioxolano or dioxano heterocyclic ring;

where R and R' are hydrogen or ($C_1$-$C_6$)alkyl; Z and Z' are ($C_1$ to $C_4$)alkyl; and "amino" means -NRR';

and agronomically acceptable salts thereof; provided that when X and X' are O, and A and B are unsubstituted phenyl, $R^1$ is not isopropyl (-$CH(CH_3)_2$); 2-methylpropyl (-$CH_2CH(CH_3)_2$); 3-methylbutyl (-$CH_2CH_2CH(CH_3)_2$); cyclohexylmethyl (-$CH_2C_6H_{11}$); or neopentyl (2,2-dimethylpropyl -$CH_2C(CH_3)_3$); and further provided that when X and X' are O and $R^1$ is t -butyl (-$C(CH_3)_3$) and A is unsubstituted phenyl, B is not 4-nitrophenyl.

Further, in accordance with the present invention, there are provided methods of using these compounds and compositions.

The term "halo" should be understood as including chloro, fluoro, bromo and iodo. The term "alkyl" by itself or as a part of another substituent, unless otherwise stated, includes straight or branched chain groups such as methyl, ethyl, n -propyl, isopropyl, n -butyl, t -butyl, isobutyl, neopentyl and the like and where indicated higher homologues and isomers such as n -octyl, isooctyl and the like. The term "haloalkyl" by itself or as part of another substituent is an alkyl group of the stated number of carbon atoms having one or more halo atoms bonded thereto such as chloromethyl, 1- or 2-bromoethyl, trifluoromethyl and the like. Analogously, "cyanoalkyl" by itself or as part of another group is an alkyl group of the stated number of carbon atoms having one ore more cyano groups bonded thereto; "haloalkoxy" by itself or as part of another group is an alkoxy group of the stated number of carbon atoms having one or more halo atoms bonded thereto such as difluoromethoxy, trifluoromethoxy, 2-fluoroethoxy, 2,2,2-trifluoroethoxy and the like. "Alkenyl" and "alkynyl" by themselves or as part of another substituent comprise straight and branched chain groups of the stated number of carbon atoms.

In another group of preferred compositions and compounds of the invention,

X, X' and R are as above defined and

A and B are the same or different

unsubstituted or substituted naphthyl

where the substituents can be from one to three of the same or different halo; nitro; ($C_1$-$C_4$)alkoxy; ($C_1$-$C_4$)alkyl; or amino;

unsubstituted or substituted phenyl

where the substituents can be from one to five of the same or different halo; nitro; cyano; hydroxy; ($C_1$ to $C_6$)alkyl; halo($C_1$ to $C_6$)alkyl; cyano($C_1$ to $C_6$)alkyl; ($C_1$ to $C_6$)alkoxy; halo($C_1$ to $C_6$)alkoxy; alkoxyalkyl having, independently, 1 to 6 carbon atoms in each alkyl group; alkoxyalkoxy having independently, 1 to 6 carbon atoms in each alkyl group; -$OCO_2$R group; ($C_2$-$C_6$)alkenyl,

optionally substituted with halo, cyano; ($C_1$ to $C_4$) alkyl, or ($C_1$ to $C_4$)alkoxy;

($C_2$ to $C_6$)alkenyl-carbonyl; ($C_2$ to $C_6$)alkynyl,

optionally substituted with halo or ($C_1$ to $C_4$)alkyl;

-$RCO_2$R' group; -COR group; halo($C_1$ to $C_6$)alkyl-carbonyl; -$CO_2$R group; halo($C_1$ to $C_6$)alkoxy-carbonyl; -OCOR group; -NRR' group; -CONRR' group; -OCONRR' group; -NRCOR' group; -$NRCO_2$R' group; thiocyanato; ($C_1$ to $C_6$)alkyl-thio; -S(O)R group; -$SO_2$R group; -$OSO_2$R group; -$SO_2$NRR' group; -CSR group; -NRCSR' group;

unsubstituted or substituted phenyl,

where the substituents can be one to three of the same or different halo, cyano, nitro, ($C_1$ to $C_4$)alkyl, ($C_1$ to $C_4$)alkoxy, carboxy, -$NH_2$ group, -NHZ group or -NZZ' group;

phenoxy where the phenyl ring is unsubstituted or substituted,

where the substituents can be one to three of the same or different halo, cyano, nitro, ($C_1$ to $C_4$)alkyl, ($C_1$ to $C_4$)alkoxy, carboxy, -$NH_2$ group, -NHZ group or -NZZ' group;

benzoyl where the phenyl ring is unsubstituted or substituted,

where the substituents can be one to three of the same or different halo, cyano, nitro, ($C_1$ to $C_4$)alkyl, ($C_1$ to $C_4$)alkoxy, carboxy, -$NH_2$ group, -NHZ group or NZZ' group;

-CR=N-$R^2$ where $R^2$ is hydroxy, ($C_1$ to $C_4$)alkyl, ($C_1$ to $C_4$)alkoxy, amino, phenylamino, -COR, or benzoyl; or, when two adjacent positions on the phenyl ring are substituted with alkoxy groups, these groups may be joined to form, together with the carbon atoms to which they are both

attached, a 5 or 6 membered dioxolano or dioxano heterocyclic ring; where R and R' are hydrogen or ($C_1$ to $C_6$)alkyl; Z and Z' are ($C_1$ to $C_4$)alkyl; and "amino" means -NRR';

and agronomically acceptable salts thereof. Typical compounds within the scope of the present invention include, but are not limited to:

N'-t -butyl-N,N'-bis(4-chlorobenzoyl)hydrazine
N'-t -butyl-N,N'-bis(3-chlorobenzoyl)hydrazine
N'-t -butyl-N,N'-dibenzoylhydrazine
N'-t -butyl-N,N'-bis(3,4-dichlorobenzoyl)hydrazine
N'-t -butyl-N,N'-bis(4-toluoyl)hydrazine
N'-t -butyl-N,N'-bis(4-nitrobenzoyl)hydrazine
N'-t -butyl-N,N'-bis(4-anisoyl)hydrazine
N'-t -butyl-N,N'-bis(3-nitrobenzoyl)hydrazine
N'-t -butyl-N,N'-bis(3-anisoyl)hydrazine
N'-t -butyl-N,N'-bis(2-nitrobenzoyl)hydrazine
N'-t -butyl-N,N'-bis(2-chlorobenzoyl)hydrazine
N'-t -butyl-N,N'-bis(2-anisoyl)hydrazine
N'-t -butyl-N-(4-toluoyl)-N'-benzoylhydrazine
N'-t -butyl-N,N'-bis(4-cyanobenzoyl)hydrazine
N'-t -butyl-N-(4-toluoyl)-N'-(4-chlorobenzoyl)hydrazine
N'-t -butyl-N-benzoyl-N'-(4-chlorobenzoyl)hydrazine
N'-t -butyl-N-benzoyl-N'-(3-chlorobenzoyl)hydrazine
N'-t -butyl-N-benzoyl-N'-(2-chlorobenzoyl)hydrazine
N'-t -butyl-N,N'-bis(3-toluoyl)hydrazine
N'-t -butyl-N,N'-bis(2-toluoyl)hydrazine
N'-t -butyl-N-benzoyl-N'-(4-toluoyl)hydrazine
N'-t -butyl-N-benzoyl-N'-(3-toluoyl)hydrazine
N'-t -butyl-N-benzoyl-N'-(2-toluoyl)hydrazine
N'-t -butyl-N-benzoyl-N'-(4-anisoyl)hydrazine
N'-t -butyl-N-benzoyl-N'-(3-anisoyl)hydrazine
N'-t -butyl-N-benzoyl-N'-(2-anisoyl)hydrazine
N'-t -butyl-N-benzoyl-N'-(4-n -butylbenzoyl)hydrazine
N'-t -butyl-N-benzoyl-N'-(4-cyanobenzoyl)hydrazine
N'-t -butyl-N-benzoyl-N'-(4-nitrobenzoyl)hydrazine
N'-t -butyl-N-benzoyl-N'-(3-nitrobenzoyl)hydrazine
N'-t -butyl-N-benzoyl-N'-(2-nitrobenzoyl)hydrazine
N'-t -butyl-N,N'-bis(4-t -butylbenzoyl)hydrazine
N'-t -butyl-N-(4-toluoyl)-N'-(3,4-dichlorobenzoyl)hydrazine
N'-t -butyl-N-benzoyl-N'-(4-fluorobenzoyl)hydrazine
N'-t -butyl-N-benzoyl-N'-(3-fluorobenzoyl)hydrazine
N'-t -butyl-N-benzoyl-N'-(2-fluorobenzoyl)hydrazine
N'-t -butyl-N-benzoyl-N'-(2,4-dichlorobenzoyl)hydrazine
N'-isopropyl-N,N'-dibenzoylhydrazine
N'-t -butyl-N-benzoyl-N'-(4-trifluoromethylbenzoyl)hydrazine
N'-t -butyl-N-benzoyl-N'-(3-trifluoromethylbenzoyl)hydrazine
N'-t -butyl-N-benzoyl-N'-(2-trifluoromethybenzoyl)hydrazine
N'-t -butyl-N-benzoyl-N'-(2,5-difluorobenzoyl)hydrazine
N'-(2,2-dimethylethyl)-N,N'-dibenzoylhydrazine
N'-t -butyl-N-benzoyl-N'-(3-cyanobenzoyl)hydrazine
N'-(1-methylpropyl)-N,N'-dibenzoylhydrazine
N'-t -butyl-N-benzoyl-N'-(2,6-difluorobenzoyl)hydrazine
N'-t -butyl-N-(4-chlorobenzoyl)-N'-benzoylhydrazine
N'-t -butyl-N-benzoyl-N'-(3,4-dichlorobenzoyl)hydrazine
N'-t -butyl-N-benzoyl-N'-(3,5-dichlorobenzoyl)hydrazine
N'-t -butyl-N-benzoyl-N'-(2,6-dichlorobenzoyl)hydrazine
N'-t -butyl-N-(4-t -butylbenzoyl)-N'-benzoylhydrazine
N'-t -butyl-N-(2-chlorobenzoyl)-N'-benzoylhydrazine
N'-t -butyl-N-(1-naphthoyl)-N'-benzoylhydrazine
N'-t -butyl-N,N'-dinaphthoylhydrazine

N'-t -butyl-N-(3-chlorobenzoyl)-N'-benzoylhydrazine
N'-t -butyl-N-(4-chlorobenzoyl)-N'-(3,4-dichlorobenzoyl)hydrazine
N'-t -butyl-N-(2-chlorobenzoyl)-N'-(3,4-dichlorobenzoyl) hydrazine
N'-t -butyl-N-(2-toluoyl)-N'-benzoylhydrazine
N'-t -butyl-N-benzoyl-N'-(2-chloro-4-nitrobenzoyl)hydrazine
N'-t -butyl-N-benzoyl-N'-(3,5-dinitrobenzoyl)hydrazine
N'-t -butyl-N-benzoyl-N'-(2,3-dichlorobenzoyl)hydrazine
N'-(1,2,2-trimethylethyl)-N,N'-dibenzoylhydrazine
N'-t -butyl-N-benzoyl-N'-(2-chloro-5-methylbenzoyl)hydrazine
N'-t -butyl-N-benzoyl-N'-(3,5-dimethylbenzoyl)hydrazine
N'-t -butyl-N-benzoyl-N'-(2-nitro-5-methylbenzoyl)hydrazine
N'-t -butyl-N-benzoyl-N'-(2-methyl-3-chlorobenzoyl)hydrazine
N'-t -butyl-N-benzoyl-N'-(3-chloro-4-methylbenzoyl)hydrazine
N'-t -butyl-N-benzoyl-N'-(2-nitro-3-chlorobenzoyl)hydrazine
N'-t -butyl-N-benzoyl-N'-(3-methoxy-4-nitrobenzoyl)hydrazine
N'-t -butyl-N-benzoyl-N'-(2-nitro-3-methoxybenzoyl)hydrazine
N'-t -butyl-N-benzoyl-N'-(2,4-dinitrobenzoyl)hydrazine
N'-t -butyl-N-(4-chlorobenzoyl)-N'-(2-chlorobenzoyl)hydrazine
N'-t -butyl-N-(4-chlorobenzoyl)-N'-(3-chlorobenzoyl) hydrazine
N'-t -butyl-N-(4-chlorobenzoyl)-N'-(4-toluoyl)hydrazine
N'-t -butyl-N-(4-chlorobenzoyl)-N'-(3,5-dichlorobenzoyl)hydrazine
N'-t -butyl-N-(4-chlorobenzoyl)-N'-(2,4-dichlorobenzoyl)hydrazine
N'-t -butyl-N-(4-chlorobenzoyl)-N'-(4-trifluoromethylbenzoyl)hydrazine
N'-t -butyl-N-benzoyl-N'-(4-methanesulfonyloxybenzoyl)hydrazine
N'-t -butyl-N-benzoyl-N'-(4-isopropylbenzoyl)hydrazine
N'-t -butyl-N-benzoyl-N'-(2-acetoxybenzoyl)hydrazine
N'-t -butyl-N-benzoyl-N'-(4-ethylbenzoyl)hydrazine
N'-t -butyl-N-benzoyl-N'-(2-bromobenzoyl)hydrazine
N'-t -butyl-N-benzoyl-N'-(4-hydroxybenzoyl)hydrazine
N'-t -butyl-N-(4-toluoyl)-N'-(2-toluoyl)hydrazine
N'-t -butyl-N-(4-toluoyl)-N'-(3-toluoyl)hydrazine
N'-t -butyl-N-(4-toluoyl)-N'-(2,4-dichlorobenzoyl)hydrazine
N'-t -butyl-N-(4-toluoyl)-N'-(3,5-dichlorobenzoyl)hydrazine
N'-t -butyl-N-(4-toluoyl)-N'-(2-chlorobenzoyl)hydrazine
N'-t -butyl-N-(4-toluoyl)-N'-(4-fluorobenzoyl)hydrazine
N'-t -butyl-N-(4-toluoyl)-N'-(4-trifluoromethylbenzoyl)hydrazine
N'-t -butyl-N-(4-toluoyl)-N'-(3-chlorobenzoyl)hydrazine
N'-t -butyl-N-(4-chlorobenzoyl)-N'-(3-chlorobenzoyl)hydrazine
N'-t -butyl-N-(4-chlorobenzoyl)-N'-(4-chloromethylbenzoyl)hydrazine
N'-t -butyl-N-(4-chlorobenzoyl)-N'-(2-toluoyl)hydrazine
N'-t -butyl-N-(4-chlorobenzoyl)-N'-(3-anisoyl)hydrazine
N'-t -butyl-N-(4-chlorobenzoyl)-N'-(3-toluoyl)hydrazine
N'-t -butyl-N,N'-bis(4-fluorobenzoyl)hydrazine
N'-t -butyl-N,N'-bis(3-fluorobenzoyl)hydrazine
N'-t -butyl-N,N'-bis(2-fluorobenzoyl)hydrazine
N'-t -butyl-N,N'-bis(2-naphthoyl)hydrazine
N'-t -butyl-N-(4-isobutylbenzoyl)-N'-(2-nitrobenzoyl)hydrazine
N'-t -butyl-N-(2-bromobenzoyl)-N'-(4-ethenylbenzoyl)hydrazine
N'-t -butyl-N-(4-toluoyl)-N'-(4-ethynylbenzoyl)hydrazine
N'-t -butyl-N-[4-(1-hydroxy-2-propynyl)benzoyl]-N'-(3,4-methylenedioxybenzoyl)hydrazine
N'-t -butyl-N-(3-phenoxybenzoyl)-N'-(2-bromobenzoyl)hydrazine
N'-t -butyl-N-(2,4-dichlorobenzoyl)-N'-(4-trifluoromethoxybenzoyl)hydrazine
N'-t -butyl-N-(4-ethylbenzoyl)-N'-(2-difluoromethoxy-4-chlorobenzoyl)hydrazine
N'-isopropyl-N'-(4-chloro-2-bromobenzoyl)-N-benzoyl)hydrazine
N'-(2,2-dimethylethyl)-N-(3-bromomethylbenzoyl)-N'-(4-isopropyloxybenzoyl)hydrazine
N'-t -butyl-N-(4-chloromethylbenzoyl)-N'-(2-carboxybenzoyl)hydrazine
N'-(1-methylpropyl)-N-(4-carboxybenzoyl)-N'-(3,4,5-trichlorobenzoyl)hydrazine
N'-t -butyl-N-(4-propanoylbenzoyl)-N'-[4-(4-pentenyl)-benzoyl]hydrazine

N'-(1,2,2,-trimethylpropyl)-N-[2-(ethoxy-1-ethoxyl)-benzoyl]-N'-[4-(2-ethylbutanoyl)-benzoyl]hydrazine
N'-t -butyl-N-(6-bromo-2-naphthoyl)-N'-(4-benzoylbenzoyl)hydrazine
N'-isopropyl-N-(4-(2[pentynoyl)benzoyl)-N'-(3-nitrobenzoyl)hydrazine
N'-(2,2-dimethylpropyl)-N-(4-t -butyloxycarbonylbenzoyl)-N'-(4-chloro-3-trifluoromethoxybenzoyl)hydrazine
N'-t -butyl-N-(2-benzyloxycarbonylbenzoyl)-N'-(2-methoxy-4-bromobenzoyl)hydrazine
N'-t -butyl-N-(4-(2,2,2-trifluoroethoxycarbonyl)-3-methyl-benzoyl)-N'-(2,4-dichloro-3-hydroxybenzoyl)-hydrazine
N'-isopropyl-N-(3-propanoyloxybenzoyl)-N'-(2,5-dibromobenzoyl)hydrazine
N'-(1,2,2,-trimethylpropyl)-N-(4-n -propylbenzoyl)-N'-(3-ethoxycarbonyloxybenzoyl)hydrazine
N'-t -butyl-N-(3,5-dimethylbenzoyl)-N'-(4-t -butylcarbonyl-oxybenzoyl)hydrazine
N'-(1-methylpropyl)-N-(2-aminobenzoyl)-N'-(3,4,-dichlorobenzoyl)hydrazine
N'-t -butyl-N-(4-chloro-2-trifluoromethoxybenzoyl)-N'-(4-methylaminobenzoyl)hydrazine
N'-t -butyl-N-(4-dimethylaminobenzoyl)-N'-(4-acetylaminobenzoyl)hydrazine
N'-t -butyl-N-(2-methanesulfonylaminobenzoyl)-N'(2-chloro-3-(1-(formylidene)-2-phenylhydrazine)benzoyl)-hydrazine
N'-(1-methylpropyl)-N-(2-aminocarbonylbenzoyl)-N'-(2-chloro-4-ethylaminocarbonylbenzoyl)hydrazine
N'-isopropyl-N-(4-methyl-3-dimethylaminocarbonylbenzoyl)-N'-(4-trifluoromethylbenzoyl)hydrazine
N'-(1,2,2-trimethylpropyl)-N-(4-trifluoromethoxy-2-chlorobenzoyl)-N'-(4-methoxycarbonylaminobenzoyl)-hydrazine
N'-t -butyl-N-(2-carboxymethylbenzoyl)-N'-(4-dimethylaminocarbonyloxybenzoyl)hydrazine
N'-t -butyl-N-(3-methylaminocarbonyloxybenzoyl)-N'-(2-chloro-4-(N-acetoxyaminocarbonyloxy)-benzoylhydrazine
N'-isopropyl-N-(4-methoxy-3-bromobenzoyl)-N'-(4-sulfhydrylbenzoyl)hydrazine
N'-(2,2-dimethylpropyl)-N-(2-methylthiobenzoyl)-N'-(2-chloro-4-(1,3-dioxolano-2-yl)benzoyl)hydrazine
N'-t -butyl-N-(3-methanesulfinylbenzoyl)-N'-(3,4,5-trimethoxybenzoyl)hydrazine
N'-(1,2,2-trimethylpropyl)-N-(3-phenylsulfonylbenzoyl)-N'-(3,4-dichlorobenzoyl(hydrazine
N'-t -butyl-N-(2-iodobenzoyl)-N'-(4-aminosulfonyloxybenzoyl)hydrazine
N'-(1,2,2-trimethylpropyl)-N-(4-acetylthiobenzoyl)-N'-(3,4-dichlorobenzoyl)hydrazine
N'-t -butyl-N-(3-methylthiocarbonylbenzoyl)-N'-(4-pentafluoroethoxybenzoyl)hydrazine
N'-t -butyl-N-(pentafluorobenzoyl)-N'-(4-phenylaminobenzoyl)hydrazine
N'-t -butyl-N-(5-chlorophenylbenzoyl)-N'-(3-chloro-4-acetylaminobenzoyl)hydrazine
N'-(1-methylpropy)-N-(4-fluoro-3-bromochloromethylbenzoyl)-N'-(3-cyanomethylbenzoyl)hydrazine
N'-t -butyl-N-((4-n -propyl)thiobenzoyl)-N'-(3,4-dichlorobenzoyl)hydrazine
N'-t -butyl-N-(4-chloromethylcarbonylbenzoyl)-N'-(2-bromobenzoyl)hydrazine
N'-t -butyl-N-(3-trichloroethenylbenzoyl)-N'-(4-fluorobenzoyl)hydrazine
N'-isopropyl-N-(4-(1,3-dimethylbutyl)benzoyl)-N'-(2-nitrobenzoyl)hydrazine
N'-isopropyl-N-(2,6-dichlorobenzoyl)-N'-(4-trifluoromethoxybenzoyl)hydrazine
N'-t -butyl-(2,3,4-trichlorobenzoyl)-N'-(2-nitrobenzoyl)hydrazine
N'-t -butyl-N-benzoyl-N'-(4-bromobenzoyl)hydrazine
N'-t -butyl-N-benzoyl-N'-(3-bromobenzoyl)hydrazine
N'-t -butyl-N-benzoyl-N'-(4-n butylbenzoyl)hydrazine
N'-t -butyl-N-(4-ethylbenzoyl)-N'-benzoyl hydrazine
N'-t -butyl-N-(3,4-dichlorobenzoyl)-N'-benzoyl hydrazine
N'-t -butyl-N-benzoyl-N'-(4-acetylbenzoyl)hydrazine
N'-(2,2-dimethylpropyl)-N-benzoyl-N'-(2-bromobenzoyl)hydrazine
N'-(2,2-dimethylpropyl)-N-benzoyl-N'-(2-nitrobenzoyl)hydrazine
N'-(2,2-dimethylpropyl)-N-benzoyl-N'-(2-methoxybenzoyl)hydrazine
N'-t -butyl-N-benzoyl-N'-(2-iodobenzoyl)hydrazine
N'-(2-methylpropyl)-N,N'-dibenzoylhydrazine
N'-isopropyl-N-benzoyl-N'-(2-bromobenzoyl)hydrazine
N'-isopropyl-N-benzoyl-N'-(3,4-dichlorobenzoyl)hydrazine
N'-t -butyl-N-benzoyl-N'-(4-phenoxybenzoyl)hydrazine
N'-t -butyl-N-(4-trifluoromethylbenzoyl)-N'-benzoylhydrazine
N'-t -butyl-N-(4-trifluoromethylbenzoyl)-N'-(3,4-dichlorobenzoyl)hydrazine
N'-dicyclopropylmethyl-N,N'-dibenzoylhydrazine
N'-t -butyl-N-benzoyl-N'-(2-chloro-4-bromobenzoyl)hydrazine
N'-t -butyl-N-((4-chloro)thiobenzoyl)-N'-benzoylhydrazine
N'-t -butyl-N-benzoyl-N'-(thiobenzoyl)hydrazine

N'-t -butyl-N-benzoyl-N'-(4-phenylbenzoyl)hydrazine
N'-t -butyl-N-benzoyl-N'-(3,4,5-trimethoxybenzoyl)hydrazine
N'-(1,2,2-trimethylpropyl)-N-benzoyl-N'-(2-nitrobenzoyl)hydrazine
N'-t -butyl-N-benzoyl-N'-(3-cyanothiomethylbenzoyl)hydrazine
N'-t -butyl-N-benzoyl-N'-(3-cyanomethylbenzoyl)hydrazine
N'-(1,2,2-trimethylpropyl)-N,N'-dibenzoylhydrazine N'-(diisopropylmethyl)-N,N'-dibenzoylhydrazine
N'-(1-cyclopropylethyl)-N,N'-dibenzoylhydrazine
N'-t -butyl-N-benzoyl-N'-(4-n -butylbenzoyl)hydrazine
N'-t -butyl-N-(4-ethylbenzoyl)-N'-(3-toluoyl)hydrazine
N'-t -butyl-N-(4-ethylbenzoyl)-N'-(4-chlorobenzoyl)hydrazine
N'-t -butyl-N-(4-ethylbenzoyl)-N'-(2-nitrobenzoyl)hydrazine
N'-t -butyl-N-benzoyl-N'-(3-toluoyl)hydrazine
N'-t -butyl-N-(4-ethylbenzoyl)-N'-(3-bromobenzoyl)hydrazine
N'-t -butyl-N-(4-ethylbenzoyl)-N'-(2-iodobenzoyl)hydrazine
N'-(1,2,2-trimethylpropyl)-N-benzoyl-N'-(2-bromobenzoyl)hydrazine
N'-t -butyl-N-benzoyl-N'-(4-carbomethoxybenzoyl)hydrazine
N'-t -butyl-N-(2-bromobenzoyl)-N'-benzoylhydrazine
N'-t -butyl-N-(2-trifluoromethylbenzoyl)-N'-benzoylhydrazine
N'-t -butyl-N-benzoyl-N'-(3-iodobenzoyl)hydrazine
N'-t -butyl-N-benzoyl-N'-(2-ethylbenzoyl)hydrazine
N'-t -butyl-N-benzoyl-N'-(3-methoxymethoxybenzoyl)hydrazine
N,'-dibenzoyl-N'-(1-cyclohexylethyl)hydrazine
N-benzoyl-N'-t -N'-(4-allyloxybenzoyl)hydrazine
N,N'-bis(4-phenylbenzoyl)-N'-t -butylhydrazine
N-benzoyl-N'-t -butyl-N'-(4-(4-trifluoromethyl-2-chlorophenyl)-2-nitrobenzoyl)hydrazine
N-(4-(4-trifluoromethyl-2-chlorophenyl)-2-nitrobenzoyl)-N'-t -butyl-N'-benzoylhydrazine
N-(2-benzoyloxymethylbenzoyl)-N'-t -butyl-N'-benzoylhydrazine
N-benzoyl-N'-t -butyl-N'-(4-methanesulfonylbenzoyl)hydrazine
N-benzoyl-N'-t -butyl-N'-(4-methylthiobenzoyl)hydrazine
N,N-dibenzoyl-N'-(1-cyclohexylethyl)hydrazine
N-benzoyl-N'-t -butyl-N'-(4-allyloxybenzoyl)hydrazine
N,N'-bis(4-phenylbenzoyl)-N'-t -butylhydrazine
N-benzoyl-N'-t -butyl-N'-(4-(4-trifluoromethyl-2-chlorophenyl)-2-nitrobenzoyl)hydrazine
N-(4-(4-trifluoromethyl-2-chlorophenyl)-2-nitrobenzoyl)-N'-t -butyl-N'-benzoylhydrazine
N-(2-benzoyloxymethylbenzoyl)-N'-t -N'-benzoylhydrazine
N-benzoyl-N'-t -butyl-N'-(4-methanesulfonylbenzoyl)hydrazine
N-benzoyl-N'-t -butyl-N'-(4-methylthiobenzoyl)hydrazine
N-benzoyl-N'-t -butyl-N'-(2-hydroxybenzoyl)hydrazine
N-benzoyl-N'-t -butyl-N'-(3-bromo-4-methylbenzoyl)hydrazine
N-benzoyl-N'-t -butyl-N'-(3-methyl-4-bromobenzoyl)hydrazine
N-benzoyl-N'-t -butyl-N'-(2,4-dibromobenzoyl)hydrazine
N-benzoyl-N'-isopropyl-N'-(2,6-dichlorobenzoyl)hydrazine
N-benzoyl-N'-iospropyl-N'-(3,4-dichlorobenzoyl)hydrazine
N-benzoyl-N'-(1,2,2-trimethylpropyl)-N'-(4-cyanobenzoyl)hydrazine
N-benzoyl-N'-isopropyl-N'-(4-ethylbenzoyl)hydrazine
N-benzoyl-N'-t -butyl-N'-(3-(4-methylphenylthiomethyl)benzoylhydrazine
N-benzoyl-N'-t -butyl-N'-(4-phenoxybenzoyl)hydrazine N-benzoyl-N'-t -butyl-N'-(4-methoxycarbonyloxymethylbenzoyl)hydrazine
N-benzoyl-N'-t -butyl-N'-(4-hydroxybenzoyl)hydrazine
N-benzoyl-N'-t -butyl-N'-(4-formylbenzoyl)hydrazine
N-benzoyl-N'-t -butyl-N'-(4-carboxybenzoyl)hydrazine
N-benzoyl-N'-(1,2,2-trimethylpropyl)-N'-(2-hydroxybenzoyl)hydrazine
N-benzoyl-N'-t -butyl-N'-(4-(2,2-dichlorovinyl)benzoyl)hydrazine
N-benzoyl-N'-t -N'-(2-benzoyloxybenzoyl)hydrazine
N-3,4-dimethoxybenzoyl)-N'-t -butyl-N'-benzoylhydrazine
N,N'-bis(2-chloromethylbenzoyl)-N'-t -butylhydrazine
N-(2-chloromethylbenzoyl)-N'-t -butyl-N'-benzoylhydrazine
N-(4-n -propylbenzoyl)-N'-t -butyl-N'-benzoylhydrazine

N-(2-nitrobenzoyl)-N'-t -butyl-N'-benzoylhydrazine
N-(2-methyl-5-trifluoromethylbenzoyl)-N'-t -butyl-N'-(2-toluoyl)hydrazine
N,N'-bis(2-bromobenzoyl)-N'-t -butylhydrazine
N-(4-toluoyl)-N'-t -butyl-N'-(3-ethylbenzoyl)hydrazine
N-benzoyl-N'-t -butyl-N'(4-n-propylbenzoyl)hydrazine
N-(4-toluoyl)-N'-t -butyl-N'-(3-bromobenzoyl)hydrazine
N-(4-toluoyl)-N'-t -butyl-N'-(3-5-dimethylbenzoyl)hydrazine
N-benzoyl-N'-t -butyl-N'-(4-iodobenzoyl)hydrazine
N-(4-toluoyl)-N'-t -butyl-N'-(4-ethylbenzoyl)hydrazine
N,N'-bis(4-ethylbenzoyl)-N'-t -butylhydrazine
N-(4-toluoyl)-N'-t -N'-(3-ethylbenzoyl)hydrazine
N-benzoyl-N'-t -butyl-N'-(2-isopropylbenzoyl)hydrazine
N-(3-ethylbenzoyl)-N'-t -butyl-N'-benzoylhydrazine
N-benzoyl-N'-(1,2,2-trimethylpropyl)-N'-(3-nitrobenzoyl)hydrazine
N-benzoyl-N'-(2,2-dimethylpropyl)-N'-(3-nitrobenzoyl)hydrazine
N-benzoyl-N'-(2,2-dimethylpropyl)-N'-(3-toluoyl)hydrazine
N-(4-ethylbenzoyl)-N'-t -butyl-N'-(2,4-dichlorobenzoyl)hydrazine
N-(3,.4-dichlorobenzoyl)-N'-t -butyl-N'-(4-chlorobenzoyl)hydrazine
N-(4-heptylbenzoyl)-N'-t -butyl-N'-(4-chlorobenzoyl)hydrazine
N-(4-n-propylbenzoyl)-N'-t -butyl-N'-(4-chlorobenzoyl)hydrazine
N-(4-methoxybenzoyl)-N'-t -butyl-N'-(3-toluoyl)hydrazine
N-(4-methoxybenzoyl)-N'-t -butyl-N'-benzoylhydrazine
N-(2-chlorobenzoyl)-N'-t -butyl-N'-(3-toluoyl)hydrazine
N-(2-chlorobenzoyl)-N'-t -butyl-N'-(2-nitrobenzoyl)hydrazine
N-(2-chlorobenzoyl)-N'-t -N'-(4-toluoyl)hydrazine
N-(2-chlorobenzoyl)-N'-t -butyl-N'-(4-ethylbenzoyl)hydrazine
N-(4-ethylbenzoyl)-N'-t -butyl-N'-(3,5-dimethylbenzoyl)hydrazine
N-(4-ethylbenzoyl)-N'-t -butyl-N'-(3-methyl-6-chlorobenzoyl)hydrazine
N-benzoyl-N'-t -butyl-N'-(3-acetyloxybenzoyl)hydrazine
N-benzoyl-N'-t -butyl-N'-(2-hydroxylbenzoyl)hydrazine
N-(4-ethylbenzoyl)-N'-t -butyl-N'-(2-nitro-3-methylbenzoyl)hydrazine
N-(4-methoxybenzoyl)-N'-t -butyl-N'-(3,5-dimethylbenzoyl)hydrazine
N-(4-methoxybenzoyl)-N'-t -butyl-N'-(2,4-dichlorobenzoyl)hydrazine
N-(2-chlorobenzoyl)-N'-t -butyl-N'-(2,6-dichlorobenzoyl)hydrazine
N-(2-chlorobenzoyl)-N'-t -butyl-N'-(2-bromobenzoyl)hydrazine
N-(2-chlorobenzoyl)-N'-t -butyl-N'-(2,4-difluorobenzoyl)hydrazine
N-(2-chlorobenzoyl)-N'-t -butyl-N'-(4-methoxybenzoyl)hydrazine
N-(2-chlorobenzoyl)-N'-t -butyl-N'-(2-methylbenzoyl)hydrazine
N-(2-fluorobenzoyl)-N'-t -butyl-N'-(4-chlorobenzoyl)hydrazine
N-(2,4-dichlorobenzoyl)-N'-t -butyl-N'-(4-chlorobenzoyl)hydrazine
N-(2,4-dichlorobenzoyl)-N'-t -butyl-N'-(2,4-dichlorobenzoyl)hydrazine
N-(2,4-dichlorobenzoyl)-N'-t -butyl-N'-(3-methoxybenzoyl)hydrazine
N-(2,4-dichlorobenzoyl)-N'-t -butyl-N'-(3-chlorobenzoyl)hydrazine
N-(2,4-dichlorobenzoyl)-N'-t -butyl-N'-(2-trifluoromethylbenzoyl)hydrazine
N-(2,4-dichlorobenzoyl)-N'-t -butyl-N'-(3-trifluoromethylbenzoyl)hydrazine
N-(2,4-dichlorobenzoyl)-N'-t -butyl-N'-(4-trifluoromethylbenzoyl)hydrazine
N-(3-toluoyl)-N'-t -butyl-N'-benzoylhydrazine
N-(3-nitrobenzoyl)-N'-t -butyl-N'-benzoylhydrazine
N-(2,6-dichlorobenzoyl)-N'-t -butyl-N'-benzoylhydrazine
N-(2,4-difluorobenzoyl)-N'-t -butyl-N'-benzoylhydrazine
N-(2-chlorobenzoyl)-N'-t -butyl-N'-(4-cyanobenzoyl)hydrazine
N-(2-chlorobenzoyl)-N'-t -butyl-N'-(4-fluorobenzoyl)hydrazine
N-(2-chlorobenzoyl)-N'-t -butyl-N'-(4-bromobenzoyl)hydrazine
N-(2-chlorobenzoyl)-N'-t -butyl-N'-(4-chlorobenzoyl)hydrazine
N-(2-chlorobenzoyl)-N'-t -butyl-N'-(2-methoxybenzoyl)hydrazine
N-(2-chlorobenzoyl)-N'-t -butyl-N'-(4-nitrobenzoyl)hydrazine
N-(2-chlorobenzoyl)-N'-t -butyl-N'-(2-fluorobenzoyl)hydrazine
N-(2-chlorobenzoyl)-N'-t -butyl-N'-(2,6-dichlorobenzoyl)hydrazine

N-(4-nitrobenzoyl)-N'-t -butyl-N'-benzoylhydrazine
N-(4-cyanobenzoyl)-N'-t -butyl-N'-benzoylhydrazine
N,N'-bis(3-methoxybenzoyl)-N'-t -butylhydrazine
N-(3-methoxybenzoyl)-N'-t -butyl-N'-(4-chlorobenzoyl)hydrazine
N-(3-methoxybenzoyl)-N'-t -butyl-N'-(3,4-dichlorobenzoyl)hydrazine
N-(2-methoxybenzoyl)-N'-t -butyl-N'-benzoylhydrazine
N-(2-methoxybenzoyl)-N'-t -butyl-N'-(3-toluoyl)hydrazine
N-(2-methoxybenzoyl)-N'-t -butyl-N'-(3,4-dichlorobenzoyl)hydrazine
N-(2-methoxybenzoyl)-N'-t -butyl-N'-(4-chlorobenzoyl)hydrazine
N-(2-methoxybenzoyl)-N'-t -butyl-N'-(2-nitrobenzoyl)hydrazine
N-benzoyl-N'-t -butyl-N'-(4-trifluoromethoxybenzoyl)hydrazine
N-(4-trifluoromethoxybenzoyl)-N'-t -butyl-N'-benzoylhydrazine
N-(4-trifluoromethoxybenzoyl)-N'-t -butyl-N'-(3-toluoyl)hydrazine
N-(4-trifluoromethoxybenzoyl)-N'-t -butyl-N'-(4-chlorobenzoyl)hydrazine
N-(4-trifluoromethoxybenzoyl)-N'-t -butyl-N'-(3,4-dichlorobenzoyl)hydrazine
N-(4-trifluoromethylbenzoyl)-N'-t -butyl-N'-(4-chlorobenzoyl)hydrazine
N-(4-trifluoromethylbenzoyl)-N'-t -butyl-N'-(3-toluoyl)hydrazine
N,N'-dibenzoyl-N'-(1,1-dimethylpentyl)hydrazine
N-(4-ethoxybenzoyl)-N'-t -butyl-N'(3-toluoyl)hydrazine
N-(4-ethoxybenzoyl)-N'-t -butyl-N'-(3,5-dimethylbenzoyl)hydrazine
N-(4-ethoxybenzoyl)-N'-t -butyl-N'-benzoylhydrazine
N-(4-ethylbenzoyl)-N'-t -butyl-N'-(2-nitro-4-chlorobenzoyl)hydrazine
N-(4-methoxy-3-chlorobenzoyl)-N'-t -butyl-N'-benzoylhydrazine
N-(4-methylthiobenzoyl)-N'-t -butyl-N'-benzoylhydrazine
N-(4-n -butoxybenzoyl)-N'-t -butyl-N'-(4-chlorobenzoyl)hydrazine
N-(2-methylthiobenzoyl)-N'-t -butyl-N'-benzoylhydrazine
N-(2-nitro-4-chlorobenzoyl)-N'-t -butyl-N'-benzoylhydrazine
N,N'-bis(2-nitro-4-chlorobenzoyl)-N'-t -butylhydrazine
N-(2-nitro-4-chlorobenzoyl)-N'-t -butyl-N'-(4-t -butylbenzoyl)hydrazine
N-(2-nitro-4-chlorobenzoyl)-N'-t -butyl-N'-(4-chlorobenzoyl)hydrazine
N-(4-toluoyl)-N'-t -butyl-N'-(3-chloro-6-methylbenzoyl)hydrazine
N,N'-bis(2,6-difluorobenzoyl)-N'-t -butylhydrazine
N-(4-phenoxybenzoyl)-N'-t -butyl-N'-benzoylhydrazine
N-(4-phenoxybenzoyl)-N'-t -butyl-N'-(4-toluoyl)hydrazine
N-(4-n -butylbenzoyl)-N'-t -butyl-N'-benzoylhydrazine
N-(4-n -butylbenzoyl)-N'-t -butyl-N'-(4-chlorobenzoyl)hydrazine
N-(4-isopropylbenzoyl)-N'-t -butyl-N'-benzoylhydrazine
N-(4-isopropylbenzoyl)-N'-t -butyl-N'-(3-toluoyl)hydrazine
N-(4-hydroxybenzoyl)-N'-t -butyl-N'-benzoylhydrazine
N-(4-cyanobenzoyl)-N'-t -butyl-N'-benzoylhydrazine
N-(4-cyanobenzoyl)-N'-t -butyl-N'-(3-toluoyl)hydrazine
N-(2-methyl-4-chlorobenzoyl)-N'-t -butyl-N'-benzoylhydrazine
N-(4-ethylbenzoyl)-N'-t -butyl-N'-(4-trifluoromethoxybenzoyl)hydrazine
N-benzoyl-N'-t -butyl-N'-(2,3,4,5,6-pentafluorobenzoyl)hydrazine
N-(2,3,4,5,6-pentafluorobenzoyl)-N'-t -butyl-N'-benzoylhydrazine
N-(4-cyanobenzoyl)-N'-t -butyl-N'-(3,4-dichlorobenzoyl)hydrazine
N-(2-methyl-4-chlorobenzoyl)-N'-t -butyl-N'-(3-toluoyl)hydrazine
N-(4-trifluoromethylbenzoyl)-N'-t -butyl-N'-(3,5-dimethylbenzoyl)hydrazine
N-(2-methyl-4-chlorobenzoyl)-N'-t -butyl-N'-(3,5-dimethylbenzoyl)hydrazine
N-benzoyl-N'-t-butyl-N'-(3-vinylbenzoyl)hydrazine
N-(4-ethylbenzoyl)-N'-t -butyl-N'-(3-vinylbenzoyl)hydrazine
N-(4-trifluoromethylbenzoyl)-N'-t -butyl-N'-(3-vinylbenzoyl)hydrazine
N-(4-hydroxybenzoyl)-N'-t -butyl-N'-(3-toluoyl)hydrazine
N-(4-allyloxybenzoyl)-N'-t -butyl-N'-(3-toluoyl)hydrazine
N-(4-n -propylbenzoyl)-N'-t -butyl-N'-(3,4-dichlorobenzoyl)hydrazine
N-(4-n -butylbenzoyl)-N'-t -butyl-N'-(3-toluoyl)hydrazine
N,N'-bis(4-vinylbenzoyl)-N'-t -butylhydrazine
N-(4-vinylbenzoyl)-N'-t -butyl-N'-(3-toluoyl)hydrazine

N-(2,6-difluorobenzoyl)-N'-t -butyl-N'-(3,4-dichlorobenzoyl)hydrazine
N-(2,6-difluorobenzoyl)-N'-t -butyl-N'-(3-toluoyl)hydrazine
N-(2-N,N'-diethylaminoethylbenzoyl)-N'-t -butyl-N'-(4-chlorobenzoyl)hydrazine
N,N'-dibenzoyl-N'-(1,1-dimethylpropyl)hydrazine
N-(4-chlorobenzoyl)-N'-t -butyl-N'-(2-bromobenzoyl)hydrazine
N,N'-bis(3-toluoyl)-N'-(1,1-dimethylpropyl)hydrazine
N,N'-bis(2-bromobenzoyl)-N'-(1,1-dimethylpropyl)hydrazine
N-(4-chlorobenzoyl)-N'-t -butyl-N'-(3,5-dimethylbenzoyl)hydrazine
N-(3-methylbenzoyl)-N'-t -butyl-N'-(3,5-dimethylbenzoyl)hydrazine
N-(4-chlorobenzoyl)-N'-t -butyl-N'-(4-fluorobenzoyl)hydrazine
N-(4-ethylbenzoyl)-N'-t -butyl-N'-(3,5-dimethylbenzoyl)hydrazine
N-(4-vinylbenzoyl)-N'-t -butyl-N'-(3,5-dimethylbenzoyl)hydrazine
N-(4-acetoxybenzoyl)-N'-t -butyl-N'-(3,5-dimethylbenzoyl)hydrazine
N-(3,5-dimethylbenzoyl)-N'-t -butyl-N'-benzoylhydrazine
N-(3,5-dimethylbenzoyl)-N'-t -butyl-N'-(3-toluoyl)hydrazine
N-(3,5-dimethylbenzoyl)-N'-t -butyl-N'-(4-ethylbenzoyl)hydrazine
N,N'-(4-(1-propenylbenzoyl))-N'-t -butylhydrazine
N-(4-isopropylbenzoyl)-N'-t -butyl-N'-(3,5-dimethylbenzoyl)hydrazine
N-(4-isopropylbenzoyl)-N'-t -butyl-N'-(2-bromobenzoyl)hydrazine
N-(3-chloro-4-methylbenzoyl)-N'-t -butyl-N'-benzoylhydrazine
N-(3-chloro-4-methylbenzoyl)-N'-t -butyl-N'-(3-toluoyl)hydrazine
N-(3-chloro-4-methylbenzoyl)-N'-t -butyl-N'-(4-chlorobenzoyl)hydrazine
N-(3-chloro-4-methylbenzoyl)-N'-t -butyl-N'-(3,4-dichlorobenzoyl)hydrazine
N-(4-ethylbenzoyl)-N'-(1,1-dimethylpropyl)-N'-(2-nitrobenzoyl)hydrazine
N-(2,6-difluorobenzoyl)-N'-t -butyl-N'-(2-chlorobenzoyl)hydrazine
N-(2,6-difluorobenzoyl)-N'-t -butyl-N'-(3-chlorobenzoyl)hydrazine
N-(2,6-difluorobenzoyl)-N'-t -butyl-N'-(4-chlorobenzoyl)hydrazine
N-(2,6-difluorobenzoyl)-N'-t -butyl-N'-(3,5-dimethylbenzoyl)hydrazine
N-(4-ethylbenzoyl)-N'-(1,1-dimethylpropyl)-N'-(3,5-dimethylbenzoyl)hydrazine
N-(3-trifluoromethylbenzoyl)-N'-t -butyl-N'-(4-chlorobenzoyl)hydrazine
N-(3-trifluoromethylbenzoyl)-N'-t -butyl-N'-(3,5-dimethylbenzoyl)hydrazine
N-thiobenzoyl-N'-t -butyl-N'-(4-chlorobenzoyl)hydrazine
N-thiobenzoyl-N'-t -butyl-N'-(3-toluoyl)hydrazine
N-(2-nitro-3-methoxybenzoyl)-N'-t -butyl-N'-(3-toluoyl)hydrazine
N-(3-bromo-4-methylbenzoyl)-N'-t -butyl-N'-benzoylhydrazine
N-(4-chlorobenzoyl)-N'-t -butyl-N'-(4-methoxycarbonylbenzoyl)hydrazine
N-(4-ethylbenzoyl)-N'-t -butyl-N'-(4-methoxycarbonylbenzoyl)hydrazine
N-benzoyl-N'-t -butyl-N'-(3-aminobenzoyl)hydrazine
N-benzoyl-N'-t -butyl-N'-(2-aminobenzoyl)hydrazine
N-(4-fluorobenzoyl)-N'-t -butyl-N'-benzoylhydrazine
N-(4-toluoyl)-N'-t -butyl-N'-(4-methoxycarbonylbenzoyl)hydrazine
N-(3-hydroxybenzoyl)-N'-t -butyl-N'-benzoylhydrazine
N-(3-allyloxybenzoyl-N'-t -butyl-N'-benzoylhydrazine
N-(4-toluoyl)-N'-(1,2,2-trimethylpropyl)-N'-(3,5-dimethylbenzoyl)hydrazine
N-(4-toluoyl)-N'-(1,2,2-trimethylpropyl-N'-(2-nitro-3-methylbenzoyl)hydrazine
N-(4-toluoyl-N'-(1,2,2-trimethylpropyl)-N'-(2-nitro-5-methylbenzoyl)hydrazine
N-(4-toluoyl)-N'-(1,2,2-trimethylpropyl)-N'-(2-iodobenzoyl)hydrazine
N-(4-chlorobenzoyl)-N'-t -butyl-N'-(2-fluorobenzoyl)hydrazine
N-(4-ethylbenzoyl)-N'-t -butyl-N'-(3,4-dichlorobenzoyl)hydrazine
N-(4-ethylbenzoyl)-N'-t -butyl-N'-(2-fluorobenzoyl)hydrazine
N-(4-N,N-dimethylaminocarbonyloxybenzoyl)-N'-t -butyl-N'-benzoylhydrazine
N-(4-vinyloxycarbonyloxybenzoyl)-N'-t -butyl-N'-benzoylhydrazine
N-(4-methoxycarbonyl)-N'-t -butyl-N'-(4-chlorobenzoylhydrazine
N-(4-methoxycarbonyl-N'-t -butyl-N'-(3,5-dimethylbenzoyl)hydrazine
N-(4-methoxycarbonyl)-N'-t -butyl-N'-(3-toluoyl)hydrazine
N-(4-carboxybenzoyl)-N'-t -butyl-N'-(3-toluoyl)hydrazine
N-(2-amino-3-methylbenzoyl)-N'-t -butyl-N'-(3-toluoyl)hydrazine
N-(4-aminobenzoyl)-N'-t -butyl-N'-(4-chlorobenzoyl)hydrazine

N-(4-methoxycarbonylaminobenzoyl)-N'-t -butyl-N'-(4-chlorobenzoyl)hydrazine
N-(4-acetylaminobenzoyl)-N'-t -butyl-N'-(4-chlorobenzoyl)hydrazine
N-(3-methoxy-2-acetylamino)-N'-t -butyl-N'-(3-toluoyl)hydrazine
N-(3-phenoxybenzoyl)-N'-t -butyl-N'-benzoylhydrazine
N-(3-phenoxybenzoyl)-N'-t -butyl-N'-(3-toluoyl)hydrazine
N-(4-acetylbenzoyl)-N'-t -butyl-N'-(3-toluoyl)hydrazine
N-(4-methoxymethoxybenzoyl)-N'-t -butyl-N'-benzoylhydrazine
N-(4-dimethylaminocarbonyloxybenzoyl)-N'-t -butyl-N'-benzoylhydrazine
N-(4-methoxycarbonylmethoxybenzoyl)-N'-t -butyl-N'-benzoylhydrazine
N-(4-acetyloxymethylbenzoyl)-N'-t -butyl-N'-benzoylhydrazine
N-(4-thiocyanatomethylbenzoyl)-N'-t -butyl-N'-benzoylhydrazine
N-(4-hydroxymethylbenzoyl)-N'-t -butyl-N'-benzoylhydrazine
N,N'-bis(4-bromobenzoyl)-N'-t -butylhydrazine
N-(4-methylthiomethoxybenzoyl)-N'-t -butyl-N'-benzoylhydrazine
N-(4-isobutyloxybenzoyl)-N'-t -butyl-N'-benzoylhydrazine
N-(4-cyanomethylbenzoyl)-N'-t -butyl-N'-benzoylhydrazine
N-(4-(1,2-epoxypropyl)benzoyl)-N'-t -butyl-N'-benzoylhydrazine
N-(4-acetylsemicarbazonebenzoyl)-N'-t -butyl-N'-(3-toluoyl)hydrazine
N-(4-phenylbenzoyl)-N'-t -butyl-N'-(3-toluoyl)hydrazine
N-(3-cyanobenzoyl)-N'-t -butyl-N'-(3-toluoyl)hydrazine
N-(3-aminobenzoyl)-N'-t -butyl-N'-(3-toluoyl)hydrazine
N-(4-(2-hydroxy-1,1-dimethylethylaminocarbonyl)benzoyl)-N'-t -butyl-N'-(3-toluoyl)hydrazine
N-(4-(2-hydroxyethyl)benzoyl)-N'-t -butyl-N'-(3-toluoyl)hydrazine
N-(3-methacrolyaminobenzoyl)-N'-t -butyl-N'-(3-toluoyl)hydrazine
N-(3-carboxybenzoyl)-N'-t -butyl-N'-(3-toluoyl)hydrazine
N-(3-chloromethylbenzoyl)-N'-t -butyl-N'-(3-toluoyl)hydrazine
N-(4-ethylbenzoyl)-N'-t -butyl-N'-(3-toluoyl)hydrazine
N-(2,3-dimethylbenzoyl)-N'-t -butyl-N'-(3-toluoyl)hydrazine
N-benzoyl-N'-t -butyl-N'-(2,3-dimethylbenzoyl)hydrazine
N-(4-toluoyl)-N'-t -butyl-N'-(2,3-dimethylbenzoyl)hydrazine
N-(4-toluoyl)-N'-(1,2,2-trimethylpropyl)-N'-(2-toluoyl)hydrazine
N-(4-toluoyl)-N'-(1,2,2-trimethylpropyl)-N'-(2-trifluoromethylbenzoyl)hydrazine
N-(4-toluoyl)-N'-(1,2,2-trimethylpropyl)-N'-benzoylhydrazine
N-(4-methoxymethoxybenzoyl)-N'-t -butyl-N'-(3-toluoyl)hydrazine
N-(4-(1-methylethenyl)benzoyl)-N'-t -butyl-N'-(3-toluoyl)hydrazine
N-benzoyl-N'-t -butyl-N'-(1-naphthoyl)hydrazine
N-(1-naphthoyl)-N'-t -butyl-N'-(3-toluoyl)hydrazine
N-(4-isothiocyanatobenzoyl-N'-(3-toluoyl)hydrazine
N,N'-bis(3,5-dimethylbenzoyl)-N'-t -butylhydrazine
N,N'-bis(2,4-dichlorobenzoyl)-N'-t -butylhydrazine
N-(2-fluorobenzoyl)-N'-t -butyl-N'-(2-bromobenzoyl)hydrazine
N-(2-fluorobenzoyl)-N'-t -butyl-N'-(3-toluoyl)hydrazine
N,N'-bis(2,3-dimethylbenzoyl)-N'-t -butylhydrazine
N-(2-fluorobenzoyl)-N'-t -butyl-N'-(2-nitrobenzoyl)hydrazine
N-(2-fluorobenzoyl)-N'-t -butyl-N'-benzoylhydrazine
N-(2-methyl-3-chlorobenzoyl)-N'-t -butyl-N'-(3-toluoyl)hydrazine
N-(2-methyl-3-chlorobenzoyl)-N'-t -butyl-N'-benzoylhydrazine
N-(4-toluoyl)-N'-(1,2,2,-trimethylpropyl)N'-(3,5-dimethylbenzoylhydrazine
N-(4-toluoyl)-N'-(1,2,2-trimethylpropyl)-N'-(3,4-dichlorobenzoyl)hydrazine
N-(4-bromobenzoyl)-N'-t -butyl-N'-benzoylhydrazine
N-(2,3-dichlorobenzoyl)-N'-t -butyl-N'-(2-bromobenzoyl)hydrazine
N-(2,3-dichlorobenzoyl)-N'-t -butyl-N'-benzoylhydrazine
N-(2-fluorobenzoyl)-N'-t -butyl-N'-(3,5-dimethylbenzoyl)hydrazine
N-(2,3-dichlorobenzoyl)-N'-t -butyl-N'-(3-toluoyl)hydrazine
N-benzoyl-N'-t -butyl-N'-(2-naphthoyl)hydrazine
N-(2-naphthoyl)-N'-t -butyl-N'-benzoylhydrazine
N-benzoyl-N'-t -butyl-N'-(4-trifluoromethoxybenzoyl)hydrazine
N-(4-isothiocyanatobenzoyl)-N ' -t -butyl-N'-benzoylhydrazine

N-(2,6-difluorobenzoyl)-N'-t -butyl-N'-(2,4-dichlorobenzoyl)hydrazine
N-(2,6-difluorobenzoyl)-N'-t -butyl-N'-(3,4-dichlorobenzoyl)hydrazine
N-(4-toluoyl)-N'-t -butyl-N'-(3,5-trifluoromethylbenzoyl)hydrazine
N-benzoyl-N'-t -butyl-N'-(3,5-trifluoromethylbenzoyl)hydrazine
N-(4-toluoyl)-N'-(1,2-dimethyl-3-ethylbutyl)-N'-benzoylhydrazine
N-(4-toluoyl)-N'(1-2,-dimethyl-3-ethylbutyl)-N'-(3,5-dimethylbenzoyl)hydrazine
N-(4-toluoyl)-N'-(1,2,2-trimethylpropyl)-N'-(3,5-dimethylbenzoyl)hydrazine
N-(4-toluoyl)-N'-(1,2,2-trimethylpropyl)-N'-(2-nitro-5-methylbenzoyl)hydrazine
N-benzoyl-N'-t -butyl-N'-(3-chloro-4-fluorobenzoyl)hydrazine
N-(4-chlorobenzoyl)-N'-t -butyl-N'-(3-chloro-4-fluorobenzoyl)hydrazine
N-(4-ethylbenzoyl)-N'-t -butyl-N'-(3,5-dimethylbenzoyl)hydrazine
N-(4-chlorobenzoyl)-N'-t -butyl-N'-(3,5-trifluoromethylbenzoyl)hydrazine
N-(2,3-dimethylbenzoyl)-N'-t -butyl-N'-(3,4-dichlorobenzoyl)hydrazine
N-(4-toluoyl)-N'-t -butyl-N'-(2-methyl-3-chlorobenzoyl)hydrazine
N-(3-chloro-4-fluorobenzoyl)-N'-t -butyl-N'-benzoylhydrazine
N-(2,6-dimethylbenzoyl)-N'-t -butyl-N'-(3-toluoyl)hydrazine
N-(2,6-dimethylbenzoyl)-N'-t -butyl-N'-(3,4-dimethylbenzoyl)hydrazine
N-(2,3-dimethylbenzoyl)-N'-t -butyl-N'-(2-bromobenzoyl)hydrazine
N-(2,3-dimethylbenzoyl)-N'-t -butyl-N'-(3,5-dimethylbenzoyl)hydrazine
N-(2,3-dimethylbenzoyl)-N'-t -butyl-N'-(3-chlorobenzoyl)hydrazine
N-(3-trifluoromethoxybenzoyl)-N'-t -butyl-N'-benzoylhydrazine
N-(3-trifluoromethoxybenzoyl)-N'-t -butyl-N'-(3-toluoyl)hydrazine
N-(4-ethoxycarbonylmethylbenzoyl)-N'-t -butyl-N'-(3,4dichlorobenzoyl)hydrazine
N-(4-ethylbenzoyl)-N'-(1,2,2,-trimethylpropyl)-N'-(2,4dichlorobenzoyl)hydrazine
N-(4-toluoyl)-N'-(1,2-dimethyl-2-ethylbutyl-N'-(2-nitro-5-methylbenzoyl)hydrazine
N-(4-toluoyl)-N'-(1,2-dimethyl-2-ethylbutyl)-N'-(2-nitro-3-methylbenzoyl)hydrazine
N-(2,6-difluorobenzoyl)-N'-t -butyl-N'-(2-bromobenzoyl)hydrazine
N-(2-methyl-3-chlorobenzoyl)-N'-t -butyl-N'-(3,5-dimethylbenzoyl)hydrazine
N-(2-methyl-3-chlorobenzoyl)-N'-t -butyl-N'-(3-chloro-4-fluorobenzoyl)hydrazine
N-(2,3-dimethylbenzoyl)-N'-t -butyl-N'-(3-chloro-4-fluorobenzoyl)hydrazine
N-(4-ethylbenzoyl)-N'-t -butyl-N'-(3-chloro-4-fluorobenzoyl)hydrazine
N-(3,4-dimethylbenzoyl)-N'-t -butyl-N'-benzoylhydrazine
N-(3,4-dimethylbenzoyl)-N'-t -butyl-N'-(3-toluoyl)hydrazine
N-(3,4-dimethylbenzoyl)-N'-t -butyl-N'-(2-chlorobenzoylhydrazine
N-(3,4-dimethylbenzoyl)-N'-t -butyl-N-(2,4-dichlorobenzoyl)hydrazine
N-(3,4-dimethylbenzoyl)-N'-t -butyl-N'-(2-bromobenzoyl)hydrazine
N-(3,4-dimethylbenzoyl)-N'-t -butyl-N'-(3,5-dimethylbenzoyl)hydrazine
N-(3,4-dimethylbenzoyl)-N'-t -butyl-N'-(2-nitrobenzoylhydrazine
N,N'-bis(3,4-dimethylbenzoyl)-N'-t -butylhydrazine
N-benzoyl-N'-t -butyl-N'-(3,4-dimethylbenzoyl)hydrazine
N-(4-ethylbenzoyl)-N'-t -butyl-N'-(3,4-dimethylbenzoyl)hydrazine
N-(2-chloro-6-fluorobenzoyl)-N'-t -butyl-N'-(3-toluoyl)hydrazine
N-(4-toluoyl)-N'-(1,2,2-trimethylpropyl-N'-benzoylhydrazine
N-(4-chloromethylbenzoyl)-N'-t -butyl-N'-benzoylhydrazine
N-(2,3-dimethoxybenzoyl)-N'-t -butyl-N'-benzoylhydrazine
N-(2,3-dimethoxybenzoyl)-N'-t -butyl-N'-(4-chlorobenzoyl)hydrazine
N-(2,3-dimethoxybenzoyl)-N'-t -butyl-N'-(2-bromobenzoyl)hydrazine
N-(3-chloro-4-fluorobenzoyl)-N'-t -butyl-N'-(3,4-dichlorobenzoyl)hydrazine
N-(4-t -butylbenzoyl)-N'-t -butyl-N'-(3,4-dimethylbenzoyl)hydrazine
N-(4-t -butylbenzoyl)-N'-t -butyl-N'-(3,5-dimethylbenzoyl)hydrazine
N-(4-t -butylbenzoyl)-N'-t -butyl-N'-(2,4-dichlorobenzoyl)hydrazine
N-(4-t -butylbenzoyl)-N'-t -butyl-N'-(2-nitrobenzoyl)hydrazine
N-(4-t -butylbenzoyl)-N'-t -butyl-N'-(2-bromobenzoyl)hydrazine
N-(4-t -butylbenzoyl)-N'-t -butyl-N'-(3-toluoyl)hydrazine
N-(4-t -butylbenzoyl)-N'-t -butyl-N'-(2-chlorobenzoyl)hydrazine
N-(4-t -butylbenzoyl)-N'-t -butyl-N'-(3,4-dichlorobenzoyl)hydrazine
N-(3,4-dimethylbenzoyl)-N'-t -butyl-N'-(3,4-dichlorobenzoyl)hydrazine
N-(3,4-dimethylbenzoyl)-N'-t -butyl-N'-(4-fluorobenzoyl)hydrazine

N-(4-t -butylbenzoyl)-N'-t -butyl-N'-(4-fluorobenzoyl)hydrazine
N-(4-toluoyl)-N'-(2,2-dimethyl-1-ethylpropyl)-N'-(2-nitrobenzoyl)hydrazine
N-(4-toluoyl)-N'-(2,2-dimethyl-1-ethylpropyl)-N'-(3-nitro-5-methylbenzoyl)hydrazine
N-(4-toluoyl)-N'-(2,2-dimethyl-1-ethylpropyl)-N'-(3,5-dimethylbenzoyl)hydrazine
N-(2-amino-4-methoxybenzoyl)-N'-t -butyl-N'-benzoylhydrazine
N-(1-naphthoyl)-N'-t -butyl-N'-(2,4-dichlorobenzoyl)hydrazine
N-(1-naphthoyl)-N'-t -butyl-N'-(2-bromobenzoyl)hydrazine
N-(2-methyl-3-nitrobenzoyl)-N'-t -butyl-N'-benzoylhydrazine
N-(2-amino-4-methoxybenzoyl)-N'-t -butyl-N'-benzoylhydrazine
N-(1-naphthoyl)-N'-t -butyl-N'-(2,4-dichlorobenzoyl)hydrazine
N(1-naphthoyl)-N'-t -butyl-N'-(2-bromobenzoyl)hydrazine
N-(2-methyl-3-nitrobenzoyl)-N'-t -butyl-N'-benzoylhydrazine
N-(2-methyl-3-nitrobenzoyl)-N'-t -butyl-N'-(3-toluoyl)hydrazine
N-(2-methyl-3-nitrobenzoyl)-N'-t -butyl-N'-(4-chlorobenzoyl)hydrazine
N-(2-methyl-3-nitrobenzoyl)-N'-t -butyl-N'-(2,4-dichlorobenzoyl)hydrazine
N-(2-methyl-3-bromobenzoyl)-N'-t -butyl-N'-benzoylhydrazine
N-(2-methyl-3-bromobenzoyl)-N'-t -butyl-N'-(3-toluoyl)hydrazine
N-(2-methyl-3-bromobenzoyl)-N'-t -butyl-N'-(4-chlorobenzoyl)hydrazine
N-(2-methyl-3-bromobenzoyl)-N'-t -butyl-N'-(2,4-dichlorobenzoyl)hydrazine
N-(2-methyl-3-bromobenzoyl)-N'-t -butyl-N'-(3-toluoyl)hydrazine
N-(2-methylbenzoyl)-N'-t -butyl-N'-(2-bromobenzoyl)hydrazine
N-(2-methylbenzoyl)-N'-t -butyl-N'-(2-nitrobenzoyl)hydrazine
N(-2-methylbenzoyl)-N'-t -butyl-N'-(2-chlorobenzoyl)hydrazine
N-(2-methylbenzoyl)-N'-t -butyl-N'-(4-chlorobenzoyl)hydrazine
N-(2-methylbenzoyl)-N'-t -butyl-N'-(3-toluoyl)hydrazine
N-(2-methylbenzoyl)-N'-t -butyl-N'-(3,5-dimethylbenzoyl)hydrazine
N-(2-methylbenzoyl)-N'-t -butyl-N'-(3,4-dichlorobenzoyl)hydrazine
N-(2-methylbenzoyl)-N'-t -butyl-N'-(3,5-dichlorobenzoyl)hydrazine
N-(2-methyl-3-fluorobenzoyl)-N'-t -butyl-N'-benzoyl)hydrazine
N-(2-methyl-3-fluorobenzoyl)-N'-t -butyl-N'-(3-toluoyl)hydrazine
N-(2-fluoro-6-chlorobenzoyl)-N'-t -butyl-N'-benzoyl)hydrazine
N-(2-fluoro-6-chlorobenzoyl)-N'-t -butyl-N'-(2,4-dichlorobenzoyl)hydrazine
N-(2-fluoro-6-chlorobenzoyl)-N'-t -butyl-N'-(4-fluorobenzoyl)hydrazine
N-(4-(2-chloroethyl)benzoyl)-N'-t -butyl-N'-(3,5-dimethylbenzoyl)hydrazine
N-(2,4,6-trifluorobenzoyl)-N'-t -butyl-N'-benzoylhydrazine
N-(2,4,6-trifluorobenzoyl)-N'-t -butyl-N'-(2,4-dichlorobenzoyl)hydrazine
N-(2,4,6-trifluorobenzoyl)-N'-t -butyl-N'-(2-bromobenzoyl)hydrazine
N-(2,4,6-trifluorobenzoyl)-N'-t -butyl-N'-(3,5-dimethylbenzoyl)hydrazine
N-(2-nitro-3-chlorobenzoyl)-N'-t -butyl-N'-benzoylhydrazine
N-(2-nitro-3-chlorobenzoyl)-N'-t -butyl-N'-(3-bromobenzoyl)hydrazine
N-(2-nitro-3-chlorobenzoyl)-N'-t -butyl-N'-(2-nitrobenzoyl)hydrazine
N-(2-nitro-3-chlorobenzoyl)-N'-t -butyl-N'-(3-toluoyl)hydrazine
N-(2-nitro-3-chlorobenzoyl)-N'-t -butyl-N'-(3-chlorobenzoyl)hydrazine
N-(2-nitro-3-chlorobenzoyl)-N'-t -butyl-N'-(2,4-dichlorobenzoyl)hydrazine
N-(2-nitro-3-chlorobenzoyl)-N'-t -butyl-N'-(3,5-dichlorobenzoyl)hydrazine
N-(2-nitro-3-chlorobenzoyl)-N'-t -butyl-N'-(3-5-dimethylbenzoyl)hydrazine
N-(4-ethylbenzoyl)-N'-t -butyl-N'-(2,3-difluorobenzoyl)hydrazine
N-(4-ethylbenzoyl)-N'-t -butyl-N'-(2,3-dichlorobenzoyl)hydrazine
N-(2,3-difluorobenzoyl)-N'-t -butyl-N'-benzoylhydrazine
N-(2,3-dichlorobenzoyl)-N'-t -butyl-N'-(2-nitrobenzoyl)hydrazine
N-benzoyl-N'-t -butyl-N'-(2,3-difluorobenzoyl)hydrazine
N-(2,3-dichlorobenzoyl)-N'-t -butyl-N'-(3,5-dimethylbenzoyl)hydrazine
N-(2,3-dichlorobenzoyl-N'-t -butyl-N'-(2,4-dichlorobenzoyl)hydrazine
N-(2,3-dichlorobenzoyl)-N'-t -butyl-N'-(3,5-dichlorobenzoyl)hydrazine
N-(2,3-dichlorobenzoyl)-N'-t -butyl-N'-(3-chlorobenzoyl)hydrazine
N-(2,3-dichlorobenzoyl)-N'-t -butyl-N'-(2,3-dimethylbenzoyl)hydrazine
N-(2,3-difluorobenzoyl)-N'-t -butyl-N'-(2-bromobenzoyl)hydrazine
N-(2,3-dimethylbenzoyl)-N'-t -butyl-N'-(4-chlorobenzoyl)hydrazine

N-(2,3-dimethylbenzoyl)-N'-t -butyl-N'-(2,4-dichlorobenzoyl)hydrazine
N-(2,3-dimethylbenzoyl)-N'-t -butyl-N'-(2,6-difluorobenzoyl)hydrazine
N-(2,3-dimethylbenzoyl)-N'-t -butyl-N'-(2,4-difluorobenzoyl)hydrazine
N-(2,3-dimethylbenzoyl)-N'-t -butyl-N'-(3-methoxybenzoyl)hydrazine
N-(2,3-dimethylbenzoyl)-N'-t -butyl-N'-(2-methoxybenzoyl)hydrazine
N-(2,3-dimethylbenzoyl)-N'-t -butyl-N'-(2-toluoyl)hydrazine
N-(2,3-dimethylbenzoyl)-N'-t -butyl-N'-(4-toluoyl)hydrazine
N-(2-methyl-3-chlorobenzoyl)-N'-t -butyl-N'-(2,4-dichlorobenzoyl)hydrazine
N-(4-(2-chloroethyl)benzoyl)-N'-t -butyl-N'-benzoylhydrazine
N-4-(2-chloroethyl)benzoyl)-N'-t -butyl-N'-(2,4-dichlorobenzoyl)hydrazine
N-(3-fluoro-4-methylbenzoyl)-N'-t -butyl-N'-(3,5-dimethylbenzoyl)hydrazine
N-(3-fluoro-4-methylbenzoyl)-N'-t -butyl-N'-(3,5-difluorobenzoyl)hydrazine
N-(3-fluoro-4-methylbenzoyl)-N'-t -butyl-N'-(3-toluoyl)hydrazine
N-(3-fluoro-4-methylbenzoyl)-N'-t -butyl-N'-(3,5-dichlorobenzoyl)hydrazine
N-(3-fluoro-4-methylbenzoyl)-N'-t -butyl-N'-(2-nitrobenzoyl)hydrazine
N-(3-fluoro-4-methylbenzoyl)-N'-t -butyl-N'-(2,4-dichlorobenzoyl)hydrazine
N-(4-ethylbenzoyl)-N'-t -butyl-N'-(3,5-difluorobenzoyl)hydrazine
N-(2,3-dimethylbenzoyl)-N'-t -butyl-N'-(2-iodobenzoyl)hydrazine
N-(4-(2-hydroxyethyl)benzoyl)-N'-t -butyl-N'-(3,5-dimethylbenzoyl)hydrazine
N-(2,6-difluoro-3-methylbenzoyl)-N'-t -butyl-N'-benzoylhydrazine
N-(2,6-difluoro-3-methylbenzoyl)-N'-t -butyl-N'-(3-toluoyl)hydrazine
N-(2,6-difluoro-3-methylbenzoyl)-N'-t -butyl-N'-(2,4-dichlorobenzoyl)hydrazine
N-(4-chlorobenzoyl)-N'-t -butyl-N'-(2,3-dimethylbenzoyl)hydrazine
N-(2,3-dimethylbenzoyl)-N'-(1,2,2-trimethylpropyl)-N'-(3,5-dimethylbenzoyl)hydrazine
N-(2,3-dimethylbenzoyl)-N'-(1,2,2-trimethylpropyl)-N'-(3-toluoyl)hydrazine
N-(2-fluoro-6-chlorobenzoyl)-N'-t -butyl-N'-(2,3-difluorobenzoyl)hydrazine
N-(2,3-difluorobenzoyl)-N'-t -butyl-N'-(2-nitrobenzoyl)hydrazine
N-(2,3-difluorobenzoyl)-N'-t -butyl-N'-(3-toluoyl)hydrazine
N-(2,3-difluorobenzoyl)-N'-t -butyl-N'-(3,5-dimethylbenzoyl)hydrazine
N-(2,3-difluorobenzoyl)-N'-t -butyl-N'-(2,4-dichlorobenzoyl)hydrazine
N-(2,3-dimethylbenzoyl)-N'-t -butyl-N'-(2,3-difluorobenzoyl)hydrazine
N-(2,3-dimethylbenzoyl)-N'-t -butyl-N'-(2,3-dichlorobenzoyl)hydrazine
N-(2,3-dimethylbenzoyl)-N'-t -butyl-N'-(2,4-dimethylbenzoyl)hydrazine
N-(2,3-dimethylbenzoyl)-N'-t -butyl-N'-(2-nitrobenzoyl)hydrazine
N-(4-ethylbenzoyl)-N'-t -butyl-N'-(2-methyl-5-chlorobenzoyl)hydrazine
N-benzoyl-N'-t -butyl-N'-(2-methyl-5-chlorobenzoyl)hydrazine
N-(2,3-difluorobenzoyl)-N'-t -butyl-N'-(2-methyl-5-chlorobenzoyl)hydrazine
N-(2,3-dimethylbenzoyl)-N'-t -butyl-N'-(2,-methyl-5-chlorobenzoyl)hydrazine
N-(2-methyl-3-chlorobenzoyl)-N'-t -butyl-N'-(2-methyl-5-chlorobenzoyl)hydrazine
N-(2,6-difluorobenzoyl)-N'-t -butyl-N'-(2-methyl-5-chlorobenzoyl)hydrazine
N-(2,3-dimethylbenzoyl)-N'-t -butyl-N'-(2-methyl-5-chlorobenzoyl)hydrazine
N-(4-chlorobenzoyl)-N'-t -butyl-N'-(2-methyl-5-chlorobenzoyl)hydrazine
N-(4-chlorobenzoyl)-N'-t -butyl-N'-(2-methyl-5-chlorobenzoyl)hydrazine
N-(2-fluoro-4-chlorobenzoyl)-N'-t -butyl-N'-benzoyl)hydrazine
N-(2-fluoro-4-chlorobenzoyl)-N'-t -butyl-N'-(3-toluoyl)hydrazine
N-(2-fluoro-4-chlorobenzoyl)-N'-t -butyl-N'-(3,5-dimethylbenzoyl)hydrazine
N-(2-fluoro-4-chlorobenzoyl)-N'-t -butyl-N'-(3,5-dichlorobenzoyl)hydrazine
N-(2-fluoro-4-chlorobenzoyl)-N'-t -butyl-N'-(2-bromobenzoyl)hydrazine
N-(2-fluoro-4-chlorobenzoyl)-N'-t -butyl-N'-(2-nitrobenzoyl)hydrazine
N-(2-chloro-3-methylbenzoyl)-N'-t -butyl-N'-(2,4-dichlorobenzoyl)hydrazine
N-(2-chloro-3-methylbenzoyl)-N'-t -butyl-N'-benzoylhydrazine
N-(2-chloro-3-methylbenzoyl)-N'-t -butyl-N'-(3-toluoyl)hydrazine
N-(2-chloro-3-methylbenzoyl)-N'-t -butyl-N'-(3,5-dimethylbenzoyl)hydrazine
N-(2-chloro-3-methylbenzoyl)-N'-t -butyl-N'-(3,5-dichlorobenzoyl)hydrazine
N-(2-bromo-3-methylbenzoyl)-N'-t -butyl-N'-(3,5-dimethylbenzoyl)hydrazine
N-(2-bromo-3-methylbenzoyl)-N'-t -butyl-N'-(2,4-dichlorobenzoyl)hydrazine
N-(2-bromo-3-methybenzoyl)-N'-t -butyl-N'-(3-toluoyl)hydrazine
N-(2-bromo-3-methylbenzoyl)-N'-t -butyl-N'-(3,4-dichlorobenzoyl)hydrazine

N-(2-bromo-3-methylbenzoyl)-N'-t -butyl-N'-(2-bromobenzoyl)hydrazine
N-(2,3-dimethylbenzoyl)-N'-t -butyl-N'-(2-chlorobenzoyl)hydrazine
N-(2,3-dimethylbenzoyl)-N'-t -butyl-N'-(2-trifluoromethylbenzoyl)hydrazine
N-(2,3-dimethylbenzoyl)-N'-t -butyl-N'-(4-ethylbenzoyl)hydrazine
N-(2,3-dimethylbenzoyl)-N'-t -butyl-N'-(3,5-dichlorobenzoyl)hydrazine
N-(2,6-difluorobenzoyl)-N'-t -butyl-N'-(3-chloro-4-fluorobenzoyl)hydrazine
N-(4-chlorobenzoyl)-N'-t -butyl-N'-(3,5-difluorobenzoyl)hydrazine
N-(2,3-dimethylbenzoyl)-N'-t -butyl-N'-(3,5-difluorobenzoyl)hydrazine
N-(2-methyl-3-chlorobenzoyl)-N'-t -butyl-N'-(3,5-difluorobenzoyl)hydrazine
N-benzoyl-N'-t -butyl-N'-(3,5-difluorobenzoyl)hydrazine
N-(4-ethylbenzoyl)-N'-t -butyl-N'-(2,5-dimethylbenzoyl)hydrazine
N-(2,6-difluorobenzoyl)-N'-t -butyl-N'-(3,5-difluorobenzoyl)hydrazine
N-(3-chloro-2-methylbenzoyl)-N'-t -butyl-N'-(3,4-dichlorobenzoyl)hydrazine
N-(2,3-dimethylbenzoyl)-N'-t -butyl-N'-(3,5-dimethylbenzoyl)hydrazine
N-(2-bromobenzoyl)-N'-t -butyl-N'-(3,5-dimethylbenzoyl)hydrazine
N-(2-bromo-3-methylbenzoyl)-N'-t -butyl-N'-(3-chlorobenzoyl)hydrazine
N-(2-chloro-3-methylbenzoyl)-N'-t -butyl-N'-(3-chlorobenzoyl)hydrazine

Because of their good insecticidal activity, compounds of the present invention for use in the insecticidal compositions and formulations include those where, any one or any combination of two or more of the substituents conforms to the following definitions:

X and X' are O or S;

$R^1$ is unsubstituted ($C_3$-$C_8$) branched alkyl or a ($C_1$-$C_4$) straight chain alkyl substituted with one or two of the same or different ($C_3$-$C_4$)-cycloalkyl; preferably $R^1$ has no more than 10 carbon atoms;

A and B are the same or different unsubstituted naphthyl; or

unsubstituted or substituted phenyl where the substituents can be from one to three of the same or different halo; nitro; cyano; ($C_1$-$C_4$) alkyl; halo($C_1$-$C_4$)alkyl; cyano ($C_1$-$C_4$) alkyl; ($C_1$-$C_4$)alkoxy; alkoxyalkyl having independently 1 to 4 carbon atoms in each alkyl group; -COD4; carboxy; ($C_1$-$C_4$)alkoxy-carbonyl; ($C_1$-$C_4$)-alkanoyloxy; ($C_2$-$C_6$)alkenyl; ($C_2$-$C_6$)alkynyl; -$ND^4D^5$; thiocyanato; ($C_1$-$C_4$)alkylthio; $CSD^4$; unsubstituted or substituted phenyl having one to two of the same or different halo, nitro, $C_1$-$C_4$)alkyl, ($C_1$-$C_4$)alkoxy, carboxy, -$NH_2$, -NHZ or NZZ'; phenoxy where the phenyl ring is unsubstituted or substituted with one or two of the same or different halo, nitro ($C_1$-$C_4$)alkyl, ($C_1$-$C_4$)alkoxy, carboxy, -$NH_2$, -NHZ or NZZ'; or when two adjacent positions on the phenyl ring are substituted with alkoxy groups, these groups may be joined to form together with the carbon atoms to which they are attached a 5-or 6-membered dioxolano or dioxano heterocyclic ring; where $D^4$ and $D^5$ are hydrogen or ($C_1$-$C_4$)alkyl; Z and Z' are as hereinbefore defined, and agronomically acceptable salts thereof.

Insecticidal compounds of the present invention having very good activity for use in the insecticidal compositions and formulations of the present invention include those where any one or any combination of two or more, of the substituents conforms to the following definitions:

X and X' are O or S;

$R^1$ is branched ($C_3$-$C_8$)alkyl;

unsubstituted naphthyl;

unsubstituted or substituted phenyl having one to three of the same or different halo; nitro; cyano; ($C_1$-$C_4$)-alkyl; halo($C_1$-$C_4$)alkyl; cyano($C_1$-$C_4$)alkyl; ($C_1$-$C_4$)alkoxy; alkoxyalkyl having independently 1 to 4 carbon atoms in each alkyl group; -$COD^4$; ($C_1$-$C_4$)alkoxy-carbonyl; ($C_1$-$C_4$)alkanoyloxy; thiocyanato; unsubstituted or substituted phenyl having one or two of the same or different halo, nitro ($C_1$-$C_4$)alkyl, ($C_1$-$C_4$)alkoxy, carboxy, -$NH_2$, -NHZ or NZZ'; or phenoxy where the phenyl ring is unsubstituted or substituted with one or two of the same or different halo, nitro, ($C_1$-$C_4$)alkyl, ($C_1$-$C_4$)alkoxy, carboxy, -$NH_2$, -NHZ or -NZZ'; where $D^4$, Z and Z' are as hereinbefore defined; and

agronomically acceptable salts thereof.

Because of their excellent insecticidal activity, preferred compounds of the present invention for use in the insecticidal compositions and formulations of the present invention include those where any one or any combination of two or more of the substituents conforms to the following definitions:

X and X' are O;

$R^1$ is branched ($C_4$-$C_7$)alkyl; and

A and B are the same or different phenyl or substituents phenyl where the substituents can be from one to three of the same or different halo, nitro, ($C_1$-$C_4$)alkyl, ($C_1$-$C_4$)alkoxy, or halo($C_1$-$C_4$)alkyl; and

agronomically acceptable salts thereof..

Because of their outstanding insecticidal activity, particularly preferred compounds of the present

invention for use in the insecticidal compositions and formulations of the present invention include those where any one or any combination of two or more of the substituents conforms to the following definitions:
X and X' are O;
$R^1$ is t -butyl, neopentyl (2,2-dimethylpropyl) or 1,2,2-trimethylpropyl;
A and B are the same or different phenyl or substituted phenyl where the substituents can be one, two or three of the same or different chloro, fluoro, bromo, iodo, nitro, methyl, ethyl, methoxy or trifluoromethyl; an agronomically acceptable salts thereof.

Those N'-substituted-N,N'-diacylhydrazines of Formula I which possess acidic or basic functional groups may be further reacted to form novel salts with appropriate bases or acids. These salts also exhibit pesticidal activity. Typical salts are the agronomically acceptable metal salts, ammonium salts and acid addition salts. Among the metal salts are those in which the metal cation is an alkali metal cation such as sodium, potassium, lithium or the like; alkaline earth metal cation such as calcium, magnesium, barium, strontium or the like; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$, and $R^8$, are independently hydrogen, hydroxy, $(C_1\text{-}C_4)$-alkoxy, $(C_1\text{-}C_{20})$alkyl, $(C_3\text{-}C_8)$alkenyl, $(C_3\text{-}C_8)$hydroxyalkyl, $(C_2\text{-}C_8)$alkoxyalkyl, $(C_2\text{-}C_6)$aminoalkyl, $(C_2\text{-}C_6)$-haloalkyl, amino, $(C_1\text{-}C_4)$alkyl- or $(C_1\text{-}C_4)$dialkylamino, substituted or unsubstituted phenyl, substituted or unsubstituted phenylalkyl, having up to four carbon atoms in the alkyl moiety, or any two or $R^5$, $R^6$, $R^7$ or $R^8$ can be taken together to form with the nitrogen atom a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, $(C_1\text{-}C_8)$alkyl, $(C_1\text{-}C_4)$alkoxy, hydroxy, nitro, trifluoromethyl, cyano, amino, $(C_1\text{-}C_4)$alkylthio and the like. Such substituted phenyl groups preferably have up to two such substituents. Representative ammonium cations include ammonium, dimethylammonium, 2-ethylhexylammonium, bis(2-hydroxyethyl)-ammonium, tris(2-hydroxyethyl)ammonium, dicyclohexylammonium, t -octylammonium, 2-hydroxyethylammonium, morpholinium, piperidinium, 2-phenethylammonium, 2-methylbenzylammonium, n-hexylammonium, triethylammonium, trimethylammonium, tri( n -butylammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium, 4-methylbenzyltrimethylammonium, and the like. Among the acid addition salts are those in which the anion is an agronomically acceptable anion such as hydrochloride, hydrobromide, sulfate, nitrate, perchlorate, acetate, oxalate and the like.

The compounds of this invention or their precursors can be prepared according to the following processes. Process A can be used when preparing compounds according to Formula I where X and X' are both oxygen and A and B are the same (for example, both A and B are phenyl or 4-chlorophenyl) or different (for example, A is 4-methylphenyl and B is 4-bromophenyl).

Process A:

Step 1

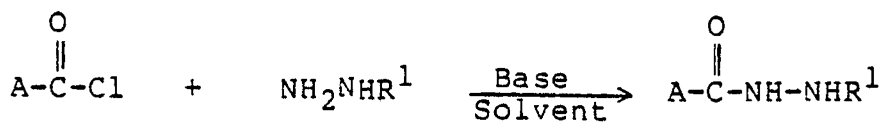


II III IV

Step 2

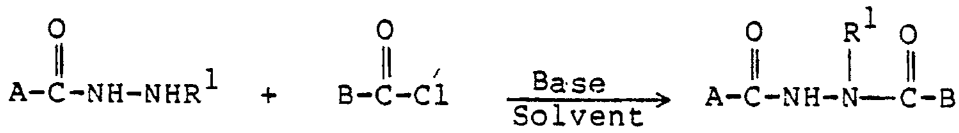


IV V I

where $R^1$, A and B are as defined above for Formula I and X and X' are oxygen.

Process B can be used when preparing compounds according to Formula I where X and X' are oxygen, and $R^1$, A and B are as defined above for Formula I.

Process B:

Method 1

Step 1

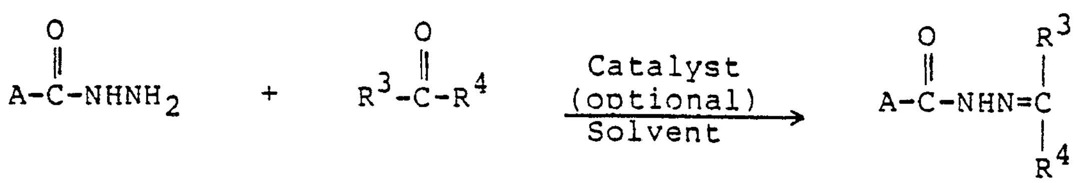


VI VII VIII

Step 2

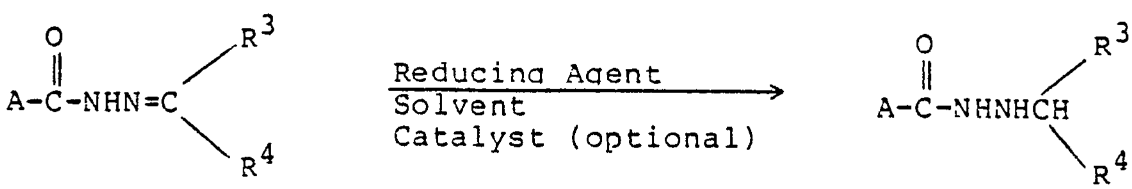


VIII IX (IV)

Step 3

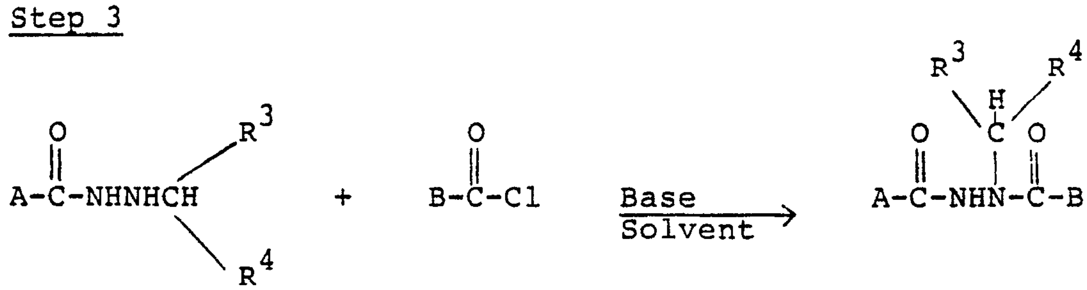


IX (IV) V X (I)

where X and X' are oxygen, A and B are as defined above for Formula I, and $R^3$ and $R^4$ are the same or different hydrogen or ($C_3$ to $C_9$) unsubstituted straight chain alkyl or ($C_1$ to $C_8$) unsubstituted straight chain alkyl or ($C_3$ to $C_6$)cycloalkyl or ($C_1$ to $C_3$) straight chain alkyl substituted by one or two ($C_3$ to $C_6$)cycloalkyl and, further, the whole group

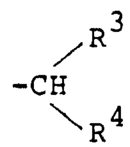

is a group $R^1$ as defined above for Formula I. As can be seen above, the intermediate product of Step 2, the compounds of Formula IX, corresponds to the compounds of Formula IV. In addition, the compound of Formula X corresponds to the compounds of Formula I where X and X' are oxygen.

Method 2

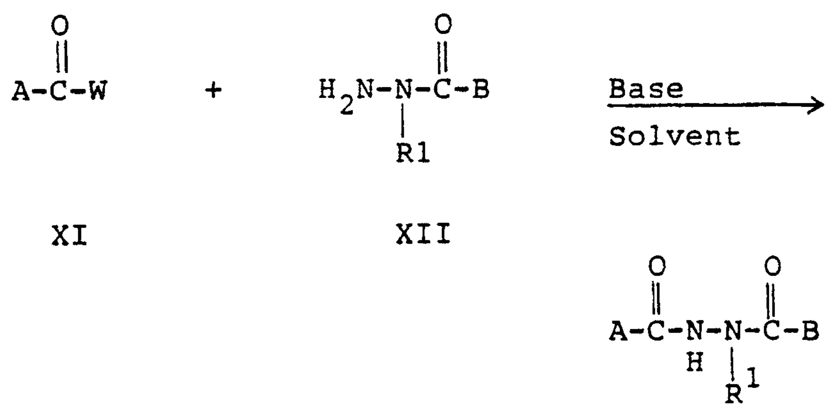


XI XII

I

where $R^1$, A and B are as defined above for Formula I and W is a good leaving group such as halo, for example, chloro; an alkoxy, for example, ethoxy; methyl sulfonate ($-OSO_2CH_3$); or an ester, for example, acetate ($-OC(O)CH_3$).

Process C can be used when preparing compounds according to Formula I where A, B and $R^1$ are as defined for Formula I and one or both X and X' are sulfur.

## Process C:

### Step 1:

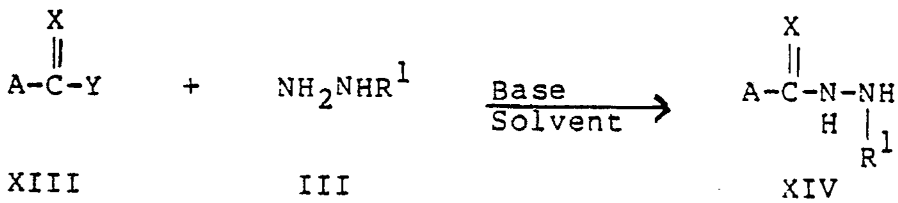


XIII III XIV

### Step 2:

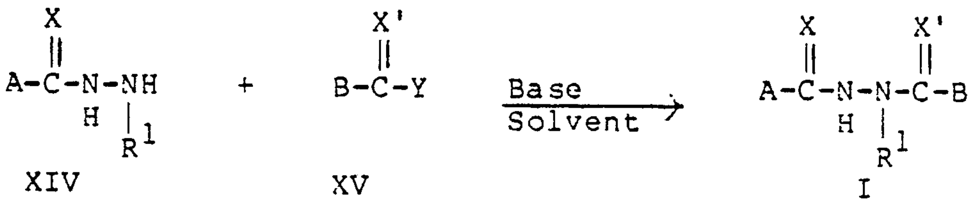


XIV XV I

where A, B and $R^1$ are as defined above for Formula I and one or both X and X' are sulfur, and Y is a good leaving group such as carboxyalkylthio (for example, carboxymethylthio, $-SCH_2CO_2H$); alkylthio (for example, methylthio); or halo (for example, chloro).

Process D can be used when preparing compounds according to Formula I where X and X' are oxygen and $R^1$, A and B are as defined above for Formula I.

Process D

Step 1

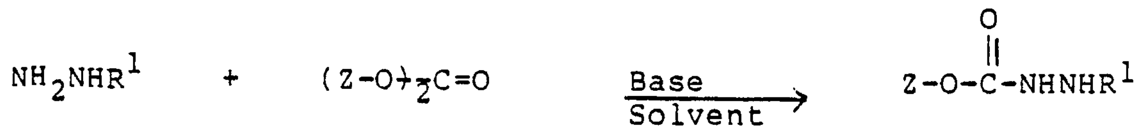


III XVI XVII

Step 2

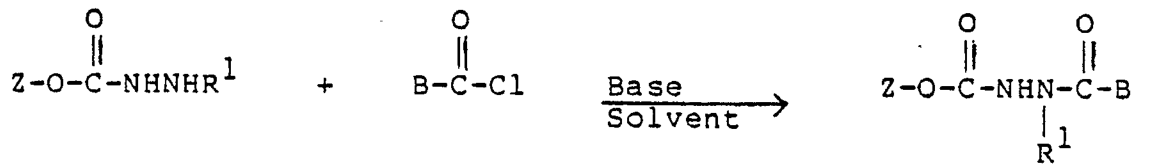


XVII V XVIII

Step 3

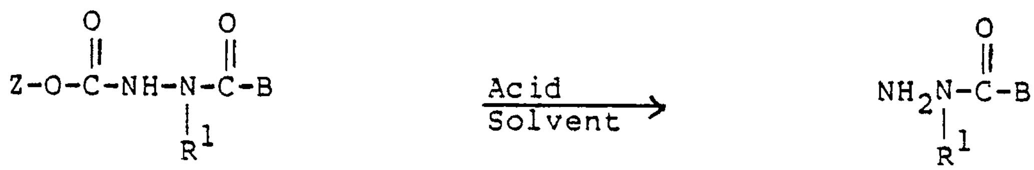


XVIII XIX

Step 4

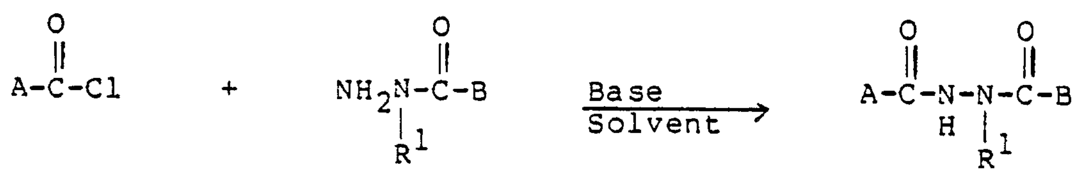


II XIX I

wherein A, B and $R^1$ are as defined above for Formula I and Z is t butyl; ethyl; phenyl; or benzyl.

In process A, a compound of Formula II is reacted with a monosubstituted hydrazine of Formula III or a corresponding acid addition salt such as the hydrochloride salt or the like in the presence of a base in an inert or substantially inert solvent or mixture of solvents to afford an intermediate product of Formula IV which can be isolated or further reacted with a compound of Formula V in the presence of a base in an inert or substantially inert solvent or mixture of solvents to afford the desired product of Formula I.

When A and B are the same, for example, both A and B are 4-chlorophenyl, two equivalents of a compound of Formula II or V are reacted with a monosubstituted hydrazine of Formula III in the presence of a base in an inert or substantially inert solvent or mixture of solvents to afford the desired product of Formula I.

Examples of the compounds of Formula II and/or Formula V which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride and the like. The compounds of Formula II and/or Formula V are generally commercially available or can be prepared by known procedures.

Examples of the compounds of Formula III which can be used in the above processes include isopropylhydrazine, t -butylhydrazine, neopentylhydrazine, alpha-methylneopentylhydrazine, isobutyl-

hydrazine, isopentylhydrazine, isooctylhydrazine, and the like. The compounds of Formula III are generally commerically available or can be prepared by known procedures. For example, the Grignard reagent addition product of acetone azine in diethyl ether is hydrolyzed by the adition of an acid (such as oxalic acid), in a suitable solvent or mixture of solvents (such as ethanol and diethyl ether, 1:1) to afford the monosubstituted hydrazine of Formula III.

Suitable solvents for use in the above processes include water; alcohols such as methanol, ethanol, isopropanol and the like; hydrocarbons such as toluene, xylene, hexane, heptane and the like; glyme; tetrahydrofuran; acetonitrile; pyridine; or haloalkanes such as methylene chloride or mixtures of these solvents.

Preferred solvents are water, toluene, methylene chloride or a mixture of these solvents.

Examples of bases for use in the above processes include tertiary amines such as triethylamine; pyridine; potassium carbonate; sodium carbonate; sodium bicarbonate; sodium hydroxide; or potassium hydroxide. Preferred bases are sodium hydroxide, potassium hydroxide or triethylamine.

In Process B, Method 1, a compound of Formula VI is reacted with a ketone or aldehyde of Formula VII in an inert or substantially inert solvent or mixture of solvents and optionally in the present of a catalyst to afford an intermediate product of Formula VIII. The intermediate product of Formula VIII is then further reacted with a reducing agent in an inert or substantially inert solvent or mixture of solvents to afford a second intermediate product of Formula IX (IV) which can be isolated or further reacted with a compound of Formula V in the presence of a base in an inert or substantially inert solvent or mixture of solvents to afford the desired product of Formula X (I).

Examples of the compounds of Formula VI which can be used in the above Process B, Method 1, include benzoylhydrazine, 4-chlorobenzoylhydrazine, 2-methylbenzoylhydrazine, 4-methylbenzoylhydrazine, 3,5-dichlorobenzoylhydrazine and the like. The compounds of Formula VI are generally commercially available or can be prepared by known procedures.

Examples of the compounds of Formula VII which can be used in the above Process B, Method I, include 1,1,1-trimethylacetaldehyde, methylethylketone, diethylketone and the like. The compounds of Formula VII are generally commerically available or can be prepared by known procedures.

Optionally, a catalyst may be used in Step 1, Method 1 of of Process B. Suitable catalysts generally include organic acids such as acetic acid, trifluoroacetic acid, oxalic acid and the like; mineral acids such as hydrochloric acid, sulfuric acid, nitric acid and the like; arylsulfonic acids such as toluenesulfonic acid; or pyridinium toluenesulfonate. Preferred catalysts are organic acids or arylsulfonic acids. Most preferred catalysts are acetic acid or trifluoroacetic acid.

Suitable solvents for use in the above Process B, Method 1, Step 1, include alcohols such as methanol, ethanol, isopropanol and the like; hydrocarbons such as toluene, benzene; ethers such as tetrahydrofuran (THF), glyme and the like; or dimethylformamide. Preferred solvents are alcohols and hydrocarbons. Most preferred solvents are alcohols such as methanol or ethanol.

Examples of suitable reducing agents for use in the above Process B, Method 1, Step 2, include hydrides such as sodium borohydride and derivatives thereof such as sodium cyanoborohydride, lithium aluminum hydride and derivatives thereof and the like; or diborane. Preferred reducing agents are sodium borohydride and derivatives thereof or lithium aluminum hydride and derivatives thereof. Most preferred as a reducing agent is sodium cyanoborohydride.

Optionally, in Process B, Method 1, Step 2, a catalyst may be included. Examples of suitable catalysts include organic acids such as acetic acid, trifluoroacetic acid; or mineral acids such as hydrochloric acid, sulfuric acid and the like. Preferred catalysts are organic acids or hydrochloric acid. Most preferred catalysts are acetic acid, trifluoroacetic acid or hydrochloric acid.

Suitable solvents for use in the above Process B, Method 1, Step 2, include alcohols such as methanol, ethanol, isopropanol and the like; ethers such as tetrahydrofuran (THF), diethylether, glyme and the like; or halohydrocarbons such as methylene chloride, chloroform and the like. Preferred solvents are alcohols and most preferred are methanol or ethanol.

Step 3 of Process B, Method 1 corresponds to Step 2 of Process A. Consequently, those bases and solvents suitable for use in Step 2 of Process A are suitable for use in Step 2, Method 1 of Process B including the preferred bases and solvents described above.

In Process B, Method 2, N'-substituted-N'-benzoylhydrazine of Formula XII is reacted with a compound of Formula XI in the presence of a base in an inert or substantially inert solvent or mixture of solvents to afford the desired product of Formula I.

The compounds of Formula XI are generally commercially available or can be prepared from commerically available compounds by procedures well known to those skilled in the art as described below.

Examples of the compounds of Formula XII which can be used in the above Process B, Method 2,

include N'-t -butyl-N'-benzoylhydrazine; N'-t -butyl-N'-(3-methylbenzoyl)hydrazine; N'-t -butyl-N'-(4-chlorobenzoyl)hydrazine; N'-t -butyl-N'-(2-fluorobenzoyl)hydrazine; N'-isopropyl-N'-benzoyl)hydrazine; N'-neopentyl-N'-(4-chlorobenzoyl)hydrazine, and the like.

Suitable solvents for use in the above Process B, Method 2, include water; hydrocarbons such as toluene, xylene, hexane, heptane and the like; alcohols such as methanol, ethanol, isopropanol, and the like; glyme; tetrahydrofuran; acetonitrile; pyridine; or haloalkanes such as methylene chloride; or mixtures of these solvents. Preferred solvents are water, toluene, methylene chloride or a mixture of these solvents.

Examples of bases suitable for use in the above Process C includes tertiary amines such as triethylamine; pyridine; potassium carbonate; sodium carbonate; sodium bicarbonate; sodium hydroxide; or potassium hydroxide. Preferred bases are sodium hydroxide, or triethylamine.

The compounds of Formula XI are commercially available, such as nicotinoyl chloride hydrochloride, isonicotinoyl chloride hydrochloride and ethyl picolinate or can be prepared from commerically available materials by procedures known to those skilled in the art.

The compounds of Formula XII can be prepared by procedures known to those skilled in the art from commerically available reactants. By way of example, a suitably substituted hydrazine (such as t -butyl-hydrazine) is reacted with an aldehyde or ketone (such as acetone) in the presence of a base (such as triethylamine) to afford a hydrazone which is then reacted with a benzoyl chloride in an inert or substantially inert solvent or mixture of solvents in the presence of a base (such as sodium hydroxide) to afford an N'-substituted-N'-benzoylhydrazone which is then reacted with an acid (such as hydrochloric acid) to afford the compound of Formula XII. Alternatively, a suitable substituted hydrazine (such as t -butyl-hydrazine) is reacted with di tert -butyldicarbonate in an inert or substantially inert solvent or mixture of solvents (such as toluene/water) to afford an N'-t -butyl-N-t -butoxycarbonylhydrazine which is then reacted with a benzoylchloride in an inert or substantially inert solvent or mixture of solvents to afford an N'-t -butyl-N'-benzoyl-N -t-butoxycarbonyl hydrazine which is then reacted with an acid to afford the desired compound of Formula XII.

In Process C, a compound of Formula XII is reacted with a monosubstituted hydrazine of Formula III or a corresponding acid addition salt such as the hydrochloride salt or the like in the presence of a base in an inert or substantially inert solvent or mixture of solvents to afford an intermediate compound of Formula XIV which can be isolated or further reacted with a compound of Formula XV in the presence of a base in an inert or substantially inert solvent or mixture of solvents to afford the desired product of Formula I.

In Process D, a monosubstituted hydrazine of Formula III or a corresponding acid addition salt, such as the hydrochloride salt or the like, is reacted with a compound of Formula XVI in the presence of a base in an inert or substantially inert solvent or mixture of solvents to afford an intermediate product of Formula XVII. The intermediate product of Formula XVII is then further reacted with a compound of Formula V in the presence of a base in an inert or substantially inert solvent or mixture of solvents to afford a second intermediate product of Formula XVIII. The second intermediate product of Formula XVIII is then further reacted with an acid in an inert or substantially inert solvents or mixture of solvents to afford a third intermediate product of Formula XIX. The third intermediate product of Formula XIX is then further reacted with a compound of Formula II in the presence of a base in an inert or substantially inert solvent or mixture of solvents to afford the desired product of Formula I.

Examples of the compounds of Formula XVI which can be used in the above Process D include di-t -butylcarbonate, diethylcarbonate, diphenylcarbonate, dibenzylcarbonate and the like. The compounds of Formula XVI are generally commercially available or can be prepared by known procedures.

Suitable solvents for use in the above Process D, Steps 1, 2 and 4 include water; tetrahydrofuran; dioxane; toluene; alcohols such as methanol, ethanol and isopropanol; hexane; acetonitrile; pyridine; and haloalkanes such as methylene chloride; or mixtures of these solvents.

Preferred solvents are dioxane; toluene; tetrahydrofuran; pyridine; methylene chloride or water.

Most preferred solvents are dioxane; water or toluene.

Examples of the bases for use in the above Process D, Steps 1, 2 and 4 include tertiary amines such as triethylamine; pyridine; potassium carbonate, sodium carbonate; sodium bicarbonate; sodium hydroxide; and potassium hydroxide.

Preferred bases are sodium hydroxide; potassium hydroxide; pyridine or triethylamine.

Suitable solvents for use in the above Process D, Step 3 include alcohols such as methanol, ethanol and isopropanol; water; tetrahydrofuran; dioxane; and acetonitrile.

Preferred solvents are methanol or ethanol.

Examples of acids for use in the above Process D, Step 3 include concentrated hydrochloric acid or concentrated sulfuric acid.

When A and B are the same, for example, both A and B are unsubstituted phenyl, two equivalents of a compound Formula XIII or XV are reacted with a monosubstituted hydrazine of Formula III in the presence

of a base in an inert or substantially inert solvent or mixture of solvents to afford the desired product of Formula I.

Examples of the compounds of Formula XIIII and/or Formula XV which can be used in the above Process C include 3-methyl-methylthio-thiobenzoate, 4-chloro-methylthio-thiobenzoate, 4-methyl-methylthio-thiobenzoate, carboxymethylthio-thiobenzoate and the like. The compounds of Formula XIII and/or Formula XIV are generally commerically available or can be prepared by known procedures.

Suitable solvents for use in the above Process C are generally polar high-boiling solvents such as dimethylformamide (DMF); glyme; tetrahydrofuran (THF); and pyridine. The preferred solvent is pyridine.

Suitable bases for use in the above Process C include tertiary amines such as triethylamine; and pyridine. The preferred base is pyridine.

The above Processes A and B, Method 1, can be carried out at temperatures between about -20 °C and about 100 °C. Preferably, these reactions are carried out between about -5 °C and about 50 °C.

The above Process B, Method 2, can be carried out at temperatures between about -50 °C and about 150 °C. Preferably when W is a halo radical, the reaction is carried out between about 0 °C and about 30 °C. When W is alkoxy, the reaction is preferably carried out between about 100 °C and about 150 °C. When W is methyl sulfonate, the reaction is preferably carried out between about -20 °C to about 20 °C. When W is an ester, the reaction is preferably carried out between about 0 °C and about 50 °C.

Process C can be carried out at temperatures between about 10 °C and 200 °C. Preferably, this reaction is carried out between about 70 °C and about 100 °C.

Process D can be carried out at temperatures between about 0 °C and 100 °C. Preferably, these reactions are carried out between about 0 °C and about 50 °C.

Preparation of the compounds of the present invention by processes A, B, C and D is preferably carried out at about atmospheric pressure, although higher or lower pressures can be used if desired.

Substantially equimolar amounts of reactants are preferably used in processes A, B and C, although higher or lower amounts can be used if desired.

Generally, about one equivalent of base is used per equivalent of starting material of Formula II, V, XI and/or XIII. Where the acid addition salt of the monosubstituted hydrazine of Formula III is used, one additional equivalent of base is used. For example, in Process A, when substituents A and B are the same and a monosubstituted hydrazine is used, about two equivalents of base are used since about two equivalents of a suitably substituted benzoyl chloride of Formula II or V are employed. In Process A, when substituents A and B are different and an acid addition salt of the monosubstituted hydrazines of Formula III is used, about two equivalents of base are used in Step 1 and about one equivalent of base is used in Step 2.

Modifications to the above processes may be necessary to accommodate reactive funtionalities of particular A and/or B substituents. Such modifications would be apparent and known to those skilled in the art.

It will be appreciated by those skilled in the art that electronic forces may give rise to more than one isomer of the compounds of Formula I such as enantiomers, conformers and the like. There may be a difference in properties such as physical characteristics and degree of biological activity between such isomers. Separation of a specific isomer can be accomplished by standard techniques well known to those skilled in the art such as silica gel chromatography.

The agronomically acceptable salts embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt of a compound of Formula I in a suitable solvent. When metal hydroxides are used as reagents, useful solvents include water; ethers such as glyme and the like; dioxane; tetrahydrofuran; alcohols such as methanol, ethanol, isopropanol and the like. When metal hydrides are used as reagents, useful solvents include nonhydroxylic solvents, for example, ethers such as dioxane, glyme, diethylether and the like; tetrahydrofuran; hydrocarbons such as toluene, xylene, hexane, pentane, heptane, octane and the like; dimethylformamide, and the like. When amines are used as reagents, useful solvents include alcohols, such as methanol or ethanol; hydrocarbons, such as toluene, xylene, hexane and the like; tetrahydrofuran; glyme; dioxane; or water. When ammonium salts are used as reagents, useful solvents include water; alcohols, such as methanol or ethanol; glyme; tetrahydrofuran; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about

0° C to about 100° C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones, haloalkanes and the like. The particular choice of solvent will depend on the relative solubilities of the starting materials and the resulting salts and slurries rather then solutions of certain reagents may be used to obtain the salts. Generally, equivalent molar amounts of starting materials are used and the salt-forming reaction is carried out at from about -10° C to about 100° C, preferably at about room temperature.

The following Examples, and the individual substituents mentioned therein and each and every combination of, or group containing, two or more of these substituents, will further illustrate this invention but are not intended to limit it in any way. In Table I, some N'-substituted-N,N'-diacyl hydrazines of the present invention that have been made are listed. The structure of these compounds was confirmed by NMR and in some cases by IR and/or elemental analysis. Specific illustrative preparation of the compounds of Examples 1, 3, 16, 44, 102, 103, 148, 220, 295, 324 and 625 are described after Table I.

TABLE I

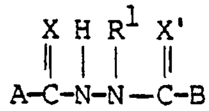

| Ex. No. | X | X' | $R^1$ | A | B |
|---|---|---|---|---|---|
| 1 | O | O | $-C(CH_3)_3$ | $-C_6H_4Cl$-4 | $-C_6H_4Cl$-4 |
| 2 | O | O | $-C(CH_3)_3$ | $-C_6H_4Cl$-3 | $-C_6H_4Cl$-3 |
| 3 | O | O | $-C(CH_3)_3$ | $-C_6H_5$ | $-C_6H_5$ |
| 4 | O | O | $-C(CH_3)_3$ | $-C_6H_3Cl_2$-3,4 | $-C_6H_3Cl_2$-3,4 |
| 5 | O | O | $-C(CH_3)_3$ | $-C_6H_4CH_3$-4 | $-C_6H_4CH_3$-4 |
| 6 | O | O | $-C(CH_3)_3$ | $-C_6H_4NO_2$-4 | $-C_6H_4NO_2$-4 |
| 7 | O | O | $-C(CH_3)_3$ | $-C_6H_4OCH_3$-4 | $-C_6H_4OCH_3$-4 |
| 8 | O | O | $-C(CH_3)_3$ | $-C_6H_4NO_2$-3 | $-C_6H_4NO_2$-3 |
| 9 | O | O | $-C(CH_3)_3$ | $-C_6H_4OCH_3$-3 | $-C_6H_4OCH_3$-3 |
| 10 | O | O | $-C(CH_3)_3$ | $-C_6H_4NO_2$-2 | $-C_6H_4NO_2$-2 |
| 11 | O | O | $-C(CH_3)_3$ | $-C_6H_4Cl$-2 | $-C_6H_4Cl$-2 |
| 12 | O | O | $-C(CH_3)_3$ | $-C_6H_4OCH_3$-2 | $-C_6H_4OCH_3$-2 |
| 13 | O | O | $-C(CH_3)_3$ | $-C_6H_4CH_3$-4 | $-C_6H_5$ |
| 14 | O | O | $-C(CH_3)_3$ | $-C_6H_4CN$-4 | $-C_6H_4CN$-4 |
| 15 | O | O | $-C(CH_3)_3$ | $-C_6H_4CH_3$-4 | $-C_6H_4Cl$-4 |
| 16 | O | O | $-C(CH_3)_3$ | $-C_6H_5$ | $-C_6H_4Cl$-4 |
| 17 | O | O | $-C(CH_3)_3$ | $-C_6H_5$ | $-C_6H_4Cl$-3 |
| 18 | O | O | $-C(CH_3)_3$ | $-C_6H_5$ | $-C_6H_4Cl$-2 |
| 19 | O | O | $-C(CH_3)_3$ | $-C_6H_4CH_3$-3 | $-C_6H_4CH_3$-3 |
| 20 | O | O | $-C(CH_3)_3$ | $-C_6H_4CH_3$-2 | $-C_6H_4CH_3$-2 |
| 21 | O | O | $-C(CH_3)_3$ | $-C_6H_5$ | $-C_6H_4CH_3$-4 |
| 22 | O | O | $-C(CH_3)_3$ | $-C_6H_5$ | $-C_6H_4CH_3$-3 |
| 23 | O | O | $-C(CH_3)_3$ | $-C_6H_5$ | $-C_6H_4CH_3$-2 |
| 24 | O | O | $-C(CH_3)_3$ | $-C_6H_5$ | $-C_6H_4OCH_3$-4 |
| 25 | O | O | $-C(CH_3)_3$ | $-C_6H_5$ | $-C_6H_4OCH_3$-3 |
| 26 | O | O | $-C(CH_3)_3$ | $-C_6H_5$ | $-C_6H_4OCH_3$-2 |
| 27 | O | O | $-C(CH_3)_3$ | $-C_6H_5$ | $-C_6H_4C(CH_3)_3$-4 |

| Ex. No. | X | X' | $R^1$ | A | B |
|---|---|---|---|---|---|
| 28 | O | O | $-C(CH_3)_3$ | $-C_6H_5$ | $-C_6H_4CN$-4 |
| 29 | O | O | $-C(CH_3)_3$ | $-C_6H_5$ | $-C_6H_4NO_2$-4 |
| 30 | O | O | $-C(CH_3)_3$ | $-C_6H_5$ | $-C_6H_4NO_2$-3 |
| 31 | O | O | $-C(CH_3)_3$ | $-C_6H_5$ | $-C_6H_4NO_2$-2 |
| 32 | O | O | $-C(CH_3)_3$ | $-C_6H_4C(CH_3)_3$-4 | $-C_6H_4C(CH_3)_3$-4 |
| 33 | O | O | $-C(CH_3)_3$ | $-C_6H_4CH_3$-4 | $-C_6H_3Cl_2$-3,4 |
| 34 | O | O | $-C(CH_3)_3$ | $-C_6H_5$ | $-C_6H_4F$-4 |
| 35 | O | O | $-C(CH_3)_3$ | $-C_6H_5$ | $-C_6H_4F$-3 |
| 36 | O | O | $-C(CH_3)_3$ | $-C_6H_5$ | $-C_6H_4F$-2 |
| 37 | O | O | $-C(CH_3)_3$ | $-C_6H_3Cl_2$-3,5 | $-C_6H_3Cl_2$-3,5 |
| 38 | O | O | $-C(CH_3)_3$ | $-C_6H_5$ | $-C_6H_3Cl_2$-2,4 |
| 39 | O | O | $-CH(CH_3)_2$ | $-C_6H_5$ | $-C_6H_5$ |
| 40 | O | O | $-C(CH_3)_3$ | $-C_6H_5$ | $-C_6H_4CF_3$-4 |
| 41 | O | O | $-C(CH_3)_3$ | $-C_6H_5$ | $-C_6H_4CF_3$-3 |
| 42 | O | O | $-C(CH_3)_3$ | $-C_6H_5$ | $-C_6H_4CF_3$-2 |
| 43 | O | O | $-C(CH_3)_3$ | $-C_6H_5$ | $-C_6H_3F_2$-2,5 |
| 44 | O | O | $-CH_2C(CH_3)_3$ | $-C_6H_5$ | $-C_6H_5$ |
| 45 | O | O | $-C(CH_3)_3$ | $-C_6H_5$ | $-C_6H_4CN$-3 |
| 46 | O | O | $-CH(CH_3)CH_2CH_3$ | $-C_6H_5$ | $-C_6H_5$ |
| 47 | O | O | $-C(CH_3)_3$ | $-C_6H_5$ | $-C_6H_3F_2$-2,6 |
| 48 | O | O | $-C(CH_3)_3$ | $-C_6H_4Cl$-4 | $-C_6H_5$ |
| 49 | O | O | $-C(CH_3)_3$ | $-C_6H_5$ | $-C_6H_3Cl_2$-3,4 |
| 50 | O | O | $-C(CH_3)_3$ | $-C_6H_5$ | $-C_6H_3Cl_2$-3,5 |
| 51 | O | O | $-C(CH_3)_3$ | $-C_6H_5$ | $-C_6H_3Cl_2$-2,6 |
| 52 | O | O | $-C(CH_3)_3$ | $-C_6H_4C(CH_3)_3$-4 | $-C_6H_5$ |
| 53 | O | O | $-C(CH_3)_3$ | $-C_6H_4Cl$-2 | $-C_6H_5$ |
| 54 | O | O | $-C(CH_3)_3$ | 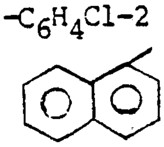 | $-C_6H_5$ |
| 55 | O | O | $C(CH_3)_3$ | 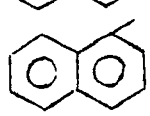 | 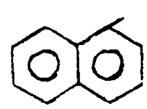 |
| 56 | O | O | $-C(CH_3)_3$ | $-C_6H_4Cl$-3 | $-C_6H_5$ |

| Ex. No. | X | X' | $R^1$ | A | B |
|---|---|---|---|---|---|
| 57 | O | O | $-C(CH_3)_3$ | $-C_6H_4Cl$-4 | $-C_6H_3Cl_2$-3,4 |
| 58 | O | O | $-C(CH_3)_3$ | $-C_6H_4Cl$-2 | $-C_6H_3Cl_2$-3,4 |
| 59 | O | O | $-C(CH_3)_3$ | $-C_6H_4CH_3$-2 | $-C_6H_5$ |
| 60 | O | O | $-C(CH_3)_3$ | $-C_6H_5$ | $-C_6H_3Cl$-2-$NO_2$-4 |
| 61 | O | O | $-C(CH_3)_3$ | $-C_6H_5$ | $-C_6H_3(NO_2)_2$-3,5 |
| 62 | O | O | $-C(CH_3)_3$ | $-C_6H_5$ | $-C_6H_3Cl_2$-2,3 |
| 63 | O | O | $-CH(CH_3)C(CH_3)_3$ | $-C_6H_5$ | $-C_6H_5$ |
| 64 | O | O | $-C(CH_3)_3$ | $-C_6H_5$ | $-C_6H_3Cl$-2-$CH_3$-5 |
| 65 | O | O | $-C(CH_3)_3$ | $-C_6H_5$ | $-C_6H_3(CH_3)_2$-3,5 |
| 66 | O | O | $-C(CH_3)_3$ | $-C_6H_5$ | $-C_6H_3NO_2$-2-$CH_3$-5 |
| 67 | O | O | $-C(CH_3)_3$ | $-C_6H_5$ | $-C_6H_3CH_3$-2-Cl-3 |
| 68 | O | O | $-C(CH_3)_3$ | $-C_6H_5$ | $-C_6H_3Cl$-3-$CH_3$-4 |
| 69 | O | O | $-C(CH_3)_3$ | $-C_6H_5$ | $-C_6H_3NO_2$-2-Cl-3 |
| 70 | O | O | $-C(CH_3)_3$ | $-C_6H_5$ | $-C_6H_3OCH_3$-3-$NO_2$-4 |
| 71 | O | O | $-C(CH_3)_3$ | $-C_6H_5$ | $-C_6H_3NO_2$-2-$OCH_3$-3 |
| 72 | O | O | $-C(CH_3)_3$ | $-C_6H_5$ | $-C_6H_3(NO_2)_2$-2,4 |
| 73 | O | O | $-C(CH_3)_3$ | $-C_6H_4Cl$-4 | $-C_6H_4Cl$-2 |
| 74 | O | O | $-C(CH_3)_3$ | $-C_6H_4Cl$-4 | $-C_6H_4Cl$-3 |
| 75 | O | O | $-C(CH_3)_3$ | $-C_6H_4Cl$-4 | $-C_6H_4CH_3$-4 |
| 76 | O | O | $-C(CH_3)_3$ | $-C_6H_4Cl$-4 | $-C_6H_3Cl_2$-3,5 |
| 77 | O | O | $-C(CH_3)_3$ | $-C_6H_4Cl$-4 | $-C_6H_3Cl_2$-2,4 |
| 78 | O | O | $-C(CH_3)_3$ | $-C_6H_4Cl$-4 | $-C_6H_4CF_3$-4 |
| 79 | O | O | $-C(CH_3)_3$ | $-C_6H_5$ | $-C_6H_4OSO_2CH_3$-4 |
| 80 | O | O | $-C(CH_3)_3$ | $-C_6H_5$ | $-C_6H_4CH(CH_3)_2$-4 |
| 81 | O | O | $-C(CH_3)_3$ | $-C_6H_5$ | $-C_6H_4OCOCH_3$-2 |
| 82 | O | O | $-C(CH_3)_3$ | $-C_6H_5$ | $-C_6H_4CH_2CH_3$-4 |
| 83 | O | O | $-C(CH_3)_3$ | $-C_6H_5$ | $-C_6H_4Br$-2 |
| 84 | O | O | $-C(CH_3)_3$ | $-C_6H_5$ | $-C_6H_4OH$-4 |
| 85 | O | O | $-C(CH_3)_3$ | $-C_6H_4CH_3$-4 | $-C_6H_4CH_3$-2 |
| 86 | O | O | $-C(CH_3)_3$ | $-C_6H_4CH_3$-4 | $-C_6H_4CH_3$-3 |
| 87 | O | O | $-C(CH_3)_3$ | $-C_6H_4CH_3$-4 | $-C_6H_3Cl_2$-2,4 |
| 88 | O | O | $-C(CH_3)_3$ | $-C_6H_4CH_3$-4 | $-C_6H_3Cl_2$-3,5 |

| Ex. No. | X | X' | $R^1$ | A | B |
|---|---|---|---|---|---|
| 89 | O | O | $-C(CH_3)_3$ | $-C_6H_4CH_3$-4 | $-C_6H_4Cl$-2 |
| 90 | O | O | $-C(CH_3)_3$ | $-C_6H_4CH_3$-4 | $-C_6H_4F$-4 |
| 91 | O | O | $-C(CH_3)_3$ | $-C_6H_4CH_3$-4 | $-C_6H_4CF_3$-4 |
| 92 | O | O | $-C(CH_3)_3$ | $-C_6H_4CH_3$-4 | $-C_6H_4Cl$-3 |
| 93 | O | O | $-C(CH_3)_3$ | $-C_6H_4Cl$-4 | $-C_6H_4CH_2Cl$-3 |
| 94 | O | O | $-C(CH_3)_3$ | $-C_6H_4Cl$-4 | $-C_6H_4CH_2Cl$-4 |
| 95 | O | O | $-C(CH_3)_3$ | $-C_6H_4Cl$-4 | $-C_6H_4CH_3$-2 |
| 96 | O | O | $-C(CH_3)_3$ | $-C_6H_4Cl$-4 | $-C_6H_4OCH_3$-3 |
| 97 | O | O | $-C(CH_3)_3$ | $-C_6H_4Cl$-4 | $-C_6H_4CH_3$-3 |
| 98 | O | O | $-C(CH_3)_3$ | $-C_6H_4F$-4 | $-C_6H_4F$-4 |
| 99 | O | O | $-C(CH_3)_3$ | $-C_6H_4F$-3 | $-C_6H_4F$-3 |
| 100 | O | O | $-C(CH_3)_3$ | $-C_6H_4F$-2 | $-C_6H_4F$-2 |
| 101 | O | O | $-C(CH_3)_3$ | 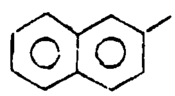 | 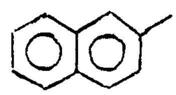 |
| 102 | S | O | $-C(CH_3)_3$ | $C_6H_4Cl$-4 | $C_6H_5$ |
| 103 | O | S | $-C(CH_3)_3$ | $C_6H_5$ | $C_6H_5$ |
| 104 | O | O | $-C(CH_3)_3$ | $-C_6H_5$ | $-C_6H_4Br$-4 |
| 105 | O | O | $-C(CH_3)_3$ | $-C_6H_5$ | $-C_6H_4Br$-3 |
| 106 | O | O | $-C(CH_3)_3$ | $-C_6H_5$ | $-C_6H_4-CH_2CH_2CH_2CH_3$-4 |
| 107 | O | O | $-C(CH_3)_3$ | $-C_6H_4CH_2CH_3$-4 | $-C_6H_5$ |
| 108 | O | O | $-C(CH_3)_3$ | $-C_6H_3Cl_2$-3,4 | $-C_6H_5$ |
| 109 | O | O | $-C(CH_3)_3$ | $-C_6H_5$ | $-C_6H_4COCH_3$-4 |
| 110 | O | O | $-CH_2-C(CH_3)_3$ | $-C_6H_5$ | $-C_6H_4Br$-2 |
| 111 | O | O | $-CH_2-C(CH_3)_3$ | $-C_6H_5$ | $-C_6H_4NO_2$-2 |
| 112 | O | O | $-CH_2-C(CH_3)_3$ | $-C_6H_5$ | $-C_6H_4OCH_3$-2 |
| 113 | O | O | $-C(CH_3)_3$ | $-C_6H_5$ | $-C_6H_4I$-2 |
| 114 | O | O | $-CH_2CH(CH_3)_2$ | $-C_6H_5$ | $-C_6H_5$ |
| 115 | O | O | $-CH(CH_3)_2$ | $-C_6H_5$ | $-C_6H_4Br$-2 |
| 116 | O | O | $-CH(CH_3)_2$ | $-C_6H_5$ | $-C_6H_3Cl_2$-3,4 |
| 117 | O | O | $-C(CH_3)_3$ | $-C_6H_5$ | $-C_6H_4OC_6H_5$-4 |
| 118 | O | O | $-C(CH_3)_3$ | $-C_6H_4CF_3$-4 | $-C_6H_5$ |

| Ex. No. | X | X' | $R^1$ | A | B |
|---|---|---|---|---|---|
| 119 | O | O | $-C(CH_3)_3$ | $-C_6H_4CF_3$-4 | $-C_6H_3Cl_2$-3,4 |
| 120 | O | O | 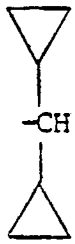 | $-C_6H_5$ | $-C_6H_5$ |
| 121 | O | O | $-C(CH_3)_3$ | $-C_6H_5$ | $-C_6H_3$Cl-2-Br-4 |
| 122 | O | O | $-C(CH_3)_3$ | $-C_6H_5$ | $-C_6H_4C_6H_5$-4 |
| 123 | O | O | $-C(CH_3)_3$ | $-C_6H_5$ | $-C_6H_2(OCH_3)_3$-3,4,5 |
| 124 | O | O | $-CH(CH_3)C(CH_3)_3$ | $-C_6H_5$ | $-C_6H_4NO_2$-2 |
| 125 | O | O | $-C(CH_3)_3$ | $-C_6H_5$ | $-C_6H_4CH_2SCN$-3 |
| 126 | O | O | $-C(CH_3)_3$ | $-C_6H_5$ | $-C_6H_4CH_2CN$-3 |
| 127 | O | O | $-CH(CH_3)C(CH_3)_3$ | $-C_6H_5$ | $-C_6H_5$ |
| 128 | O | O | $-CH[CH(CH_3)_2]_2$ | $-C_6H_5$ | $-C_6H_5$ |
| 129 | O | O | 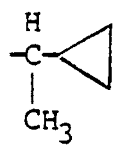 | $-C_6H_5$ | $-C_6H_5$ |
| 130 | O | O | $-C(CH_3)_3$ | $-C_6H_4CH_2CH_3$-4 | $-C_6H_4CH_3$-3 |
| 131 | O | O | $-C(CH_3)_3$ | $-C_6H_4CH_2CH_3$-4 | $-C_6H_4$Cl-4 |
| 132 | O | O | $-C(CH_3)_3$ | $-C_6H_4CH_2CH_3$-4 | $-C_6H_4NO_2$-2 |
| 133 | O | O | $-C(CH_3)_3$ | $-C_6H_5$ | $-C_6H_4CH_2CH_3$-3 |
| 134 | O | O | $-C(CH_3)_3$ | $-C_6H_4CH_2CH_3$-4 | $-C_6H_4$Br-3 |
| 135 | O | O | $-C(CH_3)_3$ | $-C_6H_4CH_2CH_3$-4 | $-C_6H_4$I-2 |
| 136 | O | O | $-CH(CH_3)C(CH_3)_3$ | $-C_6H_5$ | $-C_6H_4$Br-2 |
| 137 | O | O | $-C(CH_3)_3$ | $-C_6H_5$ | $-C_6H_4CO_2CH_3$-4 |
| 138 | O | O | $-C(CH_3)_3$ | $-C_6H_4$Br-2 | $-C_6H_5$ |
| 139 | O | O | $-C(CH_3)_3$ | $-C_6H_4CF_3$-2 | $-C_6H_5$ |
| 140 | O | O | $-C(CH_3)_3$ | $-C_6H_5$ | $-C_6H_4$I-3 |
| 141 | O | O | $-C(CH_3)_3$ | $-C_6H_5$ | $-C_6H_4CH_2CH_3$-2 |
| 142 | O | O | $-C(CH_3)_3$ | $-C_6H_5$ | $-C_6H_4CH_2OCH_3$-3 |

| Ex. No. | X | X' | $R^1$ | A | B |
|---|---|---|---|---|---|
| 143 | 0 | 0 | $-CH(CH_3)-$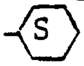 | $-C_6H_5$ | $-C_6H_5$ |
| 144 | 0 | 0 | $-C(CH_3)_3$ | $-C_6H_5$ | $-C_6H_4OCH_2CH{=}CH_2-4$ |
| 145 | 0 | 0 | $-C(CH_3)_3$ | $-C_6H_4C_6H_5-4$ | $-C_6H_4C_6H_5-4$ |
| 146 | 0 | 0 | $-C(CH_3)_3$ | $-C_6H_5$ | 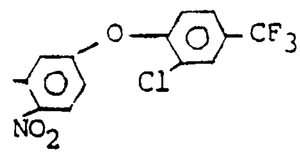 |
| 147 | 0 | 0 | $-C(CH_3)_3$ | 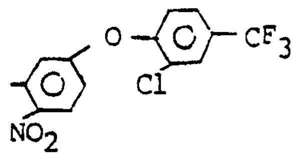 | $C_6H_5$ |
| 148 | 0 | 0 | $-C(CH_3)_3$ | $-C_6H_4(-CH_2OC(O)C_6H_5)-2$ | $-C_6H_5$ |
| 149 | 0 | 0 | $-C(CH_3)_3$ | $-C_6H_5$ | $-C_6H_4SO_2CH_3-4$ |
| 150 | 0 | 0 | $-C(CH_3)_3$ | $-C_6H_5$ | $-C_6H_4OH-2$ |
| 151 | 0 | 0 | $-C(CH_3)_3$ | $-C_6H_5$ | $-C_6H_4SCH_3-4$ |
| 152 | 0 | 0 | $-C(CH_3)_3$ | $-C_6H_5$ | $-C_6H_3Br-3-CH_3-4$ |
| 153 | 0 | 0 | $-C(CH_3)_3$ | $-C_6H_5$ | $-C_6H_3CH_3-3-Br-4$ |
| 154 | 0 | 0 | $-C(CH_3)_3$ | $-C_6H_5$ | $-C_6H_3Br_2-2,4$ |
| 155 | 0 | 0 | $-CH(CH_3)_2$ | $-C_6H_5$ | $-C_6H_3Cl_2-2,6$ |
| 156 | 0 | 0 | $-CH_2C(CH_3)_3$ | $-C_6H_5$ | $-C_6H_3Cl_2-3,4$ |
| 157 | 0 | 0 | $-CH(CH_3)C(CH_3)_3$ | $-C_6H_5$ | $-C_6H_4CN-4$ |
| 158 | 0 | 0 | $-CH_2C(CH_3)_3$ | $-C_6H_5$ | $-C_6H_4CH_2CH_3-4$ |
| 159 | 0 | 0 | $-C(CH_3)_3$ | $-C_6H_5$ | 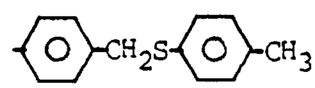 |
| 160 | 0 | 0 | $-C(CH_3)_3$ | $-C_6H_5$ | $-C_6H_4OC_6H_5-3$ |
| 161 | 0 | 0 | $-C(CH_3)_3$ | $-C_6H_5$ | $-C_6H_4(CH_2OC(O)CH_3)-3$ |

| Ex. No. | X | X' | $R^1$ | A | B |
|---|---|---|---|---|---|
| 162 | 0 | 0 | $-C(CH_3)_3$ | $-C_6H_5$ | $-C_6H_4CH_2OH$-4 |
| 163 | 0 | 0 | $-C(CH_3)_3$ | $-C_6H_5$ | $-C_6H_4CHO$-4 |
| 164 | 0 | 0 | $-C(CH_3)_3$ | $-C_6H_5$ | $-C_6H_4CO_2H$-4 |
| 165 | 0 | 0 | $-CH(CH_3)C(CH_3)_3$ | $-C_6H_5$ | $-C_6H_4OH$-2 |
| 166 | 0 | 0 | $-C(CH_3)_3$ | $-C_6H_5$ | $-C_6H_4CH{=}CCl_2$-4 |
| 167 | 0 | 0 | $-CH(CH_3)C(CH_3)_3$ | $-C_6H_5$ | $-C_6H_4(OC(O)CH_3)$-2 |
| 168 | 0 | 0 | $-C(CH_3)_3$ | $-C_6H_3(OCH_3)_2$-3,4 | $-C_6H_5$ |
| 169 | 0 | 0 | $-C(CH_3)_3$ | $-C_6H_4CH_2Cl$-2 | $-C_6H_4CH_2Cl$-2 |
| 170 | 0 | 0 | $-C(CH_3)_3$ | $-C_6H_4CH_2Cl$-2 | $-C_6H_5$ |
| 171 | 0 | 0 | $-C(CH_3)_3$ | $-C_6H_4NO_2$-2 | $-C_6H_5$ |
| 172 | 0 | 0 | $-C(CH_3)_3$ | $-C_6H_4CH_2CH_2CH_3$-4 | $-C_6H_5$ |
| 173 | 0 | 0 | $-C(CH_3)_3$ | $-C_6H_3CH_3$-2-$CF_3$-5 | $-C_6H_4CH_3$-2 |
| 174 | 0 | 0 | $-C(CH_3)_3$ | $-C_6H_4Br$-2 | $-C_6H_4Br$-2 |
| 175 | 0 | 0 | $-C(CH_3)_3$ | $-C_6H_4CH_2CH_3$-4 | $-C_6H_4CH_2CH_3$-3 |
| 176 | 0 | 0 | $-C(CH_3)_3$ | $-C_6H_5$ | $-C_6H_4CH_2CH_2CH_3$-4 |
| 177 | 0 | 0 | $-C(CH_3)_3$ | $-C_6H_4CH_3$-4 | $-C_6H_4Br$-3 |
| 178 | 0 | 0 | $-C(CH_3)_3$ | $-C_6H_4CH_3$-4 | $-C_6H_3(CH_3)_2$-3,5 |
| 179 | 0 | 0 | $-C(CH_3)_3$ | $-C_6H_5$ | $-C_6H_4I$-4 |
| 180 | 0 | 0 | $-C(CH_3)_3$ | $-C_6H_4CH_3$-4 | $-C_6H_4CH_2CH_3$-4 |
| 181 | 0 | 0 | $-C(CH_3)_3$ | $-C_6H_4CH_2CH_3$-4 | $-C_6H_4CH_2CH_3$-4 |
| 182 | 0 | 0 | $-C(CH_3)_3$ | $-C_6H_4CH_3$-4 | $-C_6H_4CH_2CH_3$-3 |
| 183 | 0 | 0 | $-C(CH_3)_3$ | $-C_6H_5$ | $-C_6H_4CH(CH_3)_2$-2 |
| 184 | 0 | 0 | $-C(CH_3)_3$ | $-C_6H_4CH_2CH_3$-3 | $-C_6H_5$ |
| 185 | 0 | 0 | $-CH(CH_3)C(CH_3)_3$ | $-C_6H_5$ | $-C_6H_4NO_2$-3 |
| 186 | 0 | 0 | $-CH_2C(CH_3)_3$ | $-C_6H_5$ | $-C_6H_4NO_2$-3 |
| 187 | 0 | 0 | $-CH_2C(CH_3)_3$ | $-C_6H_5$ | $-C_6H_4CH_3$-3 |
| 188 | 0 | 0 | $-CH_2C(CH_3)_3$ | $-C_6H_5$ | $-C_6H_4Cl$-4 |
| 189 | 0 | 0 | $-CH_2C(CH_3)_3$ | $-C_6H_5$ | $-C_6H_3(NO_2)_2$-2,4 |
| 190 | 0 | 0 | $-C(CH_3)_3$ | $-C_6H_4CH_2CH_3$-4 | $-C_6H_3Cl_2$-2,4 |
| 191 | 0 | 0 | $-C(CH_3)_3$ | $-C_6H_3Cl_2$-3,4 | $-C_6H_4Cl$-4 |
| 192 | 0 | 0 | $-C(CH_3)_3$ | $-C_6H_4(CH_2)_6CH_3$-4 | $-C_6H_4Cl$-4 |
| 193 | 0 | 0 | $-C(CH_3)_3$ | $-C_6H_4CH_2CH_2CH_3$-4 | $-C_6H_4Cl$-4 |

| Ex. No. | X | X' | $R^1$ | A | B |
|---|---|---|---|---|---|
| 194 | 0 | 0 | $-C(CH_3)_3$ | $-C_6H_4OCH_3$-4 | $-C_6H_4CH_3$-3 |
| 195 | 0 | 0 | $-C(CH_3)_3$ | $-C_6H_4OCH_3$-4 | $-C_6H_5$ |
| 196 | 0 | 0 | $-C(CH_3)_3$ | $-C_6H_4Cl$-2 | $-C_6H_4CH_3$-3 |
| 197 | 0 | 0 | $-C(CH_3)_3$ | $-C_6H_4Cl$-2 | $-C_6H_4NO_2$-2 |
| 198 | 0 | 0 | $-C(CH_3)_3$ | $-C_6H_4Cl$-2 | $-C_6H_4CH_3$-4 |
| 199 | 0 | 0 | $-C(CH_3)_3$ | $-C_6H_4Cl$-2 | $-C_6H_4CH_2CH_3$-4 |
| 200 | 0 | 0 | $-C(CH_3)_3$ | $-C_6H_4CH_2CH_3$-4 | $-C_6H_3(CH_3)_2$-3,5 |
| 201 | 0 | 0 | $-C(CH_3)_3$ | $-C_6H_4CH_2CH_3$-4 | $-C_6H_3CH_3$-3-Cl-6 |
| 202 | 0 | 0 | $-C(CH_3)_3$ | $-C_6H_5$ | $-C_6H_4(OC(O)CH_3)$-3 |
| 203 | 0 | 0 | $-C(CH_3)_3$ | $-C_6H_5$ | $-C_6H_4OH$-3 |
| 204 | 0 | 0 | $-C(CH_3)_3$ | $-C_6H_4CH_2CH_3$-4 | $-C_6H_3NO_2$-2-$CH_3$-3 |
| 205 | 0 | 0 | $-C(CH_3)_3$ | $-C_6H_4OCH_3$-4 | $-C_6H_3(CH_3)_2$-3,5 |
| 206 | 0 | 0 | $-C(CH_3)_3$ | $-C_6H_4OCH_3$-4 | $-C_6H_3Cl_2$-2,4 |
| 207 | 0 | 0 | $-C(CH_3)_3$ | $-C_6H_4Cl$-2 | $-C_6H_3Cl_2$-2,6 |
| 208 | 0 | 0 | $-C(CH_3)_3$ | $-C_6H_4Cl$-2 | $-C_6H_4Br$-2 |
| 209 | 0 | 0 | $-C(CH_3)_3$ | $-C_6H_4Cl$-2 | $-C_6H_3F_2$-2,5 |
| 210 | 0 | 0 | $-C(CH_3)_3$ | $-C_6H_4Cl$-2 | $-C_6H_4OCH_3$-4 |
| 211 | 0 | 0 | $-C(CH_3)_3$ | $-C_6H_4Cl$-2 | $-C_6H_4CH_3$-2 |
| 212 | 0 | 0 | $-C(CH_3)_3$ | $-C_6H_4F$-2 | $-C_6H_4Cl$-4 |
| 213 | 0 | 0 | $-C(CH_3)_3$ | $-C_6H_3Cl_2$-2,4 | $-C_6H_4Cl$-4 |
| 214 | 0 | 0 | $-C(CH_3)_3$ | $-C_6H_4Cl$-2 | $-C_6H_3Cl_2$-2,4 |
| 215 | 0 | 0 | $-C(CH_3)_3$ | $-C_6H_4Cl$-2 | $-C_6H_4OCH_3$-3 |
| 216 | 0 | 0 | $-C(CH_3)_3$ | $-C_6H_4Cl$-2 | $-C_6H_4Cl$-3 |
| 217 | 0 | 0 | $-C(CH_3)_3$ | $-C_6H_4Cl$-2 | $-C_6H_4CF_3$-2 |
| 218 | 0 | 0 | $-C(CH_3)_3$ | $-C_6H_4Cl$-2 | $-C_6H_4CF_3$-3 |
| 219 | 0 | 0 | $-C(CH_3)_3$ | $-C_6H_4Cl$-2 | $-C_6H_4CF_3$-4 |
| 220 | 0 | 0 | $-C(CH_3)_3$ | $-C_6H_4CH_3$-3 | $-C_6H_5$ |
| 221 | 0 | 0 | $-C(CH_3)_3$ | $-C_6H_4NO_2$-3 | $-C_6H_5$ |
| 222 | 0 | 0 | $-C(CH_3)_3$ | $-C_6H_3Cl_2$-2,6 | $-C_6H_5$ |
| 223 | 0 | 0 | $-C(CH_3)_3$ | $-C_6H_3F_2$-2,4 | $-C_6H_5$ |
| 224 | 0 | 0 | $-C(CH_3)_3$ | $-C_6H_4Cl$-2 | $-C_6H_4CN$-4 |
| 225 | 0 | 0 | $-C(CH_3)_3$ | $-C_6H_4Cl$-2 | $-C_6H_4F$-4 |

| Ex. No. | X | X' | $R^1$ | A | B |
|---|---|---|---|---|---|
| 226 | 0 | 0 | $-C(CH_3)_3$ | $-C_6H_4Cl-2$ | $-C_6H_4Br-4$ |
| 227 | 0 | 0 | $-C(CH_3)_3$ | $-C_6H_4Cl-2$ | $-C_6H_4Cl-4$ |
| 228 | 0 | 0 | $-C(CH_3)_3$ | $-C_6H_4Cl-2$ | $-C_6H_4OCH_3-2$ |
| 229 | 0 | 0 | $-C(CH_3)_3$ | $-C_6H_4Cl-2$ | $-C_6H_4NO_2-4$ |
| 230 | 0 | 0 | $-C(CH_3)_3$ | $-C_6H_4Cl-2$ | $-C_6H_4F-2$ |
| 231 | 0 | 0 | $-C(CH_3)_3$ | $-C_6H_4Cl-2$ | $-C_6H_3F_2-2,6$ |
| 232 | 0 | 0 | $-C(CH_3)_3$ | $-C_6H_4NO_2-4$ | $-C_6H_5$ |
| 233 | 0 | 0 | $-C(CH_3)_3$ | $-C_6H_4CN-4$ | $-C_6H_5$ |
| 234 | 0 | 0 | $-C(CH_3)_3$ | $-C_6H_4OCH_3-3$ | $-C_6H_4OCH_3-3$ |
| 235 | 0 | 0 | $-C(CH_3)_3$ | $-C_6H_4OCH_3-3$ | $-C_6H_5$ |
| 236 | 0 | 0 | $-C(CH_3)_3$ | $-C_6H_4OCH_3-3$ | $-C_6H_4Cl-4$ |
| 237 | 0 | 0 | $-C(CH_3)_3$ | $-C_6H_4OCH_3-3$ | $-C_6H_4Cl_2-3,4$ |
| 238 | 0 | 0 | $-C(CH_3)_3$ | $-C_6H_4OCH_3-2$ | $-C_6H_5$ |
| 239 | 0 | 0 | $-C(CH_3)_3$ | $-C_6H_4OCH_3-2$ | $-C_6H_4CH_3-3$ |
| 240 | 0 | 0 | $-C(CH_3)_3$ | $-C_6H_4OCH_3-2$ | $-C_6H_3Cl_2-3,4$ |
| 241 | 0 | 0 | $-C(CH_3)_3$ | $-C_6H_4OCH_3-2$ | $-C_6H_4Cl-4$ |
| 242 | 0 | 0 | $-C(CH_3)_3$ | $-C_6H_4OCH_3-2$ | $-C_6H_4NO_2-2$ |
| 243 | 0 | 0 | $-C(CH_3)_3$ | $-C_6H_5$ | $-C_6H_4OCF_3-4$ |
| 244 | 0 | 0 | $-C(CH_3)_3$ | $-C_6H_4OCF_3-4$ | $-C_6H_5$ |
| 245 | 0 | 0 | $-C(CH_3)_3$ | $-C_6H_4OCF_3-4$ | $-C_6H_4CH_3-3$ |
| 246 | 0 | 0 | $-C(CH_3)_3$ | $-C_6H_4OCF_3-4$ | $-C_6H_4Cl-4$ |
| 247 | 0 | 0 | $-C(CH_3)_3$ | $-C_6H_4OCF_3-4$ | $-C_6H_3Cl_2-3,4$ |
| 248 | 0 | 0 | $-C(CH_3)_3$ | $-C_6H_4CF_3-4$ | $-C_6H_4Cl-4$ |
| 249 | 0 | 0 | $-C(CH_3)_3$ | $-C_6H_4CF_3-4$ | $-C_6H_4CH_3-3$ |
| 250 | 0 | 0 | $-C(CH_3)_2CH_2CH_2CH_2CH_3$ | $-C_6H_5$ | $-C_6H_5$ |
| 251 | 0 | 0 | $-C(CH_3)_3$ | $-C_6H_4OCH_2CH_3-4$ | $-C_6H_4CH_3-3$ |
| 252 | 0 | 0 | $-C(CH_3)_3$ | $-C_6H_4OCH_2CH_3-4$ | $-C_6H_3(CH_3)_2-3,5$ |
| 253 | 0 | 0 | $-C(CH_3)_3$ | $-C_6H_4OCH_2CH_3-4$ | $-C_6H_5$ |
| 254 | 0 | 0 | $-C(CH_3)_3$ | $-C_6H_4CH_2CH_3-4$ | $-C_6H_3NO_2-2-Cl-4$ |
| 255 | 0 | 0 | $-C(CH_3)_3$ | $-C_6H_3Cl-3-OCH_3-4$ | $-C_6H_5$ |
| 256 | 0 | 0 | $-C(CH_3)_3$ | $-C_6H_4SCH_3-4$ | $-C_6H_5$ |

| Ex. No. | X | X' | $R^1$ | A | B |
|---|---|---|---|---|---|
| 257 | O | O | $-C(CH_3)_3$ | $-C_6H_4OCH_2CH_2CH_2CH_3$-4 | $-C_6H_4Cl$-4 |
| 258 | O | O | $-C(CH_3)_3$ | $-C_6H_4SCH_3$-2 | $-C_6H_5$ |
| 259 | O | O | $-C(CH_3)_3$ | $-C_6H_3NO_2$-2-Cl-4 | $-C_6H_5$ |
| 260 | O | O | $-C(CH_3)_3$ | $-C_6H_3NO_2$-2-Cl-4 | $-C_6H_3NO_2$-2-Cl-4 |
| 261 | O | O | $-C(CH_3)_3$ | $-C_6H_3NO_2$-2-Cl-4 | $-C_6H_4C(CH_3)_3$-4 |
| 262 | O | O | $-C(CH_3)_3$ | $-C_6H_3NO_2$-2-Cl-4 | $-C_6H_4Cl$-4 |
| 263 | O | O | $-C(CH_3)_3$ | $-C_6H_4CH_3$-4 | $-C_6H_3CH_3$-3-Cl-6 |
| 264 | O | O | $-C(CH_3)_3$ | $-C_6H_3F_2$-2,6 | $-C_6H_3F_2$-2,6 |
| 265 | O | O | $-C(CH_3)_3$ | $-C_6H_4OC_6H_5$-4 | $-C_6H_5$ |
| 266 | O | O | $-C(CH_3)_3$ | $-C_6H_4OC_6H_5$-4 | $-C_6H_4CH_3$-4 |
| 267 | O | O | $-C(CH_3)_3$ | $-C_6H_4CH_2CH_2CH_2CH_3$-4 | $-C_6H_5$ |
| 268 | O | O | $-C(CH_3)_3$ | $-C_6H_4CH_2CH_2CH_2CH_3$-4 | $-C_6H_4Cl$-4 |
| 269 | O | O | $-C(CH_3)_3$ | $-C_6H_4C(CH_3)_2$-4 | $-C_6H_5$ |
| 270 | O | O | $-C(CH_3)_3$ | $-C_6H_4CH(CH_3)_2$-4 | $-C_6H_4CH_3$-3 |
| 271 | O | O | $-C(CH_3)_3$ | $-C_6H_4OH$-4 | $-C_6H_5$ |
| 272 | O | O | $-C(CH_3)_3$ | $-C_6H_4CN$-4 | $-C_6H_5$ |
| 273 | O | O | $-C(CH_3)_3$ | $-C_6H_4CN$-4 | $-C_6H_4CH_3$-3 |
| 274 | O | O | $-C(CH_3)_3$ | $-C_6H_3CH_3$-2-Cl-4 | $-C_6H_5$ |
| 275 | O | O | $-C(CH_3)_3$ | $-C_6H_4CH_2CH_3$-4 | $-C_6H_4OCF_3$-4 |
| 276 | O | O | $-C(CH_3)_3$ | $-C_6H_5$ | $-C_6F_5$-2,3,4,5,6 |
| 277 | O | O | $-C(CH_3)_3$ | $-C_6F_5$-2,3,4,5,6 | $-C_6H_5$ |
| 278 | O | O | $-C(CH_3)_3$ | $-C_6H_4CN$-4 | $-C_6H_3Cl_2$-3,4 |
| 279 | O | O | $-C(CH_3)_3$ | $-C_6H_3CH_3$-2-Cl-4 | $-C_6H_4CH_3$-3 |
| 280 | O | O | $-C(CH_3)_3$ | $-C_6H_4CF_3$-4 | $-C_6H_3(CH_3)_2$-3,5 |
| 281 | O | O | $-C(CH_3)_3$ | $-C_6H_3CH_3$-2-Cl-4 | $-C_6H_3(CH_3)_2$-3,5 |
| 282 | O | O | $-C(CH_3)_3$ | $-C_6H_5$ | $-C_6H_4CH=CH_2$-3 |
| 283 | O | O | $-C(CH_3)_3$ | $-C_6H_4CH_2CH_3$-4 | $-C_6H_4CH=CH_2$-3 |
| 284 | O | O | $-C(CH_3)_3$ | $-C_6H_4CF_3$-4 | $-C_6H_4CH=CH_2$-3 |
| 285 | O | O | $-C(CH_3)_3$ | $-C_6H_4OH$-4 | $-C_6H_4CH_3$-3 |

| Ex. No. | X | X' | $R^1$ | A | B |
|---|---|---|---|---|---|
| 286 | 0 | 0 | $-C(CH_3)_3$ | $-C_6H_4OCH_2CH=CH_2-4$ | $-C_6H_4CH_3-3$ |
| 287 | 0 | 0 | $-C(CH_3)_3$ | $-C_6H_4CH_2CH_2CH_3-4$ | $-C_6H_3Cl_2-3,4$ |
| 288 | 0 | 0 | $-C(CH_3)_3$ | $-C_6H_4CH_2CH_2CH_3-4$ | $-C_6H_4CH_3-3$ |
| 289 | 0 | 0 | $-C(CH_3)_3$ | $-C_6H_4CH=CH_2-4$ | $-C_6H_4CH=CH_2-4$ |
| 290 | 0 | 0 | $-C(CH_3)_3$ | $-C_6H_4CH=CH_2-4$ | $-C_6H_4CH_3-3$ |
| 291 | 0 | 0 | $-C(CH_3)_3$ | $-C_6H_3F_2-2,6$ | $-C_6H_3Cl_2-3,4$ |
| 292 | 0 | 0 | $-C(CH_3)_3$ | $-C_6H_3F_2-2,6$ | $-C_6H_4CH_3-3$ |
| 293 | 0 | 0 | $-C(CH_3)_3$ | $-C_6H_4CH_2Cl-2$ | $-C_6H_4Cl-4$ |
| 294 | 0 | 0 | $-C(CH_3)_3$ | $-C_6H_4CH_2N(CH_2CH_3)_2-2$ | $-C_6H_4Cl-4$ |
| 295 | 0 | 0 | $-C(CH_3)_2CH_2CH_3$ | $-C_6H_5$ | $-C_6H_5$ |
| 296 | 0 | 0 | $-C(CH_3)_3$ | $-C_6H_4Cl-4$ | $-C_6H_4Br-2$ |
| 297 | 0 | 0 | $-C(CH_3)_2CH_2CH_3$ | $-C_6H_4CH_3-3$ | $-C_6H_4CH_3-3$ |
| 298 | 0 | 0 | $-C(CH_3)_2CH_2CH_3$ | $-C_6H_4Br-2$ | $-C_6H_4Br-2$ |
| 299 | 0 | 0 | $-C(CH_3)_3$ | $-C_6H_4Cl-4$ | $-C_6H_3(CH_3)_2-3,5$ |
| 300 | 0 | 0 | $-C(CH_3)_3$ | $-C_6H_4OCH_3-3$ | $-C_6H_3(CH_3)_2-3,5$ |
| 301 | 0 | 0 | $-C(CH_3)_3$ | $-C_6H_4Cl-4$ | $-C_6H_4F-4$ |
| 302 | 0 | 0 | $-C(CH_3)_3$ | $-C_6H_4CH_2CH_3-4$ | $-C_6H_3(CH_3)_2-3,5$ |
| 303 | 0 | 0 | $-C(CH_3)_3$ | $-C_6H_4CH=CH_2-4$ | $-C_6H_3(CH_3)_2-3,5$ |
| 304 | 0 | 0 | $-C(CH_3)_3$ | $-C_6H_4(OC(O)CH_3)-4$ | $-C_6H_3(CH_3)_2-3,5$ |
| 305 | 0 | 0 | $-C(CH_3)_3$ | $-C_6H_3(CH_3)_2-3,5$ | $-C_6H_5$ |
| 306 | 0 | 0 | $-C(CH_3)_3$ | $-C_6H_3(CH_3)_2-3,5$ | $-C_6H_4CH_3-3$ |
| 307 | 0 | 0 | $-C(CH_3)_3$ | $-C_6H_3(CH_3)_2-3,5$ | $-C_6H_4CH_2CH_3-4$ |
| 308 | 0 | 0 | $-C(CH_3)_3$ | $-C_6H_4CH=CHCH_3-4$ | $-C_6H_4CH=CHCH_3-4$ |
| 309 | 0 | 0 | $-C(CH_3)_3$ | $-C_6H_4CH(CH_3)_2-4$ | $-C_6H_3(CH_3)_2-3,5$ |
| 310 | 0 | 0 | $-C(CH_3)_3$ | $-C_6H_4CH_2CH_3-4$ | $-C_6H_4Br-2$ |
| 311 | 0 | 0 | $-C(CH_3)_3$ | $-C_6H_3Cl-3-CH_3-4$ | $-C_6H_5$ |
| 312 | 0 | 0 | $-C(CH_3)_3$ | $-C_6H_3Cl-3-CH_3-4$ | $-C_6H_4CH_3-3$ |
| 313 | 0 | 0 | $-C(CH_3)_3$ | $-C_6H_3Cl-3-CH_3-4$ | $-C_6H_4Cl-4$ |
| 314 | 0 | 0 | $-C(CH_3)_3$ | $-C_6H_3Cl-3-CH_3-4$ | $-C_6H_3Cl_2-3,4$ |
| 315 | 0 | 0 | $-C(CH_3)_2CH_2CH_3$ | $-C_6H_4CH_2CH_3-4$ | $-C_6H_4NO_2-2$ |
| 316 | 0 | 0 | $-C(CH_3)_3$ | $-C_6H_3F_2-2,6$ | $-C_6H_4Cl-2$ |

| Ex. No. | X | X' | $R^1$ | A | B |
|---|---|---|---|---|---|
| 317 | O | O | $-C(CH_3)_3$ | $-C_6H_3F_2$-2,6 | $-C_6H_4Cl$-3 |
| 318 | O | O | $-C(CH_3)_3$ | $-C_6H_3F_2$-2,6 | $-C_6H_4Cl$-4 |
| 319 | O | O | $-C(CH_3)_3$ | $-C_6H_3F_2$-2,6 | $-C_6H_3(CH_3)_2$-3,5 |
| 320 | O | O | $-C(CH_3)_2CH_2CH_3$ | $-C_6H_4CH_2CH_3$-4 | $-C_6H_3(CH_3)_2$-3,5 |
| 321 | O | O | $-C(CH_3)_3$ | $-C_6H_4CF_3$-3 | $-C_6H_4Cl$-4 |
| 322 | O | O | $-C(CH_3)_3$ | $-C_6H_4CF_3$-3 | $-C_6H_3(CH_3)_2$-3,5 |
| 323 | S | O | $-C(CH_3)_3$ | $-C_6H_5$ | $-C_6H_4Cl$-4 |
| 324 | S | O | $-C(CH_3)_3$ | $-C_6H_5$ | $-C_6H_4CH_3$-3 |
| 325 | O | O | $-C(CH_3)_3$ | $-C_6H_3NO_2$-2-$OCH_3$-3 | $-C_6H_4CH_3$-3 |
| 326 | O | O | $-C(CH_3)_3$ | $-C_6H_3Br$-3-$CH_3$-4 | $-C_6H_5$ |
| 327 | O | O | $-C(CH_3)_3$ | $-C_6H_4Cl$-4 | $-C_6H_4CO_2CH_3$-4 |
| 328 | O | O | $-C(CH_3)_3$ | $-C_6H_4CH_2CH_3$-4 | $-C_6H_4CO_2CH_3$-4 |
| 329 | O | O | $-C(CH_3)_3$ | $-C_6H_5$ | $-C_6H_4NH_2$-3 |
| 330 | O | O | $-C(CH_3)_3$ | $-C_6H_5$ | $-C_6H_4NH_2$-2 |
| 331 | O | O | $-C(CH_3)_3$ | $-C_6H_4F$-4 | $-C_6H_5$ |
| 332 | O | O | $-C(CH_3)C(CH_3)_3$ | $-C_6H_4CH_3$-4 | $-C_6H_4NO_2$-2 |
| 333 | O | O | $-C(CH_3)_3$ | $-C_6H_4OH$-3 | $-C_6H_5$ |
| 334 | O | O | $-C(CH_3)_3$ | $-C_6H_4CH_2CH_3$-4 | $-C_6H_3Cl_2$-3,5 |
| 335 | O | O | $-C(CH_3)_3$ | $-C_6H_4OCH_2CH{=}CH_2$-3 | $-C_6H_5$ |
| 336 | O | O | $-CH(CH_3)C(CH_3)_3$ | $-C_6H_4CH_3$-4 | $-C_6H_3(CH_3)_2$-3,5 |
| 337 | O | O | $-CH(CH_3)C(CH_3)_3$ | $-C_6H_4CH_3$-4 | $-C_6H_3NO_2$-2-$CH_3$-3 |
| 338 | O | O | $-CH(CH_3)C(CH_3)_3$ | $-C_6H_4CH_3$-4 | $-C_6H_3NO_2$-2-$CH_3$-5 |
| 339 | O | O | $-CH(CH_3)C(CH_3)_3$ | $-C_6H_4CH_3$-4 | $-C_6H_4CH_3$-3 |
| 340 | O | O | $-CH(CH_3)C(CH_3)_3$ | $-C_6H_4CH_3$-4 | $-C_6H_4I$-2 |
| 341 | O | O | $-C(CH_3)_3$ | $-C_6H_4Cl$-4 | $-C_6H_4F$-2 |
| 342 | O | O | $-C(CH_3)_3$ | $-C_6H_4CH_2CH_3$-4 | $-C_6H_3Cl_2$-3,4 |
| 343 | O | O | $-C(CH_3)_3$ | $-C_6H_4CH_2CH_3$-4 | $-C_6H_4F$-2 |
| 344 | O | O | $-C(CH_3)_3$ | $-C_6H_4OC(O)N(CH_3)_2$-3 | $-C_6H_5$ |
| 345 | O | O | $-C(CH_3)_3$ | $-C_6H_4OCO_2CH{=}CH_2$-3 | $-C_6H_5$ |
| 346 | O | O | $-C(CH_3)_3$ | $-C_6H_4CO_2CH_3$-4 | $-C_6H_4Cl$-4 |
| 347 | O | O | $-C(CH_3)_3$ | $-C_6H_4CO_2CH_3$-4 | $-C_6H_3(CH_3)_2$-3,5 |

| Ex. No. | X | X' | $R^1$ | A | B |
|---|---|---|---|---|---|
| 348 | 0 | 0 | $-C(CH_3)_3$ | $-C_6H_4CO_2CH_3-4$ | $-C_6H_4CH_3-3$ |
| 349 | 0 | 0 | $-C(CH_3)_3$ | $-C_6H_4CO_2H-4$ | $-C_6H_4CH_3-3$ |
| 350 | 0 | 0 | $-C(CH_3)_3$ | $-C_6H_3NH_2-2-OCH_3-3$ | $-C_6H_4CH_3-3$ |
| 351 | 0 | 0 | $-C(CH_3)_3$ | $-C_6H_4NH_2-4$ | $-C_6H_4Cl-4$ |
| 352 | 0 | 0 | $-C(CH_3)_3$ | $-C_6H_4NHCO_2CH_3-4$ | $-C_6H_4Cl-4$ |
| 353 | 0 | 0 | $-C(CH_3)_3$ | $-C_6H_4NHC(O)CH_3-4$ | $-C_6H_4Cl-4$ |
| 354 | 0 | 0 | $-C(CH_3)_3$ | $-C_6H_3NHC(O)CH_3-2-OCH_3-3$ | $-C_6H_4CH_3-3$ |
| 355 | 0 | 0 | $-C(CH_3)_3$ | $-C_6H_4OC_6H_5-3$ | $-C_6H_5$ |
| 356 | 0 | 0 | $-C(CH_3)_3$ | $-C_6H_4OC_6H_5-3$ | $-C_6H_4CH_3-3$ |
| 357 | 0 | 0 | $-C(CH_3)_3$ | $-C_6H_4C(O)CH_3-4$ | $-C_6H_4CH_3-3$ |
| 358 | 0 | 0 | $-C(CH_3)_3$ | $-C_6H_4OCH_2OCH_3-4$ | $-C_6H_5$ |
| 359 | 0 | 0 | $-C(CH_3)_3$ | $-C_6H_4OC(O)N(CH_3)_2-4$ | $-C_6H_5$ |
| 360 | 0 | 0 | $-C(CH_3)_3$ | $-C_6H_4OCH_2CO_2CH_3-4$ | $-C_6H_5$ |
| 361 | 0 | 0 | $-C(CH_3)_3$ | $-C_6H_4CH_2OC(O)CH_3-4$ | $-C_6H_5$ |
| 362 | 0 | 0 | $-C(CH_3)_3$ | $-C_6H_4CH_2SCN-4$ | $-C_6H_5$ |
| 363 | 0 | 0 | $-C(CH_3)_3$ | $-C_6H_4CH_2OH-4$ | $-C_6H_5$ |
| 364 | 0 | 0 | $-C(CH_3)_3$ | $-C_6H_4Br-4$ | $-C_6H_4Br-4$ |
| 365 | 0 | 0 | $-C(CH_3)_3$ | $-C_6H_4OCH_2SCH_3-4$ | $-C_6H_5$ |
| 366 | 0 | 0 | $-C(CH_3)_3$ | $-C_6H_4OCH_2C(CH_3)_2-4$ | $-C_6H_5$ |
| 367 | 0 | 0 | $-C(CH_3)_3$ | $-C_6H_4CH_2CN-4$ | $-C_6H_5$ |
| 368 | 0 | 0 | $-C(CH_3)_3$ | 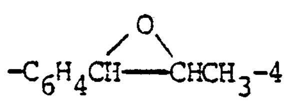 | $-C_6H_5$ |
| 369 | 0 | 0 | $-C(CH_3)_3$ | $-C_6H_4C(CH_3)=NNHC(O)NH_2-4$ | $-C_6H_4CH_3-3$ |
| 370 | 0 | 0 | $-C(CH_3)_3$ | $-C_6H_4C_6H_5-4$ | $-C_6H_4CH_3-3$ |
| 371 | 0 | 0 | $-C(CH_3)_3$ | $-C_6H_4CN-3$ | $-C_6H_4CH_3-3$ |
| 372 | 0 | 0 | $-C(CH_3)_3$ | $-C_6H_4NH_2-3$ | $-C_6H_4CH_3-3$ |
| 373 | 0 | 0 | $-C(CH_3)_3$ | $-C_6H_4C(O)NHC(CH_3)_2CH_2OH-4$ | $-C_6H_4CH_3-3$ |
| 374 | 0 | 0 | $-C(CH_3)_3$ | $-C_6H_4CH(OH)CH_3-4$ | $-C_6H_4CH_3-3$ |

| Ex. No. | X | X' | $R^1$ | A | B |
|---|---|---|---|---|---|
| 375 | 0 | 0 | $-C(CH_3)_3$ | $-C_6H_4NHC(O)C(CH_3)=CH_2-3$ | $-C_6H_4CH_3-3$ |
| 376 | 0 | 0 | $-C(CH_3)_3$ | $-C_6H_4CO_2H-3$ | $-C_6H_4CH_3-3$ |
| 377 | 0 | 0 | $-C(CH_3)_3$ | $-C_6H_4CH_2Cl-3$ | $-C_6H_4CH_3-3$ |
| 378 | 0 | 0 | $-C(CH_3)_3$ | $-C_6H_4CH_2CH_3-4$ | $-C_6H_3(CH_3)_2-2,3$ |
| 379 | 0 | 0 | $-C(CH_3)_3$ | $-C_6H_3(CH_3)_2-2,3$ | $-C_6H_4CH_3-3$ |
| 380 | 0 | 0 | $-C(CH_3)_3$ | $-C_6H_5$ | $-C_6H_3(CH_3)_2-2,3$ |
| 381 | 0 | 0 | $-C(CH_3)_3$ | $-C_6H_4CH_3-4$ | $-C_6H_3(CH_3)_2-2,3$ |
| 382 | 0 | 0 | $-CH(CH_3)C(CH_3)_3$ | $-C_6H_4CH_3-4$ | $-C_6H_4CH_3-2$ |
| 383 | 0 | 0 | $-CH(CH_3)C(CH_3)_3$ | $-C_6H_4CH_3-4$ | $-C_6H_4CF_3-2$ |
| 384 | 0 | 0 | $-CH(CH_3)CH_2C(CH_3)_3$ | $-C_6H_4CH_3-4$ | $-C_6H_5$ |
| 385 | 0 | 0 | $-C(CH_3)_3$ | $-C_6H_4OCH_2OCH_3-4$ | $-C_6H_4CH_3-3$ |
| 386 | 0 | 0 | $-C(CH_3)_3$ | $-C_6H_4C(CH_3)=CH_2-4$ | $-C_6H_4CH_3-3$ |
| 387 | 0 | 0 | $-C(CH_3)_3$ | $-C_6H_5$ | 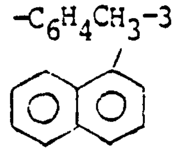 |
| 388 | 0 | 0 | $-C(CH_3)_3$ | 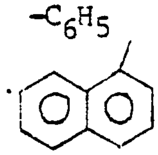 | $-C_6H_4CH_3-3$ |
| 389 | 0 | 0 | $-C(CH_3)_3$ | $-C_6H_4NCS-4$ | $-C_6H_4CH_3-3$ |
| 390 | 0 | 0 | $-C(CH_3)_3$ | $-C_6H_3(CH_3)_2-3,5$ | $-C_6H_3(CH_3)_2-3,5$ |
| 391 | 0 | 0 | $-C(CH_3)_3$ | $-C_6H_3Cl_2-2,4$ | $-C_6H_3Cl_2-2,4$ |
| 392 | 0 | 0 | $-C(CH_3)_3$ | $-C_6H_4F-2$ | $-C_6H_4Br-2$ |
| 393 | 0 | 0 | $-C(CH_3)_3$ | $-C_6H_4F-2$ | $-C_6H_4CH_3-3$ |
| 394 | 0 | 0 | $-C(CH_3)_3$ | $-C_6H_3(CH_3)_2-2,3$ | $-C_6H_3(CH_3)_2-2,3$ |
| 395 | 0 | 0 | $-C(CH_3)_3$ | $-C_6H_4F-2$ | $-C_6H_4NO_2-2$ |
| 396 | 0 | 0 | $-C(CH_3)_3$ | $-C_6H_4F-2$ | $-C_6H_5$ |
| 397 | 0 | 0 | $-C(CH_3)_3$ | $-C_6H_3CH_3-2-Cl-3$ | $-C_6H_4CH_3-3$ |
| 398 | 0 | 0 | $-C(CH_3)_3$ | $-C_6H_3CH_3-2-Cl-3$ | $-C_6H_5$ |
| 399 | 0 | 0 | $-CH(CH_3)CH_2C(CH_3)_3$ | $-C_6H_4CH_3-4$ | $-C_6H_3(CH_3)_2-3,5$ |

| Ex. No. | X | X' | $R^1$ | A | B |
|---|---|---|---|---|---|
| 400 | 0 | 0 | $-CH(CH_3)CH_2C(CH_3)_3$ | $-C_6H_4CH_3$-4 | $-C_6H_3Cl_2$-3,4 |
| 401 | 0 | 0 | $-C(CH_3)_3$ | $-C_6H_4Br$-4 | $-C_6H_5$ |
| 402 | 0 | 0 | $-C(CH_3)_3$ | $-C_6H_3Cl_2$-2,3 | $-C_6H_4Br$-2 |
| 403 | 0 | 0 | $-C(CH_3)_3$ | $-C_6H_3Cl_2$-2,3 | $-C_6H_5$ |
| 404 | 0 | 0 | $-C(CH_3)_3$ | $-C_6H_4F$-2 | $-C_6H_3(CH_3)_2$-3,5 |
| 405 | 0 | 0 | $-C(CH_3)_3$ | $-C_6H_3Cl_2$-2,3 | $-C_6H_4CH_3$-3 |
| 406 | 0 | 0 | $-C(CH_3)_3$ | $-C_6H_5$ | 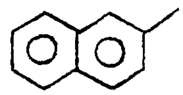 |
| 407 | 0 | 0 | $-C(CH_3)_3$ | 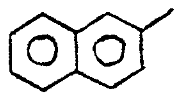 | $-C_6H_4CH_3$-3 |
| 408 | 0 | 0 | $-C(CH_3)_3$ | $-C_6H_5$ | $-C_6H_4OCF_3$-3 |
| 409 | 0 | 0 | $-C(CH_3)_3$ | $-C_6H_4NCS$-4 | $-C_6H_5$ |
| 410 | 0 | 0 | $-C(CH_3)_3$ | $-C_6H_3F_2$-2,6 | $-C_6H_3Cl_2$-2,4 |
| 411 | 0 | 0 | $-C(CH_3)_3$ | $-C_6H_3F_2$-2,6 | $-C_6H_3Cl_2$-3,5 |
| 412 | 0 | 0 | $-C(CH_3)_3$ | $-C_6H_4CH_3$-4 | $-C_6H_3(CF_3)_2$-3,5 |
| 413 | 0 | 0 | $-C(CH_3)_3$ | $-C_6H_5$ | $-C_6H_3(CF_3)_2$-3,5 |
| 414 | 0 | 0 | $-CH(CH_3)C(CH_3)(CH_2CH_3)_2$ | $-C_6H_4CH_3$-4 | $-C_6H_5$ |
| 415 | 0 | 0 | $-CH(CH_3)C(CH_3)(CH_2CH_3)_2$ | $-C_6H_4CH_3$-4 | $-C_6H_3(CH_3)_2$-3,5 |
| 416 | 0 | 0 | $-CH(CH_3)C(CH_3)(CH_2CH_3)_2$ | $-C_6H_4CH_3$-4 | $-C_6H_4NO_2$-2 |
| 417 | 0 | 0 | $-CH(CH_3)C(CH_3)_3$ | $-C_6H_4CH_2CH_3$-4 | $-C_6H_3(CH_3)_2$-3,5 |
| 418 | 0 | 0 | $-CH(CH_3)C(CH_3)_3$ | $-C_6H_4CH_2CH_3$-4 | $-C_6H_3NO_2$-2-$CH_3$-5 |
| 419 | 0 | 0 | $-C(CH_3)_3$ | $-C_6H_5$ | $-C_6H_3Cl$-3-F-4 |
| 420 | 0 | 0 | $-C(CH_3)_3$ | $-C_6H_4Cl$-4 | $-C_6H_3Cl$-3-F-4 |
| 421 | 0 | 0 | $-CH(CH_3)_2$ | $-C_6H_4CH_2CH_3$-4 | $-C_6H_3(CH_3)_2$-3,5 |
| 422 | 0 | 0 | $-C(CH_3)_3$ | $-C_6H_4Cl$-4 | $-C_6H_3(CF_3)_2$-3,5 |
| 423 | 0 | 0 | $-C(CH_3)_3$ | $-C_6H_3(CH_3)_2$-2,3 | $-C_6H_3Cl_2$-3,4 |

| Ex. No. | X | X' | $R^1$ | A | B |
|---|---|---|---|---|---|
| 424 | 0 | 0 | $-C(CH_3)_3$ | $-C_6H_4CH_3-4$ | $-C_6H_3CH_3-2-Cl-3$ |
| 425 | 0 | 0 | $-C(CH_3)_3$ | $-C_6H_3Cl-3-F-4$ | $-C_6H_5$ |
| 426 | 0 | 0 | $-C(CH_3)_3$ | $-C_6H_3(CH_3)_2-2,6$ | $-C_6H_4CH_3-3$ |
| 427 | 0 | 0 | $-C(CH_3)_3$ | $-C_6H_3(CH_3)_2-2,6$ | $-C_6H_3(CH_3)_2-3,5$ |
| 428 | 0 | 0 | $-C(CH_3)_3$ | $-C_6H_3(CH_3)_2-2,3$ | $-C_6H_4Br-2$ |
| 429 | 0 | 0 | $-C(CH_3)_3$ | $-C_6H_3(CH_3)_2-2,3$ | $-C_6H_3(CH_3)_2-3,5$ |
| 430 | 0 | 0 | $-C(CH_3)_3$ | $-C_6H_3(CH_3)_2-2,3$ | $-C_6H_4Cl-3$ |
| 431 | 0 | 0 | $-C(CH_3)_3$ | $-C_6H_4OCF_3-3$ | $-C_6H_5$ |
| 432 | 0 | 0 | $-C(CH_3)_3$ | $-C_6H_4OCF_3-3$ | $-C_6H_4CH_3-3$ |
| 433 | 0 | 0 | $-C(CH_3)_3$ | $-C_6H_3(CH_3)_2-2,3$ | $-C_6H_3CH_3-2-Cl-3$ |
| 434 | 0 | 0 | $-CH(CH_3)C(CH_3)_3$ | $-C_6H_4CH_2CH_3-4$ | $-C_6H_3Cl_2-2,4$ |
| 435 | 0 | 0 | $-CH(CH_3)C(CH_3)(CH_2CH_3)_2$ | $-C_6H_4CH_3-4$ | $-C_6H_3NO_2-2-CH_3-5$ |
| 436 | 0 | 0 | $-CH(CH_3)C(CH_3)(CH_2CH_3)_2$ | $-C_6H_4CH_3-4$ | $-C_6H_3NO_2-2-CH_3-3$ |
| 437 | 0 | 0 | $-C(CH_3)_3$ | $-C_6H_3F_2-2,6$ | $-C_6H_4Br-2$ |
| 438 | 0 | 0 | $-C(CH_3)_3$ | $-C_6H_3CH_3-2-Cl-3$ | $-C_6H_3(CH_3)_2-3,5$ |
| 439 | 0 | 0 | $-C(CH_3)_3$ | $-C_6H_3CH_3-2-Cl-3$ | $-C_6H_3Cl-3-F-4$ |
| 440 | 0 | 0 | $-C(CH_3)_3$ | $-C_6H_3(CH_3)_2-2,3$ | $-C_6H_3Cl-3-F-4$ |
| 441 | 0 | 0 | $-C(CH_3)_3$ | $-C_6H_4CH_2CH_3-4$ | $-C_6H_3Cl-3-F-4$ |
| 442 | 0 | 0 | $-C(CH_3)_3$ | $-C_6H_3(CH_3)_2-3,4$ | $-C_6H_5$ |
| 443 | 0 | 0 | $-C(CH_3)_3$ | $-C_6H_3(CH_3)_2-3,4$ | $-C_6H_4CH_3-3$ |
| 444 | 0 | 0 | $-C(CH_3)_3$ | $-C_6H_3(CH_3)_2-3,4$ | $-C_6H_4Cl-2$ |
| 445 | 0 | 0 | $-C(CH_3)_3$ | $-C_6H_3(CH_3)_2-3,4$ | $-C_6H_3Cl_2-2,4$ |
| 446 | 0 | 0 | $-C(CH_3)_3$ | $-C_6H_3(CH_3)_2-3,4$ | $-C_6H_4Br-2$ |
| 447 | 0 | 0 | $-C(CH_3)_3$ | $-C_6H_3(CH_3)_2-3,4$ | $-C_6H_3(CH_3)_2-3,5$ |
| 448 | 0 | 0 | $-C(CH_3)_3$ | $-C_6H_3(CH_3)_2-3,4$ | $-C_6H_4NO_2-2$ |
| 449 | 0 | 0 | $-C(CH_3)_3$ | $-C_6H_3(CH_3)_2-3,4$ | $-C_6H_3(CH_3)_2-3,4$ |
| 450 | 0 | 0 | $-C(CH_3)_3$ | $-C_6H_5$ | $-C_6H_3(CH_3)_2-3,4$ |
| 451 | 0 | 0 | $-C(CH_3)_3$ | $-C_6H_4CH_2CH_3-4$ | $-C_6H_3(CH_3)_2-3,4$ |
| 452 | 0 | 0 | $-C(CH_3)_3$ | $-C_6H_3F-2-Cl-6$ | $-C_6H_4CH_3-3$ |

| Ex. No. | X | X' | $R^1$ | A | B |
|---|---|---|---|---|---|
| 453 | 0 | 0 | $-CH(CH_2CH_3)C(CH_3)_3$ | $-C_6H_4CH_3$-4 | $-C_6H_5$ |
| 454 | 0 | 0 | $-C(CH_3)_3$ | $-C_6H_4CH_2Cl$-4 | $-C_6H_5$ |
| 455 | 0 | 0 | $-C(CH_3)_3$ | $-C_6H_3(OCH_3)_2$-2,3 | $-C_6H_5$ |
| 456 | 0 | 0 | $-C(CH_3)_3$ | $-C_6H_3(OCH_3)_2$-2,3 | $-C_6H_4Cl$-4 |
| 457 | 0 | 0 | $-C(CH_3)_3$ | $-C_6H_3(OCH_3)_2$-2,3 | $-C_6H_4Br$-2 |
| 458 | 0 | 0 | $-C(CH_3)_3$ | $-C_6H_3Cl$-3-F-4 | $-C_6H_3Cl_2$-3,4 |
| 459 | 0 | 0 | $-C(CH_3)_3$ | $-C_6H_4C(CH_3)_3$-4 | $-C_6H_3(CH_3)_2$-3,4 |
| 460 | 0 | 0 | $-C(CH_3)_3$ | $-C_6H_4C(CH_3)_3$-4 | $-C_6H_3(CH_3)_2$-3,5 |
| 461 | 0 | 0 | $-C(CH_3)_3$ | $-C_6H_4C(CH_3)_3$-4 | $-C_6H_3Cl_2$-2,4 |
| 462 | 0 | 0 | $-C(CH_3)_3$ | $-C_6H_4C(CH_3)_3$-4 | $-C_6H_4NO_2$-2 |
| 463 | 0 | 0 | $-C(CH_3)_3$ | $-C_6H_4C(CH_3)_3$-4 | $-C_6H_4Br$-2 |
| 464 | 0 | 0 | $-C(CH_3)_3$ | $-C_6H_4C(CH_3)_3$-4 | $-C_6H_4CH_3$-3 |
| 465 | 0 | 0 | $-C(CH_3)_3$ | $-C_6H_4C(CH_3)_3$-4 | $-C_6H_4Cl$-2 |
| 466 | 0 | 0 | $-C(CH_3)_3$ | $-C_6H_4C(CH_3)_3$-4 | $-C_6H_3Cl_2$-3,4 |
| 467 | 0 | 0 | $-C(CH_3)_3$ | $-C_6H_3(CH_3)_2$-3,4 | $-C_6H_3Cl_2$-3,4 |
| 468 | 0 | 0 | $-C(CH_3)_3$ | $-C_6H_3(CH_3)_2$-3,4 | $-C_6H_4F$-4 |
| 469 | 0 | 0 | $-C(CH_3)_3$ | $-C_6H_4C(CH_3)_3$-4 | $-C_6H_4F$-4 |
| 470 | 0 | 0 | $-CH(CH_2CH_3)C(CH_3)_3$ | $-C_6H_4CH_3$-4 | $-C_6H_4NO_2$-2 |
| 471 | 0 | 0 | $-CH(CH_2CH_3)C(CH_3)_3$ | $-C_6H_4CH_3$-4 | $-C_6H_3NO_2$-2-$CH_3$-5 |
| 472 | 0 | 0 | $-CH(CH_2CH_3)C(CH_3)_3$ | $-C_6H_4CH_3$-4 | $-C_6H_3(CH_3)_2$-3,5 |
| 473 | 0 | 0 | $-C(CH_3)_3$ | $-C_6H_3NH_2$-2-$OCH_3$-3 | $-C_6H_5$ |
| 474 | 0 | 0 | $-C(CH_3)_3$ | 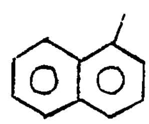 | $-C_6H_3Cl_2$-2,4 |
| 475 | 0 | 0 | $-C(CH_3)_3$ | 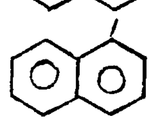 | $-C_6H_4Br$-2 |
| 476 | 0 | 0 | $-C(CH_3)_3$ | $-C_6H_3CH_3$-2-$NO_2$-3 | $-C_6H_5$ |
| 477 | 0 | 0 | $-C(CH_3)_3$ | $-C_6H_3CH_3$-2-$NO_2$-3 | $-C_6H_4CH_3$-3 |
| 478 | 0 | 0 | $-C(CH_3)_3$ | $-C_6H_3CH_3$-2-$NO_2$-3 | $-C_6H_4Cl$-4 |

| Ex. No. | X | X' | $R^1$ | A | B |
|---|---|---|---|---|---|
| 479 | 0 | 0 | $-C(CH_3)_3$ | $-C_6H_3CH_3$-2-$NO_2$-3 | $-C_6H_3Cl_2$-2,4 |
| 480 | 0 | 0 | $-C(CH_3)_3$ | $-C_6H_3CH_3$-2-Br-3 | $-C_6H_5$ |
| 481 | 0 | 0 | $-C(CH_3)_3$ | $-C_6H_3CH_3$-2-Br-3 | $-C_6H_4CH_3$-3 |
| 482 | 0 | 0 | $-C(CH_3)_3$ | $-C_6H_3CH_3$-2-Br-3 | $-C_6H_4Cl$-4 |
| 483 | 0 | 0 | $-C(CH_3)_3$ | $-C_6H_3CH_3$-2-Br-3 | $-C_6H_3Cl_2$-2,4 |
| 484 | 0 | 0 | $-C(CH_3)_3$ | $-C_6H_3CH_3$-2-$NH_2$-3 | $-C_6H_4CH_3$-3 |
| 485 | 0 | 0 | $-C(CH_3)_3$ | $-C_6H_4CH_3$-2 | $-C_6H_4Br$-2 |
| 486 | 0 | 0 | $-C(CH_3)_3$ | $-C_6H_4CH_3$-2 | $-C_6H_4NO_2$-2 |
| 487 | 0 | 0 | $-C(CH_3)_3$ | $-C_6H_4CH_3$-2 | $-C_6H_4Cl$-3 |
| 488 | 0 | 0 | $-C(CH_3)_3$ | $-C_6H_4CH_3$-2 | $-C_6H_4Cl$-4 |
| 489 | 0 | 0 | $-C(CH_3)_3$ | $-C_6H_4CH_3$-2 | $-C_6H_4CH_3$-3 |
| 490 | 0 | 0 | $-C(CH_3)_3$ | $-C_6H_4CH_3$-2 | $-C_6H_3(CH_3)_2$-3,5 |
| 491 | 0 | 0 | $-C(CH_3)_3$ | $-C_6H_4CH_3$-2 | $-C_6H_3Cl_2$-3,4 |
| 492 | 0 | 0 | $-C(CH_3)_3$ | $-C_6H_4CH_3$-2 | $-C_6H_3Cl_2$-3,5 |
| 493 | 0 | 0 | $-C(CH_3)_3$ | $-C_6H_3CH_3$-2-F-3 | $-C_6H_5$ |
| 494 | 0 | 0 | $-C(CH_3)_3$ | $-C_6H_3CH_3$-2-F-3 | $-C_6H_4CH_3$-3 |
| 495 | 0 | 0 | $-C(CH_3)_3$ | $-C_6H_3F$-2-Cl-6 | $-C_6H_5$ |
| 496 | 0 | 0 | $-C(CH_3)_3$ | $-C_6H_3F$-2-Cl-6 | $-C_6H_3Cl_2$-2,4 |
| 497 | 0 | 0 | $-C(CH_3)_3$ | $-C_6H_3F$-2-Cl-6 | $-C_6H_4F$-4 |
| 498 | 0 | 0 | $-C(CH_3)_3$ | $-C_6H_4CH_2CH_2Cl$-4 | $-C_6H_3(CH_3)_2$-3,5 |
| 499 | 0 | 0 | $-C(CH_3)_3$ | $-C_6H_2F_3$-2,4,6 | $-C_6H_5$ |
| 500 | 0 | 0 | $-C(CH_3)_3$ | $-C_6H_2F_3$-2,4,6 | $-C_6H_3Cl_2$-2,4 |
| 501 | 0 | 0 | $-C(CH_3)_3$ | $-C_6H_2F_3$-2,4,6 | $-C_6H_4Br$-2 |
| 502 | 0 | 0 | $-C(CH_3)_3$ | $-C_6H_2F_3$-2,4,6 | $-C_6H_3(CH_3)_2$-3,5 |
| 503 | 0 | 0 | $-C(CH_3)_3$ | $-C_6H_3NO_2$-2-Cl-3 | $-C_6H_5$ |
| 504 | 0 | 0 | $-C(CH_3)_3$ | $-C_6H_3NO_2$-2-Cl-3 | $-C_6H_4Br$-2 |
| 505 | 0 | 0 | $-C(CH_3)_3$ | $-C_6H_3NO_2$-2-Cl-3 | $-C_6H_4NO_2$-2 |
| 506 | 0 | 0 | $-C(CH_3)_3$ | $-C_6H_3NO_2$-2-Cl-3 | $-C_6H_4CH_3$-3 |
| 507 | 0 | 0 | $-C(CH_3)_3$ | $-C_6H_3NO_2$-2-Cl-3 | $-C_6H_4Cl$-3 |
| 508 | 0 | 0 | $-C(CH_3)_3$ | $-C_6H_3NO_2$-2-Cl-3 | $-C_6H_3Cl_2$-2,4 |
| 509 | 0 | 0 | $-C(CH_3)_3$ | $-C_6H_3NO_2$-2-Cl-3 | $-C_6H_3Cl_2$-3,5 |
| 510 | 0 | 0 | $-C(CH_3)_3$ | $-C_6H_3NO_2$-2-Cl-3 | $-C_6H_3(CH_3)_2$-3,5 |

| Ex. No. | X | X' | $R^1$ | A | B |
|---|---|---|---|---|---|
| 511 | 0 | 0 | $-C(CH_3)_3$ | $-C_6H_4CH_2CH_3$-4 | $-C_6H_3F_2$-2,3 |
| 512 | 0 | 0 | $-C(CH_3)_3$ | $-C_6H_4CH_2CH_3$-4 | $-C_6H_3Cl_2$-2,3 |
| 513 | 0 | 0 | $-C(CH_3)_3$ | $-C_6H_3F_2$-2,3 | $-C_6H_5$ |
| 514 | 0 | 0 | $-C(CH_3)_3$ | $-C_6H_3Cl_2$-2,3 | $-C_6H_4NO_2$-2 |
| 515 | 0 | 0 | $-C(CH_3)_3$ | $-C_6H_5$ | $-C_6H_3F_2$-2,3 |
| 516 | 0 | 0 | $-C(CH_3)_3$ | $-C_6H_3Cl_2$-2,3 | $-C_6H_3(CH_3)_2$-3,5 |
| 517 | 0 | 0 | $-C(CH_3)_3$ | $-C_6H_3Cl_2$-2,3 | $-C_6H_3Cl_2$-2,4 |
| 518 | 0 | 0 | $-C(CH_3)_3$ | $-C_6H_3Cl_2$-2,3 | $-C_6H_3Cl_2$-3,5 |
| 519 | 0 | 0 | $-C(CH_3)_3$ | $-C_6H_3Cl_2$-2,3 | $-C_6H_4Cl$-3 |
| 520 | 0 | 0 | $-C(CH_3)_3$ | $-C_6H_3Cl_2$-2,3 | $-C_6H_3(CH_3)_2$-2,3 |
| 521 | 0 | 0 | $-C(CH_3)_3$ | $-C_6H_3F_2$-2,3 | $-C_6H_4Br$-2 |
| 522 | 0 | 0 | $-C(CH_3)_3$ | $-C_6H_3(CH_3)_2$-2,3 | $-C_6H_4Cl$-4 |
| 523 | 0 | 0 | $-C(CH_3)_3$ | $-C_6H_3(CH_3)_2$-2,3 | $-C_6H_3Cl_2$-2,4 |
| 524 | 0 | 0 | $-C(CH_3)_3$ | $-C_6H_3(CH_3)_2$-2,3 | $-C_6H_3F_2$-2,6 |
| 525 | 0 | 0 | $-C(CH_3)_3$ | $-C_6H_3(CH_3)_2$-2,3 | $-C_6H_3F_2$-2,4 |
| 526 | 0 | 0 | $-C(CH_3)_3$ | $-C_6H_3(CH_3)_2$-2,3 | $-C_6H_4OCH_3$-3 |
| 527 | 0 | 0 | $-C(CH_3)_3$ | $-C_6H_3(CH_3)_2$-2,3 | $-C_6H_4OCH_3$-2 |
| 528 | 0 | 0 | $-C(CH_3)_3$ | $-C_6H_3(CH_3)_2$-2,3 | $-C_6H_4CH_3$-2 |
| 529 | 0 | 0 | $-C(CH_3)_3$ | $-C_6H_3(CH_3)_2$-2,3 | $-C_6H_4CH_3$-4 |
| 530 | 0 | 0 | $-C(CH_3)_3$ | $-C_6H_3CH_3$-2-Cl-3 | $-C_6H_3Cl_2$-2,4 |
| 531 | 0 | 0 | $-C(CH_3)_3$ | $-C_6H_4CH_2CH_2Cl$-4 | $-C_6H_5$ |
| 532 | 0 | 0 | $-C(CH_3)_3$ | $-C_6H_4CH_2CH_2Cl$-4 | $-C_6H_3Cl_2$-2,4 |
| 533 | 0 | 0 | $-C(CH_3)_3$ | $-C_6H_3F$-3-$CH_3$-4 | $-C_6H_3(CH_3)_2$-3,5 |
| 534 | 0 | 0 | $-C(CH_3)_3$ | $-C_6H_3F$-3-$CH_3$-4 | $-C_6H_3F_2$-3,5 |
| 535 | 0 | 0 | $-C(CH_3)_3$ | $-C_6H_5F$-3-$CH_3$-4 | $-C_6H_4CH_3$-3 |
| 536 | 0 | 0 | $-C(CH_3)_3$ | $-C_6H_3F$-3-$CH_3$-4 | $-C_6H_3Cl_2$-3,5 |
| 537 | 0 | 0 | $-C(CH_3)_3$ | $-C_6H_3F$-3-$CH_3$-4 | $-C_6H_4NO_2$-2 |
| 538 | 0 | 0 | $-C(CH_3)_3$ | $-C_6H_3F$-3-$CH_3$-4 | $-C_6H_3Cl_2$2,4 |
| 539 | 0 | 0 | $-C(CH_3)_3$ | $-C_6H_4CH_2CH_3$-4 | $-C_6H_3F_2$-3,5 |
| 540 | 0 | 0 | $-C(CH_3)_3$ | $-C_6H_3(CH_3)_2$-2,3 | $-C_6H_4I$-2 |
| 541 | 0 | 0 | $-C(CH_3)_3$ | $-C_6H_4CH_2CH_2OH$-4 | $-C_6H_3(CH_3)_2$-3,5 |
| 542 | 0 | 0 | $-C(CH_3)_3$ | $-C_6H_2F_2$-2,6-$CH_3$-3 | $-C_6H_5$ |

| Ex. No. | X | X' | $R^1$ | A | B |
|---|---|---|---|---|---|
| 543 | 0 | 0 | $-C(CH_3)_3$ | $-C_6H_2F_2$-2,6-$CH_3$3 | $-C_6H_4CH_3$-3 |
| 544 | 0 | 0 | $-C(CH_3)_3$ | $-C_6H_2F_2$-2,6-$CH_3$-3 | $-C_6H_3Cl_2$-2,4 |
| 545 | 0 | 0 | $-C(CH_3)_3$ | $-C_6H_4Cl$-4 | $-C_6H_3(CH_3)_2$-2,3 |
| 546 | 0 | 0 | $-CH(CH_3)C(CH_3)_3$ | $-C_6H_3(CH_3)_2$-2,3 | $-C_6H_3(CH_3)_2$-3,5 |
| 547 | 0 | 0 | $-CH(CH_3)C(CH_3)_3$ | $-C_6H_3(CH_3)_2$-2,3 | $-C_6H_4CH_3$-3 |
| 548 | 0 | 0 | $-C(CH_3)_3$ | $-C_6H_3F$-2-Cl-6 | $-C_6H_3F_2$-2,3 |
| 549 | 0 | 0 | $-C(CH_3)_3$ | $-C_6H_3F_2$-2,3 | $-C_6H_4NO_2$-2 |
| 550 | 0 | 0 | $-C(CH_3)_3$ | $-C_6H_3F_2$-2,3 | $-C_6H_4CH_3$-3 |
| 551 | 0 | 0 | $-C(CH_3)_3$ | $-C_6H_3F_2$-2,3 | $-C_6H_3(CH_3)_2$-3,5 |
| 552 | 0 | 0 | $-C(CH_3)_3$ | $-C_6H_3F_2$-2,3 | $-C_6H_3Cl_2$-2,4 |
| 553 | 0 | 0 | $-C(CH_3)_3$ | $-C_6H_3(CH_3)_2$-2,3 | $-C_6H_3F_2$-2,3 |
| 554 | 0 | 0 | $-C(CH_3)_3$ | $-C_6H_3(CH_3)_2$-2,3 | $-C_6H_3Cl_2$-2,3 |
| 555 | 0 | 0 | $-C(CH_3)_3$ | $-C_6H_3(CH_3)_2$-2,3 | $-C_6H_3(CH_3)_2$-3,4 |
| 556 | 0 | 0 | $-C(CH_3)_3$ | $-C_6H_3(CH_3)_2$-2,3 | $-C_6H_4NO_2$-2 |
| 557 | 0 | 0 | $-C(CH_3)_3$ | $-C_6H_4CH_2CH_3$-4 | $-C_6H_3CH_3$-2-Cl-5 |
| 558 | 0 | 0 | $-C(CH_3)_3$ | $-C_6H_3(CH_3)_2$-2,3 | $-C_6H_5$ |
| 559 | 0 | 0 | $-C(CH_3)_3$ | $-C_6H_5$ | $-C_6H_3CH_3$-2-Cl-5 |
| 560 | 0 | 0 | $-C(CH_3)_3$ | $-C_6H_3F_2$-2,6 | $-C_6H_3CH_3$-2-Cl-5 |
| 561 | 0 | 0 | $-C(CH_3)_3$ | $-C_6H_3(CH_3)_2$-2,3 | $-C_6H_3CH_3$-2-Cl-5 |
| 562 | 0 | 0 | $-C(CH_3)_3$ | $-C_6H_3CH_3$-2-Cl-3 | $-C_6H_3CH_3$-2-Cl-5 |
| 563 | 0 | 0 | $-C(CH_3)_3$ | $-C_6H_3F_2$-2,6 | $-C_6H_3Cl$-2-$CH_3$-5 |
| 564 | 0 | 0 | $-C(CH_3)_3$ | $-C_6H_3(CH_3)_2$-2,3 | $-C_6H_3Cl$-2-$CH_3$-5 |
| 565 | 0 | 0 | $-C(CH_3)_3$ | $-C_6H_3CH_3$-2-Cl-3 | $-C_6H_3Cl$-2-$CH_3$-5 |
| 566 | 0 | 0 | $-C(CH_3)_3$ | $-C_6H_4Cl$-4 | $-C_6H_3CH_3$-2-Cl-5 |
| 567 | 0 | 0 | $-C(CH_3)_3$ | $-C_6H_4Cl$-4 | $-C_6H_3Cl$-2-$CH_3$-5 |
| 568 | 0 | 0 | $-C(CH_3)_3$ | $-C_6H_3F$-2-Cl-4 | $-C_6H_5$ |
| 569 | 0 | 0 | $-C(CH_3)_3$ | $-C_6H_3F$-2-Cl-4 | $-C_6H_4CH_3$-3 |
| 570 | 0 | 0 | $-C(CH_3)_3$ | $-C_6H_3F$-2-Cl-4 | $-C_6H_3(CH_3)_2$-3,5 |
| 571 | 0 | 0 | $-C(CH_3)_3$ | $-C_6H_3F$-2-Cl-4 | $-C_6H_3Cl_2$-3,5 |
| 572 | 0 | 0 | $-C(CH_3)_3$ | $-C_6H_3F$-2-Cl-4 | $-C_6H_4Br$-2 |
| 573 | 0 | 0 | $-C(CH_3)_3$ | $-C_6H_3F$-2-Cl-4 | $-C_6H_4NO_2$-2 |
| 574 | 0 | 0 | $-C(CH_3)_3$ | $-C_6H_3Cl$-2-$CH_3$-3 | $-C_6H_3Cl_2$-2,4 |

| Ex. No. | X | X' | $R^1$ | A | B |
|---|---|---|---|---|---|
| 575 | O | O | $-C(CH_3)_3$ | $-C_6H_3Cl-2-CH_3-3$ | $-C_6H_5$ |
| 576 | O | O | $-C(CH_3)_3$ | $-C_6H_3Cl-2-CH_3-3$ | $-C_6H_4CH_3-3$ |
| 577 | O | O | $-C(CH_3)_3$ | $-C_6H_3Cl-2-CH_3-3$ | $-C_6H_3(CH_3)_2-3,5$ |
| 578 | O | O | $-C(CH_3)_3$ | $-C_6H_3Cl-2-CH_3-3$ | $-C_6H_3Cl_2-3,5$ |
| 579 | O | O | $-C(CH_3)_3$ | $-C_6H_3Br-2-CH_3-3$ | $-C_6H_3(CH_3)_2-3,5$ |
| 580 | O | O | $-C(CH_3)_3$ | $-C_6H_3Br-2-CH_3-3$ | $-C_6H_3Cl_2-2,4$ |
| 581 | O | O | $-C(CH_3)_3$ | $-C_6H_3Br-2-CH_3-3$ | $-C_6H_4CH_3-3$ |
| 582 | O | O | $-C(CH_3)_3$ | $-C_6H_3Br-2-CH_3-3$ | $-C_6H_3Cl_2-3,5$ |
| 583 | O | O | $-C(CH_3)_3$ | $-C_6H_3Br-2-CH_3-3$ | $-C_6H_4Br-2$ |
| 584 | O | O | $-C(CH_3)_3$ | $-C_6H_3(CH_3)_2-2,3$ | $-C_6H_4Cl-2$ |
| 585 | O | O | $-C(CH_3)_3$ | $-C_6H_3(CH_3)_2-2,3$ | $-C_6H_4CF_3-2$ |
| 586 | O | O | $-C(CH_3)_3$ | $-C_6H_3(CH_3)_2-2,3$ | $-C_6H_4CH_2CH_3-4$ |
| 587 | O | O | $-C(CH_3)_3$ | $-C_6H_3(CH_3)_2-2,3$ | $-C_6H_3Cl_2-3,5$ |
| 588 | O | O | $-C(CH_3)_3$ | $-C_6H_3F_2-2,6$ | $-C_6H_3Cl-3-F-4$ |
| 589 | O | O | $-C(CH_3)_3$ | $-C_6H_4Cl-4$ | $-C_6H_3F_2-3,5$ |
| 590 | O | O | $-C(CH_3)_3$ | $-C_6H_3(CH_3)_2-2,3$ | $-C_6H_3F_2-3,5$ |
| 591 | O | O | $-C(CH_3)_3$ | $-C_6H_3CH_3-2-Cl-3$ | $-C_6H_3F_2-3,5$ |
| 592 | O | O | $-C(CH_3)_3$ | $-C_6H_5$ | $-C_6H_3F_2-3,5$ |
| 593 | O | O | $-C(CH_3)_3$ | $-C_6H_4CH_2CH_3-4$ | $-C_6H_3(CH_3)_2-2,5$ |
| 594 | O | O | $-C(CH_3)_3$ | $-C_6H_3F_2-2,6$ | $-C_6H_3F_2-3,5$ |
| 595 | O | O | $-C(CH_3)_3$ | $-C_6H_3CH_3-2-Cl-3$ | $-C_6H_3Cl_2-3,5$ |
| 596 | O | O | $-C(CH_3)_3$ | $-C_6H_3(CH_3)_2-2,3$ | $-C_6H_3(CH_3)_2-2,5$ |
| 597 | O | O | $-C(CH_3)_3$ | $-C_6H_4Br-2$ | $-C_6H_3(CH_3)_2-3,5$ |
| 598 | O | O | $-C(CH_3)_3$ | $-C_6H_3Br-2-CH_3-3$ | $-C_6H_4Cl-3$ |
| 599 | O | O | $-C(CH_3)_3$ | $-C_6H_3Cl-2-CH_3-3$ | $-C_6H_4Cl-3$ |
| 600 | O | O | $-C(CH_3)_3$ | $-C_6H_3Br-2-CH_3-3$ | $-C_6H_4NO_2-2$ |
| 601 | O | O | $-C(CH_3)_3$ | $-C_6H_3Cl-2-CH_3-3$ | $-C_6H_4NO_2-2$ |
| 602 | O | O | $-C(CH_3)_3$ | $-C_6H_3(CH_3)_2-2,3$ | $-C_6H_3NO_2-2-CH_3-3$ |
| 603 | O | O | $-C(CH_3)_3$ | $-C_6H_3(CH_3)_2-2,3$ | $-C_6H_3NO_2-2-CH_3-5$ |
| 604 | O | O | $-C(CH_3)_3$ | $-C_6H_3F_2-2,6$ | $-C_6H_5$ |
| 605 | O | O | $-C(CH_3)_3$ | $-C_6H_3(CH_3)_2-2,6$ | $-C_6H_3Cl_2-2,4$ |
| 606 | O | O | $-C(CH_3)_3$ | $-C_6H_2(CH_3)_3-2,4,6$ | $-C_6H_3(CH_3)_2-3,5$ |

| Ex. No. | X | X' | $R^1$ | A | B |
|---|---|---|---|---|---|
| 607 | 0 | 0 | $-C(CH_3)_3$ | $-C_6H_2(CH_3)_3$-2,4,6 | $-C_6H_3Cl_2$-2,4 |
| 608 | 0 | 0 | $-C(CH_3)_3$ | $-C_6H_3F_2$-2,6 | $-C_6H_4CH_3$-4 |
| 609 | 0 | 0 | $-C(CH_3)_3$ | $-C_6H_3F_2$-2,6 | $-C_6H_3Cl_2$-2,5 |
| 610 | 0 | 0 | $-C(CH_3)_3$ | $-C_6H_2(CH_3)_3$-2,4,6 | $-C_6H_4Cl$-4 |
| 611 | 0 | 0 | $-C(CH_3)_3$ | $-C_6H_2(CH_3)_3$-2,4,6 | $-C_6H_4CH_3$-3 |
| 612 | 0 | 0 | $-C(CH_3)_3$ | $-C_6H_2(CH_3)_3$-2,4,6 | $-C_6H_3Cl_2$-3,4 |
| 613 | 0 | 0 | $-C(CH_3)_3$ | $-C_6H_4OC(O)CH_3$-2 | $-C_6H_4OC(O)CH_3$-2 |
| 614 | 0 | 0 | $-C(CH_3)_3$ | $-C_6H_4OH$-2 | $-C_6H_4OH$-2 |
| 615 | 0 | 0 | $-C(CH_3)_3$ | $-C_6H_3(CH_3)_2$-2,4 | $-C_6H_4CH_3$-3 |
| 616 | 0 | 0 | $-C(CH_3)_3$ | $-C_6H_3(CH_3)_2$-2,4 | $-C_6H_3Cl_2$-2,4 |
| 617 | 0 | 0 | $-C(CH_3)_3$ | $-C_6H_3(CH_3)_2$-2,4 | $-C_6H_3(CH_3)_2$-3,5 |
| 618 | 0 | 0 | $-C(CH_3)_3$ | $-C_6H_3(CH_3)_2$-2,4 | $-C_6H_3Cl_2$-3,5 |
| 619 | 0 | 0 | $-C(CH_3)_3$ | $-C_6H_3(CH_3)_2$-2,4 | $-C_6H_4Br$-3 |
| 620 | 0 | 0 | $-C(CH_3)_3$ | $-C_6H_3(CH_3)_2$-2,4 | $-C_6H_3Cl_2$-3,4 |
| 621 | 0 | 0 | $-C(CH_3)_3$ | $-C_6H_3Br$-2-$CH_3$-3 | $-C_6H_5$ |
| 622 | 0 | 0 | $-C(CH_3)_3$ | 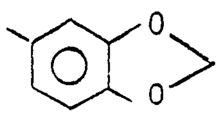 | $-C_6H_5$ |
| 623 | 0 | 0 | $-C(CH_3)_3$ | 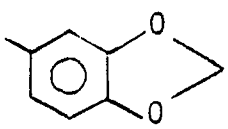 | $-C_6H_4Cl$-4 |
| 624 | 0 | 0 | $-C(CH_3)_3$ | 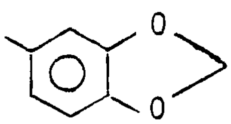 | $-C_6H_4CH_3$-3 |
| 625 | 0 | 0 | $-C(CH_3)_3$ | 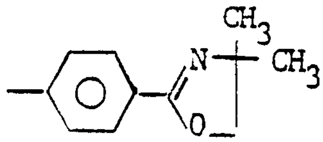 | $-C_6H_4CH_3$-3 |
| 626 | 0 | 0 | $-C(CH_3)_3$ | 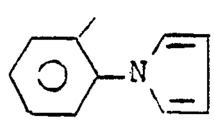 | $-C_6H_5$ |

| Ex. No. | X | X' | $R^1$ | A | B |
|---|---|---|---|---|---|
| 627 | O | O | $-C(CH_3)_3$ | 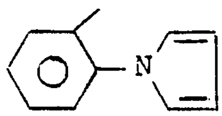 | $-C_6H_4CH_3-3$ |
| 628 | O | O | $-C(CH_3)_3$ | $-C_6H_3(CH_3)_2-2,3$ | $-C_6H_3Br-2-Cl-5$ |
| 629 | O | O | $-C(CH_3)_3$ | $-C_6H_3CH_3-2-Cl-3$ | $-C_6H_4Cl-3$ |
| 630 | O | O | $-C(CH_3)_3$ | $-C_6H_3CH_3-2-Cl-3$ | $-C_6H_4F-3$ |
| 631 | O | O | $-C(CH_3)_3$ | $-C_6H_3CH_3-2-Cl-3$ | $-C_6H_4Br-2$ |
| 632 | O | O | $-C(CH_3)_3$ | $-C_6H_3CH_3-2-Cl-3$ | $-C_6H_3CH_3-2-Cl-3$ |
| 633 | O | O | $-C(CH_3)_3$ | $-C_6H_3CH_3-2-Cl-3$ | $-C_6H_2(CH_3)_2-3,5-Cl-4$ |
| 634 | O | O | $-CH(CH_3)C(CH_3)_3$ | $-C_6H_5$ | $-C_6H_3CH_3-2-Cl-3$ |

EXAMPLE NO. 1 - Preparation of N'-t-butyl-N,N'-(4-chloro benzoyl)hydrazine

A suspension of t -butylhydrazine hydrochloride (12.5 g, 0.1 mole) in toluene (100 ml) at 0-5° C was treated slowly with 1 equivalent of NaOH solution, prepared from diluting 8 g of 50% NaOH commercially available solution to 20 ml of the volume with $H_2O$. At 0 to 5° C with mechanical stirring, 2 equivalents of 4-chlorobenzoyl chloride (35.9 g, 0.2 mole) and 2 equivalents of NaOH (16 g of 50% NaOH diluted with $H_2O$ to 40 ml) were added dropwise separately and simultaneously from dropping funnels. The exothermic reaction was cooled down by an ice-water bath through the entire addition. After the addition was completed, the resulting suspension was stirred at room temperature (RT) for one hour. The white precipitate (p.p.t.) was collected by suction-filtration and washed with a small amount of toluene and 100 ml of $H_2O$. The material was then air-dried, then crystallized from 95% aqueous $CH_3OH$ to afford 24.65 g of N'-t -butyl-N,N'-(4-chlorobenzoyl)hydrazine as needles: m.p. 246-248° C

Additional product can be obtained by concentrating the mother liquid of crystallization.

EXAMPLE NO. 3 - Preparation of N'-t-butyl-N,N'-dibenzoyl hydrazine

To a stirred suspension of t -butylhydrazine hydrochloride (1.24 g, 10 mmoles) in toluene (50 ml) at room temperature, was added dropwise a solution of 50% aqueous sodium hydroxide (0.8 g, 10 ml). After 15 minutes, the reaction mixture was cooled to 5° C and solutions of benzoyl chloride (2.82 gm, 20 ml) in toluene (7 ml) and 50% aqueous sodium hydroxide (1.6 g) were added dropwise and simultaneously from separate addition funnels while maintaining the temperature below 10° C. Following the addition, the reaction mixture was warmed to room temperature and stirred for 1 hr. The reaction mixture was diluted with ether and the product isolated by filtration. The product was washed with water and ether and dried. The product was recrystallized from ethermethanol to afford N'-t -butyl-N,N'-dibenzoylhydrazine as a white powder: m.p. 174-176° C.

EXAMPLE NO. 16 - Preparation of N'-t-butyl-N'-(4-chloro benzoyl)-N-benzoylhydrazine

To a stirred suspension of t -butylhydrazine hydrochloride (1.24 g, 10 mmoles) in toluene (30 ml) at room temperature was added dropwise a 50% aqueous solution of sodium hydroxide (0.8 g, 10 mmole). After 15 min., the reaction mixture was cooled to 5° C and a solution of benzoyl chloride (1.42 g 10 mmoles) in toluene (5 ml) and a solution of aqueous 50% sodium hydroxide (0.8 g, 10 mmole) were added

dropwise simultaneously from separate addition funnels while maintaining the temperature at or below 10°C. Following the addition, the reaction mixture was warmed to room temperature and stirred for 1 hr. The reaction mixture was diluted with toluene washed with water. The organic layer was separated, dried over anhydrous magnesium sulfate, and the solvent removed under vacuum to afford a yellow oil which slowly solidifies on standing. The product was recrystallized from etherhexane to afford white crystals.

To a stirred solution of the monobenzoylated compound (1.92 g, 10 mmoles) in toluene (30 ml) at 5°C, were added dropwise simultaneously from separate addition funnels, solutions of p -chlorobenzoyl chloride (1.75 g, 10 mmoles) in toluene (5 ml) and aqueous 50% sodium hydroxide solution (0.8 g) while maintaining the temperature below 10°C. Following the addition, the reaction mixture was warmed to room temperature and stirred for 1 hr. The mixture was then diluted with hexane and the precipitated product isolated by filtration. The product was washed with water and hexane and dried. The crude product was recrystallized from ether-methanol to afford N'-t -butyl-N'-(4-chlorobenzoyl)-N-benzoylhydrazine as a white powder: m.p. 201-204°C.

EXAMPLE NO. 44 - Preparation of N'-neopentyl-N,N'-dibenzoylhydrazine

A solution of benzoylhydrazine (1.36 g, 10 mmoles), 1,1,1-trimethylacetaldehyde (0.86 g, 10 mmoles), and acetic acid (catalytic amount) in methanol are stirred at room temperature until hydrazone formation is complete. The reaction mixture is brought to a pH of 4 and sodium cyanoborohydride (0.75 g, 12.5 mmoles) is added slowly portionwise (the reaction is connected to an aqueous sodium hydroxide trap). Upon completion, the reaction is diluted with excess aqueous sodium hydroxide and the methanol is removed under vacuum. The product is partitioned into methylene chloride and washed with aqueous base and water. The organic layer is separated and dried over anhydrous magnesium sulfate. The magnesium sulfate is filtered, and the methylene chloride removed under vacuum to afford the product as a yellow oil which solidifies on standing. The crude 2-neopentyl-1-benzoylhydrazine is benzoylated directly.

To a stirred solution of the 2-neopentyl-1-benzoylhydrazine in toluene (40 ml) at 5°C, were added dropwise and simultaneously solutions of benzoyl chloride (1.4 g, 10 mmoles) in toluene (5 ml) and aqueous 50% sodium hydroxide solution (0.8 g) while maintaining the temperature below 10°C. After the addition, the reaction mixture was warmed to room temperature and stirred for 1 hour. The reaction mixture was diluted with hexane and the precipitated product isolated by filtration. The product was washed with water and hexane and dried. The crude product was recrystallized from methanol to afford N'-neopentyl-N,N'-dibenzoylhydrazine as a white powder: m.p. 237-239°C.

EXAMPLE NO. 102 - Preparation of N'-t-butyl-N'-benzoyl-N-4-chlorothiobenzoylhydrazine

A mixture of 4-chloro-methylthio-thiobenzoate (3.0 g, 0.015 mol) and t -butyl hydrazine hydrochloride (2.0 g, 0.016 mol) in 5 ml of pyridine was heated at 90°C for 18 hours. The mixture was poured into 0.1 N HCl/ether. The layers were separated and the organic extracts were washed with 3 portions of 0.1 N HCl followed by saturated aqueous NaHCO. After the extracts were dried over anhydrous magnesium sulfate, the solvents were removed under vacuum to afford 1.9 g of a brown solid. Chromatography on silica gel using ether (25%)-methylene chloride (25%)-hexane as eluant afforded 0.8 g of a golden yellow solid. The solid was dissolved in 3 ml of methylene chloride and treated with pyridine (1 ml) and benzoyl chloride (0.6 ml). After 24 hours at 23°C, the reaction mixture was poured onto 0.1 N HCl/ether. The organic layer was washed with saturated aqueous sodium bicarbonate and was dried over anhydrous magnesium sulfate. Evaporation of solvents gave a yellow oil which was chromatographed on silica gel using ether (25%)-methylene chloride (25%)-hexane as eluant to give 0.15 g of N' t -butyl-N'-benzoyl-N-4-chlorothiobenzoyl-hydrazine as a yellow solid: m.p. 160-162°C.

EXAMPLE NO. 103 - Preparation of N'-t-butyl-N'-thiobenzoyl-N-benzoylhydrazine

A mixture of N'-t -butyl-N-benzoyl hydrazine (60% purity, 1.0 g, 0.0031 mol) and S-(thiobenzoyl)-thioglycolic acid (1.0 g, 0.0047 mol) in 3 ml of pyridine was heated at about 90°C for 24 hours. The dark colored mixture was cooled and poured into 0.1 N HCl/ether. The organic layer was washed with three 15 ml portions of 0.1 N HCl followed by saturated aqueous sodium bicarbonate. The organic extracts were dried over anhydrous magnesium sulfate. Evaporation of the solvents afforded 0.5 g of a brown oil which

was recrystallized from ether-hexane to yield 0.2 g of N'-t -butyl-N'-thiobenzoyl-N-benzoylhydrazine as a tan solid m.p. 169-171 °C.

EXAMPLE NO. 148 - Preparation of N'-t-butyl-N-(2-hydroxy methylbenzoyl)-N'-benzoylhydrazine

t -Butylhydrazine (0.1 mol) in 75 ml ethanol was treated with 50% aqueous sodium hydroxide (0.11 mol). Phthalide (0.1 mol) was added and the mixture was refluxed for 5 days. After cooling, water was added and the crude product was isolated by filtration. Filtration through silica gel afforded N'-t -butyl-N-(2-hydroxymethylbenzoyl)hydrazine (3.0 g). m.p. 116-118 °C.

0.7 g of N'-t -butyl-N-(2-hydroxymethylbenzoyl)hydrazine and 1.1 g benzoyl chloride are combined in 10 ml of 5% NaOH and stirred at room temperature for 1.5 hours. The solids are filtered off, washed with water, then ether, to afford 0.6 g of white solid N'-t -butyl-N-(2-(benzoyloxymethyl)benzoyl)-N'-benzoylhydrazine. m.p. 190-191 °C.

EXAMPLE NO. 220 - Preparation of N-(3-toluoyl)-N'-t-butyl-N'-benzoylhydrazine

Step 1

To a stirred suspension of t -butylhydrazine (51 g) in a mixture of dioxane and water (2:1) (150 ml) was added sodium hydroxide (32 g of a 50% aqueous solution). After 10 min., the solution was cooled to 5 °C and di-t -butyl-dicarbonate (42 g) was added dropwise so as to maintain the reaction temperature below 10 °C. The reaction mixture was warmed and stirred 2 hours at room temperature. The reaction mixture was filtered, washed with water and dried to afford N-t -butyloxycarbonyl-N'-t -butylhydrazine (74 g) as a white crystalline solid. m.p. 69-71 °C.

Step 2

To a stirred solution of N-t -butyloxycarbonyl-N'-t -butylhydrazine (61 g) in toluene (120 ml) was added benzoyl chloride (45 g) and sodium hydroxide (31 g of a 50% aqueous sodium hydroxide solution) dropwise and simultaneously. After stirring for 1 hour at room temperature, the solid N-t -butyloxycarbonyl-N'-t -butyl-N'-benzoylhydrazine was filtered, washed with water, hexane and dried to afford 52 g of product. m.p. 167-170 °C.

Step 3

The N-t -butoxycarbonyl-N'-t -butyl-N'-benzoylhydrazine (52 g, 0.18 mol) was stirred at room temperature in a methanolic hydrochloric acid solution for 4 days. The reaction mixture was neutralized with saturated aqueous sodium bicarbonate. The white precipitate was filtered, washed with water and dried *in vacuo* to give 30 g of N'-t -butyl-N'-benzoylhydrazine. m.p. 124-125 °C.

Step 4

To a stirred mixture of N'-t -butyl-N'-benzoylhydrazine (1.0 g) in 15 ml toluene and aqueous sodium hydroxide (0.5 g of 50% NaOH) was added 3-toluoylchloride (0.9 g). After stirring for 2 hours, the product was isolated by filtration to give N'-t -butyl-N-(3-toluoyl)-N'-benzoylhydrazine in good yield. m.p. 111-114 °C.

EXAMPLE NO. 295 - Preparation of N'-(1,1-dimethylethyl)-N,N'-dibenzoylhydrazine

To a gently refluxing solution of ethyl magnesium bromide (150 ml of 1 M solution) was added acetone azine (20 g) dissolved in diethyl ether (80 ml). The solution was refluxed for three days. Upon cooling, a

saturated solution of ammonium chloride (75 ml) was added. The aqueous layer was separated and washed twice with diethyl ether (150 ml). The combined ether extracts were dried over anhydrous magnesium sulfate, filtered and the ether removed at reduced pressure. The product was distilled through a vigreux column at 3 torr and collected in a dry ice/acetone cooled receiving flask. The boiling point was 40-50°. 15 g of product was collected.

Oxalic acid (17 g) was dissolved in a solution of ethanol:diethyl ether (1:1) (150 ml) and water (3.3 g) was added. To this acid solution was added the hydrazone (13 g) dissolved in diethyl ether (30 ml). The solution was stirred for 24 hours then filtered. The solid is washed once with diethyl ether. The filtrate was concentrated and combined with the solid to afford a 77% yield (16.3 g) of the hydrazine oxalate.

The 1,1-dimethylethylhydrazine oxalate (2 g) was dissolved in toluene and neutralized with 50% aqueous sodium hydroxide. To this solution was added benzoyl chloride (4.02 g) and sodium hydroxide (50% Aq. solution) (2.45 g) at 25° C. The reaction mixture was warmed to room temperature and stirred 3 hours. The mixture was diluted with hexane and filtered to afford the product as a white solid (0.5 g).

EXAMPLE NO. 324 - Preparation of N'-t-butyl-N-(thio benzoyl)-N'-(3-toluoyl)hydrazine

S-(thiobenzoyl)thioglycolic acid (3.0 g) was dissolved in 20 ml pyridine, treated with t -butyl hydrazine hydrochloride (excess, ca. 4 g) and then was heated at ca. 120° C for 14 hours. Water (120 ml) was added and the mixture was extracted with ether. The organic extracts were dried over anhydrous magnesium sulfate, filtered and evaporated to give crude N'-t -butyl-N-(thiobenzoyl)hydrazine as a viscous yellow oil.

N' t -butyl-N-(thiobenzoyl)hydrazine (ca. 1 g), m -toluoyl chloride (approx. 0.7 g) and 50% aqueous sodium hydroxide (6 drops) were mixed in 1 ml water and 10 ml toluene at 23° C. After stirring for 3 hours, ether-hexane was added and the product was isolated by filtration (0.25 g). m.p. 165-168° C.

EXAMPLE NO. 625 - Preparation of N'-t-butyl-N-(4-(4,4-dimethyloxazol-2-yl)benzoyl)-N'-(3-toluoyl)hydrazine

1.2 g of N'-t -butyl-N-(4-carbomethoxybenzoyl)-N'-(3-toluoyl)hydrazine was heated in 2 ml of 2-amino-2-methyl-1-propanol at 90-100° C for 5 hours. After cooling, the mixture was diluted with ether/methylene chloride and washed with 0.1 N HCl. The organic layer was evaporated to afford 1.0 g of the corresponding amide.

The amide in 10 ml of chloroform was treated with 0.25 g of thionyl chloride and stirred at 23° C for 1.5 hours. Saturated aqueous sodium bicarbonate was added and the layers separated. Evaporation of the organic layer afforded the product as a foam.

By following substantially the procedures in Examples 1 and 3 and using the reactants shown below in Table II the products of Example Nos. 2, 4 through 12, 14, 19, 20, 32, 37, 55, 98 through 101, 145, 169, 174, 181, 182, 234, 250, 260, 264, 289, 295, 298, 308, 364, 390, 391, 394, 449, 613 and 632 were prepared.

TABLE II

| Ex. No. | Compound of Formula II or V | Compound of Formula III | Base | Solvent | m.p. |
|---|---|---|---|---|---|
| 2 | 3-chlorobenzoyl chloride | *t*-butylhydrazine hydrochloride | sodium hydroxide | toluene and water | 207-208°C |
| 4 | 3,4-dichlorobenzoyl chloride | *t*-butylhydrazine hydrochloride | sodium hydroxide | toluene and water | 225-227°C |
| 5 | 4-methylbenzoyl chloride | *t*-butylhydrazine hydrochloride | sodium hydroxide | toluene and water | 222-223°C |
| 6 | 4-nitrobenzoyl chloride | *t*-butylhydrazine hydrochloride | sodium hydroxide | toluene and water | 237-240°C |
| 7 | 4-methoxybenzoyl chloride | *t*-butylhydrazine hydrochloride | sodium hydroxide | toluene and water | 210-211°C |
| 8 | 3-nitrobenzoyl chloride | *t*-butylhydrazine hydrochloride | sodium hydroxide | toluene and water | 225-227°C |
| 9 | 3-methoxybenzoyl chloride | *t*-butylhydrazine hydrochloride | sodium hydroxide | toluene and water | 164-166°C |
| 10 | 2-nitrobenzoyl chloride | *t*-butylhydrazine hydrochloride | sodium hydroxide | toluene and water | 228-230°C |
| 11 | 2-chlorobenzoyl chloride | *t*-butylhydrazine hydrochloride | sodium hydroxide | toluene and water | 217-218°C |
| 12 | 2-methoxybenzoyl chloride | *t*-butylhydrazine hydrochloride | sodium hydroxide | toluene and water | 96-97°C |
| 14 | 4-cyanobenzoyl chloride | *t*-butylhydrazine hydrochloride | sodium hydroxide | toluene and water | 241-244°C |
| 19 | 3-methylbenzoyl chloride | *t*-butylhydrazine hydrochloride | sodium hydroxide | toluene and water | 146-148°C |
| 20 | 2-methylbenzoyl chloride | *t*-butylhydrazine hydrochloride | sodium hydroxide | toluene and water | 194-195°C |

| Ex. No. | Compound of Formula II or V | Compound of Formula III | Base | Solvent | m.p. |
|---|---|---|---|---|---|
| 32 | 4-*t*-butylbenzoyl chloride | *t*-butylhydrazine hydrochloride | sodium hydroxide | toluene and water | 194-196°C |
| 37 | 3,5-dichlorobenzoyl-chloride | *t*-butylhydrazine hydrochloride | sodium hydroxide | toluene and water | >250°C |
| 55 | 1-naphthoylchloride | *t*-butylhydrazine hydrochloride | sodium hydroxide | toluene and water | 105-108°C |
| 98 | 4-fluorobenzoyl chloride | *t*-butylhydrazine hydrochloride | sodium hydroxide | toluene and water | 210-214°C |
| 99 | 3-fluorobenzoyl chloride | *t*-butylhydrazine hydrochloride | sodium hydroxide | toluene and water | 130-145°C |
| 100 | 2-fluorobenzoyl chloride | *t*-butylhydrazine hydrochloride | sodium hydroxide | toluene and water | 141-145°C |
| 101 | 2-naphthoylchloride | *t*-butylhydrazine hydrochloride | sodium hydroxide | toluene and water | 246-249°C |
| 145 | 4-biphenylbenzoyl chloride | *t*-butylhydrazine hydrochloride | sodium hydroxide | toluene and water | |
| 169 | 2-chloromethyl-benzoyl chloride | *t*-butylhydrazine hydrochloride | sodium hydroxide | toluene and water | 144-146°C |
| 174 | 2-bromobenzoyl chloride | *t*-butylhydrazine hydrochloride | sodium hydroxide | toluene and water | 220°C |
| 181 | 4-ethylbenzoyl chloride | *t*-butylhydrazine hydrochloride | sodium hydroxide | toluene and water | |
| 234 | 3-methoxy benzoyl chloride | *t*-butylhydrazine hydrochloride | sodium hydroxide | toluene and water | 179-180°C |
| 250 | benzoyl chloride | 2,2-dimethyl-pentylhydrazine oxalic acid salt | sodium hydroxide | toluene and water | low melting solid |
| 260 | 2-nitro-4-chloro-benzoyl chloride | *t*-butylhydrazine hydrochloride | sodium hydroxide | toluene and water | |
| 264 | 2,6-difluorobenzoyl chloride | *t*-butylhydrazine hydrochloride | sodium hydroxide | toluene and water | 210-212°C |

| Ex. No. | Compound of Formula II or V | Compound of Formula III | Base | Solvent | m.p. |
|---|---|---|---|---|---|
| 289 | 4-vinylbenzoyl chloride | t-butylhydrazine hydrochloride | sodium hydroxide | toluene and water | low melting solid |
| 295 | benzoyl chloride | 2,2-dimethyl-propylhydrazine oxalic acid salt | sodium hydroxide | toluene and water | 150°C |
| 298 | 2-bromobenzoyl chloride | 2,2-dimethyl-propylhydrazine oxalic acid salt | sodium hydroxide | toluene and water | 172°C |
| 308 | 4-(1-propenyl)-benzoyl chloride | t-butylhydrazine hydrochloride | sodium hydroxide | toluene and water | low melting solid |
| 364 | 4-bromobenzoyl chloride | t-butylhydrazine hydrochloride | sodium hydroxide | toluene and water | >250°C |
| 390 | 3,5-dimethyl-benzoyl chloride | t-butylhydrazine hydrochloride | sodium hydroxide | toluene and water | 209-211°C |
| 391 | 2,4-dichlorobenzoyl chloride | t-butylhydrazine hydrochloride | sodium hydroxide | toluene and water | 170°C |
| 394 | 2,3-dimethyl-benzoyl chloride | t-butylhydrazine hydrochloride | sodium hydroxide | toluene and water | |
| 449 | 3,4-dimethyl-benzoyl chloride | t-butylhydrazine hydrochloride | sodium hydroxide | toluene and water | 190°C |
| 613 | 2-acetoxybenzoyl chloride | t-butylhydrazine hydrochloride | $NaHCO_3$ i.e. sodium bicarbonate | toluene and water | low melting solid |
| 632 | 2-methyl-3-chloro-benzoyl chloride | t-butylhydrazine hydrochloride | sodium hydroxide | toluene and water | |

By following substantially the procedures in Example 16 and using the reactants shown below in Table III, the products of Example Nos. 13, 15, 17, 18, 21 through 31, 33 through 36, 38, 40 through 43, 45, 47 through 54, 57 through 62, 64 through 97, 104 through 109, 113, 117, 118, 119, 121, 122, 123, 125, 126, 130 through 135, 137 through 142, 146, 147, 150, 152 through 154, 160, 163, 167, 173, 175, through 180, 182, 183, 184, 190, 194 through 202, 204, through 211, 214 through 220, 224 through 231, 235 through 249, 251 through 259, 261 through 263, 265 through 270, 272 through 284, 287, 288, 290 through 292, 296, 297, 299 through 307, 309 through 322, 325, 327, 328, 334 341 through 343, 346 through 348, 355 through 357, 370, 371, 377, 378, 380 381, 387 through 389, 392, 393, 395, 396, 401 through 413, 419, 420, 422 through 425, 426 through 432, 437 through 448, 450 through 452, 454 through 457, 459 through 469, 474 through 479, 485 through 492, 494 through 528, 531 through 540, 542 through 545, 548 through 597, 599, 601 through 612, 615 through 624, 626 through 631 and 633 were prepared.

TABLE III

| Example No. | Compound of Formula II | Compound of Formula V | Compound of Formula III | Base | Solvents | m.p. |
|---|---|---|---|---|---|---|
| 13 | 4-toluoyl chloride | benzoylchloride | t-butylhydrazine hydrochloride | sodium hydroxide | toluene and water | |
| 15 | 4-toluoyl chloride | 4-chlorobenzoyl chloride | t-butylhydrazine hydrochloride | sodium hydroxide | toluene and water | |
| 17 | benzoylchloride | 3-chlorobenzoyl chloride | t-butylhydrazine hydrochloride | sodium hydroxide | toluene and water | 177-179°C |
| 18 | benzoylchloride | 2-chlorobenzoyl chloride | t-butylhydrazine hydrochloride | sodium hydroxide | toluene and water | 182-184°C |
| 21 | benzoylchloride | 4-methylbenzoyl chloride | t-butylhydrazine hydrochloride | sodium hydroxide | toluene and water | 177-179°C |
| 22 | benzoylchloride | 3-methylbenzoyl chloride | t-butylhydrazine hydrochloride | sodium hydroxide | toluene and water | 198-200°C |
| 23 | benzoylchloride | 2-methylbenzoyl chloride | t-butylhydrazine hydrochloride | sodium hydroxide | toluene and water | 200-202°C |
| 24 | benzoylchloride | 4-methoxybenzoyl chloride | t-butylhydrazine hydrochloride | sodium hydroxide | toluene and water | 214-216°C |
| 25 | benzoylchloride | 3-methoxybenzoyl chloride | t-butylhydrazine hydrochloride | sodium hydroxide | toluene and water | 178.5-181°C |
| 26 | benzoylchloride | 2-methoxybenzoyl chloride | t-butylhydrazine hydrochloride | sodium hydroxide | toluene and water | 175-177°C |
| 27 | benzoylchloride | 4-t-butylbenzoyl chloride | t-butylhydrazine hydrochloride | sodium hydroxide | toluene and water | 185-188°C |

| Example No. | Compound of Formula II | Compound of Formula V | Compound of Formula III | Base | Solvents | m.p. |
|---|---|---|---|---|---|---|
| 28 | benzoylchloride | 4-cyanobenzoyl chloride | _t_-butylhydrazine hydrochloride | sodium hydroxide | toluene and water | 185-190°C |
| 29 | benzoylchloride | 4-nitrobenzoyl chloride | _t_-butylhydrazine hydrochloride | sodium hydroxide | toluene and water | 216-218°C |
| 30 | benzoylchloride | 3-nitrobenzoyl chloride | _t_-butylhydrazine hydrochloride | sodium hydroxide | toluene and water | 194-198°C |
| 31 | benzoylchloride | 2-nitrobenzoyl chloride | _t_-butylhydrazine hydrochloride | sodium hydroxide | toluene and water | 135-137°C |
| 33 | 4-methylbenzoyl chloride | 3,4-dichlorobenzoyl chloride | _t_-butylhydrazine hydrochloride | sodium hydroxide | toluene and water | 238-240°C |
| 34 | benzoylchloride | 4-fluorobenzoyl chloride | _t_-butylhydrazine hydrochloride | sodium hydroxide | toluene and water | 202-203°C |
| 35 | benzoylchloride | 3-fluorobenzoyl chloride | _t_-butylhydrazine hydrochloride | sodium hydroxide | toluene and water | 155-158°C |
| 36 | benzoylchloride | 2-fluorobenzoyl chloride | _t_-butylhydrazine hydrochloride | sodium hydroxide | toluene and water | 168-170°C |
| 38 | benzoylchloride | 2,4-dichlorobenzoyl chloride | _t_-butylhydrazine hydrochloride | sodium hydroxide | toluene and water | 82-84°C |
| 40 | benzoylchloride | 4-trifluoromethyl benzoylchloride | _t_-butylhydrazine hydrochloride | sodium hydroxide | toluene and water | 216-218°C |
| 41 | benzoylchloride | 3-trifluoromethyl benzoylchloride | _t_-butylhydrazine hydrochloride | sodium hydroxide | toluene and water | 186-190°C |
| 42 | benzoylchloride | 2-trifluoromethyl benzoylchloride | _t_-butylhydrazine hydrochloride | sodium hydroxide | toluene and water | 163-165°C |

| Example No. | Compound of Formula II | Compound of Formula V | Compound of Formula III | Base | Solvents | m.p. |
|---|---|---|---|---|---|---|
| 43 | benzoylchloride | 2,5-difluorobenzoyl chloride | _t_-butylhydrazine hydrochloride | sodium hydroxide | toluene and water | 147-150°C |
| 45 | benzoylchloride | 3-cyanobenzoyl chloride | _t_-butylhydrazine hydrochloride | sodium hydroxide | toluene and water | 138-140°C |
| 47 | benzoylchloride | 2,6-difluorobenzoyl chloride | _t_-butylhydrazine hydrochloride | sodium hydroxide | toluene and water | 179-182°C |
| 48 | 4-chlorobenzoyl chloride | benzoylchloride | _t_-butylhydrazine hydrochloride | sodium hydroxide | toluene and water | |
| 49 | benzoylchloride | 3,4-dichlorobenzoyl chloride | _t_-butylhydrazine hydrochloride | sodium hydroxide | toluene and water | 211-213°C |
| 50 | benzoylchloride | 3,5-dichlorobenzoyl chloride | _t_-butylhydrazine hydrochloride | sodium hydroxide | toluene and water | >250°C |
| 51 | benzoylchloride | 2,6-dichlorobenzoyl chloride | _t_-butylhydrazine hydrochloride | sodium hydroxide | toluene and water | 183-185°C |
| 52 | 4-_t_-butylbenzoyl chloride | benzoylchloride | _t_-butylhydrazine hydrochloride | sodium hydroxide | toluene and water | glassy solid |
| 53 | 2-chlorobenzoyl chloride | benzoylchloride | _t_-butylhydrazine hydrochloride | sodium hydroxide | toluene and water | glassy solid |
| 54 | 1-naphthoylchloride | benzoylchloride | _t_-butylhydrazine hydrochloride | sodium hydroxide | toluene and water | 212-214°C |
| 56 | 3-chlorobenzoyl chloride | benzoylchloride | _t_-butylhydrazine hydrochloride | sodium hydroxide | toluene and water | 182-183°C |
| 57 | 4-chlorobenzoyl chloride | 3,4-dichlorobenzoyl chloride | _t_-butylhydrazine hydrochloride | sodium hydroxide | toluene and water | 261-263°C |

| Example No. | Compound of Formula II | Compound of Formula V | Compound of Formula III | Base | Solvents | m.p. |
|---|---|---|---|---|---|---|
| 58 | 2-chlorobenzoyl chloride | 3,4-dichlorobenzoyl chloride | _t_-butylhydrazine hydrochloride | sodium hydroxide | toluene and water | 256-258°C |
| 59 | 2-methylbenzoyl chloride | benzoylchloride | _t_-butylhydrazine hydrochloride | sodium hydroxide | toluene and water | 182-185°C |
| 60 | benzoylchloride | 2-chloro-4-nitro benzoylchloride | _t_-butylhydrazine hydrochloride | sodium hydroxide | toluene and water | glassy solid |
| 61 | benzoylchloride | 3,5-dinitrobenzoyl chloride | _t_-butylhydrazine hydrochloride | sodium hydroxide | toluene and water | 223-226°C |
| 62 | benzoylchloride | 2,3-dichlorobenzoyl chloride | _t_-butylhydrazine hydrochloride | sodium hydroxide | toluene and water | 185-188°C |
| 64 | benzoylchloride | 2-chloro-5-methyl benzoylchloride | _t_-butylhydrazine hydrochloride | sodium hydroxide | toluene and water | 212-214°C |
| 65 | benzoylchloride | 3,5-dimethylbenzoyl chloride | _t_-butylhydrazine hydrochloride | sodium hydroxide | toluene and water | 194-197°C |
| 66 | benzoylchloride | 2-nitro-5-methyl benzoylchloride | _t_-butylhydrazine hydrochloride | sodium hydroxide | toluene and water | 190-195°C |
| 67 | benzoylchloride | 2-methyl-3-chloro benzoylchloride | _t_-butylhydrazine hydrochloride | sodium hydroxide | toluene and water | 207-209°C |
| 68 | benzoylchloride | 3-chloro-4-methyl benzoylchloride | _t_-butylhydrazine hydrochloride | sodium hydroxide | toluene and water | glassy solid |
| 69 | benzoylchloride | 2-nitro-3-chloro benzoylchloride | _t_-butylhydrazine hydrochloride | sodium hydroxide | toluene and water | 80-83°C |
| 70 | benzoylchloride | 3-methoxy-4-nitro benzoylchloride | _t_-butylhydrazine hydrochloride | sodium hydroxide | toluene and water | 205-207°C |

| Example No. | Compound of Formula II | Compound of Formula V | Compound of Formula III | Base | Solvents | m.p. |
|---|---|---|---|---|---|---|
| 71 | benzoylchloride | 2-nitro-3-methoxy benzoylchloride | *t*-butylhydrazine hydrochloride | sodium hydroxide | toluene and water | 158-159°C |
| 72 | benzoylchloride | 2,4-dinitrobenzoyl chloride | *t*-butylhydrazine hydrochloride | sodium hydroxide | toluene and water | >250°C |
| 73 | 4-chlorobenzoyl chloride | 2-chlorobenzoyl chloride | *t*-butylhydrazine hydrochloride | sodium hydroxide | toluene and water | 190-192°C |
| 74 | 4-chlorobenzoyl chloride | 3-chlorobenzoyl chloride | *t*-butylhydrazine hydrochloride | sodium hydroxide | toluene and water | 195-197° |
| 75 | 4-chlorobenzoyl chloride | 4-methylbenzoyl chloride | *t*-butylhydrazine hydrochloride | sodium hydroxide | toluene and water | 204-205.5°C |
| 76 | 4-chlorobenzoyl chloride | 3,5-dichlorobenzoyl chloride | *t*-butylhydrazine hydrochloride | sodium hydroxide | toluene and water | 215-219°C |
| 77 | 4-chlorobenzoyl chloride | 2,4-dichlorobenzoyl chloride | *t*-butylhydrazine hydrochloride | sodium hydroxide | toluene and water | 185-188°C |
| 78 | 4-chlorobenzoyl chloride | 4-trifluoromethyl benzoylchloride | *t*-butylhydrazine hydrochloride | sodium hydroxide | toluene and water | 257-259°C |
| 79 | benzoylchloride | 4-methanesulfonyl benzoylchloride | *t*-butylhydrazine hydrochloride | sodium hydroxide | toluene and water | 180-183°C |
| 80 | benzoylchloride | 4-isopropylbenzoyl chloride | *t*-butylhydrazine hydrochloride | sodium hydroxide | toluene and water | 220-224°C |
| 81 | benzoylchloride | 2-acetoxybenzoyl chloride | *t*-butylhydrazine hydrochloride | sodium hydroxide | toluene and water | 154-156°C |
| 82 | benzoylchloride | 4-ethylbenzoyl chloride | *t*-butylhydrazine hydrochloride | sodium hydroxide | toluene and water | glassy solid |

| Example No. | Compound of Formula II | Compound of Formula V | Compound of Formula III | Base | Solvents | m.p. |
|---|---|---|---|---|---|---|
| 83 | benzoylchloride | 2-bromobenzoyl chloride | t-butylhydrazine hydrochloride | sodium hydroxide | toluene and water | 85-90°C |
| 84 | benzoylchloride | 4-hydroxybenzoyl chloride | t-butylhydrazine hydrochloride | sodium hydroxide | toluene and water | 229-233°C |
| 85 | 4-methylbenzoyl chloride | 2-methylbenzoyl chloride | t-butylhydrazine hydrochloride | sodium hydroxide | toluene and water | 207-208°C |
| 86 | 4-methylbenzoyl chloride | 3-methylbenzoyl chloride | t-butylhydrazine hydrochloride | sodium hydroxide | toluene and water | 173-174°C |
| 87 | 4-methylbenzoyl chloride | 2,4-dichlorobenzoyl chloride | t-butylhydrazine hydrochloride | sodium hydroxide | toluene and water | 147-148°C |
| 88 | 4-methylbenzoyl chloride | 3,5-dichlorobenzoyl chloride | t-butylhydrazine hydrochloride | sodium hydroxide | toluene and water | 228-230°C |
| 89 | 4-methylbenzoyl chloride | 2-chlorobenzoyl chloride | t-butylhydrazine hydrochloride | sodium hydroxide | toluene and water | 206-207°C |
| 90 | 4-methylbenzoyl chloride | 4-fluorobenzoyl chloride | t-butylhydrazine hydrochloride | sodium hydroxide | toluene and water | 173-174°C |
| 91 | 4-methylbenzoyl chloride | 4-trifluoromethyl chloride | t-butylhydrazine hydrochloride | sodium hydroxide | toluene and water | 147-148°C |
| 92 | 4-methylbenzoyl chloride | 3-chlorobenzoyl chloride | t-butylhydrazine hydrochloride | sodium hydroxide | toluene and water | 158-161°C |
| 93 | 4-chlorobenzoyl chloride | 3-chloromethyl benzoylchloride | t-butylhydrazine hydrochloride | sodium hydroxide | toluene and water | 203-204°C |

| Example No. | Compound of Formula II | Compound of Formula V | Compound of Formula III | Base | Solvents | m.p. |
|---|---|---|---|---|---|---|
| 94 | 4-chlorobenzoyl chloride | 4-chloromethyl benzoylchloride | t-butylhydrazine hydrochloride | sodium hydroxide | toluene and water | 216-217°C |
| 95 | 4-chlorobenzoyl chloride | 2-methylbenzoyl chloride | t-butylhydrazine hydrochloride | sodium hydroxide | toluene and water | 190-195°C |
| 96 | 4-chlorobenzoyl chloride | 3-methoxybenzoyl chloride | t-butylhydrazine hydrochloride | sodium hydroxide | toluene and water | 170-172°C |
| 97 | 4-chlorobenzoyl chloride | 3-methylbenzoyl chloride | t-butylhydrazine hydrochloride | sodium hydroxide | toluene and water | 186-188°C |
| 104 | benzoylchloride | 4-bromobenzoyl chloride | t-butylhydrazine hydrochloride | sodium hydroxide | toluene and water | 216-219°C |
| 105 | benzoylchloride | 3-bromobenzoyl chloride | t-butylhydrazine hydrochloride | sodium hydroxide | toluene and water | 207-210°C |
| 106 | benzoylchloride | 4-n-butylbenzoyl chloride | t-butylhydrazine hydrochloride | sodium hydroxide | toluene and water | glassy solid |
| 107 | 4-ethylbenzoyl chloride | benzoylchloride | t-butylhydrazine hydrochloride | sodium hydroxide | toluene and water | 197-200°C |
| 108 | 3,4-dichloro-benzoylchloride | benzoylchloride ~~chloride~~ | t-butylhydrazine hydrochloride | sodium hydroxide | toluene and water | 119-130°C |
| 109 | benzoylchloride | 4-acetylbenzoyl chloride | t-butylhydrazine hydrochloride | sodium hydroxide | toluene and water | >250°C |
| 113 | benzoylchloride | 2-iodobenzoyl chloride | t-butylhydrazine hydrochloride | sodium hydroxide | toluene and water | 80-82°C |
| 117 | benzoylchloride | 4-phenoxybenzoyl chloride | t-butylhydrazine hydrochloride | sodium hydroxide | toluene and water | 240-250°C |

| Example No. | Compound of Formula II | Compound of Formula V | Compound of Formula III | Base | Solvents | m.p. |
|---|---|---|---|---|---|---|
| 118 | 4-trifluoromethyl-benzoylchloride | benzoylchloride | _t_-butylhydrazine hydrochloride | sodium hydroxide | toluene and water | 173-175°C |
| 119 | 4-trifluoromethyl-benzoylchloride | 3,4-dichlorobenoyl-chloride | _t_-butylhydrazine hydrochloride | sodium hydroxide | toluene and water | 223-227°C |
| 121 | benzoylchloride | 2-chloro-4-bromo-benzoylchloride | _t_-butylhydrazine hydrochloride | sodium hydroxide | toluene and water | glassy solid |
| 122 | benzoylchloride | 4-phenylbenzoyl chloride | _t_-butylhydrazine hydrochloride | sodium hydroxide | toluene and water | low melting solid |
| 123 | benzoylchloride | 3,4,5-trimethoxy-benzoylchloride | _t_-butylhydrazine hydrochloride | sodium hydroxide | toluene and water | 175-177°C |
| 125 | benzoylchloride | 3-thiocyanomethyl-benzoylchloride | _t_-butylhydrazine hydrochloride | sodium hydroxide | toluene and water | low melting solid |
| 126 | benzoylchloride | 3-cyanomethyl-benzoylchloride | _t_-butylhydrazine hydrochloride | sodium hydroxide | toluene and water | 160-162°C |
| 130 | 4-ethylbenzoyl chloride | 3-toluoylchloride | _t_-butylhydrazine hydrochloride | sodium hydroxide | toluene and water | low melting solid |
| 131 | 4-ethylbenzoyl chloride | 4-chlorobenzoyl chloride | _t_-butylhydrazine hydrochloride | sodium hydroxide | toluene and water | 196-197°C |
| 132 | 4-ethylbenzoyl chloride | 2-nitrobenzoyl chloride | _t_-butylhydrazine hydrochloride | sodium hydroxide | toluene and water | low melting solid |

| Example No. | Compound of Formula II | Compound of Formula V | Compound of Formula III | Base | Solvents | m.p. |
|---|---|---|---|---|---|---|
| 133 | benzoylchloride | 3-ethylbenzoyl chloride | t-butylhydrazine hydrochloride | sodium hydroxide | toluene and water | 141-144°C |
| 134 | 4-ethylbenzoyl chloride | 3-bromobenzoyl chloride | t-butylhydrazine hydrochloride | sodium hydroxide | toluene and water | 171-174°C |
| 135 | 4-ethylbenzoyl chloride | 2-iodobenzoyl chloride | t-butylhydrazine hydrochloride | sodium hydroxide | toluene and water | glassy solid |
| 137 | benzoylchloride | 4-carbomethoxy benzoylchloride | t-butylhydrazine hydrochloride | sodium hydroxide | toluene and water | 169-180°C |
| 138 | 2-bromobenzoyl chloride | benzoylchloride | t-butylhydrazine hydrochloride | sodium hydroxide | toluene and water | 207°C |
| 139 | 2-trifluoromethyl-benzoylchloride | benzoylchloride | t-butylhydrazine hydrochloride | sodium hydroxide | toluene and water | 212-214°C |
| 140 | benzoylchloride | 3-iodobenzoyl chloride | t-butylhydrazine hydrochloride | sodium hydroxide | toluene and water | >250°C |
| 141 | benzoylchloride | 2-ethylbenzoyl chloride | t-butylhydrazine hydrochloride | sodium hydroxide | toluene and water | oil |
| 142 | benzoylchloride | 3-methoxymethylbenzoyl chloride | t-butylhydrazine hydrochloride | sodium hydroxide | toluene and water | oil |
| 146 | benzoylchloride | 2-nitro-4-(2-chloro -4-trifluoro-methyl phenoxy) benzoylchloride | t-butylhydrazine hydrochloride | sodium hydroxide | toluene and water | 50-55°C |

| Example No. | Compound of Formula II | Compound of Formula V | Compound of Formula III | Base | Solvents | m.p. |
|---|---|---|---|---|---|---|
| 147 | 2-nitro-4-(2-chloro -4-trifluoromethyl phenoxy) benzoyl chloride | benzoylchloride | *t*-butylhydrazine hydrochloride | sodium hydroxide | toluene and water | 255-258°C |
| 150 | benzoylchloride | 4-methylthio-benzoylchloride | *t*-butylhydrazine hydrochloride | sodium hydroxide | toluene and water | >265°C |
| 152 | benzoylchloride | 3-bromo-4-methyl benzoylchloride | *t*-butylhydrazine hydrochloride | sodium hydroxide | toluene and water | 220-223°C |
| 153 | benzoylchloride | 3-methyl-4-bromo benzoylchloride | *t*-butylhydrazine hydrochloride | sodium hydroxide | toluene and water | 225-227°C |
| 154 | benzoylchloride | 2,4-dibromo benzoylchloride | *t*-butylhydrazine hydrochloride | sodium hydroxide | toluene and water | 223-225°C |
| 160 | benzoylchloride | 3-phenoxy benzoylchloride | *t*-butylhydrazine hydrochloride | sodium hydroxide | toluene and water | 201-203°C |
| 163 | benzoylchloride | 4-formyl benzoyl chloride | *t*-butylhydrazine hydrochloride | sodium hydroxide | toluene and water | 158-161°C |
| 167 | benzoylchloride | 2-acetoxybenzoyl chloride | *t*-butylhydrazine hydrochloride | sodium hydroxide | toluene and water | |
| 173 | 2-methyl-5-tri-fluoromethyl benzoylchloride | 2-toluoyl chloride | *t*-butylhydrazine hydrochloride | sodium hydroxide | toluene and water | 175°C |
| 175 | 4-ethylbenzoyl chloride | 3-ethyl benzoylchloride | *t*-butylhydrazine hydrochloride | sodium hydroxide | toluene and water | oil |
| 176 | benzoylchloride | 4-*n*-propyl benzoylchloride | *t*-butylhydrazine hydrochloride | sodium hydroxide | toluene and water | 145-146°C |

| Example No. | Compound of Formula II | Compound of Formula V | Compound of Formula III | Base | Solvents | m.p. |
|---|---|---|---|---|---|---|
| 177 | 4-toluoyl chloride | 3-bromo benzoylchloride | t-butylhydrazine hydrochloride | sodium hydroxide | toluene and water | 151-153°C |
| 178 | 4-toluoyl chloride | 3,5-dimethyl benzoylchloride | t-butylhydrazine hydrochloride | sodium hydroxide | toluene and water | 225-227.5°C |
| 179 | benzoylchloride | 4-iodobenzoyl chloride | t-butylhydrazine hydrochloride | sodium hydroxide | toluene and water | 223-225°C |
| 180 | 4-toluoyl chloride | 4-ethyl benzoyl chloride | t-butylhydrazine hydrochloride | sodium hydroxide | toluene and water | 180-182°C |
| 182 | 4-toluoyl chloride | 3-ethyl benzoyl chloride | t-butylhydrazine hydrochloride | sodium hydroxide | toluene and water | 183-185°C |
| 183 | benzoylchloride | 2-isopropylbenzoyl chloride | t-butylhydrazine hydrochloride | sodium hydroxide | toluene and water | low melting solid |
| 184 | 3-ethyl benzoyl chloride | benzoylchloride | t-butylhydrazine hydrochloride | sodium hydroxide | toluene and water | oil |
| 190 | 3-ethyl benzoyl, chloride | 2,4-dichloro benzoylchloride | t-butylhydrazine hydrochloride | sodium hydroxide | toluene and water | 179-181°C |
| 194 | 4-methoxybenzoyl chloride | 3-toluoyl chloride | t-butylhydrazine hydrochloride | sodium hydroxide | toluene and water | foam |
| 195 | 4-methoxybenzoyl chloride | benzoylchloride | t-butylhydrazine hydrochloride | sodium hydroxide | toluene and water | foam |
| 196 | 2-chlorobenzoyl chloride | 3-toluoyl chloride | t-butylhydrazine hydrochloride | sodium hydroxide | toluene and water | glassy solid |

| Example No. | Compound of Formula II | Compound of Formula V | Compound of Formula III | Base | Solvents | m.p. |
|---|---|---|---|---|---|---|
| 197 | 2-chlorobenzoyl chloride | 2-nitrobenzoyl chloride | *t*-butylhydrazine hydrochloride | sodium hydroxide | toluene and water | glass |
| 198 | 2-chlorobenzoyl chloride | 4-toluoyl chloride | *t*-butylhydrazine hydrochloride | sodium hydroxide | toluene and water | 203-206°C |
| 199 | 2-chlorobenzoyl chloride | 4-ethyl benzoyl chloride | *t*-butylhydrazine hydrochloride | sodium hydroxide | toluene and water | 171-174°C |
| 200 | 4-ethyl benzoyl chloride | 3,5-dimethyl benzoylchloride | *t*-butylhydrazine hydrochloride | sodium hydroxide | toluene and water | 181°C |
| 201 | 4-ethyl benzoyl chloride | 2-chloro-4-methyl benzoylchloride | *t*-butylhydrazine hydrochloride | sodium hydroxide | toluene and water | oily solid |
| 202 | benzoylchloride | 3-acetoxybenzoyl chloride | *t*-butylhydrazine hydrochloride | sodium hydroxide | toluene and water | |
| 204 | 4-ethyl benzoyl chloride | 2-nitro-3-methyl benzoylchloride | *t*-butylhydrazine hydrochloride | sodium hydroxide | toluene and water | 85-90°C |
| 205 | 4-methoxybenzoyl chloride | 3,5-dimethyl benzoylchloride | *t*-butylhydrazine hydrochloride | sodium hydroxide | toluene and water | |
| 206 | 4-methoxybenzoyl chloride | 2,4-dichloro benzoylchloride | *t*-butylhydrazine hydrochloride | sodium hydroxide | toluene and water | |
| 207 | 2-chlorobenzoyl chloride | 2,6-dichloro benzoylchloride | *t*-butylhydrazine hydrochloride | sodium hydroxide | toluene and water | |
| 208 | 2-chlorobenzoyl chloride | 2-bromobenzoyl chloride | *t*-butylhydrazine hydrochloride | sodium hydroxide | toluene and water | 213-216°C |
| 209 | 2-chlorobenzoyl chloride | 2,5-difluoro benzoylchloride | *t*-butylhydrazine hydrochloride | sodium hydroxide | toluene and water | 190-192°C |

| Example No. | Compound of Formula II | Compound of Formula V | Compound of Formula III | Base | Solvents | m.p. |
|---|---|---|---|---|---|---|
| 210 | 2-chlorobenzoyl chloride | 4-methoxy benzoylchloride | t-butylhydrazine hydrochloride | sodium hydroxide | toluene and water | 190-192°C |
| 211 | 2-chlorobenzoyl chloride | 2-toluoyl chloride | t-butylhydrazine hydrochloride | sodium hydroxide | toluene and water | 209-211°C |
| 214 | 2-chlorobenzoyl chloride | 2,4-dichlorobenzoyl chloride | t-butylhydrazine hydrochloride | sodium hydroxide | toluene and water | 204-206°C |
| 215 | 2-chlorobenzoyl chloride | 3-methoxy benzoylchloride | t-butylhydrazine hydrochloride | sodium hydroxide | toluene and water | 170-172°C |
| 216 | 2-chlorobenzoyl chloride | 3-chlorobenzoyl chloride | t-butylhydrazine hydrochloride | sodium hydroxide | toluene and water | 212-214°C |
| 217 | 2-chlorobenzoyl chloride | 2-trifluoromethyl benzoylchloride | t-butylhydrazine hydrochloride | sodium hydroxide | toluene and water | 161-162°C |
| 218 | 2-chlorobenzoyl chloride | 3-trifluoromethyl benzoylchloride | t-butylhydrazine hydrochloride | sodium hydroxide | toluene and water | 189-190°C |
| 219 | 2-chlorobenzoyl chloride | 4-trifluoromethyl benzoylchloride | t-butylhydrazine hydrochloride | sodium hydroxide | toluene and water | 246-247°C |
| 220 | 3-toluoyl chloride | benzoylchloride | t-butylhydrazine hydrochloride | sodium hydroxide | toluene and water | |
| 224 | 2-chlorobenzoyl chloride | 4-cyanobenzoyl chloride | t-butylhydrazine hydrochloride | sodium hydroxide | toluene and water | 220-222°C |
| 225 | 2-chlorobenzoyl chloride | 4-fluorobenzoyl chloride | t-butylhydrazine hydrochloride | sodium hydroxide | toluene and water | >250°C |
| 226 | 2-chlorobenzoyl chloride | 4-bromobenzoyl chloride | t-butylhydrazine hydrochloride | sodium hydroxide | toluene and water | 228-230°C |

| Example No. | Compound of Formula II | Compound of Formula V | Compound of Formula III | Base | Solvents | m.p. |
|---|---|---|---|---|---|---|
| 227 | 2-chlorobenzoyl chloride | 4-chloro benzoylchloride | t-butylhydrazine hydrochloride | sodium hydroxide | toluene and water | 244-246°C |
| 228 | 2-chlorobenzoyl chloride | 2-methoxy benzoylchloride | t-butylhydrazine hydrochloride | sodium hydroxide | toluene and water | 199-200°C |
| 229 | 2-chlorobenzoyl chloride | 4-nitro benzoylchloride | t-butylhydrazine hydrochloride | sodium hydroxide | toluene and water | 251-253°C |
| 230 | 2-chlorobenzoyl chloride | 2-fluoro benzoylchloride | t-butylhydrazine hydrochloride | sodium hydroxide | toluene and water | 178-180°C |
| 231 | 2-chlorobenzoyl chloride | 2,6-difluoro benzoylchloride | t-butylhydrazine hydrochloride | sodium hydroxide | toluene and water | 213-214°C |
| 235 | 3-methoxybenzoyl chloride | 2,5-difluoro benzoylchloride | t-butylhydrazine hydrochloride | sodium hydroxide | toluene and water | 146-148°C |
| 236 | 3-methoxybenzoyl chloride | 4-chlorobenzoyl chloride | t-butylhydrazine hydrochloride | sodium hydroxide | toluene and water | 178-179°C |
| 237 | 3-methoxybenzoyl chloride | 3,4-dichloro benzoylchloride | t-butylhydrazine hydrochloride | sodium hydroxide | toluene and water | 213-216°C |
| 238 | 3-methoxybenzoyl chloride | benzoylchloride | t-butylhydrazine hydrochloride | sodium hydroxide | toluene and water | 168-170°C |
| 239 | 3-methoxybenzoyl chloride | 3-toluoyl chloride | t-butylhydrazine hydrochloride | sodium hydroxide | toluene and water | 158-160°C |
| 240 | 3-methoxybenzoyl chloride | 3,4-dichloro benzoylchloride | t-butylhydrazine hydrochloride | sodium hydroxide | toluene and water | >250°C |
| 241 | 3-methoxybenzoyl chloride | 4-chlorobenzoyl chloride | t-butylhydrazine hydrochloride | sodium hydroxide | toluene and water | 182-184°C |

| Example No. | Compound of Formula II | Compound of Formula V | Compound of Formula III | Base | Solvents | m.p. |
|---|---|---|---|---|---|---|
| 242 | 3-methoxybenzoyl chloride | 2-nitrobenzoyl chloride | _t_-butylhydrazine hydrochloride | sodium hydroxide | toluene and water | 152-154°C |
| 243 | benzoylchloride | 4-trifluoromethoxy benzoylchloride | _t_-butylhydrazine hydrochloride | pyridine | pyridine | 189-190°C |
| 244 | 4-trifluoromethoxy benzoylchloride | benzoylchloride | _t_-butylhydrazine hydrochloride | sodium hydroxide | toluene and water | 180-182°C |
| 245 | 4-trifluoromethoxy benzoylchloride | 3-toluoyl chloride | _t_-butylhydrazine hydrochloride | sodium hydroxide | toluene and water | 154-156°C |
| 246 | 4-trifluoromethoxy benzoylchloride | 4-chloro benzoyl chloride | _t_-butylhydrazine hydrochloride | sodium hydroxide | toluene and water | Solid |
| 247 | 4-trifluoromethoxy benzoylchloride | 3,4-dichloro benzoylchloride | _t_-butylhydrazine hydrochloride | sodium hydroxide | toluene and water | >250°C |
| 248 | 4-trifluoromethoxy benzoylchloride | 4-chlorobenzoyl chloride | _t_-butylhydrazine hydrochloride | sodium hydroxide | toluene and water | 196-200°C |
| 249 | 4-trifluoromethoxy benzoylchloride, | 3-toluoyl chloride | _t_-butylhydrazine hydrochloride | sodium hydroxide | toluene and water | |
| 251 | 4-ethoxybenzoyl chloride | 3-toluoyl chloride | _t_-butylhydrazine hydrochloride | sodium hydroxide | toluene and water | |
| 252 | 4-ethoxybenzoyl chloride | 3,5-dimethyl benzoylchloride | _t_-butylhydrazine hydrochloride | sodium hydroxide | toluene and water | oil |
| 253 | 4-ethoxybenzoyl chloride | benzoylchloride | _t_-butylhydrazine hydrochloride | sodium hydroxide | toluene and water | |
| 254 | 4-ethoxybenzoyl chloride | 2-nitro-4-chloro benzoylchloride | _t_-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 255 | 3-chloro-4-methoxy-benzoylchloride | benzoylchloride | *t*-butylhydrazine hydrochloride | sodium hydroxide | toluene and water | 158-160°C |
| 256 | 4-methylthio benzoylchloride | benzoylchloride | *t*-butylhydrazine hydrochloride | sodium hydroxide | toluene and water | 98-101°C |
| 257 | 4-*n*-butyloxy benzoylchloride | 4-chlorobenzoyl chloride | *t*-butylhydrazine hydrochloride | sodium hydroxide | toluene and water | |
| 258 | 2-methylthio-benzoylchloride | benzoylchloride | *t*-butylhydrazine hydrochloride | sodium hydroxide | toluene and water | 235°C |
| 259 | 2-nitro-4-chloro-benzoylchloride | benzoylchloride | *t*-butylhydrazine hydrochloride | sodium hydroxide | toluene and water | 240°C |
| 261 | 2-nitro-4-chloro-benzoylchloride | 4-*t*-butylbenzoyl chloride | *t*-butylhydrazine hydrochloride | triethyl-amine | methylene chloride | |
| 262 | 2-nitro-4-chloro-benzoylchloride | 4-chlorobenzoyl chloride | *t*-butylhydrazine hydrochloride | sodium hydroxide | toluene and water | 250°C |
| 263 | 4-toluoyl chloride | 2-chloro-5-methyl benzoylchloride | *t*-butylhydrazine hydrochloride | sodium hydroxide | toluene and water | 177-181°C |
| 265 | 4-phenoxybenzoyl chloride | benzoylchloride | *t*-butylhydrazine hydrochloride | sodium hydroxide | toluene and water | |
| 266 | 4-phenoxybenzoyl chloride | 4-toluoyl chloride | *t*-butylhydrazine hydrochloride | sodium hydroxide | toluene and water | |
| 267 | 4-*n*-butylbenzoyl chloride | benzoylchloride | *t*-butylhydrazine hydrochloride | sodium hydroxide | toluene and water | oily solid |
| 268 | 4-*n*-butylbenzoyl chloride | 4-chlorobenzoyl chloride | *t*-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 269 | 4-isopropylbenzoyl chloride | benzoylchloride | t-butylhydrazine hydrochloride | sodium hydroxide | toluene and water | |
| 270 | 4-isopropylbenzoyl chloride | 3-toluoyl chloride | t-butylhydrazine hydrochloride | sodium hydroxide | toluene and water | |
| 272 | 4-cyanobenzoyl chloride | benzoylchloride | t-butylhydrazine hydrochloride | sodium hydroxide | toluene and water | |
| 273 | 4-cyanobenzoyl chloride | 3-toluoyl chloride | t-butylhydrazine hydrochloride | sodium hydroxide | toluene and water | 228-232°C |
| 274 | 2-methyl-4-chloro-benzoylchloride | benzoylchloride | t-butylhydrazine hydrochloride | sodium hydroxide | toluene and water | 227-230°C |
| 275 | 4-ethylbenzoyl chloride | 4-trifluoromethoxy benzoylchloride | t-butylhydrazine hydrochloride | pyridine | pyridine | 198-200°C |
| 276 | benzoylchloride | 2,3,4,5,6-penta-fluoro benzoyl chloride | t-butylhydrazine hydrochloride | sodium hydroxide | toluene and water | 180-182°C |
| 277 | 2,3,4,5,6-penta-, fluoro benzoyl chloride | benzoylchloride | t-butylhydrazine hydrochloride | sodium hydroxide | toluene and water | 141-142°C |
| 278 | 4-cyanobenzoyl chloride | 3,4-dichlorobenzoyl | t-butylhydrazine hydrochloride | sodium hydroxide | toluene and water | 211-213°C |
| 279 | 2-methyl-4-chloro-benzoylchloride | 3-toluoyl chloride | t-butylhydrazine hydrochloride | sodium hydroxide | toluene and water | 212-215°C |
| 280 | 4-trifluoromethyl benzoylchloride | 3,5-dimethylbenzoyl chloride | t-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 281 | 2-methyl-4-chloro-benzoylchloride | 3,5-dimethylbenzoyl chloride | t-butylhydrazine hydrochloride | sodium hydroxide | toluene and water | >240°C |
| 282 | benzoylchloride | 3-vinylbenzoyl chloride | t-butylhydrazine hydrochloride | sodium hydroxide | toluene and water | |
| 283 | 4-ethylbenzoyl chloride | 3-vinylbenzoyl chloride | t-butylhydrazine hydrochloride | sodium hydroxide | toluene and water | |
| 284 | 4-trifluoromethyl-benzoylchloride | 3-vinylbenzoyl chloride | t-butylhydrazine hydrochloride | sodium hydroxide | toluene and water | |
| 287 | 4-n-propylbenzoyl chloride | 3,4-dichloro-benzoylchloride | t-butylhydrazine hydrochloride | sodium hydroxide | toluene and water | 171-174°C |
| 288 | 4-n-propylbenzoyl chloride | 3-toluoyl chloride | t-butylhydrazine hydrochloride | sodium hydroxide | toluene and water | |
| 290 | 4-vinylbenzoyl chloride | 3-toluoyl chloride | t-butylhydrazine hydrochloride | sodium hydroxide | toluene and water | low melting solid |
| 291 | 2,6-difluoro-benzoylchloride | 3,4-dichloro-benzoylchloride | t-butylhydrazine hydrochloride | sodium hydroxide | toluene and water | 240-241°C |
| 292 | 2,6-difluoro-benzoylchloride | 3-toluoyl chloride | t-butylhydrazine hydrochloride | sodium hydroxide | toluene and water | 172-174°C |
| 296 | 4-chlorobenzoyl chloride | 2-bromobenzoyl chloride | t-butylhydrazine hydrochloride | sodium hydroxide | toluene and water | 165-166°C |
| 297 | 4-toluoyl chloride | 3-toluoyl chloride | 2,2-dimethyl-propylhydrazine oxalic acid salt | sodium hydroxide | toluene and water | 154°C |

| Example No. | Compound of Formula II | Compound of Formula V | Compound of Formula III | Base | Solvents | m.p. |
|---|---|---|---|---|---|---|
| 299 | 4-chlorobenzoyl chloride | 3,5-dimethyl-benzoylchloride | _t_-butylhydrazine hydrochloride | sodium hydroxide | toluene and water | 218-220°C |
| 300 | 3-methoxybenzoyl chloride | 3,5-dimethylbenzoyl chloride | _t_-butylhydrazine hydrochloride | sodium hydroxide | toluene and water | >240°C |
| 301 | 4-chlorobenzoyl chloride | 4-fluorobenzoyl chloride | _t_-butylhydrazine hydrochloride | sodium hydroxide | toluene and water | |
| 302 | 4-_n_-propylbenzoyl chloride | 3,5-dimethylbenzoyl chloride | _t_-butylhydrazine hydrochloride | sodium hydroxide | toluene and water | oily solid |
| 303 | 4-vinylbenzoyl chloride | 3,5-dimethylbenzoyl chloride | _t_-butylhydrazine hydrochloride | sodium hydroxide | toluene and water | 185-187°C |
| 304 | 4-acetoxybenzoyl chloride | 3,5-dimethylbenzoyl chloride | _t_-butylhydrazine hydrochloride | sodium bicarb-onate | toluene and water | 73-75°C |
| 305 | 3,5-dimethyl-benzoylchloride | benzoylchloride | _t_-butylhydrazine hydrochloride | sodium hydroxide | toluene and water | 84-86°C |
| 306 | 3,5-dimethyl-benzoylchloride | 3-toluoyl chloride | _t_-butylhydrazine hydrochloride | sodium hydroxide | toluene and water | Oil |
| 307 | 3,5-dimethyl-benzoylchloride | 4-ethylbenzoyl chloride | _t_-butylhydrazine hydrochloride | sodium hydroxide | toluene and water | 168-170°C |
| 309 | 4-isopropyl-benzoylchloride | 3,5-dimethyl benzoylchloride | _t_-butylhydrazine hydrochloride | sodium hydroxide | toluene and water | Oily Solid |
| 310 | 4-ethylbenzoyl chloride | 2-bromobenzoyl chloride | _t_-butylhydrazine hydrochloride | sodium hydroxide | toluene and water | 179°C |

| Example No. | Compound of Formula II | Compound of Formula V | Compound of Formula III | Base | Solvents | m.p. |
| --- | --- | --- | --- | --- | --- | --- |
| 311 | 3-chloro-4-methyl-benzoylchloride | benzoylchloride | _t_-butylhydrazine hydrochloride | sodium hydroxide | toluene and water | 172-174°C |
| 312 | 3-chloro-4-methyl-benzoylchloride | benzoylchloride | _t_-butylhydrazine hydrochloride | sodium hydroxide | toluene and water | 210-214°C |
| 313 | 3-chloro-4-methyl-benzoylchloride | 3-toluoyl chloride | _t_-butylhydrazine hydrochloride | sodium hydroxide | toluene and water | 205-208°C |
| 314 | 3-chloro-4-methyl-benzoylchloride | 3,4-dichlorobenzoyl chloride | _t_-butylhydrazine hydrochloride | sodium hydroxide | toluene and water | 155-157°C |
| 315 | 4-ethylbenzoyl chloride | 2-nitrobenzoyl chloride | 2,2-dimethyl-propylhydrazine oxalic acid salt | sodium hydroxide | toluene and water | 145°C |
| 316 | 2,6-difluoro-benzoylchloride | 2-chlorobenzoyl chloride | _t_-butylhydrazine hydrochloride | | 255-257°C | |
| 317 | 2,6-difluoro-benzoylchloride | 3-chlorobenzoyl chloride | _t_-butylhydrazine hydrochloride | | 191-192°C | |
| 318 | 2,6-difluoro-benzoylchloride | 4-chlorobenzoyl chloride | _t_-butylhydrazine hydrochloride | | 233-235°C | |
| 319 | 2,6-difluoro-benzoylchloride | 3,5-dimethylbenzoyl chloride | _t_-butylhydrazine hydrochloride | | 214-215°C | |
| 320 | 4-ethylbenzoyl chloride | 3,5-dimethylbenzoyl chloride | 2,2-dimethyl-propylhydrazine oxalic acid salt | sodium hydroxide | toluene and water | 160°C |
| 321 | 3-trifluoromethyl-benzoylchloride | 4-chlorobenzoyl chloride | _t_-butylhydrazine hydrochloride | | 183-185°C | |

| Example No. | Compound of Formula II | Compound of Formula V | Compound of Formula III | Base | Solvents | m.p. |
| --- | --- | --- | --- | --- | --- | --- |
| 322 | 3-trifluoromethyl-benzoylchloride | 3,5-dimethyl-benzoylchloride | t-butylhydrazine hydrochloride | sodium hydroxide | toluene and water | >225°C |
| 325 | 2-nitro-3-methoxy-benzoylchloride | 3-toluoyl chloride | t-butylhydrazine hydrochloride | sodium hydroxide | toluene and water | 125-128°C |
| 327 | 4-chlorobenzoyl chloride | 4-carboxymethyl-benzoylchloride | t-butylhydrazine hydrochloride | sodium hydroxide | toluene and water | >240°C |
| 328 | 4-ethylbenzoyl chloride | 4-carboxymethyl-benzoylchloride | t-butylhydrazine hydrochloride | sodium hydroxide | toluene and water | >240°C |
| 334 | 4-ethylbenzoyl chloride | 3,5-dichloro-benzoylchloride | t-butylhydrazine hydrochloride | sodium hydroxide | toluene and water | low melting solid |
| 341 | 4-chlorobenzoyl chloride | 2-fluorobenzoyl chloride | t-butylhydrazine hydrochloride | sodium hydroxide | toluene and water | |
| 342 | 4-ethylbenzoyl chloride | 3,4-dichloro-benzoylchloride | t-butylhydrazine hydrochloride | sodium hydroxide | toluene and water | >210°C |
| 343 | 4-ethylbenzoyl chloride | 2-fluorobenzoyl chloride | t-butylhydrazine hydrochloride | sodium hydroxide | toluene and water | >210°C |
| 346 | 4-carboxymethyl-benzoylchloride | 4-chlorobenzoyl chloride | t-butylhydrazine hydrochloride | sodium bicar-bonate | toluene and water | 255-257°C |
| 347 | 4-carboxymethyl-benzoylchloride | 3,5-dimethyl-benzoylchloride | t-butylhydrazine hydrochloride | sodium bicar-bonate | toluene and water | >270°C |

| Example No. | Compound of Formula II | Compound of Formula V | Compound of Formula III | Base | Solvents | m.p. |
|---|---|---|---|---|---|---|
| 348 | 4-carboxymethyl-benzoylchloride | 3-toluoyl chloride | t-butylhydrazine hydrochloride | sodium bicar-bonate | toluene and water | >270°C |
| 355 | 3-phenoxybenzoyl chloride | benzoylchloride | t-butylhydrazine hydrochloride | sodium hydroxide | toluene and water | 126-130°C |
| 356 | 3-phenoxybenzoyl chloride | 3-toluoyl chloride | t-butylhydrazine hydrochloride | sodium hydroxide | toluene and water | 118-122°C |
| 357 | 4-acetylbenzoyl chloride | 3-toluoyl chloride | t-butylhydrazine hydrochloride | sodium hydroxide | toluene and water | 154-156°C |
| 370 | 4-biphenylbenzoyl chloride | 3-toluoyl chloride | t-butylhydrazine hydrochloride | sodium hydroxide | toluene and water | >240°C |
| 371 | 3-cyanobenzoyl chloride | 3-toluoyl chloride | t-butylhydrazine hydrochloride | sodium hydroxide | toluene and water | 195-198°C |
| 377 | 3-chloromethyl-benzoylchloride | 3-toluoyl chloride | t-butylhydrazine hydrochloride | sodium hydroxide | toluene and water | 100-102°C |
| 378 | 4-ethylbenzoyl chloride | 2,3-dimethylbenzoyl chloride | t-butylhydrazine hydrochloride | sodium hydroxide | toluene and water | 185-186°C |
| 380 | benzoylchloride | 2,3-dimethylbenzoyl chloride | t-butylhydrazine hydrochloride | sodium hydroxide | toluene and water | |
| 381 | 4-toluoyl chloride | 2,3-dimethylbenzoyl chloride | t-butylhydrazine hydrochloride | sodium hydroxide | toluene and water | |
| 387 | benzoylchloride | 1-naphthoyl chloride | t-butylhydrazine hydrochloride | sodium hydroxide | toluene and water | 236-239°C |

| Example No. | Compound of Formula II | Compound of Formula V | Compound of Formula III | Base | Solvents | m.p. |
|---|---|---|---|---|---|---|
| 388 | 1-naphthoyl chloride | 3-toluoyl chloride | *t*-butylhydrazine hydrochloride | sodium hydroxide | toluene and water | 201-205°C |
| 389 | 4-isothiocyanato-benzoylchloride | 3-toluoyl chloride | *t*-butylhydrazine hydrochloride | sodium hydroxide | toluene and water | 150-153°C |
| 392 | 2-fluorobenzoyl chloride | 2-bromobenzoyl chloride | *t*-butylhydrazine hydrochlorider | sodium hydroxide | toluene and water | 184-186°C |
| 393 | 2-fluorobenzoyl chloride | 3-toluoyl chloride | *t*-butylhydrazine hydrochloride | sodium hydroxide | toluene and water | 158-160°C |
| 395 | 2-fluorobenzoyl chloride | 2-nitrobenzoyl chloride | *t*-butylhydrazine hydrochloride | sodium hydroxide | toluene and water | 192°C |
| 396 | 2-fluorobenzoyl chloride | benzoylchloride | *t*-butylhydrazine hydrochloride | sodium hydroxide | toluene and water | >250°C |
| 401 | 4-bromobenzoyl chloride | benzoylchloride | *t*-butylhydrazine hydrochloride | sodium hydroxide | toluene and water | >250°C |
| 402 | 2,3-dichloro-benzoylchloride | 2-bromobenzoyl chloride | *t*-butylhydrazine hydrochloride | sodium hydroxide | toluene and water | >250°C |
| 403 | 2,3-dichloro-benzoylchloride | benzoylchloride | *t*-butylhydrazine hydrochloride | sodium hydroxide | toluene and water | |
| 404 | 2-fluorobenzoyl chloride | 3,5-dimethyl-benzoylchloride | *t*-butylhydrazine hydrochloride | sodium hydroxide | toluene and water | >250°C |
| 405 | 2,3-dichloro-benzoylchloride | 3-toluoyl chloride | *t*-butylhydrazine hydrochloride | sodium hydroxide | toluene and water | >250°C |
| 406 | benzoylchloride | 2-naphthoyl chloride | *t*-butylhydrazine hydrochloride | sodium hydroxide | toluene and water | 215-222°C |

| Example No. | Compound of Formula II | Compound of Formula V | Compound of Formula III | Base | Solvents | m.p. |
|---|---|---|---|---|---|---|
| 407 | 2-naphthoyl chloride | 3-toluoyl chloride | t-butylhydrazine hydrochloride | sodium hydroxide | toluene and water | >240°C |
| 408 | benzoylchloride | 3-trifluoromethoxy-benzoylchloride | t-butylhydrazine hydrochloride | pyridine | pyridine | 140-144°C |
| 409 | 4-isothiocyanato-benzoylchloride | benzoylchloride | t-butylhydrazine hydrochloride | sodium hydroxide | toluene and water | >240°C |
| 410 | 2,6-difluoro-benzoylchloride | 2,4-difluoro-benzoylchloride | t-butylhydrazine hydrochloride | sodium hydroxide | toluene and water | >240°C |
| 411 | 2,6-difluoro-benzoylchloride | 3,5-dichloro-benzoylchloride | t-butylhydrazine hydrochloride | sodium hydroxide | toluene and water | 245-249°C |
| 412 | 4-toluoyl chloride | 3,5-bis-(trifluoro-methyl)benzoyl chloride | t-butylhydrazine hydrochloride | sodium hydroxide | toluene and water | 208°C |
| 413 | benzoylchloride | 3,5-bis-(trifluoro-methyl)benzoyl chloride | t-butylhydrazine hydrochloride | sodium hydroxide | toluene and water | 230-231°C |
| 419 | benzoylchloride | 3-chloro-4-fluoro-benzoylchloride | t-butylhydrazine hydrochloride | sodium hydroxide | toluene and water | 210-211°C |
| 420 | 4-chlorobenzoyl chloride | 3-chloro-4-fluoro-benzoylchloride | t-butylhydrazine hydrochloride | sodium hydroxide | toluene and water | 204-205°C |
| 422 | 4-chlorobenzoyl chloride | 3,5-bis-(trifluoro-methyl)benzoyl chloride | t-butylhydrazine hydrochloride | sodium hydroxide | toluene and water | |
| 423 | 2,3-dimethyl-benzoylchloride | 3,4-dichloro-benzoylchloride | t-butylhydrazine hydrochloride | sodium hydroxide | toluene and water | 208-209°C |

| Example No. | Compound of Formula II | Compound of Formula V | Compound of Formula III | Base | Solvents | m.p. |
|---|---|---|---|---|---|---|
| 424 | 4-toluoyl chloride | 3-chloro-2-methyl-benzoylchloride | t-butylhydrazine hydrochloride | sodium hydroxide | toluene and water | 208-212°C |
| 426 | 2,6-dimethyl-benzoylchloride | 3-toluoyl chloride | t-butylhydrazine hydrochloride | sodium hydroxide | toluene and water | 203-204°C |
| 427 | 2,6-dimethyl-benzoylchloride | 3,5-dimethyl-benzoylchloride | t-butylhydrazine hydrochloride | sodium hydroxide | toluene and water | 217-218°C |
| 428 | 2,3-dimethyl-benzoylchloride | 2-bromobenzoyl chloride | t-butylhydrazine hydrochloride | sodium hydroxide | toluene and water | 197-198°C |
| 429 | 2,3-dimethyl-benzoylchloride | 3,5-dimethyl-benzoylchloride | t-butylhydrazine hydrochloride | sodium hydroxide | toluene and water | 182-183°C |
| 430 | 2,3-dimethyl-benzoylchloride | 3-chlorobenzoyl chloride | t-butylhydrazine hydrochloride | sodium hydroxide | toluene and water | 211-212°C |
| 431 | 3-trifluoro-methoxybenzoyl chloride | benzoylchloride | t-butylhydrazine hydrochloride | sodium hydroxide | toluene and water | >240°C |
| 432 | 3-trifluoro-methoxybenzoyl chloride | 3-toluoyl chloride | t-butylhydrazine hydrochloride | sodium hydroxide | toluene and water | 148-150°C |
| 433 | 2,3-dimethyl-benzoylchloride | 2-methyl-3-chloro-benzoylchloride | t-butylhydrazine hydrochloride | sodium hydroxide | toluene and water | |
| 437 | 2,6-difluoro-benzoylchloride | 2-bromobenzoyl chloride | t-butylhydrazine hydrochloride | sodium hydroxide | toluene and water | 271-275°C |
| 438 | 2-methyl-3-chloro-benzoylchloride | 3,5-dimethyl-benzoylchloride | t-butylhydrazine hydrochloride | sodium hydroxide | toluene and water | >250°C |

| Example No. | Compound of Formula II | Compound of Formula V | Compound of Formula III | Base | Solvents | m.p. |
|---|---|---|---|---|---|---|
| 439 | 2-methyl-3-chloro-benzoylchloride | 3-chloro-4-fluoro-benzoylchloride | t-butylhydrazine hydrochloride | sodium hydroxide | toluene and water | 240-241°C |
| 440 | 2,3-dimethyl-benzoylchloride | 3-chloro-4-fluoro-benzoylchloride | t-butylhydrazine hydrochloride | sodium hydroxide | toluene and water | 187°C |
| 441 | 4-ethylbenzoyl chloride | 3-chloro-4-fluoro-benzoylchloride | t-butylhydrazine hydrochloride | sodium hydroxide | toluene and water | 154-157°C |
| 442 | 3,4-dimethyl-benzoylchloride | benzoylchloride | t-butylhydrazine hydrochloride | sodium hydroxide | toluene and water | 213-215°C |
| 443 | 3,4-dimethyl-benzoylchloride | 3-toluoyl chloride | t-butylhydrazine hydrochloride | sodium hydroxide | toluene and water | 176-177°C |
| 444 | 3,4-dimethyl-benzoylchloride | 2-chlorobenzoyl chloride | t-butylhydrazine hydrochloride | sodium hydroxide | toluene and water | 190°C |
| 445 | 3,4-dimethyl-benzoylchloride | 2,4-dichloro-benzoylchloride | t-butylhydrazine hydrochloride | sodium hydroxide | toluene and water | 123-125°C |
| 446 | 3,4-dimethyl-benzoylchloride | 2-bromobenzoyl chloride | t-butylhydrazine hydrochloride | sodium hydroxide | toluene and water | 185°C |
| 447 | 3,4-dimethyl-benzoylchloride | 3,5-dimethyl-benzoylchloride | t-butylhydrazine hydrochloride | sodium hydroxide | toluene and water | 190°C |
| 448 | 3,4-dimethyl-benzoylchloride | 2-nitrobenzoyl chloride | t-butylhydrazine hydrochloride | sodium hydroxide | toluene and water | 165°C |
| 450 | benzoylchloride | 3,4-dimethyl-benzoylchloride | t-butylhydrazine hydrochloride | sodium hydroxide | toluene and water | 198-200°C |
| 451 | 4-ethylbenzoyl chloride | 3,4-dimethyl-benzoylchloride | t-butylhydrazine hydrochloride | sodium hydroxide | toluene and water | 228-230°C |

| Example No. | Compound of Formula II | Compound of Formula V | Compound of Formula III | Base | Solvents | m.p. |
|---|---|---|---|---|---|---|
| 452 | 2-fluoro-6-chloro-benzoylchloride | 3-toluoyl chloride | t-butylhydrazine hydrochloride | sodium hydroxide | toluene and water | >230°C |
| 454 | 4-chloromethyl benzoylchloride | benzoylchloride | t-butylhydrazine hydrochloride | sodium hydroxide | toluene and water | >250°C |
| 455 | 2,3-dimethoxy-benzoylchloride | benzoylchloride | t-butylhydrazine hydrochloride | sodium hydroxide | toluene and water | >240°C |
| 456 | 2,3-dimethoxy-benzoylchloride | 4-chlorobenzoyl chloride | t-butylhydrazine hydrochloride | sodium hydroxide | toluene and water | 180°C decom-poses |
| 457 | 2,3-dimethoxy-benzoylchloride | 2-bromobenzoyl chloride | t-butylhydrazine hydrochloride | sodium hydroxide | toluene and water | 130-131°C |
| 459 | 4-t-butylbenzoyl chloride | 3,4-dimethyl-benzoylchloride | t-butylhydrazine hydrochloride | sodium hydroxide | toluene and water | 202-203°C |
| 460 | 4-t-butylbenzoyl chloride | 3,5-dimethyl-benzoylchloride | t-butylhydrazine hydrochloride | sodium hydroxide | toluene and water | 228°C |
| 461 | 4-t-butylbenzoyl chloride | 2,4-dichloro-benzoylchloride | t-butylhydrazine hydrochloride | sodium hydroxide | toluene and water | 238°C |
| 462 | 4-t-butylbenzoyl chloride | 2-nitrobenzoyl chloride | t-butylhydrazine hydrochloride | sodium bicar-bonate | toluene and water | 192-193°C |
| 463 | 4-t-butylbenzoyl chloride | 2-bromobenzoyl chloride | t-butylhydrazine hydrochloride | sodium hydroxide | toluene and water | 220°C |
| 464 | 4-t-butylbenzoyl chloride | 3-toluoyl chloride | t-butylhydrazine hydrochloride | sodium hydroxide | toluene and water | 185-187°C |

| Example No. | Compound of Formula II | Compound of Formula V | Compound of Formula III | Base | Solvents | m.p. |
|---|---|---|---|---|---|---|
| 465 | 4-t-butylbenzoyl chloride | 2-chlorobenzoyl chloride | t-butylhydrazine hydrochloride | sodium hydroxide | toluene and water | 220°C |
| 466 | 4-t-butylbenzoyl chloride | 3,4-dichloro-benzoylchloride | t-butylhydrazine hydrochloride | sodium hydroxide | toluene and water | 300°C |
| 467 | 3,4-dimethyl-benzoylchloride | 3,4-dichloro-benzoylchloride | t-butylhydrazine hydrochloride | sodium hydroxide | toluene and water | 207-208°C |
| 468 | 3,4-dimethyl-benzoylchloride | 4-fluorobenzoyl chloride | t-butylhydrazine hydrochloride | sodium hydroxide | toluene and water | 225°C |
| 469 | 4-t-butylbenzoyl chloride | 4-fluorobenzoyl chloride | t-butylhydrazine hydrochloride | sodium hydroxide | toluene and water | >300°C |
| 474 | 1-naphthoyl chloride | 2,4-dichloro-benzoylchloride | t-butylhydrazine hydrochloride | sodium hydroxide | toluene and water | 240-241°C |
| 475 | 1-naphthoyl chloride | 2-bromobenzoyl chloride | t-butylhydrazine hydrochloride | sodium hydroxide | toluene and water | 195-198°C |
| 476 | 2-methyl-3-nitro-benzoylchloride | benzoylchloride | t-butylhydrazine hydrochloride | sodium hydroxide | toluene and water | 216-217°C |
| 477 | 2-methyl-3-nitro-benzoylchloride | 3-toluoyl chloride | t-butylhydrazine hydrochloride | sodium hydroxide | toluene and water | >250°C |
| 478 | 2-methyl-3-nitro-benzoylchloride | 4-chlorobenzoyl chloride | t-butylhydrazine hydrochloride | sodium hydroxide | toluene and water | 244-246°C |
| 479 | 2-methyl-3-nitro-benzoylchloride | 2,4-dichloro-benzoylchloride | t-butylhydrazine hydrochloride | sodium hydroxide | toluene and water | 235°C dec. |
| 480 | 3-bromo-2-methyl-benzoylchloride | benzoylchloride | t-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 481 | 3-bromo-2-methyl-benzoylchloride | 3-toluoylchloride | _t_-butylhydrazine hydrochloride | sodium hydroxide | toluene and water | |
| 482 | 3-bromo-2-methyl-benzoylchloride | 4-chlorobenzoyl chloride | _t_-butylhydrazine hydrochloride | sodium hydroxide | toluene and water | |
| 483 | 3-bromo-2-methyl-benzoylchloride | 2,4-dichlorobenzoyl chloride | _t_-butylhydrazine hydrochloride | sodium hydroxide | toluene and water | |
| 485 | 2-toluoyl chloride | 2-bromobenzoyl chloride | _t_-butylhydrazine hydrochloride | sodium hydroxide | toluene and water | 192-193°C |
| 486 | 2-toluoyl chloride | 2-nitrobenzoyl chloride | _t_-butylhydrazine hydrochloride | sodium bicar-bonate | toluene and water | >200°C |
| 487 | 2-toluoyl chloride | 3-nitrobenzoyl chloride | _t_-butylhydrazine hydrochloride | sodium hydroxide | toluene and water | 232-235°C |
| 488 | 2-toluoyl chloride | 4-chlorobenzoyl chloride | _t_-butylhydrazine hydrochloride | sodium hydroxide | toluene and water | 241-242°C |
| 489 | 2-toluoyl chloride | 3-toluoyl chloride | _t_-butylhydrazine hydrochloride | sodium hydroxide | toluene and water | 204-208°C |
| 490 | 2-toluoyl chloride | 3,5-dimethyl-benzoylchloride | _t_-butylhydrazine hydrochloride | sodium hydroxide | toluene and water | 190°C |
| 491 | 2-toluoyl chloride | 3,4-dichloro-benzoylchloride | _t_-butylhydrazine hydrochloride | sodium hydroxide | toluene and water | 276-277°C |
| 492 | 2-toluoyl chloride | 3,5-dichloro-benzoylchloride | _t_-butylhydrazine hydrochloride | sodium hydroxide | toluene and water | 215-216°C |

| Example No. | Compound of Formula II | Compound of Formula V | Compound of Formula III | Base | Solvents | m.p. |
|---|---|---|---|---|---|---|
| 493 | 2-methyl-3-fluoro-chloride | benzoylchloride | t-butylhydrazine hydrochloride | sodium hydroxide | toluene and water | |
| 494 | 2-methyl-3-fluoro-benzoylchloride | 3-toluoyl chloride | t-butylhydrazine hydrochloride | sodium hydroxide | toluene and water | >240°C |
| 495 | 2-fluoro-6-chloro-benzoylchloride | benzoylchloride | t-butylhydrazine hydrochloride | sodium hydroxide | toluened and water | >240° |
| 496 | 2-fluoro-6-chloro-benzoylchloride | 2,4-dichloro-benzoylchloride | t-butylhydrazine hydrochloride | sodium hydroxide | toluene and water | >240°C |
| 497 | 2-fluoro-6-chloro-benzoylchloride | 4-fluorobenzoyl chloride | t-butylhydrazine hydrochloride | sodium hydroxide | toluene and water | >240°C |
| 498 | 4-(2-chloroethyl)-benzoylchloride | 3,5-dimethyl-benzoylchloride | t-butylhydrazine hydrochloride | sodium hydroxide | toluene and water | Oil |
| 499 | 2,4,6-trifluoro-benzoylchloride | benzoylchloride | t-butylhydrazine hydrochloride | sodium hydroxide | toluene and water | 247-250°C |
| 500 | 2,4,6-trifluoro-benzoylchloride | 2,4-dichloro-benzoylchloride | t-butylhydrazine hydrochloride | sodium hydroxide | toluene and water | 196-197°C |
| 501 | 2,4,6-trifluoro-benzoylchloride | 2-bromobenzoyl chloride | t-butylhydrazine hydrochloride | sodium hydroxide | toluene and water | 228-231°C |
| 502 | 2,4,6-trifluoro-benzoylchloride | 3,5-dimethyl-benzoylchloride | t-butylhydrazine hydrochloride | sodium hydroxide | toluene and water | 245 dec. |
| 503 | 2-nitro-3-chloro-benzoylchloride | benzoylchloride | t-butylhydrazine hydrochloride | sodium hydroxide | toluene and water | 196-197°C |

| Example No. | Compound of Formula II | Compound of Formula V | Compound of Formula III | Base | Solvents | m.p. |
|---|---|---|---|---|---|---|
| 504 | 2-nitro-3-chloro-benzoylchloride | 2-bromobenzoyl chloride | _t_-butylhydrazine hydrochloride | sodium hydroxide | toluene and water | 214-215°C |
| 505 | 2-nitro-3-chloro-benzoylchloride | 2-nitrobenzoyl chloride | _t_-butylhydrazine hydrochloride | sodium hydroxide | toluene and water | 200-201°C |
| 506 | 2-nitro-3-chloro-benzoylchloride | 3-toluoyl chloride | _t_-butylhydrazine hydrochloride | sodium hydroxide | toluene and water | 213°C |
| 507 | 2-nitro-3-chloro-benzoylchloride | 3-chlorobenzoyl chloride | _t_-butylhydrazine hydrochloride | sodium hydroxide | toluene and water | 222° |
| 508 | 2-nitro-3-chloro-benzoylchloride | 2,4-dichloro-benzoylchloride | _t_-butylhydrazine hydrochloride | sodium hydroxide | toluene and water | 206-207°C |
| 509 | 2-nitro-3-chloro-benzoylchloride | 3,5-dichloro-benzoylchloride | _t_-butylhydrazine hydrochloride | sodium hydroxide | toluene and water | 136-138°C |
| 510 | 2-nitro-3-chloro-benzoylchloride | 3,5-dimethyl-benzoylchloride | _t_-butylhydrazine hydrochloride | sodium hydroxide | toluene and water | 232°C |
| 511 | 4-ethylbenzoyl chloride | 2,3-difluoro-benzoylchloride | _t_-butylhydrazine hydrochloride | sodium hydroxide | toluene and water | 100°C |
| 512 | 4-ethylbenzoyl chloride | 2,3-dichloro-benzoylchloride | _t_-butylhydrazine hydrochloride | sodium hydroxide | toluene and water | 164°C |
| 513 | 2,3-difluoro-benzoylchloride | benzoylchloride | _t_-butylhydrazine hydrochloride | sodium hydroxide | toluene and water | 180°C |
| 514 | 2,3-dichloro-benzoylchloride | 2-nitrobenzoyl chloride | _t_-butylhydrazine hydrochloride | sodium hydroxide | toluene and water | 210°C |
| 515 | benzoylchloride | 2,3-difluoro-benzoylchloride | _t_-butylhydrazine hydrochloride | sodium hydroxide | toluene and water | 52-53°C |

| Example No. | Compound of Formula II | Compound of Formula V | Compound of Formula III | Base | Solvents | m.p. |
|---|---|---|---|---|---|---|
| 516 | 2,3-dichloro-benzoylchloride | 3,5-dimethyl-benzoylchloride | t-butylhydrazine hydrochloride | sodium hydroxide | toluene and water | 205°C |
| 517 | 2,3-dichloro-benzoylchloride | 2,4-dichloro-benzoylchloride | t-butylhydrazine hydrochloride | sodium hydroxide | toluene and water | 240°C |
| 518 | 2,3-dichloro-benzoylchloride | 3,5-dichloro-benzoylchloride | t-butylhydrazine hydrochloride | sodium hydroxide | toluene and water | 180°C |
| 519 | 2,3-dichloro-benzoylchloride | 3-chlorobenzoyl chloride | t-butylhydrazine hydrochloride | sodium hydroxide | toluene and water | 238°C |
| 520 | 2,3-dichloro-benzoylchloride | 2,3-dimethyl-benzoylchloride | t-butylhydrazine hydrochloride | sodium hydroxide | toluene and water | 201°C |
| 521 | 2,3-difluoro-benzoylchloride | 2-bromobenzoyl chloride | t-butylhydrazine hydrochloride | sodium hydroxide | toluene and water | 192°C |
| 522 | 2,3-dimethyl-benzoylchloride | 4-chlorobenzoyl chloride | t-butylhydrazine hydrochloride | sodium hydroxide | toluene and water | >220°C |
| 523 | 2,3-dimethyl-benzoylchloride | 2,4-dichloro-benzoylchloride | t-butylhydrazine hydrochloride | sodium hydroxide | toluene and water | 173°C |
| 524 | 2,3-dimethyl-benzoylchloride | 2,6-difluoro-benzoylchloride | t-butylhydrazine hydrochloride | sodium hydroxide | toluene and water | 212-213°C |
| 525 | 2,3-dimethyl-benzoylchloride | 2,4-difluoro-benzoylchloride | t-butylhydrazine hydrochloride | sodium hydroxide | toluene and water | 186-187°C |
| 526 | 2,3-dimethyl-benzoylchloride | 3-methoxybenzoyl chloride | t-butylhydrazine hydrochloride | sodium hydroxide | toluene and water | 189-190°C |
| 527 | 2,3-dimethyl-benzoylchloride | 2-methoxybenzoyl chloride | t-butylhydrazine hydrochloride | sodium hydroxide | toluene and water | 150-151°C |

| Example No. | Compound of Formula II | Compound of Formula V | Compound of Formula III | Base | Solvents | m.p. |
|---|---|---|---|---|---|---|
| 528 | 2,3-dimethyl-benzoylchloride | 2-toluoyl chloride | t-butylhydrazine hydrochloride | sodium hydroxide | toluene and water | 173-175°C |
| 529 | 2,3-dimethyl-benzoylchloride | 4-toluoyl chloride | t-butylhydrazine hydrochloride | sodium hydroxide | toluene and water | |
| 530 | 2-methyl-3-chloro-benzoylchloride | 2,4-dichlorobenzoyl chloride | t-butylhydrazine hydrochloride | sodium hydroxide | toluene and water | |
| 531 | 4-(2-chloro-ethyl)benzoyl chloride | benzoylchloride | t-butylhydrazine hydrochloride | sodium hydroxide | toluene and water | 148-150°C |
| 532 | 4-(2-chloro-ethyl)benzoyl chloride | 2,4-dichloro-benzoylchloride | t-butylhydrazine hydrochloride | sodium hydroxide | toluene and water | >240°C |
| 533 | 3-fluoro-4-methyl-benzoylchloride | 3,5-dimethyl-benzoylchloride | t-butylhydrazine hydrochloride | sodium hydroxide | toluene and water | 174-177°C |
| 534 | 3-fluoro-4-methyl-benzoylchloride | 3,5-difluoro-benzoylchloride | t-butylhydrazine hydrochloride | sodium hydroxide | toluene and water | 178-180°C |
| 535 | 3-fluoro-4-methyl-benzoylchloride | 3-toluoyl chloride | t-butylhydrazine hydrochloride | sodium hydroxide | toluene and water | 155-158°C |
| 536 | 3-fluoro-4-methyl-benzoylchloride | 3,5-dichloro-benzoylchloride | t-butylhydrazine hydrochloride | sodium hydroxide | toluene and water | >240°C |
| 537 | 3-fluoro-4-methyl-benzoylchloride | 2-nitrobenzoyl chloride | t-butylhydrazine hydrochloride | sodium hydroxide | toluene and water | 140-142°C |
| 538 | 3-fluoro-4-methyl-benzoylchloride | 2,4-dichloro-benzoylchloride | t-butylhydrazine hydrochloride | sodium hydroxide | toluene and water | Oil |

| Example No. | Compound of Formula II | Compound of Formula V | Compound of Formula III | Base | Solvents | m.p. |
|---|---|---|---|---|---|---|
| 539 | 4-ethylbenzoyl chloride | 3,5-difluoro-benzoylchloride | _t_-butylhydrazine hydrochloride | sodium hydroxide | toluene and water | >250°C |
| 540 | 2,3-dimethyl-benzoylchloride | 2-iodobenzoyl chloride | _t_-butylhydrazine hydrochloride | sodium hydroxide | toluene and water | Sol. |
| 542 | 2,6-difluoro-3-methylbenzoyl chloride | benzoylchloride | _t_-butylhydrazine hydrochloride | sodium hydroxide | toluene and water | Sol. |
| 543 | 2,6-difluoro-3-methylbenzoyl chloride | 3-toluoyl chloride | _t_-butylhydrazine hydrochloride | sodium hydroxide | toluene and water | >240°C |
| 544 | 2,6-difluoro-3-methylbenzoyl chloride | 2,4-dichloro-benzoylchloride | _t_-butylhydrazine hydrochloride | sodium hydroxide | toluene and water | >240°C |
| 545 | 4-chlorobenzoyl chloride | 2,3-dimethyl-benzoylchloride | _t_-butylhydrazine hydrochloride | sodium hydroxide | toluene and water | 220-221°C |
| 548 | 2-fluoro-6-chloro-benzoylchloride | 2,3-difluorobenzoyl chloride | _t_-butylhydrazine hydrochloride | sodium hydroxide | toluene and water | 186-187°C |
| 549 | 2,3-difluoro-benzoylchloride | 2-nitrobenzoyl chloride | _t_-butylhydrazine hydrochloride | sodium hydroxide | toluene and water | 185-187°C |
| 550 | 2,3-difluoro-benzoylchloride | 3-toluoyl chloride | _t_-butylhydrazine hydrochloride | sodium hydroxide | toluene and water | 185-186°C |
| 551 | 2,3-difluoro-benzoylchloride | 3,5-dimethyl-benzoylchloride | _t_-butylhydrazine hydrochloride | sodium hydroxide | toluene and water | 181-182°C |
| 552 | 2,3-difluoro-benzoylchloride | 2,4-dichloro-benzoylchloride | _t_-butylhydrazine hydrochloride | sodium hydroxide | toluene and water | 186°C |

| Example No. | Compound of Formula II | Compound of Formula V | Compound of Formula III | Base | Solvents | m.p. |
|---|---|---|---|---|---|---|
| 553 | 2,3-dimethyl-benzoylchloride | 2,3-difluoro-benzoylchloride | t-butylhydrazine hydrochloride | sodium hydroxide | toluene and water | 190°C |
| 554 | 2,3-dimethyl-benzoylchloride | 2,3-dichloro-benzoylchloride | t-butylhydrazine hydrochloride | sodium hydroxide | toluene and water | 164-165°C |
| 555 | 2,3-dimethyl-benzoylchloride | 3,4-dimethyl-benzoylchloride | t-butylhydrazine hydrochloride | sodium hydroxide | toluene and water | 190-191°C |
| 556 | 2,3-dimethyl-benzoylchloride | 2-nitrobenzoyl chloride | t-butylhydrazine hydrochloride | sodium hydroxide | toluene and water | 183-184°C |
| 557 | 4-ethylbenzoyl chloride | 2-methyl-5-chloro-benzoylchloride | t-butylhydrazine hydrochloride | sodium hydroxide | toluene and water | 180°C |
| 558 | 2,3-dimethyl-benzoylchloride | benzoylchloride | t-butylhydrazine hydrochloride | sodium hydroxide | toluene and water | 210-212°C |
| 559 | benzoylchloride | 2-methyl-5-chloro-benzoylchloride | t-butylhydrazine hydrochloride | sodium hydroxide | toluene and water | 250°C |
| 560 | 2,6-difluoro-benzoylchloride | 2-methyl-5-chloro-benzoylchloride | t-butylhydrazine hydrochloride | sodium hydroxide | toluene and water | 200°C |
| 561 | 2,3-dimethyl-benzoylchloride | 2-methyl-5-chloro-benzoylchloride | t-butylhydrazine hydrochloride | sodium hydroxide | toluene and water | 184°C |
| 562 | 2-methyl-3-chloro-benzoylchloride | 2-methyl-5-chloro-benzoylchloride | t-butylhydrazine hydrochloride | sodium hydroxide | toluene and water | 213°C |
| 563 | 2,6-difluoro-benzoylchloride | 2-chloro-5-methyl-benzoylchloride | t-butylhydrazine hydrochloride | sodium hydroxide | toluene and water | 184°C |
| 564 | 2,3-dimethyl-benzoylchloride | 2-chloro-5-methyl-benzoylchloride | t-butylhydrazine hydrochloride | sodium hydroxide | toluene and water | 174°C |

| Example No. | Compound of Formula II | Compound of Formula V | Compound of Formula III | Base | Solvents | m.p. |
|---|---|---|---|---|---|---|
| 565 | 2-methyl-3-chloro-benzoylchloride | 2-chloro-5-methyl-benzoylchloride | t-butylhydrazine hydrochloride | sodium hydroxide | toluene and water | 184°C |
| 566 | 4-chlorobenzoyl chloride | 2-methyl-5-chloro-benzoylchloride | t-butylhydrazine hydrochloride | sodium hydroxide | toluene and water | 217°C |
| 567 | 4-chlorobenzoyl chloride | 2-chloro-5-methyl-benzoylchloride | t-butylhydrazine hydrochloride | sodium hydroxide | toluene and water | 205°C |
| 568 | 2-fluoro-4-chloro-benzoylchloride | benzoylchloride | t-butylhydrazine hydrochloride | sodium hydroxide | toluene and water | 200°C |
| 569 | 2-fluoro-4-chloro-benzoylchloride | 3-toluoyl chloride | t-butylhydrazine hydrochloride | sodium hydroxide | toluene and water | 195°C |
| 570 | 2-fluoro-4-chloro-benzoylchloride | 3,5-dimethyl-benzoylchloride | t-butylhydrazine hydrochloride | sodium hydroxide | toluene and water | 300°C |
| 571 | 2-fluoro-4-chloro-benzoylchloride | 3,5-dichloro-benzoylchloride | t-butylhydrazine hydrochloride | sodium hydroxide | toluene and water | 170°C |
| 572 | 2-fluoro-4-chloro-benzoylchloride | 2-bromobenzoyl chloride | t-butylhydrazine hydrochloride | sodium hydroxide | toluene and water | 185°C |
| 573 | 2-fluoro-4-chloro-benzoylchloride | 2-nitrobenzoyl chloride | t-butylhydrazine hydrochloride | sodium hydroxide | toluene and water | 165°C |
| 574 | 2-chloro-3-methyl-benzoylchloride | 2,4-dichloro-benzoylchloride | t-butylhydrazine hydrochloride | sodium hydroxide | toluene and water | 200-205°C |
| 575 | 2-chloro-3-methyl-benzoylchloride | benzoylchloride | t-butylhydrazine hydrochloride | sodium hydroxide | toluene and water | 190-193°C |
| 576 | 2-chloro-3-methyl-benzoylchloride | 3-toluoyl chloride | t-butylhydrazine hydrochloride | sodium hydroxide | toluene and water | >250°C |

| Example No. | Compound of Formula II | Compound of Formula V | Compound of Formula III | Base | Solvents | m.p. |
|---|---|---|---|---|---|---|
| 577 | 2-chloro-3-methyl-benzoylchloride | 3,5-dimethyl-benzoylchloride | t-butylhydrazine hydrochloride | sodium hydroxide | toluene and water | 202-206°C |
| 578 | 2-chloro-3-methyl-benzoylchloride | 3,5-dichloro-benzoylchloride | t-butylhydrazine hydrochloride | sodium hydroxide | toluene and water | 190-193°C |
| 579 | 2-bromo-3-methyl-benzoylchloride | 3,5-dimethyl-benzoylchloride | t-butylhydrazine hydrochloride | sodium hydroxide | toluene and water | 210-212°C |
| 580 | 2-bromo-3-methyl-benzoylchloride | 2,4-dichloro-benzoylchloride | t-butylhydrazine hydrochloride | sodium hydroxide | toluene and water | |
| 581 | 2-bromo-3-methyl-benzoylchloride | 3-toluoyl chloride | t-butylhydrazine hydrochloride | sodium hydroxide | toluene and water | |
| 582 | 2-bromo-3-methyl-benzoylchloride | 3,5-dichloro-benzoylchloride | t-butylhydrazine hydrochloride | sodium hydroxide | toluene and water | |
| 583 | 2-bromo-3-methyl-benzoylchloride | 2-bromobenzoyl chloride | t-butylhydrazine hydrochloride | sodium hydroxide | toluene and water | |
| 584 | 2,3-dimethyl-benzoylchloride | 2-chlorobenzoyl chloride | t-butylhydrazine hydrochloride | sodium hydroxide | toluene and water | 208°C |
| 585 | 2,3-dimethyl-benzoylchloride | 2-trifluoromethyl-benzoylchloride | t-butylhydrazine hydrochloride | sodium hydroxide | toluene and water | |
| 586 | 2,3-dimethyl-benzoylchloride | 4-ethylbenzoyl chloride | t-butylhydrazine hydrochloride | sodium hydroxide | toluene and water | 190°C |
| 587 | 2,3-dimethyl-benzoylchloride | 3,5-dichloro-benzoylchloride | t-butylhydrazine hydrochloride | sodium hydroxide | toluene and water | 148-149°C |
| 588 | 2,6-difluoro-benzoylchloride | 3-chloro-4-fluoro-benzoylchloride | t-butylhydrazine hydrochloride | sodium hydroxide | toluene and water | 230°C |

| Example No. | Compound of Formula II | Compound of Formula V | Compound of Formula III | Base | Solvents | m.p. |
|---|---|---|---|---|---|---|
| 589 | 4-chlorobenzoyl chloride | 3,5-difluoro-benzoylchloride | t-butylhydrazine hydrochloride | sodium hydroxide | toluene and water | 175°C |
| 590 | 2,3-dimethyl-benzoylchloride | 3,5-difluoro-benzoylchloride | t-butylhydrazine hydrochloride | sodium hydroxide | toluene and water | 200°C |
| 591 | 2-methyl-3-chloro-benzoylchloride | 3,5-difluoro-benzoylchloride | t-butylhydrazine hydrochloride | sodium hydroxide | toluene and water | 220°C |
| 592 | benzoylchloride | 3,5-difluoro-benzoylchloride | t-butylhydrazine hydrochloride | sodium hydroxide | toluene and water | 201°C |
| 593 | 4-ethylbenzoyl chloride | 2,5-dimethyl-benzoylchloride | t-butylhydrazine hydrochloride | sodium hydroxide | toluene and water | 146-147°C |
| 594 | 2,6-difluoro-benzoylchloride | 3,5-difluoro-benzoylchloride | t-butylhydrazine hydrochloride | sodium hydroxide | toluene and water | 224-225°C |
| 595 | 2-methyl-3-chloro-benzoylchloride | 3,5-dichloro-benzoylchloride | t-butylhydrazine hydrochloride | sodium hydroxide | toluene and water | 218-219°C |
| 596 | 2,3-dimethyl-benzoylchloride | 2,5-dimethyl-benzoylchloride | t-butylhydrazine hydrochloride | sodium hydroxide | toluene and water | 180-183°C |
| 597 | 2-bromobenzoyl chloride | 3,5-dimethyl-benzoylchloride | t-butylhydrazine hydrochloride | sodium hydroxide | toluene and water | 235°C |
| 598 | 2-bromo-3-methyl-benzoylchloride | 3-chlorobenzoyl chloride | t-butylhydrazine hydrochloride | sodium hydroxide | toluene and water | |
| 599 | 2-chloro-3-methyl-benzoylchloride | 3-chlorobenzoyl chloride | t-butylhydrazine hydrochloride | sodium hydroxide | toluene and water | 205-206°C |
| 600 | 2-chloro-3-methyl-benzoylchloride | 3-chlorobenzoyl chloride | t-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 601 | 2-chloro-3-methyl-benzoylchloride | 2-nitrobenzoyl chloride | _t_-butylhydrazine hydrochloride | sodium hydroxide | toluene and water | 212°C |
| 602 | 2,3-dimethyl-benzoylchloride | 2-nitro-3-methyl-benzoylchloride | _t_-butylhydrazine hydrochloride | sodium hydroxide | toluene and water | low melting solid |
| 603 | 2,3-dimethyl-benzoylchloride | 2-nitro-5-methyl-benzoylchloride | _t_-butylhydrazine hydrochloride | sodium hydroxide | toluene and water | >240°C |
| 604 | 2,6-difluoro-benzoylchloride | benzoylchloride | _t_-butylhydrazine hydrochloride | sodium hydroxide | toluene and water | >230°C |
| 605 | 2,6-dimethyl-benzoylchloride | 2,4-dichloro-benzoylchloride | _t_-butylhydrazine hydrochloride | sodium hydroxide | toluene and water | 217-219°C |
| 606 | 2,4,6-trimethyl-benzoylchloride | 3,5-dimethyl-benzoylchloride | _t_-butylhydrazine hydrochloride | sodium hydroxide | toluene and water | 221-223°C |
| 607 | 2,4,6-trimethyl-benzoylchloride | 2,4-dichloro-benzoylchloride | _t_-butylhydrazine hydrochloride | sodium hydroxide | toluene and water | 175-177°C |
| 608 | 2,6-difluoro-benzoylchloride | 4-toluoyl chloride | _t_-butylhydrazine hydrochloride | sodium hydroxide | toluene and water | 183-185°C |
| 609 | 2,6-difluoro-benzoylchloride | 2,5-dichloro-benzoylchloride | _t_-butylhydrazine hydrochloride | sodium hydroxide | toluene and water | 236-238°C |
| 610 | 2,4,6-trimethyl-benzoylchloride | 4-chlorobenzoyl chloride | _t_-butylhydrazine hydrochloride | sodium hydroxide | toluene and water | >240°C |
| 611 | 2,4,6-trimethyl-benzoylchloride | 3-toluoyl chloride | _t_-butylhydrazine hydrochloride | sodium hydroxide | toluene and water | 181-183°C |

| Example No. | Compound of Formula II | Compound of Formula V | Compound of Formula III | Base | Solvents | m.p. |
|---|---|---|---|---|---|---|
| 612 | 2,4,6-trimethyl-benzoylchloride | 3,4-dichloro-benzoylchloride | t-butylhydrazine hydrochloride | sodium hydroxide | toluene and water | 234-235°C |
| 615 | 2,4-dimethyl-benzoylchloride | 3-toluoyl chloride | t-butylhydrazine hydrochloride | sodium hydroxide | toluene and water | 188-189°C |
| 616 | 2,4-dimethyl-benzoylchloride | 2,4-dichloro-benzoylchloride | t-butylhydrazine hydrochloride | sodium hydroxide | toluene and water | 163-165°C |
| 617 | 2,4-dimethyl-benzoylchloride | 3,5-dimethyl-benzoylchloride | t-butylhydrazine hydrochloride | sodium hydroxide | toluene and water | 138-140°C |
| 618 | 2,4-dimethyl-benzoylchloride | 3,5-dichloro-benzoylchloride | t-butylhydrazine hydrochloride | sodium hydroxide | toluene and water | 175-178°C |
| 619 | 2,4-dimethyl-benzoylchloride | 3-bromobenzoyl chloride | t-butylhydrazine hydrochloride | sodium hydroxide | toluene and water | 218-220°C |
| 620 | 2,4-dimethyl-benzoylchloride | 3,4-dichloro-benzoylchloride | t-butylhydrazine hydrochloride | sodium hydroxide | toluene and water | >250°C |
| 621 | 2-bromo-3-methyl-benzoylchloride | 2-nitrobenzoyl chloride | t-butylhydrazine hydrochloride | sodium hydroxide | toluene and water | |
| 622 | piperonyl chloride | benzoylchloride | t-butylhydrazine hydrochloride | sodium hydroxide | toluene and water | |
| 623 | piperonyl chloride | 4-chlorobenzoyl chloride | t-butylhydrazine hydrochloride | sodium hydroxide | toluene and water | |
| 624 | piperonyl chloride | 3-toluoyl chloride | t-butylhydrazine hydrochloride | sodium hydroxide | toluene and water | |
| 626 | 2-pyrrolobenzoyl chloride | benzoylchloride | t-butylhydrazine hydrochloride | sodium hydroxide | toluene and water | |

| Example No. | Compound of Formula II | Compound of Formula V | Compound of Formula III | Base | Solvents | m.p. |
|---|---|---|---|---|---|---|
| 627 | 2-pyrrolobenzoyl chloride | 3-toluoyl chloride | _t_-butylhydrazine hydrochloride | sodium hydroxide | toluene and water | |
| 628 | 2,3-dimethyl-benzoylchloride | 2-bromo-5-methyl-benzoylchloride | _t_-butylhydrazine hydrochloride | sodium hydroxide | toluene and water | |
| 629 | 2-methyl-3-chloro-benzoylchloride | 3-chlorobenzoyl chloride | _t_-butylhydrazine hydrochloride | sodium hydroxide | toluene and water | |
| 630 | 2-methyl-3-chloro-benzoylchloride | 4-fluorobenzoyl chloride | _t_-butylhydrazine hydrochloride | sodium hydroxide | toluene and water | |
| 631 | 2-methyl-3-chloro-benzoylchloride | 2-bromobenzoyl chloride | _t_-butylhydrazine hydrochloride | sodium hydroxide | toluene and water | |
| 633 | 2-methyl-3-chloro-benzoylchloride | 3,5-dimethyl-4-chlorobenzoyl chloride | _t_-butylhydrazine hydrochloride | sodium hydroxide | toluene and water | |

By following substantially the procedures in Example 44 and using the reactants shown below in Table IV, the products of Example Nos. 39, 46, 63, 110, 111, 112, 114, 115, 116, 120, 124, 127, 128, 129, 136, 143, 155 through 158, 185 through 189, 332, 336 through 340, 382, 383, 384, 399, 400, 414 through 418, 421, 434, 435, 436, 470, 471, 472, 546 and 547 were prepared.

TABLE IV

| Ex. No. | m.p. | Reactants | |
|---|---|---|---|
| 39 | 163-164°C | Compound of Formula VI: | Benzoylhydrazine |
| | | Compound of Formula VII: | Dimethyl ketone |
| | | Solvent: | Methanol |
| | | Catalyst: | Acetic acid |
| | | Compound of Formula VIII: | Benzoylhydrazone of acetone |
| | | Reducing Agent: | Sodium cyanoborohydride |
| | | Solvent: | Methanol |
| | | Catalyst: | Acetic acid |
| | | Compound of Formula IX (IV): | 2-isopropyl-1-benzoylhydrazine |
| | | Compound of Formula V: | Benzoylchloride |
| | | Base: | Sodium hydroxide |
| | | Solvent: | Toluene and water |
| 46 | glassy solid | Compound of Formula VI: | Benzoylhydrazine |
| | | Compound of Formula VII: | Methylethyl ketone |
| | | Solvent: | Methanol |
| | | Catalyst: | Acetic acid |
| | | Compound of Formula VIII: | Benzoylhydrazone of 2-butanone |
| | | Reducing Agent: | Sodium cyanoborohydride |
| | | Solvent: | Methanol |
| | | Catalyst: | Acetic acid |
| | | Compound of Formula IX (IV): | 2-sec-butyl-1-benzoylhydrazine |
| | | Compound of Formula V: | Benzoylchloride |
| | | Base: | Sodium hydroxide |
| | | Solvent: | Toluene and water |

| Ex. No. | m.p. | Reactants | |
|---|---|---|---|
| 63 | 239-242°C | Compound of Formula VI: | Benzoylhydrazine |
| | | Compound of Formula VII: | Methyl-t-butyl ketone |
| | | Solvent: | Methanol |
| | | Catalyst: | Acetic acid |
| | | Compound of Formula VIII: | Benzoylhydrazone of methyl-t-butylketone |
| | | Reducing Agent: | Sodium cyanoborohydride |
| | | Solvent: | Methanol |
| | | Catalyst: | Acetic acid |
| | | Compound of Formula IX (IV): | 2-(1,2,2-trimethylpropyl)-1-benzoylhydrazine |
| | | Compound of Formula V: | Benzoylchloride |
| | | Base: | Sodium hydroxide |
| | | Solvent: | Toluene and water |
| 110 | low melting solid | Compound of Formula VI: | Benzoylhydrazine |
| | | Compound of Formula VII: | 1,1,1-trimethylacetaldehyde |
| | | Solvent: | Methanol |
| | | Catalyst: | Acetic acid |
| | | Compound of Formula VIII: | Benzoylhydrazone of 1,1,1-trimethylacetaldehyde |
| | | Reducing Agent: | Sodium cyanoborohydride |
| | | Solvent: | Methanol |
| | | Catalyst: | Acetic acid |
| | | Compound of Formula IX (IV): | 2-neopentyl-1-benzoylhydrazine |
| | | Compound of Formula V: | 2-bromobenzoylchloride |
| | | Base: | Sodium hydroxide |
| | | Solvent: | Toluene and water |

| Ex. No. | m.p. | Reactants | |
|---|---|---|---|
| 111 | glassy solid | Compound of Formula VI: | Benzoylhydrazine |
| | | Compound of Formula VII: | 1,1,1-trimethylacetaldehyde |
| | | Solvent: | Methanol |
| | | Catalyst: | Acetic acid |
| | | Compound of Formula VIII: | Benzoylhydrazone of 1,1,1-trimethylacetaldehyde |
| | | Reducing Agent: | Sodium cyanoborohydride |
| | | Solvent: | Methanol |
| | | Catalyst: | Acetic acid |
| | | Compound of Formula IX (IV): | 2-neopentyl-1-benzoylhydrazine |
| | | Compound of Formula V: | 2-nitrobenzoylchloride |
| | | Base: | Sodium hydroxide |
| | | Solvent: | Toluene and water |
| 112 | glassy solid | Compound of Formula VI: | Benzoylhydrazine |
| | | Compound of Formula VII: | 1,1,1-trimethylacetaldehyde |
| | | Solvent: | Methanol |
| | | Catalyst: | Acetic acid |
| | | Compound of Formula VIII: | Benzoylhydrazone of 1,1,1-trimethylacetaldehyde |
| | | Reducing Agent: | Sodium cyanoborohydride |
| | | Solvent: | Methanol |
| | | Catalyst: | Acetic acid |
| | | Compound of Formula IX (IV): | 2-neopentyl-1-benzoylhydrazine |
| | | Compound of Formula V: | 2-anisoyl chloride |
| | | Base: | Sodium hydroxide |
| | | Solvent: | Toluene and water |

| Ex. No. | m.p. | Reactants | |
|---|---|---|---|
| 114 | 161-163°C | Compound of Formula VI: | Benzoylhydrazine |
| | | Compound of Formula VII: | Isobutyraldehyde |
| | | Solvent: | Methanol |
| | | Catalyst: | Acetic acid |
| | | Compound of Formula VIII: | Benzoylhydrazone of isobutyr-aldehyde |
| | | Reducing Agent: | Sodium cyanoborohydride |
| | | Solvent: | Methanol |
| | | Catalyst: | Acetic Acid |
| | | Compound of Formula IX (IV): | 2-isobutyl-1-benzoylhydrazine |
| | | Compound of Formula V: | Benzoylchloride |
| | | Base: | Sodium hydroxide |
| | | Solvent: | Toluene and water |
| 115 | glassy solid | Compound of Formula VI: | Benzoylhydrazine |
| | | Compound of Formula VII: | Acetone |
| | | Solvent: | Methanol |
| | | Catalyst: | Acetic acid |
| | | Compound of Formula VIII: | Benzoylhydrazone of acetone |
| | | Reducing Agent: | Sodium cyanoborohydride |
| | | Solvent: | Methanol |
| | | Catalyst: | Acetic acid |
| | | Compound of Formula IX (IV): | 2-isopropyl-1-benzoylhydrazine |
| | | Compound of Formula V: | 2-bromobenzoylchloride |
| | | Base: | Sodium hydroxide |
| | | Solvent: | Toluene and water |

| Ex. No. | m.p. | Reactants | |
|---|---|---|---|
| 116 | 175-178°C | Compound of Formula VI: | Benzoylhydrazine |
| | | Compound of Formula VII: | Acetone |
| | | Solvent: | Methanol |
| | | Catalyst: | Acetic acid |
| | | Compound of Formula VIII: | Benzoylhydrazone of acetone |
| | | Reducing Agent: | Sodium cyanoborohydride |
| | | Solvent: | Methanol |
| | | Catalyst: | Acetic acid |
| | | Compound of Formula IX (IV): | 2-isopropyl-1-benzoylhydrazine |
| | | Compound of Formula V: | 3,4-dichlorobenzoylchloride |
| | | Base: | Sodium hydroxide |
| | | Solvent: | Toluene and water |
| 120 | >250°C | Compound of Formula VI: | Benzoylhydrazine |
| | | Compound of Formula VII: | Dicyclopropylketone |
| | | Solvent: | Methanol |
| | | Catalyst: | Acetic Acid |
| | | Compound of Formula VIII: | Benzoylhydrazone of dicyclopropylketone |
| | | Reducing Agent: | Sodium cyanoborohydride |
| | | Solvent: | Methanol |
| | | Catalyst: | Acetic acid |
| | | Compound of Formula IX (IV): | 2-dicyclopropylmethyl-1-benzoylhydrazine |
| | | Compound of Formula V: | Benzoylchloride |
| | | Base: | Sodium hydroxide |
| | | Solvent: | Toluene and water |

| Ex. No. | m.p. | Reactants | |
|---|---|---|---|
| 124 | glassy solid | Compound of Formula VI: | Benzoylhydrazine |
| | | Compound of Formula VII: | Methyl-t-butyl ketone |
| | | Solvent: | Methanol |
| | | Catalyst: | Acetic acid |
| | | Compound of Formula VIII: | Benzoylhydrazone of methyl-t-butylketone |
| | | Reducing Agent: | Sodium cyanoborohydride |
| | | Solvent: | Methanol |
| | | Catalyst: | Acetic acid |
| | | Compound of Formula IX (IV): | 2-(1,2,2-trimethylpropyl)-1-benzoylhydrazine |
| | | Compound of Formula V: | 2-nitrobenzoylchloride |
| | | Base: | Sodium hydroxide |
| | | Solvent: | Toluene and water |
| 127 | 239-242°C | Compound of Formula VI: | Benzoylhydrazine |
| | | Compound of Formula VII: | Methyl-t-butyl ketone |
| | | Solvent: | Methanol |
| | | Catalyst: | Acetic acid |
| | | Compound of Formula VIII: | Benzoylhydrazone of methyl-t-butylketone |
| | | Reducing Agent: | Sodium cyanoborohydride |
| | | Solvent: | Methanol |
| | | Catalyst: | Acetic acid |
| | | Compound of Formula IX (IV): | 2-(1,2,2-trimethylpropyl)-1-benzoylhydrazine |
| | | Compound of Formula V: | Benzoylchloride |
| | | Base: | Sodium hydroxide |
| | | Solvent: | Toluene and water |

| Ex. No. | m.p. | Reactants | |
|---|---|---|---|
| 128 | 175-177°C | Compound of Formula VI: | Benzoylhydrazine |
| | | Compound of Formula VII: | Diisopropyl ketone |
| | | Solvent: | Methanol |
| | | Catalyst: | Acetic acid |
| | | Compound of Formula VIII: | Benzoylhydrazone of diisopropyl-ketone |
| | | Reducing Agent: | Sodium cyanoborohydride |
| | | Solvent: | Methanol |
| | | Catalyst: | Acetic acid |
| | | Compound of Formula IX (IV): | 2-diisopropylmethyl-1-benzoyl hydrazine |
| | | Compound of Formula V: | Benzoylchloride |
| | | Base: | Sodium hydroxide |
| | | Solvent: | Toluene and water |
| 129 | >250°C | Compound of Formula VI: | Benzoylhydrazine |
| | | Compound of Formula VII: | Cyclopropylmethyl ketone |
| | | Solvent: | Methanol |
| | | Catalyst: | Acetic acid |
| | | Compound of Formula VIII: | Benzoylhydrazone of cyclopropyl-methylketone |
| | | Reducing Agent: | Sodium cyanoborohydride |
| | | Solvent: | Methanol |
| | | Catalyst: | Acetic acid |
| | | Compound of Formula IX (IV): | 2-(1-cyclopropylethyl)-1-benzoylhydrazine |
| | | Compound of Formula V: | Benzoylchloride |
| | | Base: | Sodium hydroxide |
| | | Solvent: | Toluene and water |

| Ex. No. | m.p. | Reactants | |
|---|---|---|---|
| 136 | 154-155.5°C | Compound of Formula VI: | Benzoylhydrazine |
| | | Compound of Formula VII: | Methyl-t-butylketone |
| | | Solvent: | Methanol |
| | | Catalyst: | Acetic acid |
| | | Compound of Formula VIII: | Benzoylhydrazone of methyl-t-butylketone |
| | | Reducing Agent: | Sodium cyanoborohydride |
| | | Solvent: | Methanol |
| | | Catalyst: | Acetic acid |
| | | Compound of Formula IX (IV): | 2-(1-methyl)neopentyl-1-benzoylhydrazine |
| | | Compound of Formula V: | 2-bromobenzoylchloride |
| | | Base: | Sodium hydroxide |
| | | Solvent: | Toluene and water |
| 143 | 155°C | Compound of Formula VI: | Benzoylhydrazine |
| | | Compound of Formula VII: | Methyl cyclohexyl ketone |
| | | Solvent: | Methanol |
| | | Catalyst: | Acetic acid |
| | | Compound of Formula VIII: | Benzoylhydrazone of methyl cyclohexyl ketone |
| | | Reducing Agent: | Sodium cyanoborohydride |
| | | Solvent: | Methanol |
| | | Catalyst: | Acetic acid |
| | | Compound of Formula IX: | 1-benzoyl-2-(1-cyclohexylethyl)hydrazine |
| | | Compound of Formula V: | benzoylchloride |
| | | Base: | Sodium hydroxide |
| | | Solvent: | Toluene and water |

| Ex. No. | m.p. | Reactants | |
|---|---|---|---|
| 155 | 165°C | Compound of Formula VI: | Benzoylhydrazine |
| | | Compound of Formula VII: | Acetone |
| | | Solvent: | Methanol |
| | | Catalyst: | Acetic acid |
| | | Compound of Formula VIII: | Benzoylhydrazone of acetone |
| | | Reducing Agent: | Sodium cyanoborohydride |
| | | Solvent: | Methanol |
| | | Catalyst: | Acetic acid |
| | | Compound of Formula IX: | 1-benzoyl-2-isopropyl hydrazine |
| | | Compound of Formula V: | 2,6-dichlorobenzoylchloride |
| | | Base: | Sodium hydroxide |
| | | Solvent: | Toluene and water |
| 156 | Low melting solid | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 3,4-dichlorobenzoylchloride |
| | | Base: | Sodium hydroxide |
| | | Solvent: | Toluene and water |

| Ex. No. | m.p. | Reactants | |
|---|---|---|---|
| 157 | 246-249°C | Compound of Formula VI: | Benzoylhydrazine |
| | | Compound of Formula VII: | Pinacolone |
| | | Solvent: | Methanol |
| | | Catalyst: | Acetic acid |
| | | Compound of Formula VIII: | Benzoylhydrazone of pinacolone |
| | | Reducing Agent: | Sodium cyanoborohydride |
| | | Solvent: | Methanol |
| | | Catalyst: | Acetic acid |
| | | Compound of Formula IX: | 1-benzoyl-2-(1,2,2-trimethyl-propyl)hydrazine |
| | | Compound of Formula V: | 4-cyanobenzoylchloride |
| | | Base: | Sodium hydroxide |
| | | Solvent: | Toluene and water |
| 158 | 212-214°C | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 4-ethylbenzoylchloride |
| | | Base: | Sodium hydroxide |
| | | Solvent: | Toluene and water |

| Ex. No. | m.p. | Reactants | |
|---|---|---|---|
| 185 | >250°C | Compound of Formula VI: | Benzoylhydrazine |
| | | Compound of Formula VII: | Pinacolone |
| | | Solvent: | Methanol |
| | | Catalyst: | Acetic acid |
| | | Compound of Formula VIII: | Benzoylhydrazone of pinacolone |
| | | Reducing Agent: | Sodium cyanoborohydride |
| | | Solvent: | Methanol |
| | | Catalyst: | Acetic acid |
| | | Compound of Formula IX: | 1-benzoyl-2-(1,2,2-trimethyl-propyl)hydrazine |
| | | Compound of Formula V: | 3-nitrobenzoylchloride |
| | | Base: | Sodium hydroxide |
| | | Solvent: | Toluene and water |
| 186 | >250°C | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 3-nitrobenzoylchloride |
| | | Base: | Sodium hydroxide |
| | | Solvent: | Toluene and water |

| Ex. No. | m.p. | Reactants | |
|---|---|---|---|
| 187 | 187-190°C | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 3-toluoyl chloride |
| | | Base: | Sodium hydroxide |
| | | Solvent: | Toluene and water |
| 188 | 245-250°C | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 4-chlorobenzoylchloride |
| | | Base: | Sodium hydroxide |
| | | Solvent: | Toluene and water |

| Ex. No. | m.p. | Reactants | |
|---|---|---|---|
| 189 | Glass | Compound of Formula VI: | Benzoylhydrazine |
| | | Compound of Formula VII: | Trimethylacetaldehyde |
| | | Solvent: | Methanol |
| | | Catalyst: | Acetic acid |
| | | Compound of Formula VIII: | Benzoylhydrazone of trimethyl acetaldehyde |
| | | Reducing Agent: | Sodium cyanoborohydride |
| | | Solvent: | Methanol |
| | | Catalyst: | Acetic acid |
| | | Compound of Formula IX: | 1-benzoyl-2-(2,2-dimethylpropyl) hydrazine |
| | | Compound of Formula V: | 2,4-dinitrobenzoylchloride |
| | | Base: | Sodium hydroxide |
| | | Solvent: | Toluene and water |
| 332 | 149°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-nitrobenzoylchloride |
| | | Base: | Sodium hydroxide |
| | | Solvent: | Toluene and water |

| Ex. No. | m.p. | Reactants | |
|---|---|---|---|
| 336 | 127°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 3,5-dimethylbenzoylchloride |
| | | Base: | Sodium hydroxide |
| | | Solvent: | Toluene and water |
| 337 | 90°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-nitro-3-methyl benzoyl chloride |
| | | Base: | Sodium hydroxide |
| | | Solvent: | Toluene and water |

| Ex. No. | m.p. | Reactants | |
|---|---|---|---|
| 338 | 158°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-nitro-5-methylbenzoyl chloride |
| | | Base: | Sodium hydroxide |
| | | Solvent: | Toluene and water |
| 339 | 180°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 3-toluoyl chloride |
| | | Base: | Sodium hydroxide |
| | | Solvent: | Toluene and water |

| Ex. No. | m.p. | Reactants | |
|---|---|---|---|
| 340 | 180°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-iodobenzoyl chloride |
| | | Base: | Sodium hydroxide |
| | | Solvent: | Toluene and water |
| 382 | 150°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-toluoyl chloride |
| | | Base: | Sodium hydroxide |
| | | Solvent: | Toluene and water |

| Ex. No. | m.p. | Reactants | |
|---|---|---|---|
| 383 | 165°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | 2-trifluoromethylbenzoyl chloride |
| | | Base: | Sodium hydroxide |
| | | Solvent: | Toluene and water |
| 384 | 151°C | Compound of Formula VI: | 4-toluoylhydrazine |
| | | Compound of Formula VII: | Pinacolone |
| | | Solvent: | Methanol |
| | | Catalyst: | Acetic acid |
| | | Compound of Formula VIII: | 4-toluoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(1,2,2-trimethylpropyl)hydrazine |
| | | Compound of Formula V: | benzoyl chloride |
| | | Base: | Sodium hydroxide |
| | | Solvent: | Toluene and water |

| Ex. No. | m.p. | Reactants | |
|---|---|---|---|
| 399 | 166°C | Compound of Formula VI: | 4-toluoylhydrazine |
| | | Compound of Formula VII: | 4,4-dimethyl-2-pentanone |
| | | Solvent: | Methanol |
| | | Catalyst: | Acetic acid |
| | | Compound of Formula VIII: | 4-toluoyl hydrazone of 3,4-dimethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(4,4-dimethyl-2-pentyl)hydrazine |
| | | Compound of Formula V: | 3,5-dimethylbenzoyl chloride |
| | | Base: | Sodium hydroxide |
| | | Solvent: | Toluene and water |
| 400 | 170°C | Compound of Formula VI: | 4-toluoylhydrazine |
| | | Compound of Formula VII: | 4,4-dimethyl-2-pentanone |
| | | Solvent: | Methanol |
| | | Catalyst: | Acetic acid |
| | | Compound of Formula VIII: | 4-toluoyl hydrazone of 4,4-dimethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(4,4-dimethyl-2-pentyl)hydrazine |
| | | Compound of Formula V: | 3,4-dichlorobenzoyl chloride |
| | | Base: | Sodium hydroxide |
| | | Solvent: | Toluene and water |

| Ex. No. | m.p. | Reactants | |
|---|---|---|---|
| 414 | 145°C | Compound of Formula VI: | 4-toluoylhydrazine |
| | | Compound of Formula VII: | 3-methyl-3-ethyl-2-pentanone |
| | | Solvent: | Methanol |
| | | Catalyst: | Acetic acid |
| | | Compound of Formula VIII: | 4-toluoyl hydrazone of 3-methyl-3-ethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(3-methyl-3-ethylpent-2-yl)hydrazine |
| | | Compound of Formula V: | benzoyl chloride |
| | | Base: | Sodium hydroxide |
| | | Solvent: | Toluene and water |
| 415 | 130°C | Compound of Formula VI: | 4-toluoylhydrazine |
| | | Compound of Formula VII: | 3-methyl-3-ethyl-2-pentanone |
| | | Solvent: | Methanol |
| | | Catalyst: | Acetic acid |
| | | Compound of Formula VIII: | 4-toluoyl hydrazone of 3-methyl-3-ethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(3-methyl-3-ethylpent-2-yl)hydrazine |
| | | Compound of Formula V: | 3,5-dimethylbenzoyl chloride |
| | | Base: | Sodium hydroxide |
| | | Solvent: | Toluene and water |

| Ex. No. | m.p. | Reactants | |
|---|---|---|---|
| 416 | 148°C | Compound of Formula VI: | 4-toluoylhydrazine |
| | | Compound of Formula VII: | 3-methyl-3-ethyl-2-pentanone |
| | | Solvent: | Methanol |
| | | Catalyst: | Acetic acid |
| | | Compound of Formula VIII: | 4-toluoyl hydrazone of 3-methyl-3-ethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(3-methyl-3-ethylpent-2-yl)hydrazine |
| | | Compound of Formula V: | 2-nitrobenzoyl chloride |
| | | Base: | Sodium hydroxide |
| | | Solvent: | Toluene and water |
| 417 | 171°C | Compound of Formula VI: | 4-ethylbenzoyl hydrazine |
| | | Compound of Formula VII: | Pinacolone |
| | | Solvent: | Methanol |
| | | Catalyst: | Acetic acid |
| | | Compound of Formula VIII: | 4-ethylbenzoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-ethylbenzoyl)-2-(1,2,2-trimethylpropyl)hydrazine |
| | | Compound of Formula V: | 3,5-dimethylbenzoyl chloride |
| | | Base: | Sodium hydroxide |
| | | Solvent: | Toluene and water |

| Ex. No. | m.p. | Reactants | |
|---|---|---|---|
| 418 | Glass | Compound of Formula VI: | 4-ethylbenzoyl hydrazine |
| | | Compound of Formula VII: | Pinacolone |
| | | Solvent: | Methanol |
| | | Catalyst: | Acetic acid |
| | | Compound of Formula VIII: | 4-ethylbenzoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-ethylbenzoyl)-2-(1,2,2-trimethylpropyl)hydrazine |
| | | Compound of Formula V: | 2-nitro-5-methylbenzoyl chloride |
| | | Base: | Sodium hydroxide |
| | | Solvent: | Toluene and water |
| 421 | Oil | Compound of Formula VI: | 4-ethylbenzoyl hydrazine |
| | | Compound of Formula VII: | Acetone |
| | | Solvent: | Methanol |
| | | Catalyst: | Acetic acid |
| | | Compound of Formula VIII: | 4-ethylbenzoyl hydrazone of acetone |
| | | Reducing Agent: | Sodium cyanoborohydride |
| | | Solvent: | Methanol |
| | | Catalyst: | Acetic acid |
| | | Compound of Formula IX: | 1-(4-ethylbenzoyl)-2-isopropyl hydrazine |
| | | Compound of Formula V: | 3,5-dimethylbenzoyl chloride |
| | | Base: | Sodium hydroxide |
| | | Solvent: | Toluene and water |

| Ex. No. | m.p. | Reactants | |
|---|---|---|---|
| 434 | 125°C | Compound of Formula VI: | 4-ethylbenzoyl hydrazine |
| | | Compound of Formula VII: | Pinacolone |
| | | Solvent: | Methanol |
| | | Catalyst: | Acetic acid |
| | | Compound of Formula VIII: | 4-ethylbenzoyl hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(4-ethylbenzoyl)-2-(1,2,2-trimethylpropyl)hydrazine |
| | | Compound of Formula V: | 2,4-dichlorobenzoyl chloride |
| | | Base: | Sodium hydroxide |
| | | Solvent: | Toluene and water |
| 435 | 110°C | Compound of Formula VI: | 4-toluoylhydrazine |
| | | Compound of Formula VII: | 3-methyl-3-ethyl-2-pentanone |
| | | Solvent: | Methanol |
| | | Catalyst: | Acetic acid |
| | | Compound of Formula VIII: | 4-toluoylhydrazone of 3-methyl -3-ethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(3-methyl -3-ethylpent-2-yl)hydrazine |
| | | Compound of Formula V: | 2-nitro-5-methylbenzoyl chloride |
| | | Base: | Sodium hydroxide |
| | | Solvent: | Toluene and water |

| Ex. No. | m.p. | Reactants | |
|---|---|---|---|
| 436 | 105°C | Compound of Formula VI: | 4-toluoylhydrazine |
| | | Compound of Formula VII: | 3-methyl-3-ethyl-2-pentanone |
| | | Solvent: | Methanol |
| | | Catalyst: | Acetic acid |
| | | Compound of Formula VIII: | 4-toluoylhydrazone of 3-methyl -3-ethyl-2-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(3-methyl -3-ethylpent-2-yl)hydrazine |
| | | Compound of Formula V: | 2-nitro-3-methylbenzoyl chloride |
| | | Base: | Sodium hydroxide |
| | | Solvent: | Toluene and water |
| 453 | | Compound of Formula VI: | 4-toluoylhydrazine |
| | | Compound of Formula VII: | 2,2-dimethyl-3-pentanone |
| | | Solvent: | Methanol |
| | | Catalyst: | Acetic acid |
| | | Compound of Formula VIII: | 4-toluoylhydrazone of 2,2-dimethyl-3-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(2,2-dimethyl-pent-3-yl)hydrazine |
| | | Compound of Formula V: | Benzoyl chloride |
| | | Base: | Sodium hydroxide |
| | | Solvent: | Toluene and water |

| Ex. No. | m.p. | Reactants | |
|---|---|---|---|
| 470 | 160°C | Compound of Formula VI: | 4-toluoylhydrazine |
| | | Compound of Formula VII: | 2,2-dimethyl-3-pentanone |
| | | Solvent: | Methanol |
| | | Catalyst: | Acetic acid |
| | | Compound of Formula VIII: | 4-toluoylhydrazone of 2,2-dimethyl-3-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(2,2-dimethylpent-3-yl)hydrazine |
| | | Compound of Formula V: | 2-nitrobenzoyl chloride |
| | | Base: | Sodium hydroxide |
| | | Solvent: | Toluene and water |
| 471 | | Compound of Formula VI: | 4-toluoylhydrazine |
| | | Compound of Formula VII: | 2,2-dimethyl-3-butanone |
| | | Solvent: | Methanol |
| | | Catalyst: | Acetic acid |
| | | Compound of Formula VIII: | 4-toluoylhydrazone of 2,2-dimethyl-3-butanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(2,2-dimethylbut-3-yl)hydrazine |
| | | Compound of Formula V: | 2-nitro-5-methylbenzoyl chloride |
| | | Base: | Sodium hydroxide |
| | | Solvent: | Toluene and water |

| Ex. No. | m.p. | Reactants | |
|---|---|---|---|
| 472 | 170°C | Compound of Formula VI: | 4-toluoylhydrazine |
| | | Compound of Formula VII: | 2,2-dimethyl-3-pentanone |
| | | Solvent: | Methanol |
| | | Catalyst: | Acetic acid |
| | | Compound of Formula VIII: | 4-toluoylhydrazone of 2,2-dimethyl-3-pentanone |
| | | Reducing Agent: | Sodium cyanoborohydride |
| | | Solvent: | Methanol |
| | | Catalyst: | Acetic acid |
| | | Compound of Formula IX: | 1-(4-toluoyl)-2-(2,2-dimethylpent-3-yl)hydrazine |
| | | Compound of Formula V: | 3,5-dimethylbenzoyl chloride |
| | | Base: | Sodium hydroxide |
| | | Solvent: | Toluene and water |
| 546 | 171°C | Compound of Formula VI: | 2,3-dimethylbenzoyl hydrazine |
| | | Compound of Formula VII: | Pinacolone |
| | | Solvent: | Methanol |
| | | Catalyst: | Acetic acid |
| | | Compound of Formula VIII: | (2,3-dimethylbenzoyl)hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(2,3-dimethylbenzoyl)-2-(1,2,2-trimethylpropyl) hydrazine |
| | | Compound of Formula V: | 3,5-dimethylbenzoyl chloride |
| | | Base: | Sodium hydroxide |
| | | Solvent: | Toluene and water |

| Ex. No. | m.p. | Reactants | |
|---|---|---|---|
| 547 | 160°C | Compound of Formula VI: | 2,3-dimethylbenzoyl hydrazine |
| | | Compound of Formula VII: | Pinacolone |
| | | Solvent: | Methanol |
| | | Catalyst: | Acetic acid |
| | | Compound of Formula VIII: | (2,3-dimethylbenzoyl)hydrazone of pinacolone |
| | | Reducing Agent: | Sodium cyanoborohydride |
| | | Solvent: | Methanol |
| | | Catalyst: | Acetic acid |
| | | Compound of Formula IX: | 1-(2,3-dimethylbenzoyl)-(1,2,2-trimethylpropyl) hydrazine |
| | | Compound of Formula V: | 3-toluoyl chloride |
| | | Base: | Sodium hydroxide |
| | | Solvent: | Toluene and water |

By following substantially the procedures in Example 220 and using the reactants shown below in Table V, the products of Examples 171, 172, 191, 192, 193, 212, 213, 221 through 223, 232, 233, 293, 326, 331, 379, 397, 398, 425 and 458 are prepared.

Table V

| Ex. No. | Compound of Formula XI | Compound of Formula XII | Base | Solvent | m.p. |
|---|---|---|---|---|---|
| 168 | 3,4-dimethoxy-benzoyl chloride | N'-t-butyl-N'-benzoylhydrazine | sodium hydroxide | toluene and water | |
| 170 | 2-chloromethyl-benzoyl chloride | N'-t-butyl-N'-benzoyl hydrazine | sodium hydroxide | toluene and water | 198-199°C |
| 171 | 4-n-propylbenzoyl chloride | N'-t-butyl-N'-benzoyl hydrazine | sodium hydroxide | toluene and water | 210°C |
| 172 | 2-nitrobenzoyl chloride | N'-t-butyl-N'-benzoyl hydrazine | sodium hydroxide | toluene and water | 215°C |
| 191 | 3,4-dichloro-benzoyl chloride | N'-t-butyl-N'-(4-chlorobenzoyl) hydrazine | sodium hydroxide | toluene and water | 238°C |
| 192 | 4-n-heptyl-benzoyl chloride | N'-t-butyl-N'-(4-chlorobenzoyl) hydrazine | sodium hydroxide | toluene and water | 135°C |
| 193 | 4-n-propyl-benzoyl chloride | N'-t-butyl-N'-(4-chlorobenzoyl) hydrazine | sodium hydroxide | toluene and water | 163°C |
| 212 | 2-fluoro-benzoyl chloride | N'-t-butyl-N'-(4-chlorobenzoyl) hydrazine | sodium hydroxide | toluene and water | 215°C |
| 213 | 2,4-dichloro-benzoyl chloride | N'-t-butyl-N'-(4-chlorobenzoyl) hydrazine | sodium hydroxide | toluene and water | 247°C |
| 221 | 3-nitrobenzoyl chloride | N'-t-butyl-N'-benzoylhydrazine | sodium hydroxide | toluene and water | 136-139°C |
| 222 | 2,6-dichloro-benzoyl chloride | N'-t-butyl-N'-benzoylhydrazine | sodium hydroxide | toluene and water | 256-258°C |

| Ex. No. | Compound of Formula XI | Compound of Formula XII | Base | Solvent | m.p. |
|---|---|---|---|---|---|
| 223 | 2,4-difluoro-benzoyl chloride | N'-t-butyl-N'-benzoylhydrazine | sodium hydroxide | toluene and water | 202-205°C |
| 232 | 4-nitrobenzoyl chloride | N'-t-butyl-N'-benzoylhydrazine | sodium hydroxide | toluene and water | solid |
| 233 | 4-cyanobenzoyl chloride | N'-t-butyl-N'-benzoylhydrazine | sodium hydroxide | toluene and water | solid |
| 293 | 2-chloromethyl-benzoyl chloride | N'-t-butyl-N'-(4-chlorobenzoyl)-hydrazine | sodium hydroxide | toluene and water | 224°C |
| 326 | 3-bromo-4-methyl-benzoic methane-sulfonic anhydride | N'-t-butyl-N'-benzoylhydrazine | triethyl amine | methylene chloride | 193-195-°C |
| 331 | 4-fluorobenzoyl chloride | N'-t-butyl-N'-benzoylhydrazine | sodium hydroxide | toluene and water | 200°C |
| 379 | 2,3-dimethyl-benzoylchloride | N'-t-butyl-N'-(3-toluoyl)-hydrazine | sodium hydroxide | toluene and water | 190-191°C |
| 397 | 2-methyl-3-chloro-benzoyl chloride | N'-t-butyl-N'-(3-toluoyl)-hydrazine | sodium hydroxide | toluene and water | 231-233°C |
| 398 | 2-methyl-3-chloro-benzoyl chloride | N'-t-butyl-N'-benzoylhydrazine | sodium hydroxide | toluene and water | 216°C |
| 425 | 3-chloro-4-fluoro-benzoyl chloride | N'-t-butyl-N'-benzoylhydrazine | sodium hydroxide | toluene and water | 198-205°C |
| 458 | 3-chloro-4-fluoro-benzoyl chloride | N'-t-butyl-N'-(3,4-dichloro-benzoyl)hydrazine | sodium hydroxide | toluene and water | 220-222°C |

It will be appreciated by those skilled in the art that compounds of Formula I can be used as precursors for preparing other compounds of Formula I by procedures well known to those skilled in the art. For example, a suitable compound of Formula I can be reduced, alkylated, substituted, esterified, hydrolyzed or the like.

Using a nitrobenzoyl compound of Formula I as a reactant and reducing it, followed in certain cases by an addition reaction (such as alkylation) under the conditions (additional reactant, base or acid, and solvent) set forth in Table VI, the products of Examples 329, 330, 350, 351, 352, 353, 372, 375, 473 and 484 are prepared.

TABLE VI

| Ex. No. | Compound of Formula I | Reactant | Base or Acid | Solvent | m.p. |
|---|---|---|---|---|---|
| 162 | N-benzoyl-N'-t-butyl-N'-(4-formylbenzoyl)-hydrazine | sodium borohydride | | methanol | 158-161°C |
| 329 | N-benzoyl-N'-t-butyl-N'-(3-nitrobenzoyl)-hydrazine | zinc dust | | acetic acid | oil |
| 330 | N-benzoyl-N'-t-butyl-N'-(2-nitrobenzoyl)-hydrazine | zinc dust | | acetic acid | >250°C |
| 350 | N-(2-nitro-3-methoxybenzoyl)-N'-t-butyl-N'-(3-toluoyl)hydrazine | zinc dust | | acetic acid | 188-192°C |
| 351 | N-(4-nitrobenzoyl)-N'-t-butyl-N'-(4-chloro-benzoyl)hydrazine | hydrogen, platinum carbon | | ethyl acetate-methanol | >260°C |
| 352 | N-(4-aminobenzoyl)-N'-t-butyl-N'-(4-chloro-benzoyl)hydrazine | methyl chloro-formate | pyridine | methylene chloride | 213-216°C |
| 353 | N-(4-aminobenzoyl)-N'-t-butyl-N'-(4-chloro-benzoyl)hydrazine | acetic anhydride | | | 248-252°C |
| 354 | N-(2-nitro-3-methoxybenzoyl)-N'-t-butyl-N'-(3-toluoyl)hydrazine | 1. $H_2$/catalyst 2. $AC_2O$ | | ethyl acetate | |

| Ex. No. | Compound of Formula I | Reactant | Base or Acid | Solvent | m.p. |
|---|---|---|---|---|---|
| 372 | N-(3-nitrobenzoyl)-N'-t-butyl-N'-(3-toluoyl)-hydrazine | zinc dust | | aqueous acetic acid | 218-221°C |
| 374 | N't-butyl-N-(4-acetyl-benzoyl)-N'-(3-toluoyl)-hydrazine | sodium borohydride | | Methanol | |
| 375 | N-(3-aminobenzoyl)-N'-t-butyl-N'-(3-toluoyl)-hydrazine | methacryloyl chloride | sodium hydroxide | water | 170-175°C |
| 473 | N-(2-nitro-3-methoxybenzoyl)-N'-t-butyl-N'-benzoylhydrazine | zinc dust | ammonium chloride | aqueous ethanol | 200°C |
| 484 | N-(2-methyl-3-nitro benzoyl)-N'-t-butyl-N'-(3-toluoyl) hydrazine | zinc dust | ammonium chloride | aqueous ethanol | 197-199°C |

Using a chloromethylbenzoyl compound of Formula I as a reactant and performing a substitution reaction under the conditions (base or acid, and solvent) set forth in Table VII, the products of Examples 159, 161, 162, 361, 362, 363 and 367 are prepared.

TABLE VII

| Ex. No. | Compound of Formula I | Reactant | Base or Acid | Solvent | m.p. |
|---|---|---|---|---|---|
| 159 | N-benzoyl-N'-t-butyl-N'-(3-chloromethylbenzoyl) hydrazine | sodium acetate | | N,N-dimethyl-formamide | oil |
| 161 | N-benzoyl-N'-t-butyl-N'-(3-chloromethylbenzoyl) hydrazine | p-thiocresol | sodium hydroxide | toluene-water | 140-143°C |
| 294 | N-(2-chloromethyl-benzoyl)-N"-t-butyl-N'-(4-chlorobenzoyl)-hydrazine | diethylamine | | tetra-hydro-furan | |
| 361 | N-(4-chloromethyl benzoyl)-N'-t-butyl-N'-benzoyl hydrazine | sodium acetate | | N,N-dimethyl-formamide | glass |
| 362 | N-(4-chloromethyl benzoyl)-N'-t-butyl-N'-benzoyl hydrazine | potassium thiocyanate | | ethanol | glass |
| 363 | N-(4-acetoxymethyl benzoyl)-N'-t-butyl-N'-benzoylhydrazine | | sodium hydroxide | methanol | oil |
| 367 | N-(4-chloromethyl benzoyl)-N'-t-butyl-N'-benzoyl hydrazine | potassium cyanide | | dimethyl formamide | 160-162°C |

Using an acetyloxybenzoyl compound of Formula I as a reactant and performing a hydrolysis, under the conditions (base and solvent) set forth in Table VIII, the products of Examples 165, 203, 271, 285, 333 and 614 are prepared.

TABLE VIII

| Ex. No. | Compound of Formula I | Reactant | Base or Acid | Solvent | m.p. |
|---|---|---|---|---|---|
| 151 | N-benzoyl-N'-t-butyl-N'-(2-acetoxybenzoyl)-hydrazine | | sodium hydroxide | methanol | |
| 165 | N-benzoyl-N'-(1,2,2-trimethylpropyl)-N'-2-acetoxybenzoyl)hydrazine | | sodium hydroxide | methanol | 220-224°C |
| 203 | N-benzoyl-N'-t-butyl-N'-(3-acetoxybenzoyl) hydrazine | | potassium hydroxide | methanol | 200°C |
| 271 | N-(4-acetoxybenzoyl)-N'-t-butyl-N'-benzoyl hydrazine | | potassium hydroxide | methanol | 210°C |
| 285 | N-(4-acetoxybenzoyl)-N'-t-butyl-N'-(3-toluoyl) hydrazine | | potassium hydroxide | methanol | 170°C |
| 333 | N-(3-acetoxybenzoyl)-N'-t-butyl-N'-benzoyl hydrazine | | potassium hydroxide | methanol | glass |
| 349 | N-(4-carbomethoxy benzoyl)-N'-t-butyl-N'-(3-toluoyl)hydrazine | | sodium hydroxide | aqueous tetra-hydro-furan | |
| 614 | N,N'-bis-(2-acetoxy-benzoyl)-N'-t-butyl hydrazine | | sodium hydroxide | methanol | oily solid |

Using a hydroxybenzoyl compound of Formula I as a reactant and performing an alkylation or esterification, under the conditions (base and solvent) set forth in Table IX, the products of Examples 144, 286, 335, 345, 358, 359, 360, 365, 366 and 385 are prepared.

TABLE IX

| Ex. No. | Compound of Formula I | Reactant | Base | Solvent | m.p. |
|---|---|---|---|---|---|
| 144 | N-benzoyl-N'-t-butyl-N'-(4-hydroxybenzoyl) hydrazine | allyl bromide | potassium t-butoxide | tetrahydrofuran | 177-180°C |
| 286 | N-(4-hydroxybenzoyl)-N'-t-butyl-N'-benzoyl-hydrazine | allyl bromide | potassium t-butoxide | tetrahydrofuran | Oil |
| 335 | N-(3-hydroxybenzoyl)-N'-t-butyl-N'-benzoyl-hydrazine | allyl bromide | sodium hydride | dimethylformamide | Oil |
| 345 | N-(3-hydroxybenzoyl)-N'-t-butyl-N'-benzoyl-hydrazine | vinyl chloroformate | potassium t-butoxide | tetrahydrofuran | low melting solid |
| 358 | N-(4-hydroxybenzoyl)-N'-t-butyl-N'-benzoyl-hydrazine | chloromethylmethyl ether | potassium t-butoxide | tetrahydrofuran | Oil |
| 359 | N-(4-hydroxybenzoyl)-N'-t-butyl-N'-benzoyl-hydrazine | N,N-dimethylcarbamoyl chloride | potassium t-butoxide | tetrahydrofuran | low melting solid |
| 360 | N-(4-hydroxybenzoyl)-N'-t-butyl-N'-benzoyl-hydrazine | ethyl bromoacetate | potassium t-butoxide | tetrahydrofuran | low melting solid |
| 365 | N-(4-hydroxybenzoyl)-N'-t-butyl-N'-benzoyl-hydrazine | chloromethylmethyl sulfide | sodium hydride | dimethylformamide | Oil |
| 366 | N-(4-hydroxybenzoyl)-N'-t-butyl-N'-benzoyl-hydrazine | isobutyl bromide | potassium t-butoxide | dimethyl formamide | Oil |

| Ex. No. | Compound of Formula I | Reactant | Base | Solvent | m.p. |
|---|---|---|---|---|---|
| 385 | N-(4-hydroxybenzoyl)-N'-t-butyl-N'-(3-toluoyl)hydrazine | chloromethylmethyl ether | potassium t-butoxide | tetrahydrofuran | Oil |

Using a compound of Formula I as a reactant and performing the stated reaction under the conditions (additional reactant, base or acid, and solvent) set forth in Table X, the products of Examples 149, 162, 164, 166, 368, 369, 373, 374, 376, 386 and 541 are prepared.

TABLE X

| Example No. | Compound Prepared, Reactants, Reaction Conducted and Conditions | m.p. °C |
|---|---|---|
| 149 | N-benzoyl-N'-t-butyl-N'-(4-methane-sulfonylbenzoyl)hydrazine was prepared from N-benzoyl-N'-t-butyl-N'-(4-methylthiobenzoyl)hydrazine using meta-chloroperbenzoic acid in methylene chloride in an oxidation reaction. | 183-185.5 |
| 164 | N-benzoyl-N'-t-butyl-N'-(4-carboxybenzoyl)-hydrazine was prepared from N-benzoyl-N'-t-butyl-N'-(4-methoxycarbonyl-benzoyl)hydrazine sodium hydroxide as a base and methanol as solvent in a hydrolysis reaction. | 212-214 |
| 166 | N-benzoyl-N'-t-butyl-N'-(4-(2,2-dichloroethenyl)benzoyl)hydrazine was prepared from N-benzoyl-N'-t-butyl-N'-(4-formylbenzoyl)hydrazine using triphenylphosphine in carbon tetra-chloride as solvent in a Wittig-type reaction. | Oily Solid |

| Example No. | Compound Prepared, Reactants, Reaction Conducted and Conditions | m.p. °C |
|---|---|---|
| 368 | N-(4-(1,2-epoxypropyl)benzoyl)-N'-t-butyl-N'-benzoylhydrazine was prepared from N-(4-(1-propenyl)benzoyl)-N'-t-butyl-N'-benzoylhydrazine using meta-chloroperbenzoic acid in methylene chloride as solvent in an oxidation reaction. | Solid |
| 369 | N-(4-acetylsemicarbazone)-N'-t-butyl-N-(3-toluoyl)hydrazine was prepared from N-(4-acetylbenzoyl)-N'-t-butyl-N'-(3-toluoyl)-hydrazine using semicarbazide in ethanol solvent with hydrochloric acid catalyst in a condensation reaction. | 180-184 |
| 373 | N-(4-(2-hydroxy-1,1-dimethylethylaminocarbonyl)benzoyl)-N'-t-butyl-N'-(3-toluoyl)-hydrazine was prepared from N-(4-methoxycarbonylbenzoyl)-N'-t-butyl-N'-(3-toluoyl)hydrazine using 2-amino-2-methylpropanol in a condensation reaction. | low melting solid |
| 374 | N-(4-(2-hydroxyethyl)benzoyl)-N'-t-butyl-N'-(3-toluoyl)hydrazine was prepared from N-(4-acetylbenzoyl)-N'-t-butyl-N'-(3-toluoyl)hydrazine using sodium borohydride in methanol solvent in a reduction reaction. | Oily solid |

| Example No. | Compound Prepared, Reactants, Reaction Conducted and Conditions | m.p. °C |
|---|---|---|
| 376 | N-(3-carboxybenzoyl)-N'-t-butyl-N'-(3-toluoyl)hydrazine was prepared from N-(3-cyanobenzoyl)-N'-t-butyl-N'-(3-toluoyl)hydrazine using potassium hydroxide as base in methanol solvent in a hydrolysis reaction. | 202-206 |
| 386 | N-(4-(1-methylethenyl)benzoyl)-N'-t-butyl-N'-(3-toluoyl)hydrazine was prepared from N-(4-acetylbenzoyl)-N'-t-butyl-N'-(3-toluoyl) hydrazine using methyltriphenylphosphonium bromide and n-butyl lithium as base and tetrahydrofuran solvent in a Wittig reaction. | low melting solid |
| 541 | N-(4-(2-hydroxyethyl)benzoyl)-N'-t-butyl-N'-(3,5-dimethylbenzoyl)hydrazine was prepared from N-(4-(2-acetoxyethyl)benzoyl)-N'-t-butyl-N'-(3,5-dimethylbenzoyl)hydrazine using sodium hydroxide as base and methanol as solvent in a hydrolysis. | 185-187 |

As previously noted, the compounds of the present invention exhibit excellent insecticidal activity and are most active against insects of the orders Lepidoptera and Coleoptera.

In general, for the control of insects in agriculture, horticulture and forestry, the compounds of the present invention may be used at a dosage corresponding to from about 10 grams to about 10 kilograms of the active substance per hectare and from about 100 grams to about 5 kilograms per hectare of the active substance is preferred. The exact amount of dosage for a given situation can be routinely determined and depends on a variety of factors, for example, the substance used, the kind of insect, the formulation used, the state of the crop infested with the insect and the prevailing weather conditions. The term "insecticidal" as employed in the specification and claims of this application is to be construed as any means which adversely affects the existence of growth of the target insects at any stage in their life cycle. Such means can comprise a complete killing action, eradiction, arresting in growth, inhibition, reducing in number, reproductive inhibition (such as ovicidal or chemisterilant) or any combination thereof. The terms "control" or "combat", which are used interchangeably in the present specification and claims are to be construed as meaning "insecticidal" or protecting plants from insect damage. By "insecticidally effective amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research," (1969), Advances in Chemistry Series No. 86, written by Wade Van Valkenburg; and the Marcel Dekker, Inc. publication "Pesticide Formulations," (1973), edited by Wade Van Valkenburg. In these compositions and formulations, the active substance or substances are mixed, for example in an amount up to 95% by weight of active insecticidal ingredient, with inert agronomically acceptable (i.e., plant compatible and/or pesticidally inert) diluents or extenders such as solid carrier material or liquid carrier material, of the type usable in conventional

compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, conventional adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behavior, feeding habits and physiology of the pest for the purpose of control.

The invert emulsions are mainly used for air application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as a conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl iscbutyl ketone, cyclohexanone, isophorone, etc.), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.); and/or dispersing agents, such as lignin, sulfite waste liquors, methyl cellulose, etc.

Adhesives such as carboxymethylcellulose and natural; and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists, etc., if desired, or in the form of particular dosage preparations for specific application made therefrom, such as solutions, emulsions, suspensions, powders, pastes, and granules which are thus ready for use.

As concerns commerically marketed preparations, these generally contemplate carrier composition mixtures in which the active compound is present in an amount substantially between about 0.1% and 99% by weight, and preferably between about 1% and 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert finely divided carrier solid, and/or (2) a dispersible carrier liquid such as inert organic solvent and/or water, preferably including a surface-active effective amount of a carrier vehicle assistant (e.g., a surface-active agent, such as an emulsifying agent and/or a dispersing agent), and an amount of the active compound generally, between about 0.0001% and about 99% by weight of the composition, preferably between about 0.001% and about 90% by weight of the composition, and more preferably between about 0.01% and about 75% by weight of the composition, which is effective for the purpose in question.

The active compounds can be applied as sprays by methods commonly employed, such as conventional high-gallonage hydraulic sprays, low gallonage sprays, ultra-low-volume sprays, airblast spray, aerial sprays, and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of killing, combatting or controlling insects, which comprises contacting insects with a correspondingly combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or to an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example

solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described, (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

Representative preparation of compositions and formulations including the compounds of the present invention are set forth below as Examples A through I by way of illustration but not limitation.

## Example A

### Granular

| Ingredient | %/wt. |
|---|---|
| Toxicant and toxicant impurities | 0.25 |
| Triton® X-305 (binder) (Octylphenyl-30-ethylene oxide ethanol) | 0.25 |
| Agsorb® 24/48 (diluent) (Montmorillonite clay) | 99.50 |

Preparation: The toxicant and Triton® X-305 are dissolved into methylene chloride and the mixture is added to the Agsorb® with continuous mixing. The methylene chloride is then allowed to evaporate.

Example B

Dust

| Ingredient | %/wt. |
|---|---|
| Toxicant and toxicant impurities | 1.0 |
| Talc | 99.0 |

Preparation: Toxicant is dissolved in excess acetone and the mixture is impregnated onto the talc. The acetone is then permitted to evaporate.

Example C

Wettable Powder

| Ingredient | %/wt. |
|---|---|
| Toxicant and toxicant impurities | 31.3 |
| Duponal® WA Dry (wetter) (Sodium lauryl sulfate) | 2.0 |
| Reax® 45A (dispersant) (Sodium lignin sulfonate) | 5.0 |
| Barden clay (diluent) | 31.7 |
| HiSil® 233 (diluent) (Sodium silica) | 30.0 |

Preparation: The toxicant, optionally dissolved in a volatile solvent, is absorbed onto the Barden clay and HiSil® carriers. The Duponal® and Reax® are then added and the entire dry mixture blended until homogeneous. The composition is then micronized to a fine particle size.

Example D

Emulsifiable Concentrate

| Ingredient | %/wt. |
|---|---|
| Toxicant and toxicant impurities | 15.0 |
| Sponto® 232T (emulsifier) (Anionic and nonionic blend of the following surfactants: calcium dodecyl benzene sulfonate; and ethoxylated alkylphenol) | 6.0 |
| Sponto® 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |

| | |
|---|---|
| Tenneco® 500-100 (solvent) (Aromatic solvent mixture principally comprising xylene, cumene and ethyl benzene having a boiling point range of 290-345°F) | 52.5 |

Preparation: All ingredients are mixed together with continuous agitation until a homogeneous clear solution is obtained.

Example E

Aerosol

| Ingredient | %/wt. |
|---|---|
| Toxicant and toxicant impurities | 0.5 |
| Freon 12 | 99.5 |

Preparation: The components are mixed and packaged under pressure in a suitable container equipped with a release spray valve.

Example F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon® 4200 (preservative) (2-*n*-octyl-4-isothiazolin-3-one) | 0.05 |

Preparation: The corn oil and corn syrup are added to the wheat bran with adequate mixing. The toxicant and Kathon® are premixed with excess acetone and this solution is added to the wheat bran base with continued mixing. The acetone is then permitted to evaporate.

Method B

| Ingredient | %/wt. |
| --- | --- |
| Toxicant and toxicant impurities | 0.06 |
| Granulated Sugar (carrier and attractant) | 99.94 |

Example H

Pellet

Same as Example G, Method A, with this addition: the bait composition is formed into 1/4" diameter by 3/8" long pellets using a suitable die and press apparatus.

Example I

Flowable

| Ingredient | %/wt. |
| --- | --- |
| Toxicant and toxicant impurities | 31.3 |
| Duponal® WA Dry (wetter) (Sodium lauryl sulfate) | 2.0 |
| Reax® 45A (dispersant) (Sodium lignin sulfonate) | 5.0 |
| HiSil® 233 (diluent) (Sodium silica) | 30.0 |
| Kelzan® (thickener) (Xanthan gum) | 0.5 |
| Water | 31.2 |

Preparation: The toxicant is absorbed onto the HiSil® carrier. The Duponal® and Reax® are then added and the entire dry mixture blended until homogeneous. The composition is then micronized to a fine particle size. The resulting powder is suspended in water and the Kelzan® added.

Compositions and formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis( p -chlorophenyl)-1,1,1-trichloroethane and hexachloroepoxyoctahydrodimethanonaphthalene;

Carbamates, for example, N-methyl-1-naphthylcarbamates;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, 0,0-diethyl-0-p -nitrophenylphosphorothioate; N-monomethylamide of 0,0-dimethyldithiophosphorylacetic acid;

Diphenylsulfides, for example, p -chlorobenzyl or p -chlorophenyl sulfide and 2,4,4',5-tetrachlorodiphenylsul-

fide;
Diphenylsulfonates, for example, p -chlorophenylbenzenesulfonate;
Methylcarbinols, for example, 4,4-dichloro-1-trichloromethylbenzhydrol;
Quinoxaline compounds, such as methylquinoxaline dithiocarbonate;
Amidines such as N'-(4-chloro-2-methylphenyl) N,N-dimethylformamidine;
Pyrethroids such as Allethrin;
Biologicals such as Bacillus thuringiensis preparations;
Organic tin compounds such as tricyclohexyltin hydroxide;
Synergists such as piperonyl butoxide;
Insect growth regulators such as N-benzoyl-phenyl ureas, for example, diflubenzuron.

Fungicides such as:
Organic mercury compounds, for example, phenylmercuryacetate and methylmercurycyanoguanide;
Organic tin compounds, for example, triphenyltin hydroxide and triphenyltin acetate;
Alkylenebisdithiocarbamates, for example, zinc ethylenebisthiocarbamate and manganese ethylenebisdithiocarbamate; and
2,4-dinitro-6-(2-octyl-phenylcrotonate), 1-bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

## Biological Activity

It has been found by biological evaluation that compounds according to the present invention have pesticidal activity and are capable of controlling larvae and adult forms of pests, especially insects from the orders Lepidoptera and Coleoptera and most especially insects from the order Lepidoptera. One skilled in the art will know how to determine the activity of a given compound against a given insect and the dosage required to obtain general or selective insecticidal effects. The compounds of the present invention in part affect the normal development of insects, particularly insects from the order Lepidoptera, by directly and/or indirectly influencing the moulting process.

As previously noted, the compounds of the present invention are particularly suitable for controlling plant destructive insects in crops of cultivated plants, such as, but not limited to, cotton, vegetables, corn and other cereals and the like; forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to fruit and/or citrus trees, raspberry bushes and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton® X-155) and a modified phthalic glycerol alkyl resin (sold under the trademark Triton® B-1956) was utilized at the equivalent of 1 ounce per 100 gal. of test solution as a surfactant.

Initial evaluations were made on one or more of the following pests:

| Code Symbol | Common Name | Latin Name |
|---|---|---|
| SAW | Southern Armyworm | Spodoptera eridania |
| MBB | Mexican Bean Beetle | Epilachna varivestis |
| BW | Boll Weevil | Anthonomus grandis grandis |

For the foliar bean beetle and armyworm tests, individual bean ( Phaseolus limensis var. Woods' Prolific) leaves are placed on moistened pieces of filter paper in Petri dishes. The leaves are then sprayed with the test solution using a rotating turntable and allowed to dry. The dishes are infested with 10 third

instar larvae of Southern armyworm or Mexican bean beetle. The dishes are then covered.

For the Boll Weevil test ten adult weevils are placed in a 0.5 pint glass Mason jar containing a small cube of apple. The weevils are confined to the jars by fiberglass screen mesh secured by a screw-type rim cap. The jars are then sprayed with the test solution using a rotating turntable, directing the spray through the mesh into the jar.

The percent mortality for the boll weevil evaluations are determined 48 hours after treatment. A second observation 96 hours after treatment may be made in the discretion of the experimenter if it is believed the effect of a test compound may not be complete or moribund insects appear to evidence some signs of recovery. The percent mortality for the bean beetle and armyworm evaluations are determined 96 hours after treatment. Evaluations are based on a scale of 0-100 percent in which 0 equals no activity and 100 equals total kill.

The rotating turntable consists of a fixed, continuously operating spray nozzle under which targets are rotated at a fixed speed and distance. If the target is a Petri dish (such as for the armyworm), the distance form the nozzle is 15 inches. If the target is a Mason jar, the distance between the screened lid and the nozzle is 6 inches (10 inches from the base of the jar to the nozzle). The nozzle is located 8 inches from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 10 psig air pressure used and with liquid siphon feed 0.5 GPH (gallons per hour) are delivered in a round spray pattern with a 21 °C spray angle. Targets are misted with spray droplets to the point that the droplets coalesce to form a uniform thin film insufficient to drown test organisms.

All treatments are maintained at 75-80 °F under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 3-inch pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control (mortality) is determined. A second observation may be made 6 days after infesting the dishes if the experimenter feels the effect may not be complete or moribund insects appear to evidence signs of some recovery. Where necessary, untreated bean leaves are introduced into dishes held for a second observation to preclude insect starvation.

The results of the initial insecticidal evaluations are given in Table XI.

Armyworm and bean beetle spray (foliar) results are 96 hour observations unless otherwise noted. Boll weevil spray results are 48 hour observations unless, in the discretion of the experimenter, particular evaluations were held for 96 hour observations. If, after 96 hours, there was a change in the percent control, it is shown in parentheses. Soil treatment results are 72 hour observations. At the discretion of the experimenter, particular evaluations were held for 144 hour observations. If, after 144 hours, there was a change in the percent control, it is shown in parentheses.

TABLE XI

Initial Biological Evaluations

| Example No. | Foliar Application Test Species SAW | MBB | BW | Soil Application Test Species MBB | SAW |
|---|---|---|---|---|---|
| 1 | 100[a] | 0 | 0 | 80 | 0 |
| 2 | 100 | 0 | 0 | 20 | 0 |
| 3 | 100 | 60[a] | 0 | 100 | 100 |
| 4 | 100 | 0 | 0 | 60 | 0 |
| 5 | 100 | 0 | 0 | 50 | 0 |
| 6 | 40 | 0 | 0 | --[b] | -- |
| 7 | 100 | 0 | 0 | 50 | 0 |
| 8 | 10 | 0 | 0 | -- | -- |
| 9 | 100 | 0 | 0 | 70 | 100 |
| 10 | 100 | 0 | 0 | 60 | 0 |
| 11 | 100 | 0 | 0 | 20 | 100 |
| 12 | 0 | 100 | 0 | 50 | 0 |
| 13 | 100 | 0 | 0 | 20 | 100 |
| 14 | 0 | 0 | 0 | -- | -- |
| 15 | 100 | 0 | 0 | 20 | 0 |
| 16 | 100 | 0 | 0 | 30 | 100 |
| 17 | 100 | 0 | 0 | 40 | 100 |
| 18 | 100 | 100 | 0 | 100 | 100 |
| 19 | 100 | 0 | 100 | 20 | 0 |
| 20 | 100 | 0 | 0 | -- | -- |
| 21 | 100 | 0 | 0 | -- | -- |
| 22 | 100 | 0 | 0 | 40 | 100 |
| 23 | 100 | 0 | 100 | -- | -- |
| 24 | 100 | 0 | 80 | -- | -- |
| 25 | 100 | 0 | 0 | -- | -- |
| 26 | 100 | 100 | 0 | 0 | 100 |
| 27 | 0 | 0 | 0 | -- | -- |

| Example No. | Foliar Application Test Species SAW | MBB | BW | Soil Application Test Species MBB | SAW |
|---|---|---|---|---|---|
| 28 | 10 | 0 | 0 | -- | -- |
| 29 | 100 | 0 | 0 | 0 | 40 |
| 30 | 100 | 0 | 0 | 0 | 10 |
| 31 | 100 | 100 | 0 | 40 | 100 |
| 32 | 0 | 0 | 0 | -- | -- |
| 33 | 100 | 40 | 0 | 20 | 0 |
| 34 | 100 | 20 | 0 | 70 | 100 |
| 35 | 100 | 100 | 0 | 100 | 100 |
| 36 | 100 | 100 | 20 | 90 | 100 |
| 37 | 100 | 0 | 0 | -- | -- |
| 38 | 100 | 90 | 20 | 0(60) | 100 |
| 39 | 100 | 0 | 0 | 0 | 20 |
| 40 | 40 | 20 | 20 | 0 | 0 |
| 41 | 100 | 0 | 20 | 20 | 100 |
| 42 | 100 | 100 | 0 | 100 | 100 |
| 43 | 100 | 100 | 0 | 100 | 100 |
| 44 | 100 | 10 | 0 | 0(20) | 0 |
| 45 | 100 | 0 | 0 | 0(40) | 0(40) |
| 46 | 100 | 0 | 60 | 40(80) | 60(80) |
| 47 | 100 | 100 | 0 | 80(90) | 100 |
| 48 | 100 | 100 | 0(40) | 40(60) | 100 |
| 49 | 100 | 100 | 40(80) | 10(60) | 100 |
| 50 | 100 | 0 | 40(80) | 40(70) | 0(10) |
| 51 | 100 | 100 | 0 | 70(100) | 30 |
| 52 | 100 | 100 | 0 | 20(30) | 20(50) |
| 53 | 100 | 0 | 40 | 20(60) | 100 |
| 54 | 100 | 20 | 0 | 0 | 0 |
| 55 | 100 | 10 | 0 | 0 | 0 |
| 56 | 100 | 100 | 0 | 60(80) | 100 |
| 57 | 100 | 0 | 0 | 40(60) | 0 |

| Example No. | Foliar Application Test Species SAW | MBB | BW | Soil Application Test Species MBB | SAW |
|---|---|---|---|---|---|
| 58 | 100 | 20 | 0 | 20 | 0 |
| 59 | 100 | 70 | 20 | 20 | 100 |
| 60 | 100 | 40 | 0 | 0 | 0 |
| 61 | 0 | 0 | 0 | 60(80) | 0 |
| 62 | 100 | 40 | 0 | 20 | 100 |
| 63 | 100 | 10 | 0 | 20 | 0 |
| 64 | 100 | 20 | 0 | 0(100) | 10(50) |
| 65 | 100 | 20 | 0 | 0(40) | 100 |
| 66 | 100 | 70 | 0 | 40(80) | 40(50) |
| 67 | 100 | 40 | 0 | 20(80) | 100 |
| 68 | 100 | 10 | 20 | 20 | 0 |
| 69 | 100 | 40 | 0 | 20 | 0 |
| 70 | 100 | 40 | 20 | 0(20) | 0 |
| 71 | 0 | 60 | 0 | 0(20) | 0 |
| 72 | 60 | 0 | 0 | 20 | 0 |
| 73 | 100 | 100 | 0 | 40(80) | 100 |
| 74 | 100 | 80 | 0 | 20 | 0 |
| 75 | 100 | 60 | 0 | 0 | 0 |
| 76 | 100 | 60 | 0 | 0(20) | 0 |
| 77 | 100 | 100 | 0 | 0 | 0 |
| 78 | 90 | 40 | 0 | 20 | 0 |
| 79 | 0 | 0 | 20 | 0 | 0 |
| 80 | 0 | 0 | 20 | 0 | 0 |
| 81 | 100 | 0 | 20 | 0 | 20 |
| 82 | 100 | 0 | 0 | 0 | 0 |
| 83 | 100 | 100 | 0 | 60(100) | 100 |
| 84 | 10 | 0 | 0 | 0 | 0 |
| 85 | 100 | 30 | 0 | 0 | 20 |
| 86 | 100 | 30 | 20 | 0 | 80(100) |
| 87 | 100 | 40 | 40 | 0 | 40(50) |

| Example No. | Foliar Application Test Species SAW | MBB | BW | Soil Application Test Species MBB | SAW |
|---|---|---|---|---|---|
| 88 | 100 | 20 | 0 | 0 | 0 |
| 89 | 100 | 0 | 0 | 0 | 100 |
| 90 | 100 | 0 | 0 | 0 | 90 |
| 91 | 0 | 0 | 40 | 0 | 0 |
| 92 | 100 | 50 | 0 | 0 | 90(100) |
| 93 | 100 | 0 | 0 | 0 | 0 |
| 94 | 0 | 10 | 0 | 0 | 0 |
| 95 | 100 | 100 | 20 | 40(100) | 100 |
| 96 | 100 | 30 | 0 | 0 | 0 |
| 97 | 100 | 0 | 20 | 0 | 0 |
| 98 | 100 | 0 | 0 | 0 | 100 |
| 99 | 100 | 60 | 0 | 40(80) | 100 |
| 100 | 100 | 100 | 0 | 80(100) | 100 |
| 101 | 0 | 0 | 0 | 20 | 0 |
| 102 | 100 | 0 | 0 | 20 | 0(40) |
| 103 | 100 | 100 | 0 | 0(60) | 100 |
| 104 | 100 | 0 | 0(20) | 0 | 40(100) |
| 105 | 100 | 0 | 40 | 0 | 100 |
| 106 | 80 | 0 | 60 | 0 | 0(100) |
| 107 | 100 | 0 | 0 | 0(40) | 100 |
| 108 | 100 | 0 | 0 | 0 | 0 |
| 109 | 100 | 20 | 0 | 0(100) | 100 |
| 110 | 100 | 70 | 0 | 100 | 90(100) |
| 111 | 100 | 100 | 0 | 0 | 0 |
| 112 | 100 | 100 | 20 | 20(40) | 90(100) |
| 113 | 100 | 100 | 0 | 100 | 100 |
| 114 | 100 | 0 | 0 | 0 | 10 |
| 115 | 100 | 100 | 0 | 20(60) | 90(100) |
| 116 | 100 | 10 | 0 | 0 | 0 |
| 117 | 100 | 10 | 0 | 40(80) | 100 |

| Example No. | Foliar Application Test Species SAW | MBB | BW | Soil Application Test Species MBB | SAW |
|---|---|---|---|---|---|
| 118 | 100 | 10 | 0 | 0 | 90(100) |
| 119 | 100 | 0 | 0 | 0 | 0 |
| 120 | 100 | 30 | 20 | 0 | 0 |
| 121 | 100 | 100 | 0 | 20(40) | 100 |
| 122 | 70 | 20 | 0 | 0 | 20(50) |
| 123 | 100 | 0 | 0 | 0(100) | 100 |
| 124 | 100 | 100 | 0 | 0 | 20(30) |
| 125 | 100 | 0 | 0 | 0 | 0 |
| 126 | 100 | 10 | 0 | 0 | 0 |
| 127 | 100 | 10 | 0 | 20 | 0 |
| 128 | 100 | 0 | 0 | 0 | 0 |
| 129 | 100 | 40 | 40(60) | 100(80) | 80(100) |
| 130 | 100 | 0 | 0 | 0(20) | 100 |
| 131 | 100 | 0 | 0 | 0 | 100 |
| 132 | 100 | 0 | 0 | 20(40) | 0 |
| 133 | 100 | 10 | 0 | 0 | 0 |
| 134 | 100 | 10 | 0 | 0 | 10(30) |
| 135 | 100 | 0 | 0 | 20(40) | 60 |
| 136 | 100 | 70 | 0 | 20 | 30(50) |
| 137 | 100 | 10 | 0 | 40(60) | 0 |
| 138 | 100 | 0 | 0 | 0 | 90(100) |
| 139 | 100 | 0 | 0 | 0(20) | 0 |
| 140 | 100 | 0 | 0 | 20 | 0 |
| 141 | 100 | 100 | 0 | 20(100) | 100 |
| 142 | 100 | 0 | 0 | 20 | 40 |
| 143 | 0 | 0 | 0 | 20 | 0 |
| 144 | 0 | 0 | 0 | 0 | 0 |
| 145 | 0 | 0 | 0 | 0 | 0 |
| 146 | 100 | 20 | 40(60) | 0 | 0 |
| 147 | 0 | 0 | 0 | 0 | 0 |

| Example No. | Foliar Application Test Species SAW | MBB | BW | Soil Application Test Species MBB | SAW |
|---|---|---|---|---|---|
| 148 | 0 | 0 | 40 | 0 | 20(0) |
| 149 | 0 | 0 | 0 | 0 | 0 |
| 150 | 0 | 0 | 0 | 0 | 0 |
| 151 | 0 | 0 | 0 | 0 | 0 |
| 152 | 100 | 0 | 0 | 0 | 0 |
| 153 | 100 | 0 | 0 | 0 | 80(90) |
| 154 | 100 | 10 | 0 | 0 | 0 |
| 155 | 0 | 30 | 0 | 0(20) | 0 |
| 156 | 0 | 0 | 0 | 0 | 0 |
| 157 | 0 | 10 | 20 | 0 | 0 |
| 158 | 20 | 0 | 0 | 0 | 0 |
| 159 | 10 | 0 | 0 | 0 | 0 |
| 160 | 0 | 0 | 0 | 20 | 0 |
| 161 | 30 | 0 | 0 | 0(20) | 0 |
| 162 | 70 | 20 | 0 | 40(60) | 0 |
| 163 | 20 | 20 | 0 | 0(20) | 0 |
| 164 | 0 | 0 | 20 | 40 | 0 |
| 165 | 0 | 10 | 0 | 0 | 0 |
| 166 | 10 | 10 | 0 | 0 | 0 |
| 167 | 30 | 0 | 0 | 0 | 0 |
| 168 | 10 | 40 | 0 | 0 | 0 |
| 169 | 0 | 30 | 0 | 0 | 0 |
| 170 | 0 | 10 | 0 | 0 | 0 |
| 171 | 100 | 40 | 0 | 0 | 0 |
| 172 | 100 | 0 | 0 | 0 | 0 |
| 173 | 60 | 30 | 20 | 0 | 0 |
| 174 | 100 | 0 | 0 | 0 | 80(100) |
| 175 | 100 | 0 | 0 | 0 | 0 |
| 176 | 0 | 10 | 0 | 0 | 0 |
| 177 | 100 | 30 | 0 | 0 | 10 |

| Example No. | Foliar Application Test Species SAW | MBB | BW | Soil Application Test Species MBB | SAW |
|---|---|---|---|---|---|
| 178 | 100 | 10 | 0 | 0 | 10 |
| 179 | 90 | 0 | 20 | 0 | 0 |
| 180 | 100 | 0 | 0 | 0 | 0 |
| 181 | 100 | 10 | 0 | 0 | 0 |
| 182 | 100 | 0 | 0 | 0 | 0 |
| 183 | 30 | 80 | 0 | 0(100) | 0(100) |
| 184 | 100 | 10 | 0 | 0 | 0 |
| 185 | 0 | 0 | 0 | 0 | 0 |
| 186 | 0 | 0 | 0 | 0 | 0 |
| 187 | 100 | 0 | 0 | 0(20) | 0(10) |
| 188 | 0 | 0 | 0 | 0 | 0 |
| 189 | 0 | 0 | 20 | 0 | 0 |
| 190 | 100 | 0 | 0 | 0(40) | 20(60) |
| 191 | 90 | 0 | 0 | 0 | 0 |
| 192 | 20 | 0 | 0 | 0 | 0 |
| 193 | 100 | 0 | 0 | 0 | 0 |
| 194 | 100 | 80 | 20 | 20(60) | 100 |
| 195 | 100 | 80 | 0 | 80(100) | 100 |
| 196 | 100 | 0 | 0 | 0 | 100 |
| 197 | 100 | 0 | 20 | 0(20) | 100 |
| 198 | 100 | 0 | 0 | 20 | 90(100) |
| 199 | 20 | 10 | 0 | 20 | 30 |
| 200 | 100 | 0 | 0 | 0(20) | 90 |
| 201 | 100 | 0 | 0(60) | 0 | 20(30) |
| 202 | 40 | 20 | 0 | 40 | 0 |
| 203 | 60 | 0 | 20(60) | 0 | 0 |
| 204 | 50 | 0 | 0 | 0 | 0 |
| 205 | 100 | 10 | 0 | 20 | 100 |
| 206 | 100 | 10 | 0 | 0 | 100 |
| 207 | 0 | 0 | 0 | 0 | 0 |

| Example No. | Foliar Application Test Species SAW | MBB | BW | Soil Application Test Species MBB | SAW |
|---|---|---|---|---|---|
| 208 | 100 | 0 | 0 | 0(20) | 100 |
| 209 | 100 | 10 | 0 | 60(20) | 100 |
| 210 | 0 | 10 | 40 | 0 | 0 |
| 211 | 20 | 10 | 0 | 0 | 0 |
| 212 | 100 | 0 | 0 | 0 | 90(100) |
| 213 | 100 | 0 | 40 | 0 | 0 |
| 214 | 100 | 0 | 0 | 0 | 40(60) |
| 215 | 100 | 10 | 0 | 0(20) | 100 |
| 216 | 100 | 0 | 20 | 20 | 50(60) |
| 217 | 100 | 60 | 0 | 20 | 100 |
| 218 | 90 | 10 | 0 | 0 | 0 |
| 219 | 0 | 0 | 0 | 0 | 0 |
| 220 | 100 | 0 | 0 | 0 | 100 |
| 221 | 60 | 10 | 0 | 20 | 0 |
| 222 | 0 | 0 | 0 | 0 | 0 |
| 223 | 100 | 10 | 0 | 40 | 100 |
| 224 | 40 | 10 | 20 | 0 | 0 |
| 225 | 100 | 20 | 0 | 0(20) | 100 |
| 226 | 100 | 10 | 0 | 0 | 0 |
| 227 | 100 | 0 | 0 | 0 | 30(40) |
| 228 | 100 | 0 | 20 | 0(40) | 100 |
| 229 | 60 | 20 | 0 | 0 | 0 |
| 230 | 100 | 50 | 0 | 60(80) | 100 |
| 231 | 60 | 20 | 0 | 40 | 60 |
| 232 | 100 | 0 | 0 | 20 | 0 |
| 233 | 100 | 0 | 0 | 40 | 60 |
| 234 | 100 | 10 | 0 | 0 | 100 |
| 235 | 100 | 60 | 0 | 100 | 100 |
| 236 | 100 | 10 | 0 | 0 | 30 |
| 237 | 100 | 20 | 0 | 0 | 0 |

| Example No. | Foliar Application Test Species SAW | MBB | BW | Soil Application Test Species MBB | SAW |
|---|---|---|---|---|---|
| 238 | 90 | 60 | 0 | 0 | 0 |
| 239 | 100 | 0 | 0 | 0 | 0 |
| 240 | 30 | 10 | 0 | 0 | 0 |
| 241 | 100 | 0 | 0 | 0 | 0 |
| 242 | 50 | 30 | 0 | 0 | 0 |
| 243 | 10 | 20 | 0 | 0 | 0 |
| 244 | 100 | 0 | 0 | 0 | 70(80) |
| 245 | 100 | 0 | 0 | 0 | 80(90) |
| 246 | 100 | 10 | 0 | 0 | 0 |
| 247 | 100 | 0 | 0 | 0 | 0 |
| 248 | 100 | 0 | 0 | 0 | 0 |
| 249 | 100 | 0 | 0 | 0 | 0 |
| 250 | 80 | 50 | 0 | 0 | 10 |
| 251 | 100 | 20 | 20 | 20(40) | 60 |
| 252 | 100 | 0 | 0 | 0(20) | 100 |
| 253 | 100 | 10 | 20(40) | 0 | 20(30) |
| 254 | 100 | 0 | 0 | 0 | 0 |
| 255 | 100 | 100 | 0 | 0 | 0 |
| 256 | 100 | 0 | 0 | 0 | 0 |
| 257 | 100 | 0 | 0 | 20 | 20 |
| 258 | 0 | 90 | 40 | 100 | 0 |
| 259 | 100 | 10 | 0 | 0 | 0 |
| 260 | 100 | 20 | 0 | 0 | 0 |
| 261 | 0 | 10 | 0 | 0 | 0 |
| 262 | 60 | 0 | 0 | 0 | 0 |
| 263 | 100 | 30 | 0 | 20 | 40 |
| 264 | 80 | 0 | 80(100) | 0 | 10(30) |
| 265 | 0 | 10 | 0 | 0 | 0 |
| 266 | 0 | 10 | 0 | 0 | 0 |
| 267 | 100 | 0 | 0 | 0 | 0 |

| Example No. | Foliar Application Test Species SAW | Foliar Application Test Species MBB | Foliar Application Test Species BW | Soil Application Test Species MBB | Soil Application Test Species SAW |
|---|---|---|---|---|---|
| 268 | 40 | 0 | 0 | 0 | 0 |
| 269 | 100 | 0 | 60(80) | 0 | 0 |
| 270 | 100 | 0 | 0 | 20(40) | 0 |
| 271 | 100 | 10 | 0 | 20(0) | 70 |
| 272 | 40 | 10 | 0 | 0 | 0 |
| 273 | 100 | 0 | 40(100) | 0 | 0 |
| 274 | 100 | 10 | 0 | 20 | 50(90) |
| 275 | 0 | 0 | 0 | 0 | 0 |
| 276 | 100 | 10 | 20(60) | 0 | 0 |
| 277 | 100 | 30 | 0 | 20 | 0 |
| 278 | 60 | 0 | 0 | 0 | 0 |
| 279 | 100 | 10 | 0 | 0 | 0 |
| 280 | 100 | 10 | 20(80) | 0 | 0 |
| 281 | 100 | 0 | 0 | 0 | 0 |
| 282 | 100 | 30 | 0 | 0 | 0 |
| 283 | 100 | 0 | 0 | 0 | 0 |
| 284 | 90 | 50 | 0 | 0 | 0 |
| 285 | 100 | 50 | 0 | 0 | 0 |
| 286 | 100 | 0 | 0 | 0 | 0 |
| 287 | 100 | 10 | 0 | 0 | 0 |
| 288 | 100 | 0 | 0 | 0 | 0 |
| 289 | 100 | 40 | 0 | 20 | 0 |
| 290 | 100 | 50 | 0 | 0 | 0 |
| 291 | 100 | 30 | 0 | 0 | 80(90) |
| 292 | 100 | 10 | 0 | 0 | 100 |
| 293 | 20 | 30 | 20 | 0 | 0 |
| 294 | 0 | 0 | 0 | 0 | 0 |
| 295 | 100 | 100 | 0 | 0 | 60(80) |
| 296 | 100 | 50 | 0 | 60(100) | 100 |
| 297 | 100 | 40 | 0 | 0 | 0 |

| Example No. | Foliar Application Test Species SAW | MBB | BW | Soil Application Test Species MBB | SAW |
|---|---|---|---|---|---|
| 298 | 80 | 30 | 0 | 0 | 0 |
| 299 | 100 | 20 | 0 | 0 | 0 |
| 300 | 100 | 0 | 0 | 0(20) | 100 |
| 301 | 100 | 0 | 0 | 0 | 70(100) |
| 302 | 100 | 0 | 0 | 0 | 0 |
| 303 | 100 | 30 | 0 | 0 | 0 |
| 304 | 70 | 90 | 0 | 0 | 0 |
| 305 | 100 | 40 | 0 | 0 | 60(100) |
| 306 | 100 | 40 | 0 | 0 | 0 |
| 307 | 100 | 60 | 0 | 0 | 0 |
| 308 | 0 | 40 | 0 | 0 | 0 |
| 309 | 100 | 60 | 0 | 0 | 0 |
| 310 | 100 | 40 | 0 | 0 | 0 |
| 311 | 100 | 100 | 0 | 40(60) | 90(100) |
| 312 | 100 | 0 | 0 | 0 | 10 |
| 313 | 100 | 20 | 0 | 0 | 0 |
| 314 | 100 | 0 | 0 | 0 | 0 |
| 315 | 100 | 0 | 0 | 20(40) | 0 |
| 316 | 100 | 0 | 0 | 20 | 100 |
| 317 | 100 | 20 | 0 | 0 | 80(100) |
| 318 | 100 | 20 | 0 | 0 | 90(100) |
| 319 | 100 | 20 | 0 | 0 | 100 |
| 320 | 100 | 0 | 0 | 40 | 0 |
| 321 | 80 | 0 | 0 | -- | -- |
| 322 | 100 | 0 | 0 | -- | -- |
| 323 | 100 | 20 | 0 | -- | -- |
| 324 | 100 | 20 | 0 | -- | -- |
| 325 | 100 | 10 | 0 | -- | -- |
| 326 | 100 | 30 | 0 | -- | -- |
| 327 | 30 | 30 | 0 | -- | -- |

| Example No. | Foliar Application<br>Test Species<br>SAW | <br><br>MBB | <br><br>BW | Soil Application<br>Test Species<br>MBB | <br><br>SAW |
|---|---|---|---|---|---|
| 328 | 0 | 50 | 0 | -- | -- |
| 329 | 100 | 30 | 0 | -- | -- |
| 330 | 100 | 30 | 0 | -- | -- |
| 331 | 100 | 30 | 0 | -- | -- |
| 332 | 100 | 70 | 0 | -- | -- |
| 333 | 30 | 0 | 0 | -- | -- |
| 334 | 100 | 0 | 0 | -- | -- |
| 335 | 90 | 70 | 0 | -- | -- |
| 336 | 100 | 30 | 0 | -- | -- |
| 337 | 0 | 30 | 0 | -- | -- |
| 338 | 100 | 40 | 0 | -- | -- |
| 339 | 100 | 0 | 20(80) | -- | -- |
| 340 | 100 | 0 | 0 | -- | -- |
| 341 | 100 | 100 | 0 | -- | -- |
| 342 | 100 | 0 | 0 | -- | -- |
| 343 | 100 | 10 | 20(60) | -- | -- |
| 344 | 0 | 0 | 40 | -- | -- |
| 345 | 20 | 0 | 0 | -- | -- |
| 346 | 0 | 0 | 0 | -- | -- |
| 347 | 100 | 20 | 0 | -- | -- |
| 348 | 0 | 0 | 0 | -- | -- |
| 349 | 60 | 0 | 0 | -- | -- |
| 350 | 100 | 20 | 0 | -- | -- |
| 351 | 100 | 0 | 0 | -- | -- |
| 352 | 50 | 20 | 0 | -- | -- |
| 353 | 100 | 0 | 0 | -- | -- |
| 354 | 100 | 30 | 0 | -- | -- |
| 355 | 0 | 90 | 0 | -- | -- |
| 356 | 0 | 0 | 0 | -- | -- |
| 357 | 100 | 20 | 0 | -- | -- |

| Example No. | Foliar Application Test Species SAW | MBB | BW | Soil Application Test Species MBB | SAW |
|---|---|---|---|---|---|
| 358 | 100 | 0 | 0 | -- | -- |
| 359 | 100 | 0 | 0 | -- | -- |
| 360 | 0 | 0 | 0 | -- | -- |
| 361 | 100 | 10 | 0 | -- | -- |
| 362 | 100 | 70 | 0 | -- | -- |
| 363 | 100 | 20 | 0 | -- | -- |
| 364 | 100 | 10 | 0 | -- | -- |
| 365 | 100 | 0 | 0 | -- | -- |
| 366 | 100 | 40 | 0 | -- | -- |
| 367 | 100 | 30 | 0 | -- | -- |
| 368 | 100 | 0 | 0 | -- | -- |
| 369 | 100 | 0 | 0 | -- | -- |
| 370 | 30 | 30 | 0 | -- | -- |
| 371 | 100 | 10 | 20 | -- | -- |
| 372 | 30 | 0 | 0 | -- | -- |
| 373 | 90 | 10 | 0 | -- | -- |
| 374 | 100 | 10 | 0 | -- | -- |
| 375 | 0 | 10 | 0 | -- | -- |
| 376 | 0 | 20 | 0 | -- | -- |
| 377 | 100 | 20 | 0 | -- | -- |
| 378 | 100 | 20 | 0 | -- | -- |
| 379 | 100 | 20 | 0 | -- | -- |
| 380 | 100 | 10 | 0 | -- | -- |
| 381 | 100 | 30 | 0 | -- | -- |
| 382 | 100 | 0 | 20 | -- | -- |
| 383 | 100 | 20 | 0 | -- | -- |
| 384 | 80 | 20 | 0 | -- | -- |
| 385 | 100 | 100 | 0 | -- | -- |
| 386 | 100 | 20 | 20 | -- | -- |
| 387 | 100 | 0 | 0 | -- | -- |

| Example No. | Foliar Application Test Species SAW | MBB | BW | Soil Application Test Species MBB | SAW |
|---|---|---|---|---|---|
| 388 | 100 | 30 | 20(40) | -- | -- |
| 389 | 0 | 70 | 0 | -- | -- |
| 390 | 100 | 30 | 0 | -- | -- |
| 391 | 100 | 0 | 20(60) | -- | -- |
| 392 | 100 | 100 | 0 | -- | -- |
| 393 | 100 | 20 | 0 | -- | -- |
| 394 | 100 | 0 | 0 | -- | -- |
| 395 | 100 | 70 | 0 | -- | -- |
| 396 | 100 | 100 | 0 | -- | -- |
| 397 | 100 | 0 | 0 | -- | -- |
| 398 | 100 | 40 | 0 | -- | -- |
| 399 | 100 | 0 | 0 | -- | -- |
| 400 | 90 | 0 | 0 | -- | -- |
| 401 | 100 | 70 | 0 | -- | -- |
| 402 | 100 | 40 | 0 | -- | -- |
| 403 | 100 | 0 | 0 | -- | -- |
| 404 | 100 | 20 | 20(40) | -- | -- |
| 405 | 100 | 10 | 0 | -- | -- |
| 406 | 100 | 0 | 20 | -- | -- |
| 407 | 100 | 20 | 0 | -- | -- |
| 408 | 90 | 60 | 0 | -- | -- |
| 409 | 40 | 0 | 0 | -- | -- |
| 410 | 100 | 90 | 0 | -- | -- |
| 411 | 100 | 10 | 0 | -- | -- |
| 412 | 30 | 30 | 0 | -- | -- |
| 413 | 100 | 10 | 0 | -- | -- |
| 414 | 60 | 50 | 40 | -- | -- |
| 415 | 60 | 20 | 20(60) | -- | -- |
| 416 | 100 | 30 | 0 | -- | -- |
| 417 | 100 | 20 | 0 | -- | -- |

| | Foliar Application | | | Soil Application | |
|---|---|---|---|---|---|
| | Test Species | | | Test Species | |
| Example No. | SAW | MBB | BW | MBB | SAW |
| 418 | 100 | 0 | 20 | -- | -- |
| 419 | 100 | 10 | 0 | -- | -- |
| 420 | 100 | 0 | 40 | -- | -- |
| 421 | 100 | 10 | 0 | -- | -- |
| 422 | 100 | 0 | 0 | -- | -- |
| 423 | 100 | 0 | 0 | -- | -- |
| 424 | 100 | 10 | 0 | -- | -- |
| 425 | 100 | 70 | 0 | -- | -- |
| 426 | 100 | 0 | 0 | -- | -- |
| 427 | 100 | 20 | 0 | -- | -- |
| 428 | 100 | 10 | 20(80) | -- | -- |
| 429 | 100 | 20 | 0 | -- | -- |
| 430 | 100 | 0 | 0 | -- | -- |
| 431 | 90 | 40 | 0 | -- | -- |
| 432 | 100 | 20 | 0 | -- | -- |
| 433 | 100 | 0 | 0 | -- | -- |
| 434 | 100 | 10 | 20 | -- | -- |
| 435 | 100 | 20 | 0 | -- | -- |
| 436 | 0 | 30 | 0 | -- | -- |
| 437 | 100 | 10 | 0 | -- | -- |
| 438 | 100 | 40 | 0 | -- | -- |
| 439 | 100 | 0 | 0 | -- | -- |
| 440 | 100 | 20 | 0 | -- | -- |
| 441 | 100 | 20 | 0 | -- | -- |
| 442 | 100 | 30 | 0 | -- | -- |
| 443 | 100 | 20 | 0 | -- | -- |
| 444 | 100 | 0 | 0 | -- | -- |
| 445 | 100 | 20 | 0 | -- | -- |
| 446 | 100 | 20 | 20 | -- | -- |
| 447 | 100 | 20 | 0 | -- | -- |

| Example No. | Foliar Application Test Species SAW | MBB | BW | Soil Application Test Species MBB | SAW |
|---|---|---|---|---|---|
| 448 | 100 | 20 | 0 | -- | -- |
| 449 | 100 | 20 | 0 | -- | -- |
| 450 | 100 | 20 | 0 | -- | -- |
| 451 | 100 | 50 | 0 | -- | -- |
| 452 | 100 | 30 | 0 | -- | -- |
| 453 | 100 | 30 | 0 | -- | -- |
| 454 | 100 | 0 | 0 | -- | -- |
| 455 | 100 | 50 | 0 | -- | -- |
| 456 | 100 | 20 | 0 | -- | -- |
| 457 | 100 | 50 | 0 | -- | -- |
| 458 | 100 | 0 | 0 | -- | -- |
| 459 | 100 | 0 | 20 | -- | -- |
| 460 | 100 | 0 | 0 | -- | -- |
| 461 | 0 | 0 | 0 | -- | -- |
| 462 | 40 | 20 | 0 | -- | -- |
| 463 | 50 | 10 | 0 | -- | -- |
| 464 | 100 | 10 | 0 | -- | -- |
| 465 | 70 | 0 | 0 | -- | -- |
| 466 | 100 | 0 | 40 | -- | -- |
| 467 | 100 | 0 | 0 | -- | -- |
| 468 | 100 | 10 | 0 | -- | -- |
| 469 | 0 | 20 | 0 | -- | -- |
| 470 | 100 | 10 | 0 | -- | -- |
| 471 | 100 | 10 | 0 | -- | -- |
| 472 | 100 | 0 | 0 | -- | -- |
| 473 | 100 | 20 | 0 | -- | -- |
| 474 | 0 | 20 | 0 | -- | -- |
| 475 | 0 | 0 | 0 | -- | -- |
| 476 | 100 | 0 | 0 | -- | -- |
| 477 | 100 | 10 | 20 | -- | -- |

| Example No. | Foliar Application Test Species SAW | MBB | BW | Soil Application Test Species MBB | SAW |
|---|---|---|---|---|---|
| 478 | 100 | 20 | 0 | -- | -- |
| 479 | 100 | 0 | 0 | -- | -- |
| 480 | 100 | 20 | 0 | -- | -- |
| 481 | 100 | 0 | 0 | -- | -- |
| 482 | 100 | 20 | 0 | -- | -- |
| 483 | 100 | 10 | 0 | -- | -- |
| 484 | 100 | 20 | 0 | -- | -- |
| 485 | 100 | 40 | 0 | -- | -- |
| 486 | 100 | 20 | 0 | -- | -- |
| 487 | 100 | 50 | 0 | -- | -- |
| 488 | 100 | 0 | 0 | -- | -- |
| 489 | 100 | 40 | 20 | -- | -- |
| 490 | 100 | 0 | 0 | -- | -- |
| 491 | 60 | 20 | 0 | -- | -- |
| 492 | 100 | 0 | 0 | -- | -- |
| 493 | 100 | 20 | 0 | -- | -- |
| 494 | 100 | 0 | 0 | -- | -- |
| 495 | 100 | 10 | 0 | -- | -- |
| 496 | 100 | 0 | 0 | -- | -- |
| 497 | 100 | 10 | 0 | -- | -- |
| 498 | 100 | 0 | 60(80) | -- | -- |
| 499 | 100 | 40 | 0 | -- | -- |
| 500 | 100 | 10 | 0 | -- | -- |
| 501 | 100 | 30 | 0 | -- | -- |
| 502 | 100 | 10 | 20 | -- | -- |
| 503 | 90 | 30 | 0 | -- | -- |
| 504 | 100 | 40 | 0 | -- | -- |
| 505 | 100 | 30 | 20 | -- | -- |
| 506 | 100 | 0 | 0 | -- | -- |
| 507 | 60 | 20 | 0 | -- | -- |

| Example No. | Foliar Application Test Species SAW | MBB | BW | Soil Application Test Species MBB | SAW |
|---|---|---|---|---|---|
| 508 | 100 | 0 | 0 | -- | -- |
| 509 | 100 | 70 | 0 | -- | -- |
| 510 | 100 | 10 | 0 | -- | -- |
| 511 | 100 | 10 | 0 | -- | -- |
| 512 | 90 | 20 | 0 | -- | -- |
| 513 | 100 | 10 | 0 | -- | -- |
| 514 | 100 | 30 | 0 | -- | -- |
| 515 | 100 | 100 | 0 | -- | -- |
| 516 | 100 | 10 | 0 | -- | -- |
| 517 | 100 | 10 | 0 | -- | -- |
| 518 | 100 | 30 | 0 | -- | -- |
| 519 | 100 | 10 | 0 | -- | -- |
| 520 | 100 | 30 | 0 | -- | -- |
| 521 | 100 | 50 | 0 | -- | -- |
| 522 | 100 | 10 | 0 | -- | -- |
| 523 | 100 | 10 | 0 | -- | -- |
| 524 | 90 | 30 | 0 | -- | -- |
| 525 | 100 | 30 | 0 | -- | -- |
| 526 | 100 | 10 | 0 | -- | -- |
| 527 | 100 | 0 | 0 | -- | -- |
| 528 | 100 | 0 | 0 | -- | -- |
| 529 | 100 | 10 | 0 | -- | -- |
| 530 | 100 | 10 | 0 | -- | -- |
| 531 | 100 | 10 | 0 | -- | -- |
| 532 | 100 | 20 | 0 | -- | -- |
| 533 | 100 | 0 | 0 | -- | -- |
| 534 | 100 | 10 | 0 | -- | -- |
| 535 | 100 | 0 | 0 | -- | -- |
| 536 | 100 | 10 | 0 | -- | -- |
| 537 | 100 | 0 | 0 | -- | -- |

| Example No. | Foliar Application Test Species SAW | MBB | BW | Soil Application Test Species MBB | SAW |
|---|---|---|---|---|---|
| 538 | 100 | 20 | 0 | -- | -- |
| 539 | 100 | 0 | 0 | -- | -- |
| 540 | 100 | 10 | 0 | -- | -- |
| 541 | 100 | 0 | 0 | -- | -- |
| 542 | 100 | 0 | 0 | -- | -- |
| 543 | 100 | 0 | 0 | -- | -- |
| 544 | 100 | 0 | 0 | -- | -- |
| 545 | 100 | 0 | 0 | -- | -- |
| 546 | 100 | 0 | 0 | -- | -- |
| 547 | 100 | 0 | 0 | -- | -- |
| 548 | 100 | 30 | 20 | -- | -- |
| 549 | 100 | 30 | 0 | -- | -- |
| 550 | 100 | 40 | 0 | -- | -- |
| 551 | 100 | 0 | 0 | -- | -- |
| 552 | 100 | 30 | 20 | -- | -- |
| 553 | 100 | 40 | 0 | -- | -- |
| 554 | 100 | 20 | 0 | -- | -- |
| 555 | 100 | 20 | 0 | -- | -- |
| 556 | 100 | 10 | 0 | -- | -- |
| 557 | 30 | 30 | 0 | -- | -- |
| 558 | 100 | 20 | 0 | -- | -- |
| 559 | 100 | 50 | 0 | -- | -- |
| 560 | 90 | 0 | 0 | -- | -- |
| 561 | 100 | 30 | 20 | -- | -- |
| 562 | 100 | 0 | 0 | -- | -- |
| 563 | 100 | 10 | 0 | -- | -- |
| 564 | 100 | 0 | 0 | -- | -- |
| 565 | 100 | 0 | 0 | -- | -- |
| 566 | 40 | 70 | 0 | -- | -- |
| 567 | 100 | 40 | 20 | -- | -- |

| Example No. | Foliar Application Test Species SAW | MBB | BW | Soil Application Test Species MBB | SAW |
|---|---|---|---|---|---|
| 568 | 100 | 0 | 0 | -- | -- |
| 569 | 100 | 20 | 0 | -- | -- |
| 570 | 100 | 10 | 0 | -- | -- |
| 571 | 100 | 50 | 0 | -- | -- |
| 572 | 100 | 80 | 0 | -- | -- |
| 573 | 100 | 20 | 0 | -- | -- |
| 574 | 100 | 0 | 0 | -- | -- |
| 575 | 100 | 0 | 20 | -- | -- |
| 576 | 100 | 20 | 0 | -- | -- |
| 577 | 100 | 0 | 0 | -- | -- |
| 578 | 100 | 40 | 0 | -- | -- |
| 579 | 100 | 40 | 0 | -- | -- |
| 580 | 100 | 0 | 0 | -- | -- |
| 581 | 100 | 0 | 0 | -- | -- |
| 582 | 100 | 0 | 0 | -- | -- |
| 583 | 100 | 30 | 0 | -- | -- |
| 584 | 100 | 0 | 0 | -- | -- |
| 585 | 100 | 0 | 0 | -- | -- |
| 586 | 100 | 0 | 0 | -- | -- |
| 587 | 100 | 10 | 0 | -- | -- |
| 588 | 100 | 40 | 0 | -- | -- |
| 589 | 100 | 40 | 0 | -- | -- |
| 590 | 100 | 30 | 0 | -- | -- |
| 591 | 100 | 0 | 0 | -- | -- |
| 592 | 100 | 40 | 0 | -- | -- |
| 593 | 100 | 40 | 0 | -- | -- |
| 594 | 100 | 10 | 0 | -- | -- |
| 595 | 100 | 10 | 20 | -- | -- |
| 596 | 100 | 80 | 0 | -- | -- |
| 597 | 100 | 40 | 0 | -- | -- |

| Example No. | Foliar Application Test Species SAW | MBB | BW | Soil Application Test Species MBB | SAW |
|---|---|---|---|---|---|
| 598 | 100 | 10 | 20 | -- | -- |
| 599 | 100 | 40 | 0 | -- | -- |
| 600 | 100 | 30 | 0 | -- | -- |
| 601 | 100 | 10 | 0 | -- | -- |
| 602 | 100 | 10 | 0 | -- | -- |
| 603 | 100 | 20 | 0 | -- | -- |
| 604 | 100 | 10 | 0 | -- | -- |
| 605 | 100 | 20 | 20(80) | -- | -- |
| 606 | 0 | 20 | 0 | -- | -- |
| 607 | 80 | 10 | 0 | -- | -- |
| 608 | 100 | 30 | 0 | -- | -- |
| 609 | 100 | 20 | 0 | -- | -- |
| 610 | 0 | 20 | 0 | -- | -- |
| 611 | 30 | 30 | 0 | -- | -- |
| 612 | 0(30)[a] | 20 | 0 | -- | -- |
| 613 | 0(70)[a] | 50 | 0 | -- | -- |
| 614 | 10 | 20 | 0 | -- | -- |
| 615 | 100 | 20 | 0 | -- | -- |
| 616 | 100 | 0 | 0 | -- | -- |
| 617 | 100 | 10 | 0 | -- | -- |
| 618 | 100 | 10 | 0 | -- | -- |
| 619 | 100 | 0 | 0 | -- | -- |
| 620 | 70 | 30 | 20(40) | -- | -- |
| 621 | | | | | |
| 622 | 100 | 70 | 0 | 60(100) | 100 |
| 623 | 100 | 0 | 0 | 0 | 20(50) |
| 624 | 100 | 80 | 0 | 0 | 80(100) |

| | Foliar Application | | | Soil Application | |
|---|---|---|---|---|---|
| | Test Species | | | Test Species | |
| Example No. | SAW | MBB | BW | MBB | SAW |
| 625 | 100 | 10 | 20 | -- | -- |
| 626 | 100 | 90 | 0 | -- | -- |
| 627 | 100 | 0 | 0 | -- | -- |
| 628 | 100 | 40 | 0 | -- | -- |
| 629 | 100 | 0 | 0 | -- | -- |
| 630 | 100 | 0 | 0 | -- | -- |
| 631 | 100 | 0 | 0 | -- | -- |
| 632 | 100 | 30 | 0 | -- | -- |
| 633 | 100 | 10 | 0 | -- | -- |
| 634 | 100 | 90 | 0 | -- | -- |

[a] 48 hour observation
[b] No data reported

**Claims**

1. An insecticidal composition comprising, as an insecticidally active ingredient, from 0.0001% to 99%, by weight of the composition, of a compound of the formula:

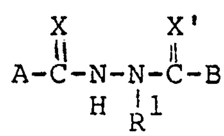

wherein
X and X' are the same or different O, S or NR;
$R^1$ is unsubstituted ($C_3$-$C_{10}$) branched alkyl or ($C_1$-$C_4$) straight chain alkyl substituted with one or two of the same or different ($C_3$-$C_6$)cycloalkyl;
A and B are the same or different unsubstituted or substituted naphthyl where the substituents can be from one to three of the same or different halo; nitro; ($C_1$-$C_4$)alkoxy; ($C_1$-$C_4$)alkyl; or amino;
unsubstituted or substituted phenyl where the substituents can be from one to five of the same or different halo; nitro; cyano; hydroxy; ($C_1$-$C_6$)alkyl; halo($C_1$-$C_6$)alkyl; cyano($C_1$-$C_6$)alkyl; hydroxy ($C_1$-$C_6$)-alkyl; ($C_1$-$C_6$)alkoxy; halo($C_1$-$C_6$)alkoxy; alkoxyalkyl having independently 1 to 6 carbon atoms in each alkyl group; alkoxyalkoxy having independently 1 to 6 carbon atoms in each alkyl group; -ORSR' group; -$OCO_2R$ group; alkanoyloxyalkyl having independently 1 to 6 carbon atoms in each alkyl group; ($C_2$-$C_6$)alkenyl, optionally substituted with halo, cyano, ($C_1$-$C_4$)alkyl, or ($C_1$-$C_4$)alkoxy; ($C_2$-$C_6$)alkenyloxy; ($C_2$-$C_6$) alkenyl-carbonyl; ($C_2$-$C_6$)alkenyl-oxycarbonyl; ($C_2$-$C_6$)alkynyl optionally substituted with halo or ($C_1$-$C_4$)alkyl; -$RCO_2R'$ group; -COR group; halo($C_1$-$C_6$)alkyl-carbonyl; -$CO_2R$ group; halo($C_1$-$C_6$)alkoxy-carbonyl; -OCOR group; -$ORCO_2R'$ group; -NRR' group; -CONRR' group; ($C_2$-$C_6$)alkenyl-carbonylamino; hydroxy($C_1$-$C_6$)alkyl-aminocarbonyl; -OCONRR' group; -NRCOR' group; -$NRCO_2R'$ group; thiocyanato; isothiocyanato; thiocyanato ($C_1$-$C_6$)alkyl; ($C_1$-$C_6$)alkyl-thio; -S(O)R group; -$SO_2R$ group;

-$OSO_2R$ group; -$SO_2NRR'$ group; -CSR group; -NRCSR' group; unsubstituted or substituted phenyl having one to three of the same or different halo, cyano, nitro, ($C_1$-$C_4$)alkyl, halo($C_1$-$C_4$) alkyl, ($C_1$-$C_4$)-alkoxy, carboxy, -$NH_2$ group, -NHZ group or -NZZ' group; phenoxy where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or NZZ' group; benzoyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; benzoyloxy($C_1$-$C_6$)alkyl; phenylthio($C_1$-$C_6$)alkyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)-alkoxy, carboxy, -$NH_2$ group, -NHZ group or -NZZ' group; -CR = N-$R^2$ group where $R^2$ is hydroxy, ($C_1$-$C_4$)alkyl, ($C_1$-$C_4$)alkoxy, -NRR', phenylamino, -COR, or benzoyl; ($C_2$-$C_6$)oxiranyl; acetylthiosemicarbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring; where R and R' are hydrogen or ($C_1$-$C_6$) alkyl, z and Z' are ($C_1$-$C_4$) alkyl and "amino" means NRR'; and agronomically acceptable salts thereof provided that when X and X' are O and A and B are unsubstituted phenyl, $R^1$ is not isopropyl (-$CH(CH_3)_2$); 2-methylpropyl (-$CH_2CH(CH_3)_2$); 3-methylbutyl (-$CH_2CH_2CH(CH_3)_2$); neopentyl (2,2-dimethylpropyl: -$CH_2C(CH_3)_3$); or cyclohexylmethyl (-$CH_2C_6H_{11}$) and further provided that when X and X' are O and $R^1$ is t -butyl (-$C(CH_3)_3$) and A is unsubstituted phenyl, B is not 4-nitrophenyl; and an agronomically acceptable diluent or carrier.

2. An insecticidal compound of the formula:

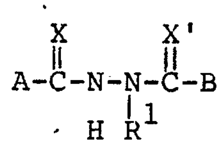

wherein X and X' are the same or different O, S or NR; $R^1$ is unsubstituted ($C_3$-$C_{10}$) branched alkyl or ($C_1$-$C_4$) straight chain alkyl substituted with one or two of the same or different ($c_3$-$c_6$)-cycloalkyl; A and B are the same or different unsubstituted or substituted naph thyl where the substituents can be from one to three of the same or different halo; nitro; ($C_1$-$C_4$) alkoxy; ($C_1$-$C_4$)-alkyl; or amino; unsubstituted or substituted phenyl where the substituents can be from one to five of the same or different halo; nitro; cyano; hydroxy; ($C_1$-$C_6$)-alkyl; halo ($C_1$-$C_6$) alkyl; ($C_1$-$c_6$) alkoxy; halo ($C_1$-$C_6$)-alkoxy; alkoxyalkyl having independently 1 to 6 carbon atoms in each alkyl group; alkoxyalkoxy having independently 1 to 6 carbon atoms in each alkyl group; -ORSR' group; -$OCO_2R$ group; alkanoyloxyalkyl having independently 1 to 6 carbon atoms in each alkyl group; ($C_2$-$C_6$) alkenyl, optionally substituted with halo, cyano, ($C_1$-$C_4$)alkyl, or ($C_1$-$C_4$)alkoxy; ($C_2$-$C_6$)alkenyloxy; ($C_2$-$C_6$)alkenyl-carbonyl; ($C_2$-$C_6$)alkenyl-oxycarbonyl; ($C_2$-$C_6$)alkynyl optionally substituted with halo or ($C_1$-$C_4$)alkyl; -$RCO_2R'$ group; -COR group; halo($C_1$-$C_6$)alkyl-carbonyl; -$CO_2R$ group; halo($C_1$-$C_6$)alkoxy-carbonyl; -OCOR group; -$ORCO_2R'$ group; -NRR' group; -CONRR' group; ($C_2$-$C_6$)alkenyl-carbonylamino; hydroxy ($C_1$-$C_6$)alkyl-aminocarbonyl; -OCONRR' group; -NRCOR' group; -$NRCO_2R'$ group; thiocyanato; isothiocyanato; thiocyanato($C_1$-$C_6$)alkyl; ($C_1$-$C_6$)-alkyl-thio; -S(O)R group; -$SO_2R$ group; -$OSO_2R$ group; -$SO_2NRR'$ group; -CSR group; -NRCSR' group; unsubstituted or substituted phenyl having one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; phenoxy where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; benzoyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; benzoyloxy($C_1$-$C_6$)alkyl; phenylthio($C_1$-$C_6$)alkyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; -CR = N-$R^2$ group where $R^2$ is hydroxy, ($C_1$-$C_4$)alkyl, ($C_1$-$C_4$)alkoxy, -NRR', phenylamino, -COR, or benzoyl; ($C_2$-$C_6$)oxiranyl; acetylthiosemicarbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;

where R and R' are hydrogen or $(C_1-C_6)$alkyl, Z and Z' are $(C_1-C_4)$alkyl and "amino" means NRR'; and agronomically acceptable salts thereof;
provided that when X and X' are O and A and B are unsubstituted phenyl, $R^1$ is not isopropyl ($-CH(CH_3)_2$); 2-methylpropyl ($-CH_2CH(CH_3)_2$); 3-methylbutyl ($-CH_2CH_2CH(CH_3)_2$); neopentyl (2,2-dimethylpropyl: $-CH_2C(CH_3)_3$); or cyclohexylmethyl ($-CH_2C_6H_{11}$) and further provided that when X and X' are O and $R^1$ is t -butyl ($-C(CH_3)_3$) and A is unsubstituted phenyl, B is not 4-nitrophenyl.

3. A composition or compound according to claim 1 or 2 in which
X, X' and R are as defined and
A and B are the same or different unsubstituted or substituted naphthyl
where the substituents can be from one to three of the same or different halo; nitro; $(C_1-C_4)$alkoxy; $(C_1-C_4)$alkyl; or amino;
unsubstituted or substituted phenyl where the substituents can be from one to five of the same or different halo; nitro; cyano; hydroxy; ($C_1$ to $C_6$)alkyl; halo($C_1$ to $C_6$)alkyl; cyano($C_1$ to $C_6$)alkyl; ($C_1$ to $C_6$)alkoxy; halo($C_1$ to $C_6$)alkoxy; alkoxyalkyl having, independently, 1 to 6 carbon atoms in each alkyl group; alkoxyalkoxy having, independently, 1 to 6 carbon atoms in each alkyl group; $-OCO_2R$ group; $(C_2-C_6)$alkenyl,
optionally substituted with halo, cyano; ($C_1$ to $C_4$)alkyl, or ($C_1$ to $C_4$)alkoxy; ($C_2$ to $C_6$)alkenyl-carbonyl; ($C_2$ to $C_6$)alkynyl,
optionally substituted with halo or ($C_1$ to $C_4$)alkyl;
$-RCO_2R'$ group; -COR group; halo($C_1$ to $C_6$)alkyl-carbonyl; $-CO_2R$ group; halo($C_1$ to $C_6$)alkoxy-carbonyl; -OCOR group; -NRR' group; -CONRR' group; -OCONRR' group; -NRCOR' group; $-NRCO_2R'$ group; thiocyanato; ($C_1$ to $C_6$)alkyl-thio; -S(O)R group; $-SO_2R$ group; $-OSO_2R$ group; $-SO_2NRR'$ group; -CSR group; -NRCSR' group;
unsubstituted or substituted phenyl, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
phenoxy where the phenyl ring is unsubstituted or substituted, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
benzoyl where the phenyl ring is unsubstituted or substituted, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
$-CR=N-R^2$ where $R^2$ is hydroxy, ($C_1$ to $C_4$)alkyl, ($C_1$ to $C_4$)alkoxy, amino, phenylamino, -COR, or benzoyl; or, when two adjacent positions on the phenyl ring are substituted with alkoxy groups, these groups may be joined to form, together with the carbon atoms to which they are both attached, a 5 or 6 membered dioxolano or dioxano heterocyclic ring.

4. A composition or compound according to any preceding claim in which $R^1$ contains no more than 10 carbon atoms.

5. A composition or compound according to claim 1, 2 or 4 wherein:
X and X' are O or S;
$R^1$ is unsubstituted $(C_3-C_8)$ branched alkyl or $(C_1-C_4)$ straight chain alkyl substituted with one or two of the same or different $(C_3-C_4)$cycloalkyl;
A and B are the same or different unsubstituted naphthyl; or
unsubstituted or substituted phenyl where the substituents can be from one to three of the same or different halo; nitro; cyano; $(C_1-C_4)$alkyl; halo$(C_1-C_4)$alkyl; cyano$(C_1-C_4)$alkyl; $(C_1-C_4)$alkoxy; alkoxyalkyl having independently 1 to 4 carbon atoms in each alkyl group; $-COD^4$; carboxy; $(C_1-C_4)$alkoxy-carbonyl; $(C_1-C_4)$alkanoyl oxy; $(C_2-C_6)$alkenyl; $(C_2-C_6)$alkynyl; $-ND^4D^5$; thiocyanato; $(C_1-C_4)$alkylthio; $-CSD^4$; unsubstituted or substituted phenyl having one or two of the same or different halo, nitro, $(C_1-C_4)$alkyl, $(C_1-C_4)$alkoxy, carboxy, $-ND^4D^5$; phenoxy where the phenyl ring is unsubstituted or substituted with one or two of the same or different halo, nitro, $(C_1-C_4)$alkyl, $(C_1-C_4)$alkoxy, carboxy, $-ND^4D^5$; or when two adjacent positions on the phenyl ring are substituted with alkoxy groups, these groups may be joined, together with the carbon atoms to which they are attached, to form a 5- or 6- membered dioxolano or dioxano heterocyclic ring;
where $D^4$ and $D^5$ are hydrogen or $(C_1-C_4)$alkyl.

6. A composition or compound according to claim 1, 2, or 4 wherein:

X and X' are O or S;
$R^1$ is branched $(C_3-C_8)$alkyl;
A and B are the same or different unsubstituted naphthyl;
unsubstituted or substituted phenyl having one to three of the same of different halo; nitro; cyano; $(C_1-C_4)$alkyl; halo$(C_1-C_4)$alkyl; cyano$(C_1-C_4)$alkyl; $(C_1-C_4)$alkoxy, alkoxyalkyl having independently 1 to 4 carbon atoms in each alkyl group; carbonyl (-COD$^4$); $(C_1-C_4)$alkoxy-carbonyl; $(C_1-C_4)$alkanoyloxy; thiocyanato; unsubstituted or substituted phenyl having one or two of the same or different halo, nitro, $(C_1-C_4)$alkyl, $(C_1-C_4)$alkoxy, carboxy, $-NH_2$, -NHZ, -NZZ'; or phenoxy where the phenyl ring is unsubstituted or substituted with one or two of the same or different halo, nitro, $(C_1-C_4)$alkyl, $(C_1-C_4)$-alkoxy, carboxy, $-NH_2$, -NHZ or -NZZ'; where $D^4$, Z and Z' are as defined in claims 5 and 3 respectively.

7. A composition or compound according to claim 1, 2 or 4 wherein:
X and X' are O;
$R^1$ is branched $(C_4-C_7)$alkyl; and
A and B are the same or different phenyl or substituted phenyl where the substituents can be from one to three of the same or different halo, nitro, $(C_1-C_4)$alkyl, $(C_1-C_4)$alkoxy, or halo$(C_1-C_4)$alkyl.

8. A composition or compound according to claim 1, 2 or 4 wherein:
X and X' are O;
$R^1$ is t -butyl, neopentyl (2,2-dimethylpropyl) or 1,2,2,-trimethylpropyl;
A and B are the same or different phenyl or substituted phenyl where the substituents can be one, two or three of the same or different chloro, fluoro, bromo, iodo, methyl, ethyl, methoxy or trifluoromethyl.

9. A composition or compound according to claim 1, 2 or 4 wherein:
X and X' are O;
$R^1$ is t -butyl; and
A and B accord to one of the following definitions:
A is 4-methylphenyl and B is 3,5-dimethylphenyl;
A is phenyl and B is phenyl;
A is 4-methylphenyl and B is 3-methylphenyl;
A is phenyl and B is 4-chlorophenyl;
A is 4-methylphenyl and B is 3,5-dimethylphenyl;
A is 2,3-dimethylphenyl and B is 3-methylphenyl;
A is 2,3-dimethylphenyl and B is 3,5-dimethylphenyl;
A is 2,3-dimethylphenyl and B is 2-bromophenyl;
A is 2,3-dimethylphenyl and B is 2,4-dichlorophenyl;
A is 2,3-dimethylphenyl and B is 2-chloro-5-methylphenyl;
A is phenyl and B is 2-bromophenyl;
A is phenyl and B is 3,4-dichlorophenyl;
A is 2-methyl-3-chlorophenyl and B is phenyl;
A is 2-methyl-3-chlorophenyl and B is 2,4-dichlorophenyl;
A is 2-methyl-3-bromophenyl and B is 2,4-dichlorophenyl;
A is 2-chloro-3-methylphenyl and B is 2,4-dichlorophenyl;
A is 2-chloro-3-methylphenyl and B is 3,5-dimethylphenyl;
A is 2,6-difluorophenyl and B is 3,4-dichlorophenyl;
A is 2,6-difluorophenyl and B is 2,4-dichlorophenyl;
A is 2,6-difluorophenyl and B is 3,5-dichlorophenyl;
A is 2-fluoro-6-chlorophenyl and B is 2,4-dichlorophenyl;
A is 2-chlorophenyl and B is 2,4-dichlorophenyl;
A is phenyl and B is 2,4-dichlorophenyl;
A is 2-methyl-3-chlorophenyl and B is 4-fluorophenyl;
A is 2-methyl-3-chlorophenyl and B is 2-bromophenyl;
A is 2-methyl-3-bromophenyl and B is 3-methylphenyl;
A is phenyl and B is 3-chloro-4-fluorophenyl;
A is 2-methyl-3-bromophenyl and B is 4-chlorophenyl;
or wherein:
X and X' are O;

$R^1$ is 1,2,2-trimethylpropyl;
A is 4-ethylphenyl or 2,3-dimethylphenyl; and
B is 3,5-dimethylphenyl.

**10.** A composition according to any of claims 1 and 3 to 9 wherein the active ingredient is present at from 0.01 to 75% by weight of the composition.

**11.** An insecticidal composition according to any of claims 1 and 3 to 10 in the form of an emulsifiable concentrate, a wettable powder, a flowable, a dust, granules or a bait.

**12.** A method of controlling insects which comprises contacting said insects with an insecticidally effective amount of active insecticidal compound as defined in claim 1 optionally in a composition according to any of claims 1 and 3 to 11.

**13.** A method according to claim 12 wherein said active insecticidal compound is applied to growing plants or an area where plants are to be grown at a dosage rate of from 10 grams to 10 kilograms per hectare.

**14.** A method according to claim 13 wherein the rate of application is from 100 grams to 5 kilograms per hectare.

**15.** A method according to any of claims 12 to 14 of controlling insects from the order Lepidoptera or Coleoptera. 16. A method according to any of claims 12 to 15 wherein the application is carried out so as to allow root absorption and transport by plants.

Claims for the following Contracting State : AT

**1.** An insecticidal composition comprising, an an insecticidally active ingredient, a compound of the formula:

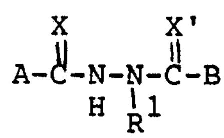

wherein
X and X' are the same or different O, S or NR;
$R^1$ is unsubstituted ($C_3$-$C_{10}$) branched alkyl or ($C_1$-$C_4$) straight chain alkyl substituted with one or two of the same or different ($C_3$-$C_6$)cycloalkyl;
A and B are the same or different unsubstituted or substituted naphthyl where the substituents can be from one to three of the same or different halo; nitro; ($C_1$-$C_4$)alkoxy; ($C_1$-$C_4$)alkyl; or amino;
unsubstituted or substituted phenyl where the substituents can be from one to five of the same or different halo; nitro; cyano; hydroxy; ($C_1$-$C_6$)alkyl; halo($C_1$-$C_6$)alkyl; cyano($C_1$-$C_6$)alkyl; hydroxy ($C_1$-$C_6$)-alkyl; ($C_1$-$C_6$)alkoxy; halo($C_1$-$C_6$)alkoxy; alkoxyalkyl having independently 1 to 6 carbon atoms in each alkyl group; alkoxyalkoxy having independently 1 to 6 carbon atoms in each alkyl group; -ORSR' group; $-OCO_2R$ group; alkanoyloxyalkyl having independently 1 to 6 carbon atoms in each alkyl group; ($C_2$-$C_6$)alkenyl, optionally substituted with halo, cyano, ($C_1$-$C_4$)alkyl, or ($C_1$-$C_4$)alkoxy; ($C_2$-$C_6$)alkenyloxy; ($C_2$-$C_6$) alkenyl-carbonyl; ($C_2$-$C_6$)alkenyl-oxycarbonyl; ($C_2$-$C_6$)alkynyl optionally substituted with halo or ($C_1$-$C_4$)alkyl; $-RCO_2R'$ group; -COR group; halo($C_1$-$C_6$)alkyl-carbonyl; $-CO_2R$ group; halo($C_1$-$C_6$)alkoxy-carbonyl; -OCOR group; $-ORCO_2R'$ group; -NRR' group; -CONRR' group; ($C_2$-$C_6$)alkenyl-carbonylamino; hydroxy($C_1$-$C_6$)alkyl-aminocarbonyl; -OCONRR' group; -NRCOR' group; $-NRCO_2R'$ group; thiocyanato; isothiocyanato; thiocyanato ($C_1$-$C_6$)alkyl; ($C_1$-$C_6$)alkyl-thio; -S(O)R group; $-SO_2R$ group; $-OSO_2R$ group; $-SO_2NRR'$ group; -CSR group; -NRCSR' group; unsubstituted or substituted phenyl having one to three of the same or different halo, cyano, nitro, ($C_1$-$C_4$)alkyl, halo($C_1$-$C_4$)alkyl, ($C_1$-$C_4$)-alkoxy, carboxy, $-NH_2$ group, -NHZ group or -NZZ' group; phenoxy where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, $-NH_2$ group, -NHZ group or -NZZ' group; benzoyl where the phenyl ring is unsubstituted or substituted with one to three of the same or different halo, cyano, nitro,

$(C_1-C_4)$alkyl, halo$(C_1-C_4)$alkyl, $(C_1-C_4)$alkoxy, carboxy, $-NH_2$ group, -NHZ group or -NZZ' group; benzoyloxy$(C_1-C_6)$alkyl; phenylthio$(C_1-C_6)$alkyl where the phenyl ring is unsubstituted or substituted with one to three of the same or different halo, cyano, nitro, $(C_1-C_4)$alkyl, halo$(C_1-C_4)$alkyl, $(C_1-C_4)$-alkoxy, carboxy, $-NH_2$ group, -NHZ group or -NZZ' group; $-CR=N-R^2$ group where $R^2$ is hydroxy, $(C_1-C_4)$alkyl, $(C_1-C_4)$alkoxy, -NRR', phenylamino, -COR, or benzoyl; $(C_2-C_6)$oxiranyl; acetylthiosemicarbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;
where R and R' are hydrogen or $(C_1-C_6)$alkyl, Z and Z' are $(C_1-C_4)$alkyl and "amino" means NRR'; and agronomically acceptable salts thereof;
and an agronomically acceptable diluent or carrier.

**2.** An insecticidal composition according to claim 1 wherein, in the insecticidally active ingredient, when X and X' are O and A and B are unsubstituted phenyl, $R^1$ is not isopropyl ($-CH(CH_3)_2$); 2-methylpropyl ($-CH_2CH(CH_3)_2$); 3-methylbutyl ($-CH_2CH_2CH(CH_3)_2$); neopentyl (2,2-dimethylpropyl: $-CH_2C(CH_3)_3$); or cyclohexylmethyl ($-CH_2C_6H_{11}$) and further provided that when X and X' are O and $R^1$ is t -butyl ($-C(CH_3)_3$) and A is unsubstituted phenyl, B is not 4-nitrophenyl.

**3.** A composition according to claim 1 or 2 in which X, X' and R are as defined and
A and B are the same or different unsubstituted or substituted naphthyl
where the substituents can be from one to three of the same or different halo; nitro; $(C_1-C_4)$alkoxy; $(C_1-C_4)$alkyl; or amino;
unsubstituted or substituted phenyl where the substituents can be from one to five of the same or different halo; nitro; cyano; hydroxy; ($C_1$ to $C_6$)alkyl; halo($C_1$ to $C_6$)alkyl; cyano($C_1$ to $C_6$)alkyl; ($C_1$ to $C_6$)alkoxy; halo($C_1$ to $C_6$)alkoxy; alkoxyalkyl having, independently, 1 to 6 carbon atoms in each alkyl group; alkoxyalkoxy having, independently, 1 to 6 carbon atoms in each alkyl group; $-OCO_2R$ group; $(C_2-C_6)$alkenyl,
optionally substituted with halo, cyano; ($C_1$ to $C_4$)alkyl, or ($C_1$ to $C_4$)alkoxy; ($C_2$ to $C_6$)alkenyl-carbonyl; ($C_2$ to $C_6$)alkynyl,
optionally substituted with halo or ($C_1$ to $C_4$)alkyl;
$-RCO_2R'$ group; -COR group; halo($C_1$ to $C_6$)alkyl-carbonyl; $-CO_2R$ group; halo($C_1$ to $C_6$)alkoxy-carbonyl; -OCOR group; -NRR' group; -CONRR' group; -OCONRR' group; -NRCOR' group; $-NRCO_2R'$ group; thiocyanato; ($C_1$ to $C_6$)alkyl-thio; -S(O)R group; $-SO_2R$ group; $-OSO_2R$ group; $-SO_2NRR'$ group; -CSR group; -NRCSR' group;
unsubstituted or substituted phenyl, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
phenoxy where the phenyl ring is unsubstituted or substituted, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
benzoyl where the phenyl ring is unsubstituted or substituted, where the substituents can be one to three of the same or different halo, cyano, nitro, ($C_1$ to $C_4$)alkyl, ($C_1$ to $C_4$)alkoxy, carboxy, $-NH_2$ group, -NHZ group or -NZZ' group;
$-CR=N-R^2$ where $R^2$ is hydroxy, ($C_1$ to $C_4$)alkyl, ($C_1$ to $C_4$)alkoxy, amino, phenylamino, -COR, or benzoyl; or, when two adjacent positions on the phenyl ring are substituted with alkoxy groups, these groups may be joined to form, together with the carbon atoms to which they are both attached, a 5 or 6 membered dioxolano or dioxano heterocyclic ring.

**4.** A composition according to any preceding claim in which $R^1$ contains no more than 10 carbon atoms.

**5.** A composition according to claim 1, 2 or 4 wherein:
X and X' are O or S;
$R^1$ is unsubstituted $(C_3-C_8)$branched alkyl or $(C_1-C_4)$ straight chain alkyl substituted with one or two or the same or different $(C_3-C_4)$cycloalkyl;
A and B are the same or different unsubstituted naphthyl; or
unsubstituted or substituted phenyl where the substituents can be from one to three of the same or different halo; nitro; cyano; $(C_1-C_4)$alkyl; halo$(C_1-C_4)$alkyl; cyano$(C_1-C_4)$alkyl; $(C_1-C_4)$alkoxy; alkoxyalkyl having independently 1 to 4 carbon atoms in each alkyl group; $-COD^4$; carboxy; $(C_1-C_4)$alkoxy-

carbonyl; $(C_1-C_4)$alkanoyl oxy; $(C_2-C_6)$alkenyl; $(C_2-C_6)$alkynyl; $-ND^4D^5$; thiocyanato; $(C_1-C_4)$alkylthio; $-CSD^4$; unsubstituted or substituted phenyl having one or two of the same of different halo, nitro, $(C_1-C_4)$alkyl, $(C_1-C_4)$alkoxy, carboxy, $-ND^4D^5$; phenoxy where the phenyl ring is unsubstituted or substituted with one or two of the same or different halo, nitro, $(C_1-C_4)$alkyl, $(C_1-C_4)$alkoxy, carboxy, $-ND^4D^5$; or when two adjacent positions on the phenyl ring are substituted with alkoxy groups, these groups may be joined, together with the carbon atoms to which they are attached, to form a 5- or 6- membered dioxolano or dioxano heterocyclic ring;
where $D^4$ and $D^5$ are hydrogen or $(C_1-C_4)$alkyl.

**6.** A composition according to claim 1, 2 or 4 wherein:
X and X' are O and S;
$R^1$ is branched $(C_3-C_8)$alkyl;
A and B are the same or different unsubstituted naphthyl;
unsubstituted or substituted phenyl having one to three of the same of different halo; nitro; cyano; $(C_1-C_4)$alkyl; halo$(C_1-C_4)$alkyl; cyano$(C_1-C_4)$alkyl; $(C_1-C_4)$alkoxy, alkoxyalkyl having independently 1 to 4 carbon atoms in each alkyl group; carbonyl $(-COD^4)$; $(C_1-C_4)$alkoxy-carbonyl; $(C_1-C_4)$alkanoyloxy; thiocyanato; unsubstituted or substituted phenyl having one or two of the same or different halo, nitro, $(C_1-C_4)$alkyl, $(C_1-C_4)$alkoxy, carboxy, $-NH_2$, -NHZ, -NZZ'; or phenoxy where the phenyl ring is unsubstituted or substituted with one or two of the same or different halo, nitro, $(C_1-C_4)$alkyl, $(C_1-C_4)$-alkoxy, carboxy, $-NH_2$, -NHZ or -NZZ'; where $D^4$, Z and Z' are ad defined in claims 5 and 3 respectively.

**7.** A composition according to claim 1, 2 or 4 wherein:
X and X' are O;
$R^1$ is branched $(C_4-C_7)$alkyl; and
A and B are the same or different phenyl or substituted phenyl where the substituents can be from one to three of the same or different halo, nitro, $(C_1-C_4)$alkyl, $(C_1-C_4)$alkoxy, or halo$(C_1-C_4)$alkyl.

**8.** A composition according to claim 1, 2 or 4 wherein:
X and X' are O;
$R^1$ is t -butyl, neopentyl (2,2-dimethylpropyl) or 1,2,2,-trimethylpropyl;
A and B are the same or different phenyl or substituted phenyl where the substituents can be one, two or three of the same or different chloro, fluoro, bromo, iodo, methyl, ethyl, methoxy or trifluoromethyl.

**9.** A composition according to claim 1, 2 or 4 wherein:
X and X' are O;
$R^1$ is t -butyl; and
A and B accord to one of the following definitions:
A is 4-methylphenyl and B is 3,5-dimethylphenyl;
A is phenyl and B is phenyl;
A is 4-methylphenyl and B is 3-methylphenyl;
A is phenyl and B is 4-chlorophenyl;
A is 4-methylphenyl and B is 3,5-dimethylphenyl;
A is 2,3-dimethylphenyl and B is 3-methylphenyl;
A is 2,3-dimethylphenyl and B is 3,5-dimethylphenyl;
A is 2,3-dimethylphenyl and B is 2-bromophenyl;
A is 2,3-dimethylphenyl and B is 2,4-dichlorophenyl;
A is 2,3-dimethylphenyl and B is 2-chloro-5-methylphenyl;
A is phenyl and B is 2-bromophenyl;
A is phenyl and B is 3,4-dichlorophenyl;
A is 2-methyl-3-chlorophenyl and B is phenyl;
A is 2-methyl-3-chlorophenyl and B is 2,4-dichlorophenyl;
A is 2-methyl-3-bromophenyl and B is 2,4-dichlorophenyl;
A is 2-chloro-3-methylphenyl and B is 2,4-dichlorophenyl;
A is 2-chloro-3-methylphenyl and B is 3,5-dimethylphenyl;
A is 2,6-difluorophenyl and B is 3,4-dichlorophenyl;
A is 2,6-difluorophenyl and B is 2,4-dichlorophenyl;
A is 2,6-difluorophenyl and B is 3,5-dichlorophenyl;

A is 2-fluoro-6-chlorophenyl and B is 2,4-dichlorophenyl;
A is 2-chlorophenyl and B is 2,4-dichlorophenyl;
A is phenyl and B is 2,4-dichlorophenyl;
A is 2-methyl-3-chlorophenyl and B is 4-fluorophenyl;
A is 2-methyl-3-chlorophenyl and B is 2-bromophenyl;
A is 2-methyl-3-bromophenyl and B is 3-methylphenyl;
A is phenyl and B is 3-chloro-4-fluorophenyl;
A is 2-methyl-3-bromophenyl and B is 4-chlorophenyl;
or wherein:
X and X' are O;
$R^1$ is 1,2,2-trimethylpropyl;
A is 4-ethylphenyl or 2,3-dimethylphenyl; and
B is 3,5-dimethylphenyl.

**10.** A composition according to any preceding claim wherein the active ingredient is present at from 0.01 to 75% by weight of the composition.

**11.** An insecticidal composition according to any preceding claim in the form of an emulsifiable concentrate, a wettable powder, a flowable, a dust, granules or a bait.

**12.** A process of controlling insects which comprises contacting said insects with an insecticidally effective amount of active insecticidal compound as defined in claim 1 optionally in a composition according to any preceding claim.

**13.** A process according to claim 12 wherein said active insecticidal compound is applied to growing plants or an area where plants are to be grown at a dosage rate of from 10 grams to 10 kilograms per hectare.

**14.** A process according to claim 13 wherein the rate of application is from 100 grams to 5 kilograms per hectare.

**15.** A process according to any of claims 12 to 14 of controlling insects from the order Lepidoptera or Coleoptera.

**16.** A process according to any of claims 12 to 15 wherein the application is carried out so as to allow root absorption and transport by plants.

Claims for the following Contracting State : ES

**1.** A process for improving the commercial value and/or profitability of vendible crops from plants whose growth is affected or likely to be affected by insects and/or improving that growth comprising (1) charging to a container, fumigation device or mechanical dissemination device an insecticidal composition comprising, as an insecticidally active ingredient, from 0.0001% to 99%, by weight of the composition, of a compound of the formula:

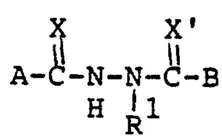

wherein
X and X' are the same or different O, S or NR;
$R^1$ is unsubstituted ($C_3$-$C_{10}$) branched alkyl or ($C_1$-$C_4$) straight chain alkyl substituted with one or two of the same or different ($C_3$-$C_6$)cycloalkyl;
A and B are the same or different unsubstituted or substituted naphthyl where the substituents can be from one to three of the same or different halo; nitro; ($C_1$-$C_4$)alkoxy; ($C_1$-$C_4$)alkyl; or amino; unsubstituted or substituted phenyl where the substituents can be from one to five of the same or different halo; nitro; cyano; hydroxy; ($C_1$-$C_6$)alkyl; halo($C_1$-$C_6$)alkyl; cyano($C_1$-$C_6$)alkyl; hydroxy ($C_1$-$C_6$)-

alkyl; ($C_1$-$C_6$)alkoxy; halo($C_1$-$C_6$)alkoxy; alkoxyalkyl having independently 1 to 6 carbon atoms in each alkyl group; alkoxyalkoxy having independently 1 to 6 carbon atoms in each alkyl group; -ORSR' group; -$OCO_2R$ group; alkanoyl oxyalkyl having independently 1 to 6 carbon atoms in each alkyl group; ($C_2$-$C_6$)alkenyl, optionally substituted with halo, cyano, ($C_1$-$C_4$)alkyl, or ($C_1$-$C_4$)alkoxy; ($C_2$-$C_6$)alkenyloxy; ($C_2$-$C_6$)alkenyl-carbonyl; ($C_2$-$C_6$)alkenyl-oxycarbonyl; ($C_2$-$C_6$)alkynyl optionally substituted with halo or ($C_1$-$C_4$)alkyl; -$RCO_2R'$ group; -COR group; halo($C_1$-$C_6$)alkyl-carbonyl; -$CO_2R$ group; halo($C_1$-$C_6$)alkoxy-carbonyl; -OCOR group; -$ORCO_2R'$ group; -NRR' group; -CONRR' group; ($C_2$-$C_6$)alkenyl-carbonylamino; hydroxy($C_1$-$C_6$)alkyl-aminocarbonyl; -OCONRR' group; -NRCOR' group; -$NRCO_2R'$ group; thiocyanato; isothiocyanato; thiocyanato ($C_1$-$C_6$)alkyl; ($C_1$-$C_6$)alkyl-thio; -S(O)R group; -$SO_2R$ group; -$OSO_2R$ group; -$SO_2NRR'$ group; -CSR group; -NRCSR' group; unsubstituted or substituted phenyl having one to three of the same or different halo, cyano, nitro, ($C_1$-$C_4$)alkyl, halo($C_1$-$C_4$) alkyl, ($C_1$-$C_4$)-alkoxy, carboxy, -$NH_2$ group, -NHZ group or -NZZ' group; phenoxy where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; benzoyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)alkoxy, carboxy, -$NH_2$ group, -NHZ group or -NZZ' group; benzoyloxy($C_1$-$C_6$)alkyl; phenylthio($C_1$-$C_6$)alkyl where the phenyl ring is unsubstituted or substituted with one to three of the same or different halo, cyano, nitro, ($C_1$-$C_4$)alkyl, halo($C_1$-$C_4$)alkyl, ($C_1$-$C_4$)-alkoxy, carboxy, -$NH_2$ group, -NHZ group or -NZZ' group; -CR=N-$R^2$ group where $R^2$ is hydroxy, ($C_1$-$C_4$)alkyl, ($C_1$-$C_4$)alkoxy, -NRR', phenylamino, -COR, or benzoyl; ($C_2$-$C_6$)oxiranyl; acetylthiosemicarbazone; pyrrolyl; oxazolyl, unsubstituted or substituted with one or two methyl groups; or when two adjacent positions on the phenyl ring are substituted with alkoxy groups, these groups may be joined to form, together with the carbon atoms to which they are attached, a 5 or 6 membered dioxolano or dioxano heterocyclic ring;

where R and R' are hydrogen or ($C_1$-$C_6$)alkyl, Z and Z' are ($C_1$-$C_4$)alkyl and "amino" means NRR'; and agronomically acceptable salts thereof;

and an agronomically acceptable diluent or carrier, (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

2. A dissemination process according to claim 1 wherein, in the insecticidal compound, when X and X' are O and A and B are unsubstituted phenyl, $R^1$ is not isopropyl (-$CH(CH_3)_2$); 2-methylpropyl (-$CH_2CH(CH_3)_2$); 3-methylbutyl (-$CH_2CH_2CH(CH_3)_2$); neopentyl (2,2-dimethylpropyl: -$CH_2C(CH_3)_3$); or cyclohexylmethyl (-$CH_2C_6H_{11}$) and further provided that when X and X' are O and $R^1$ is t -butyl (-$C(CH_3)_3$) and A is unsubstituted phenyl, B is not 4-nitrophenyl.

3. A process for the preparation of an insecticidally active compound of the formula

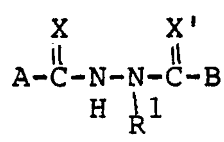

wherein X, X', $R^1$, A and B are as defined in claim 1 as limited by claim 2, which comprises reacting a first reactant (I) containing the substituent A with a second reactant (II) containing the substituent B in the presence of base and solvent, wherein, when X and X' are both O,

(a) reactant I is of the formula 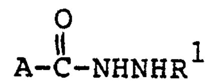 and

reactant II is of the formula 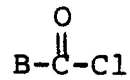; or

(b) reactant I is of the formula 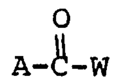 and

reactant II is of the formula 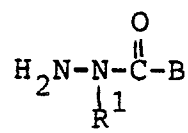

wherein W is a good leaving group; or wherein, when at least one of X and X' is S,

(c) reactant I is of the formula 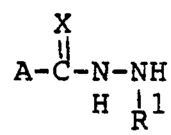 and

reactant II is of the formula 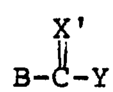

wherein Y is a good leaving group.

**4.** A preparation process according to claim 3 wherein, in reaction (a), $R^1$ is a group of the formula

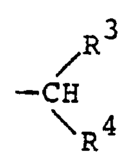

wherein $R^3$ and $R^4$ are the same or different hydrogen or ($C_3$ to $C_9$) unsubstituted branched chain alkyl or ($C_1$ to $C_8$) unsubstituted straight chain alkyl or ($C_3$ to $C_6$) cycloalkyl or ($C_1$ to $C_3$) straight chain alkyl substituted by one or two ($C_3$ to $C_6$) cycloalkyl; or, in reaction (b) W is -Cl.

**5.** A dissemination or preparation process according to any preceding claim in which
X, X' and R are as defined and
A and B are the same or different unsubstituted or substituted naphthyl where the substituents can be from one to three of the same or different halo; nitro; ($C_1$-$C_4$)alkoxy; ($C_1$-$C_4$)alkyl; or amino;
unsubstituted or substituted phenyl where the substituents can be from one to five of the same or different halo; nitro; cyano; hydroxy; ($C_1$ to $C_6$)alkyl; halo($C_1$ to $C_6$)alkyl; cyano($C_1$ to $C_6$)alkyl; ($C_1$ to $C_6$)alkoxy; halo($C_1$ to $C_6$) alkoxy; alkoxyalkyl having, independently, 1 to 6 carbon atoms in each alkyl group; alkoxyalkoxy having, independently, 1 to 6 carbon atoms in each alkyl group; $-OCO_2R$ group; ($C_2$-$C_6$)alkenyl,
optionally substituted with halo, cyano;
($C_1$ to $C_4$)alkyl, or ($C_1$ to $C_4$)alkoxy; ($C_2$ to $C_6$)alkenyl-carbonyl; ($C_2$ to $C_6$)alkynyl,
optionally substituted with halo or ($C_1$ to $C_4$)alkyl;
$-RCO_2R'$ group; -COR group; halo($C_1$ to $C_6$)alkyl-carbonyl; $-CO_2R$ group; halo ($C_1$ to $C_6$)alkoxy-carbonyl; -OCOR group; -NRR' group; -CONRR' group; -OCONRR' group; -NRCOR' group; $-NRCO_2R'$ group; thiocyanato; ($C_1$ to $C_6$) alkyl-thio; -S(O)R group; $-SO_2R$ group; $-OSO_2R$ group; $-SO_2NRR'$ group; -CSR group; -NRCSR' group;
unsubstituted or substituted phenyl,

where the substituents can be one to three of the same or different halo, cyano, nitro, ($C_1$ to $C_4$)alkyl, ($C_1$ to $C_4$)alkoxy, carboxy, -$NH_2$ group, -NHZ group or -NZZ'; group;
phenoxy where the phenyl ring is unsubstituted or substituted,
where the substituents can be one.to three of the same or different halo, cyano, nitro, ($C_1$ to $C_4$) alkyl, ($C_1$ to $C_4$) alkoxy, carboxy, -$NH_2$ group, -NHZ group or -NZZ' group;
benzoyl where the phenyl ring is unsubstituted or substituted,
where the substituents can be one to three of the same or different halo, cyano, nitro, ($C_1$ to $C_4$)alkyl, ($C_1$ to $C_4$)alkoxy, carboxy, -$NH_2$ group, -NHZ group or -NZZ' group;
-CR=N-$R^2$ where $R^2$ is hydroxy, ($C_1$ to $C_4$)alkyl, ($C_1$ to $C_4$)alkoxy, amino, phenylamino, -COR, or benzoyl; or, when two adjacent positions on the phenyl ring are substituted with alkoxy groups, these groups may be joined to form, together with the carbon atoms to which they are both attached, a 5 or 6 membered dioxolano or dioxano heterocyclic ring.

**6.** A dissemination or preparation process according to any preceding claim in which $R^1$ contains no more than 10 carbon atoms.

**7.** A dissemination or preparation process according to any of claims 1 to 4 and 6 wherein:
X and X' are O or S;
$R^1$ is unsubstituted ($C_3$-$C_8$)branched alkyl or ($C_1$-$C_4$) straight chain alkyl substituted with one or two of the same or different ($C_3$-$C_4$)cycloalkyl;
A and B are the same or different unsubstituted naphthyl; or
unsubstituted or substituted phenyl where the substituents can be from one to three of the same or different halo; nitro; cyano; ($C_1$-$C_4$)alkyl; halo($C_1$-$C_4$)alkyl; cyano($C_1$-$C_4$)alkyl; ($C_1$-$C_4$)alkoxy; alkoxyalkyl having independently 1 to 4 carbon atoms in each alkyl group; -$COD^4$; carboxy; ($C_1$-$C_4$)alkoxy-carbonyl; ($C_1$-$C_4$)alkanoyloxy; ($C_2$-$C_6$)alkenyl; ($C_2$-$C_6$)alkynyl; -$ND^4D^5$; thiocyanato; ($C_1$-$C_4$)alkylthio; -$CSD^4$; unsubstituted or substituted phenyl having one or two of the same or different halo, nitro, ($C_1$-$C_4$)alkyl, ($C_1$-$C_4$)alkoxy, carboxy, -$ND^4D^5$; phenoxy where the phenyl ring is unsubstituted or substituted with one or two of the same or different halo, nitro, ($C_1$-$C_4$)alkyl, ($C_1$-$C_4$)alkoxy, carboxy, -$ND^4D^5$; or when two adjacent positions on the phenyl ring are substituted with alkoxy groups, these groups may be joined, together with the carbon atoms to which they are attached, to form a 5- or 6- membered dioxolano or dioxano heterocyclic ring;
where $D^4$ and $D^5$ are hydrogen or ($C_1$-$C_4$)alkyl.

**8.** A dissemination or preparation process according to any of claims 1 to 4 and 6 wherein:
X and X' are O or S;
$R^1$ is branched ($C_3$-$C_8$)alkyl;
A and B are the same or different unsubstituted naphthyl;
unsubstituted or substituted phenyl having one to three of the same or different halo; nitro; cyano; ($C_1$-$C_4$)alkyl; halo($C_1$-$C_4$)alkyl; cyano($C_1$-$C_4$)alkyl; ($C_1$-$C_4$)alkoxy, alkoxyalkyl having independently 1 to 4 carbon atoms in each alkyl group; carbonyl (-$COD^4$); ($C_1$-$C_4$)alkoxy-carbonyl; ($C_1$-$C_4$)alkanoyloxy; thiocyanato; unsubstituted or substituted phenyl having one or two of the same or different halo, nitro, ($C_1$-$C_4$)alkyl, ($C_1$-$C_4$)alkoxy, carboxy, -$NH_2$, -NHZ, -NZZ'; or phenoxy where the phenyl ring is unsubstituted or substituted with one or two of the same or different halo, nitro, ($C_1$-$C_4$)alkyl, ($C_1$-$C_4$)-alkoxy, carboxy, -$NH_2$, -NHZ or -NZZ'; where $D^4$, Z and Z' are as defined in claims 5 and 3 respectively.

**9.** A dissemination or preparation process according to any of claims 1 to 4 and 6 wherein:
X and X' are O;
$R^1$ is branched ($C_4$-$C_7$)alkyl; and
A and B are the same or different phenyl or substituted phenyl where the substituents can be from one to three of the same or different halo, nitro, ($C_1$-$C_4$)alkyl, ($C_1$-$C_4$)alkoxy, or halo($C_1$-$C_4$)alkyl.

**10.** A dissemination or preparation process according to any of claims 1 to 4 and 6 wherein:
X and X' are O;
$R^1$ is t -butyl, neopentyl (2,2-dimethylpropyl) or 1,2,2,-trimethylpropyl;
A and B are the same or different phenyl or substituted phenyl where the substituents can be one, two or three of the same or different chloro, fluoro, bromo, iodo, methyl, ethyl, methoxy or trifluoromethyl.

11. A dissemination or preparation process according to any of claims 1 to 4 and 6 wherein:
X and X' are O;
$R^1$ is t -butyl; and
A and B accord to one of the following definitions:
A is 4-methylphenyl and B is 3,5-dimethylphenyl;
A is phenyl and B is phenyl;
A is 4-methylphenyl and B is 3-methylphenyl;
A is phenyl and B is 4-chlorophenyl;
A is 4-methylphenyl and B is 3,5-dimethylphenyl;
A is 2,3-dimethylphenyl and B is 3-methylphenyl;
A is 2,3-dimethylphenyl and B is 3,5-dimethylphenyl;
A is 2,3-dimethylphenyl and B is 2-bromophenyl;
A is 2,3-dimethylphenyl and B is 2,4-dichlorophenyl;
A is 2,3-dimethylphenyl and B is 2-chloro-5-methylphenyl;
A is phenyl and B is 2-bromophenyl;
A is phenyl and B is 3,4-dichlorophenyl;
A is 2-methyl-3-chlorophenyl and B is phenyl;
A is 2-methyl-3-chlorophenyl and B is 2,4-dichlorophenyl;
A is 2-methyl-3-bromophenyl and B is 2,4-dichlorophenyl;
A is 2-chloro-3-methylphenyl and B is 2,4-dichlorophenyl;
A is 2-chloro-3-methylphenyl and B is 3,5-dimethylphenyl;
A is 2,6-difluorophenyl and B is 3,4-dichlorophenyl;
A is 2,6-difluorophenyl and B is 2,4-dichlorophenyl;
A is 2,6-difluorophenyl and B is 3,5-dichlorophenyl;
A is 2-fluoro-6-chlorophenyl and B is 2,4-dichlorophenyl;
A is 2-chlorophenyl and B is 2,4-dichlorophenyl;
A is phenyl and B is 2,4-dichlorophenyl;
A is 2-methyl-3-chlorophenyl and B is 4-fluorophenyl;
A is 2-methyl-3-chlorophenyl and B is 2-bromophenyl;
A is 2-methyl-3-bromophenyl and B is 3-methylphenyl;
A is phenyl and B is 3-chloro-4-fluorophenyl;
A is 2-methyl-3-bromophenyl and B is 4-chlorophenyl;
or wherein:
X and X' are O;
$R^1$ is 1,2,2-trimethylpropyl;
A is 4-ethylphenyl or 2,3-dimethylphenyl; and
B is 3,5-dimethylphenyl.

12. A dissemination process according to any of claims 1, 2 and 5 to 11 wherein the active ingredient is present in the composition at from 0.01 to 75% by weight of the composition.

13. A dissemination process according to any of claims 1, 2 and 5 to 12 wherein the composition is used in the form of an emulsifiable concentrate, a wettable powder, a flowable, a dust, granules or a bait.

14. A dissemination process according to any of claims 1, 2 and 5 to 13 wherein said active insecticidal compound is applied in step 2 to growing plants or an area where plants are to be grown and in step 3 the dosage rate is controlled at from 10 grams to 10 kilograms per hectare.

15. A dissemination process according to any of claims 1, 2 and 5 to 14 wherein in step 3 the dosage is controlled at a rate of application from 100 grams to 5 kilograms per hectare.

16. A dissemination process according to any of claims 1,2 and 5 to 15 wherein the insects are from the order Lepidoptera or Coleoptera.

## Revendications

1. composition insecticide comprenant, comme ingrédient à action insecticide, de 0,0001% à 99% en

poids de la composition, d'un composé de formule :

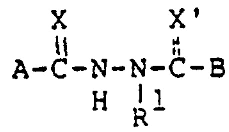

où

X et X', identiques ou différents, représentent O, S ou NR ;

$R^1$ est un alkyle ramifié en $C_{3-10}$ non-substitué ou un alkyle à chaîne droite en $C_{1-4}$ substitué par un ou deux radicaux cycloalkyle en $C_{3-6}$

A et B identiques ou différents representent des radicaux naphtyle non-substitués ou substitués ou les substituants peuvent être de un à trois radicaux halo ; nitro ; alcoxy en $C_1$ à $C_4$ ; alkyle en $C_1$ à $C_4$ ou amino ; identiques ou différents; phényle non-substitué ou substitué ou les substituants peuvent être de un à cinq, identiques ou différents, parmi les radicaux halo ; nitro ; cyano ; hydroxy ; alkyle en $C_{1-6}$ ; halo(alkyle en $C_{1-6}$) ; cyano(alkyle en $C_{1-6}$) ; hydroxy(alkyle en $C_{1-6}$) ; alcoxy en $C_{1-6}$ ; halo(alcoxy en $C_{1-6}$) ; alcoxyalkyle ayant indépendamment de 1 à 6 atomes de carbone dans chaque groupe alkyle ; alcoxyalcoxy ayant indépendamment de 1 à 6 atomes de carbone dans chaque groupe alkyle ; un groupe -ORSR' ; un groupe $-OCO_2R$ ; un alcanoyloxyalkyle ayant idépendamment de 1 à 6 atomes de carbone dans chaque groupe alkyle ; alcényle en $C_{2-6}$, éventuellement substitué par un halo, cyano, alkyle en $C_{1-4}$ ou alcoxy en $C_{1-4}$ ; alcényloxy en $C_{2-6}$ ; alcényle en $C_{2-6}$-carbonyle ; alcényle en $C_{2-6}$-oxycarbonyle ; alcynyle en $C_{2-6}$ éventuellement substitué par un halo ou un alkyle en $C_{1-4}$ ; un groupe $-RCO_2R'$ ; un groupe COR ; un halo-alkyl en $C_{1-6}$-carbonyle ; un groupe $-CO_2R$ un halo-alcoxy en $C_{1-6}$-carbonyle ; un groupe -OCOR ; un groupe $-ORCO_2R'$ ; un groupe NRR' ; un groupe -CONRR' ; un alcényle en $C_{2-6}$-carbonylamino ; hydroxyalkyl en $C_{1-6}$-aminocarbonyle ; un groupe -OCONRR' ; un groupe -NRCOR' ; un groupe $-NRCO_2R'$ ; un thiocyanato ; isothiocyanato ; thiocyanato-alkyle en $C_{1-6}$ ; alkyl en $C_{1-6}$-thio ; un groupe -S(O)R ; un groupe $-SO_2R$ ; un groupe $-OSO_2R$ ; un groupe $-SO_2NRR'$ ; un groupe -CSR ; un groupe -NRCSR' ; un phényle non-substitué ou substitué ayant de 1 à 3 groupes identiques ou différents parmi halo cyano, nitro, alkyle en $C_{1-4}$' halo-alkyle en $C_{1-4}$' alcoxy en $C_{1-4}$' carboxy, $-NH_2$, -NHZ ou -NZZ' ; phénoxy où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; benzoyle où le noyau phényle est non-substitué ou substitué par de 1 à 3 groupes identiques ou différents parmi halo, cyano, nitro, alkyle en $C_{1-4}$, haloalkyle en $C_{1-4}$, alcoxy en $C_{1-4}$, carboxy, $-NH_2$, -NHZ ou -NZZ' ; benzoyloxy-alkyle en $C_{1-6}$, Phénylthio-alkyle en $C_{1-6}$ où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; $-CR=N-R^2$ où $R^2$ est un hydroxy,alkyle en $C_{1-4}$, alcoxy en $C_{1-4}$, -NRR', phénylamino, -COR, ou benzoyle ; oxiranyle en $C_{2-6}$ ; acétylthiosemicarbazone ; pyrrolyle ; oxazolyle, non substitués ou substitués par un ou deux groupes méthyle ; ou lorsque deux positions adjacentes sur le noyau phényle sont substituées par des groupes alcoxy, ces groupes peuvent être réunis pour former, avec les atomes d'azote auxquels ils sont attachés, un noyau hétérocyclique dioxolano ou dioxano à 5 ou 6 chaînons ;

où R et R' représentent l'hydrogène ou alkyle en $C_{1-6}$ , Z et Z' représentent alkyle en $C_{1-4}$ et amino" signifie NRR' ; et leurs sels agronomiquement acceptables à condition que lorsque X et X' représentent O et A et B représentent un phényle non-substitué, $R^1$ n'est pas un isopropyle, $(-CH(CH_3)_2)$ ; 2-méthylpropyle $(-CH_2CH(CH_3)_2)$ ; 3-méthylbutyle $(-CH_2CH_2CH(CH_3)_2)$ ; néopentyle (2,2-diméthylpropyle : $-CH_2C(CH_3)_3$) ; ou cyclohexylméthyle $(-CH_2C_6H_{11})$ et à condition en outre que lorsque X et X représentent O et $R^1$ est un t -butyle $(-C(CH_3)_3)$ et A est un phényle non-substitué, B n'est pas un 4-nitrophényle ; et un diluant ou support agronomiquement acceptable.

2. Composé insecticide de formule :

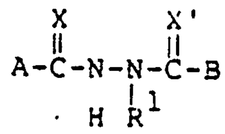

où

X et X' sont des radicaux O, S ou NR identiques ou différents ;

$R^1$ est un alkyle ramifié en $C_{3-10}$ non-substitué ou un alkyle à chaîne droite en $C_{1-4}$ substitué par un ou deux cycloalkyles en $C_{3-6}$ identiques ou différents ;

A et B identiques ou différents représentent un naphtyle non-substitué ou substitué, où les substituants peuvent être de 1 à 3 des groupes identiques ou différents suivants : halo ; nitro ; alcoxy en $C_{1-4}$ ; alkyle en $C_{1-4}$ ; ou amino ; phényle non-substitué ou substitué où les substituants peuvent être de 1 à 5 des groupes identiques ou différents parmi halo ; nitro ; cyano ; hydroxy ; alkyle en $C_{1-6}$ ; halo-alkyle en $C_{1-6}$ ; alcoxy en $C_{1-6}$ ; halo-alcoxy en $C_{1-6}$ ; alcoxyalkyle ayant indépendamment de 1 à 6 atomes de carbone dans chaque groupe alkyle ; alcoxy-alcoxy ayant indépendamment de 1 à 6 atomes de carbone dans chaque groupe alkyle ; un groupe -ORSR' ; un groupe $-OCO_2R$ ; un alcanoyloxyalkyle ayant indépendamment de 1 à 6 atomes de carbone dans chaque groupe alkyle ; un alcényle en $C_{2-6}$ , éventuellement substitué par un halo, cyano, alkyle en $C_{1-4}$ ou alcoxy en $C_{1-4}$ ; alcényloxy en $C_{2-6}$ ; un alcényl en $C_{2-6}$-carbonyle, un alcényl en $C_{2-6}$-oxycarbonyle ; un alcynyle en $C_{2-6}$ éventuellement substitué par un halo ou un alkyle en $C_{1-4}$ ; un groupe $-RCO_2R'$ ; un groupe -COR ; un halo-alkyl en $C_{1-6}$-carbonyle ; un groupe $CO_2R$ ; un halo-alcoxy en $C_{1-6}$-carbonyle ; un groupe -OCOR ; un groupe $-ORCO_2R'$ ; un groupe-NRR' ; un groupe -CONRR' ; un alcényl en $C_{2-6}$-carbonylamino ; hydroxy-alkyl en $C_{1-6}$-aminocarbonyle ; un groupe -OCONRR' ; un groupe -NRCOR' ; un groupe $-NRCO_2R'$ ; un thiocyanato, isothiocyanato ; thiocyanato- alkyle en $C_{1-6}$ ; alkyl en $C_{1-6}$-thio ; un groupe -S(O)R ; un groupe $-SO_2R$ ; un groupe $-OSO_2R$ ; un groupe $-SO_2NRR'$ ; un groupe -CSR ; un groupe -NRCSR' ; un phényle non-substitué ou substitué ayant de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; phénoxy où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ', benzoyle où le noyau phényle est non-substitué ou substitué par de 1 à 3 groupes identiques ou différents parmi halo, cyano, nitro, alkyle en $C_{1-4}$, haloalkyle en $C_{1-4}$, alcoxy en $C_{1-4}$, carboxy, $-NH_2$, -NHZ ou -NZZ' ; benzoyloxy-alkyle en $C_{1-6}$ ; phénylthioalkyle en $C_{1-6}$ où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; $-CR=N-R^2$ où $R^2$ est un hydroxy, alkyle en $C_{1-4}$, alcoxy en $C_{1-4}$, -NRR', phénylamino, -COR ou benzoyle ; oxiranyle en $C_{2-6}$ ; acétylthiosemicarbazone ; pyrrolyle ; oxazolyle, non substitués ou substitués par un ou deux groupes méthyle ; ou lorsque deux positions adjacentes sur le noyau phényle sont substituées par des groupes alcoxy, ces groupes peuvent réunis pour former, avec les atomes de carbone auxquels ils sont attachés, un noyau hétérocyclique dioxolano ou dioxano à 5 ou 6 chaînons ;

où R et R' représentent un hydrogène ou un alkyle en $C_{1-6}$ ;

Z et Z' représentent un alkyle en $C_{1-4}$ et "amino" désigne NRR' ;

et leurs sels agronomiquement acceptables ; à condition que lorsque X et X' représentent O et A et B représentent un phényle non-substitué, $R^1$ n'est pas un isopropyle ($-CH(CH_3)_2$) ; 2-méthylpropyle ($-CH_2CH(CH_3)_2$) ; 3-méthylbutyle ($-CH_2CH_2CH(CH_3)_2$) ; néopentyle (2,2-diméthylpropyle : $-CH_2C(CH_3)_3$) ; ou cyclohexylméthyle ($-CH_2C_6H_{11}$) et à condition en outre que lorsque X et X' représentent O et $R^1$ est un t -butyle ($-C(CH_3)_3$) et A est un phényle non-substitué, B n'est pas un 4-nitrophényle.

3. Composition ou composé selon la revendication 1 ou 2 où

X, X' et R sont tels que définis et

A et B identiques ou différents sont des groupes naphtyle non-substitués ou substitué, où les substituants peuvent être de 1 à 3 groupes identiques ou différents parmi halo ; nitro ; alcoxy en $C_{1-4}$ ; alkyle en $C_{1-4}$ ; ou amino; phényle non-substitué ou substitué où les substituants peuvent être de 1 à 5 groupes identiques ou différents parmi halo ; nitro ; cyano ; hydroxy ; alkyle en $C_{1-6}$ ; halo-alkyl en $C_{1-6}$ ; cyano-alkyle en $C_{1-6}$ ; alcoxy en $C_{1-6}$ ; halo-alcoxy en $C_{1-6}$ ; alcoxy-alkyle ayant, indépendamment, de 1 à 6 atomes- de carbone dans chaque groupe alkyle ; alcoxy-alcoxy ayant indépendamment de 1 à 6 atomes de carbone dans chaque groupe alkyle ; $-OCO_2R$ ; alcényle en $C_{2-6}$, éventuellement

substitué par un halo, cyano ; alkyle en $C_{1-4}$ ; ou alcoxy en $C_{1-4}$ ; alcényl en $C_{2-6}$-carbonyle ; alcynyle en $C_{2-6}$ ; éventuellement substitué par un halo ou un alkyle en $C_{1-4}$ ; $-RCO_2R'$ ; -COR ; halo-alkyle en $C_{1-6}$-carbonyle ; $-CO_2R$ ; halo-alcoxy en $C_{1-6}$-carbonyle ; -OCOR ; -NRR' ; -CONRR' ; -OCONRR' ; -NRCOR' ; $-NRCO_2R'$ ; thiocyanato ; alkyle en $C_{1-6}$-thio ; -S(O)R ; $-SO_2R$ ; $-OSO_2R$ ; $-SO_2NRR'$ ; -CSR ; -NRCSR' ; phényle non-substitué ou substitué où les substituants peuvent être de 1 à 3 groupes identiques ou différents parmi halo, cyano, nitro, alkyle en $C_{1-4}$, alcoxy en $C_{1-4}$, carboxy, $-NH_2$, -NHZ ou -NZZ' ; phénoxy où le noyau phényle est non-substitué ou substitué, où les substituants peuvent être 1 à 3 des groupes identiques ou différents parmi halo, cyano, nitro, alkyle en $C_{1-4}$, alcoxy en $C_{1-4}$, carboxy, $-NH_2$, -NHZ ou -NZZ' ; benzoyle où le noyau phényle est nonsubstitué ou substitué , où les substituants peuvent être de 1 à 3 des groupes identiques ou différents parmi halo, cyano, nitro, alkyle en $C_{1-4}$, alcoxy en $C_{1-4}$, carboxy, $-NH_2$, -NHZ ou -NZZ' ; $-CR=N-R^2$ où $R^2$ est un hydroxy, alkyle en $C_{1-4}$, alcoxy en $C_{1-4}$, amino, phénylamino, -COR, ou benzoyle ; ou ,lorsque deux positions adjacentes sur le noyau phényle sont substituées par des groupes alcoxy, ces groupes peuvent être réunis pour former, avec les atomes de carbone auxquels ils sont tous deux attachés, un noyau hétérocyclique dioxolano ou dioxano à 5 ou 6 chaînons

**4.** Composition ou composé selon l'une quelconque des revendications précédentes, où $R^1$ ne contient pas plus de 10 atomes de carbone.

**5.** Composition ou composé selon la revendication 1, 2 ou 4 où :

X et X' représentent O ou S ;

$R^1$ est un alkyle en $C_{3-8}$ ramifié non-substitué ou un alkyle à chaîne droite en $C_{1-4}$ substitué par un ou deux cycloalkyles en $C_{3-4}$ identiques ou différents ;

A et B identiques ou différents représentent des naphtyles non-substitués ; ou des phényles non-substitués ou substitués où les substituants peuvent être de 1 à 3 des groupes identiques ou différents parmi halo ; nitro ; cyano ; alkyle en $C_{1-4}$ ; halo-alkyl en $C_{1-4}$ ; cyanoalkyle en $C_{1-4}$ ; alcoxy en $C_{1-4}$ ; alcoxyalkyle ayant indépendamment de 1 à 4 atomes de carbone dans chaque groupe alkyle ; $-COD^4$ ; carboxy ; alcoxy en $C_{1-4}$-carbonyle ; alcanoyle en $C_{1-4}$-oxy ; alcényle en $C_{2-6}$ ; alcynyle en $C_{2-6}$ ; $-ND^4D^5$ ; thiocyanato ; alkylthio en $C_{1-4}$ ; $-CSD^4$ ; phényle non-substitué ou substitué ayant un ou deux groupes identiques ou différents parmi halo, nitro, alkyle en $C_{1-4}$, alcoxy en $C_{1-4}$, carboxy, $-ND^4D^5$ ; phénoxy, où le noyau phényle est non-substitué ou substitué par 1 ou 2 groupes identiques ou différents parmi halo, nitro, alkyle en $C_{1-4}$, alcoxy en $C_{1-4}$, carboxy, $-ND^4D^5$ ; ou lorsque deux positions adjacentes sur le noyau phényle sont substituées par des groupes alcoxy, ces groupes peuvent être réunis, avec les atomes de carbone auxquels ils sont attachés, pour former un noyau hétérocyclique dioxolano ou dioxano à 5 ou 6 chaînons ;

où $D^4$ et $D^5$ représentent un hydrogène ou un alkyle en $C_{1-4}$

**6.** Composition ou composé selon la revendication 1, 2 ou 4 où :

X et X' représentent O ou S ;

$R_1$ est un alkyle en $C_{3-8}$ ramifié ;

A et B identiques ou différents représentent des naphtyles non-substitués ; des phényles non-substitués ou substitués ayant de 1 à 3 groupes identiques ou différents parmi halo ; nitro ; cyano ; alkyle en $C_{1-4}$ ; halo-alkyl en $C_{1-4}$ ; cyano-alkyle en $C_{1-4}$ ; alcoxy en $C_{1-4}$ ; alcoxy-alkyle ayant indépendamment de 1 à 4 atomes de carbone dans chaque groupe alkyle ; carbonyle ($-COD^4$) ; alcoxy en $C_{1-4}$-carbonyle ; alcanoyloxy en $C_{1-4}$ ; thiocyanato ; phényle non-substitué ou substitué ayant un ou deux groupes identiques ou différents parmi halo, nitro, alkyle en $C_{1-4}$, alcoxy en $C_{1-4}$, carboxy, $-NH_2$, -NHZ, -NZZ' ou phénoxy où le noyau phényle est non-substitué ou substitué par un ou deux groupes identiques ou différents parmi halo, nitro, alkyle en $C_{1-4}$, alcoxy en $C_{1-4}$, carboxy, $-NH_2$, -NHZ ou -NZZ' ;

où $D^4$, Z et Z' sont tels que définis dans les revendications 5 et 3, respectivement.

**7.** Composition ou composé selon la revendication 1, 2 ou 4, où :

X et X' représentent O ;

$R^1$ est un alkyle en $C_{4-7}$ ramifié ; et

A et B sont des groupes phényle ou phényle substitués identiques ou différents où les substituants peuvent être de 1 à 3 groupes identiques ou différents parmi halo, nitro, alkyle en $C_{1-4}$, alcoxy en $C_{1-4}$ ou halo-alkyle en $C_{1-4}$.

8. Composition ou composé selon la revendication 1, 2 ou 4, où
X et X' représentent O ;
$R^1$ est un t -butyle, néopentyl-(2,2-diméthylpropyle) ou 1,2,2-triméthylpropyle ;
A et B représentent des phényles ou phényles substitués identiques ou différents où les substituants peuvent être 1, 2 ou 3 groupes identiques ou différents parmi chloro, fluoro, bromo, iodo, méthyle, éthyle, méthoxy ou trifluorométhyle.

9. Composition ou composé selon la revendication 1, 2 ou 4, où :
X et X' représentent O ;
$R^1$ est un t -butyle ; et
A et B se conforment à l'une des définitions suivantes ;
A est un 4-méthylphényle et B est un 3,5-diméthylphényle ;
A est un phényle et B est un phényle ;
A est un 4-méthylphényle et B est un 3-méthylphényle ;
A est un phényle et B est un 4-chloro-phényle ;
A est un 4-méthylphényle et B est un 3,5-diméthylphényle ;
A est un 2,3-diméthylphényle et B est un 3-méthylphényle ;
A est un 2,3-diméthylphényle et B est un 3,5-diméthylphényle ;
A est un 2,3-diméthylphényle et B est un 2-bromophényle ;
A est un 2,3-diméthylphényle et B est un 2,4(dichlorophényle ;
A est un 2,3-diméthylphényle et B est un 2-chloro-5-méthylphényle ;
A est un phényle et B est un 2-bromophényle ;
A est un phényle et B est un 3,4-dichlorophényle ;
A est un 2-méthyl-3-chlorophényle et B est un phényle ;
A est un 2-méthyl-3-chlorophényle et B est un 2,4-dichlorophényle ;
A est un 2-méthyl-3-bromophényle et B est un 2,4-dichlorophényle ;
A est un 2-dichloro-3-méthylphényle et B est un 2,4-dichlorophényle ;
A est un 2-chloro-3-méthylphényle et B est un 3,5-diméthylphényle ;
A est un 2,6-difluorophényle et B est un 3,4-dichlorophényle ;
A est un 2,6-difluorophényle et B est un 2,4-dichlorophényle ;
A est un 2,6-difluorophényle et B est un 3,5-dichlorophényle ;
A est un 2-fluoro-6-chlorophényle et B est un 2,4-dichlorophényle ;
A est un 2-chlorophényle et B est un 2,4-dichlorophényle ;
A est un phényle et B est un 2,4-dichlorphényle;
A est un 2-méthyl-3-chlorophényle et B est un 4-fluorophényle ;
A est un 2-méthyl-3-chlorophényle et B est un 2-bromophényle ;
A est un 2-méthyl-3-bromophényle et B est un 3-méthylphényle ;
A est un phényle et B est un 3-chloro-4-fluorophényle ;
A est un 2-méthyl-3-bromophényle et B est un 4-chlorophényle ;
ou bien où :
X et X' représentent O ;
$R^1$ est un 1,2,2-triméthylpropyle ;
A est un 4-éthylphényle ou 2,3-diméthylphényle ; et B est un 3,5-diméthylphényle.

10. Composition selon l'une quelconque des revendication 1 et 3 à 9, où l'ingrédient actif est présent à 0,01 jusqu'à 75 % en poids ce la composition.

11. Composition insecticide selon l'une quelconque des revendications 1 et 3 à 10 sous la forme d'un concentré émulsifiable, d'une poudre mouillable, d'un fluide, une poussière, de granulés ou un appât.

12. Procédé de lutte contre les insectes dans lequel on met en contact lesdits insectes avec une quantité efficace comme insecticide d'un composé actif comme insecticide tel que défini dans la revendication 1, éventuellement dans une composition selon l'une quelconque des revendications 1 et 3 à 11.

13. Procédé selon la revendication 12, dans lequel ledit composé insecticide actif est appliqué à des plantes en croissance ou à une zone où l'on doit cultiver les plantes à une dose allant de 10 g à 10 kg/hectare.

**14.** Procédé selon la revendication 13, dans lequel le taux d'application est de 100 g à 5 kg/hectare.

**15.** Procédé selon l'une quelconque des revendications 12 à 14, de lutte contre les insectes des ordres des lépidoptères ou coléoptères.

**16.** Procédé selon l'une quelconque des revendications 12 à 15, dans lequel l'application est effectuée de manière à permettre l'absorption par les racines et le transport par les plantes.

Revendications pour l'Etat contractant suivant : ES

**1.** Procédé pour améliorer la valeur commerciale et/ou la rentabilité de récoltes destinées à être vendues sur le marché provenant de plantes dont la croissance est affectée ou risque d'être affectée par des insectes, et/ou d'amélioration de cette croissance, dans lequel (1) on met dans un récipient un dispositif de fumigation ou un dispositif de dissémination mécanique une composition insecticide comprenant de 0,0001% à 99% en poids de la composition, d'un composé de formule :

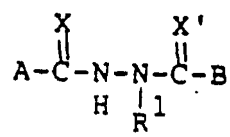

où

X et X', identiques ou différents, représentent O, S ou NR ;

$R^1$ est un alkyle ramifié en $C_{3-10}$ non-substitué ou un alkyle à chaîne droite en $C_{1-4}$ substitué par un ou deux radicaux cycloalkyle en $C_{3-6}$ identiques ou différents ;

A et B identiques ou différents représentent des radicaux naphtyle non-substitués ou substitués ou les substituants peuvent être de un à trois radicaux halo ; nitro ; alcoxy en $C_1$ à $C_4$ ; alkyle en $C_1$ à $C_4$ ou amine ; identiques ou différents phényle non-substitué ou substitué où les substituants peuvent être de un à cinq, identiques ou différents, parmi les radicaux halo ; nitro ; cyano ; hydroxy ; alkyle en $C_{1-6}$ ; halo-(alkyle en $C_{1-6}$) ; cyano(alkyle en $C_{1-6}$) ; hydroxy(alkyle en $C_{1-6}$) ; alcoxy en $C_{1-6}$ ; halo(alcoxy en $C_{1-6}$) ; alcoxyalkyle ayant indépendamment de 1 à 6 atomes de carbone dans chaque groupe alkyle ; alcoxyalcoxy ayant indépendamment de 1 à 6 atomes de carbone dans chaque groupe alkyle ; un groupe -ORSR' ; un groupe $-OCO_2R$ ; un alcanoyloxyalkyle ayant idépendamment de 1 à 6 atomes de carbone dans chaque groupe alkyle ; alcényle en $C_{2-6}$, éventuellement substitué par un halo, cyano, alkyle en $C_{1-4}$ ou alcoxy en $C_{1-4}$ ; alcényloxy en $C_{2-6}$ ; alcényle en $C_{2-6}$-carbonyle ; alcényle en $C_{2-6}$-oxycarbonyle ; alcynyle en $C_{2-6}$ éventuellement substitué par un halo ou un alkyle en $C_{1-4}$ ; un groupe $-RCO_2R'$ ; un groupe COR ; un haloalkyle en $C_{1-6}$-carbonyle ; un groupe $-CO_2R$ ; un halo-alcoxy en $C_{1-6}$-carbonyle ; un groupe -OCOR ; un groupe $-ORCO_2R'$ ; un groupe NRR' ; un groupe -CONRR' ; un alcényle en $C_{2-6}$-carbonylamino ; hydroxyalkyle en $C_{1-6}$-aminocarbonyle ; un groupe -OCONRR' ; un groupe -NRCOR' ; un groupe $-NRCO_2R'$ ; un thiocyanato ; isothiocyanato ; thiocyanato-alkyle en $C_{1-6}$ ; alkyle en $C_{1-6}$-thio ; un groupe -S(O)R ; un groupe $-SO_2R$ ; un groupe $-OSO_2R$ ; un groupe $-SO_2NRR'$ ; un groupe -CSR ; un groupe -NRCSR' ; un phényle non-substitué ou substitué ayant de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; phénoxy où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; benzoyle où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; benzyloxy-alkyle en $C_{1-6}$, phénylthio-alkyle en $C_{1-6}$ où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; $-CR=N-R^2$ où $R^2$ est un hydroxy, alkyle en $C_{1-4}$, alcoxy en $C_{1-4}$, -NRR', phénylamino, -COR, ou benzoyle ; oxiranyle en $C_{2-6}$ ; acétylthiosemicarbazone ; pyrrolyle ; oxazolyle, non substitués ou substitués par un ou deux groupes méthyle ; ou lorsque deux positions adjacentes sur le noyau phényle sont substituées par des groupes alcoxy, ces groupes peuvent être réunis pour former, avec les atomes d'azote auxquels ils sont attachés, un noyau hétérocyclique dioxolano ou dioxano à 5 ou 6 chaînons ;

où R et R' représentent hydrogène ou alkyle en $C_{1-6}$ , Z et Z' représentent alkyle en $C_{1-4}$ et "amino" signifie NRR' ; et leurs sels agronomiquement acceptables ; (2) et un support ou diluant agronomiquement acceptable, on utilise le récipient, fumigateur, ou dispositif de dissémination mécanique pour appliquer la composition insecticide, sous forme de granulés, de poussière, de fumée, de vapeur ou de préparations liquides contenant un agent tensioactif à des plantes qui croissent ou à un milieu de croissance dans lequel les plantes croissent ou sont susceptibles d'être plantées, ou aux insectes eux-mêmes; (3) on contrôle la dose d'ingrédient actif pendant cette étape d'application de manière que le taux d'application du composé insecticide actif soit suffisant pour combattre les insectes, mais soit insuffisant pour provoquer un effet nocif inacceptable sur les récoltes de plantes qui croissent ou qui doivent croître dans la zone traitée.

**2.** Procédé de dissémination selon la revendication 1, dans lequel, dans le composé insecticide, lorsque X et X' représentent O et A et B représentent un phényle non-substitué, $R^1$ n'est pas un isopropyle (-CH-$(CH_3)_2$) ; 2-méthylpropyle ($-CH_2CH(CH_3)_2$) ; 3-méthylbutyle ($-CH_2CH_2CH(CH_3)_2$) ; néopentyle (2,3-diméthylpropyle : $-CH_2C(CH_3)_3$) ; ou cyclohexylméthyle ($-CH_2C_6H_{11}$) et à condition en outre que lorsque X et X' représentent O et $R^1$ est un t -butyle ($-C(CH_3)_3$) et A est un phényle non-substitué, B n'est pas un 4-nitrophényle.

**3.** Procédé de préparation d'un composé à action insecticide de formule

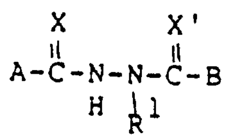

dans lequel X, X', A et B sont tels que définis dans la revendication 1 telle que limitée par la revendication 2, dans lequel on fait réagir un premier réactif (I) contenant le substituant A avec un second réactif (II) contenant le substituant B en présence d'une base et d'un solvant, où lorsque X et X'

(a) le réactif I est de formule 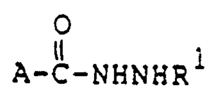
et
le réactif II est de formule 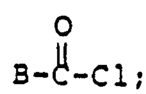 ; ou

(b) le réactif I est de formule 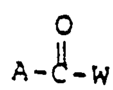
et
le réactif II est de formule 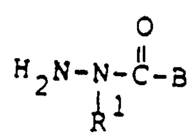

où W est un bon groupe partant ; ou bien où lorsqu'au moins l'un des radicaux X et X' représente S,

(c) le réactif I est de formule 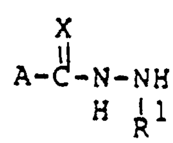
et
le réactif II est de formule 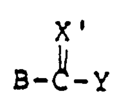

où Y est un bon groupe partant.

représentent tous deux O;

4. Procédé de préparation selon la revendication 3 dans lequel, dans la réaction (a), $R^1$ est un groupe de formule

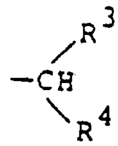

où $R^3$ et $R^4$ sont des groupes identiques ou différents parmi hydrogène ou alkyle à chaîne ramifiée non-substituée en $C_{3-9}$ ou alkyle à chaîne droite non-substitué en $C_{1-8}$, ou cycloalkyle en $C_{3-6}$, ou alkyle à chaîne droite en $C_{1-3}$ substitué par un ou deux cycloalkyle en $C_{3-6}$ ; ou, dans la réaction (b) W représente -C1.

5. Procédé de dissémination ou de préparation selon l'une quelconque des revendications précédentes, dans lequel X, X' et R sont tels que définis et A et B identiques ou différents, sont des groupes naphtyle non-substitués ou substitués où les substituants peuvent être de 1 à 3 groupes identiques ou différents parmi halo ; nitro ; alcoxy en $C_{1-4}$ ; alkyle en $C_{1-4}$ ; ou amino ; phényle non-substitué ou substitué où les substituants peuvent être de 1 à 5 groupes identiques ou différents parmi halo ; nitro ; cyano ; hydroxy ; alkyle en $C_{1-6}$ ; halo-alkyle en $C_{1-6}$ ; cyano-alkyle en $C_{1-6}$ ; alcoxy en $C_{1-6}$ ; halo-alcoxy en $C_{1-6}$ ; alcoxy-alkyle ayant, indépendamment, de 1 à 6 atomes de carbone dans chaque groupe alkyle ; alcoxy-alcoxy ayant indépendamment de 1 à 6 atomes de carbone dans chaque groupe alkyle ; $-OCO_2R$ ; alcényle en $C_{2-6}$, éventuellement substitué par un halo, cyano ; alkyle en $C_{1-4}$ ; ou alcoxy en $C_{1-4}$ ; alcényle en $C_{2-6}$-carbonyle ; alcynyle en $C_{2-6}$ ; éventuellement substitué par un halo ou un alkyle en $C_{1-4}$ ; $-RCO_2R'$ ; -COR ; halo-alkyle en $C_{1-6}$-carbonyle ; $-CO_2R$ ; halo-alcoxy en $C_{1-6}$-carbonyle ; -OCOR ; -NRR' ; -CONRR' ; -OCONRR' ; -NRCOR' ; $-NRCO_2R'$ ; thiocyanato ; alkyle en $C_{1-6}$-thio ; -S(O)R ; $-SO_2R$ ; $-OSO_2R$ ; $-SO_2NRR'$ ; -CSR ; -NRCSR' ; phényle non-substitué ou substitué où les substituants peuvent être de 1 à 3 groupes identiques ou différents parmi halo, cyano, nitro, alkyle en $C_{1-4}$, alcoxy en $C_{1-4}$, carboxy, $-NH_2$, -NHZ ou -NZZ' ; phénoxy où le noyau phényle est non-substitué ou substitué, où les substituants peuvent être 1 à 3 des groupes identiques ou différents parmi halo, cyano, nitro, alkyle en $C_{1-4}$, alcoxy en $C_{1-4}$, carboxy, $-NH_2$, -NHZ ou -NZZ' ; benzoyle où le noyau phényle est non-substitué ou substitué, où les substituants peuvent être de 1 à 3 des groupes identiques ou différents parmi halo, cyano, nitro, alkyle en $C_{1-4}$, alcoxy en $C_{1-4}$, carboxy, $-NH_2$, -NHZ ou -NZZ' ; $-CR=N-R^2$ où $R^2$ est un hydroxy, alkyle en $C_{1-4}$, alcoxy en $C_{1-4}$, amino, phényl-amino, -COR, ou benzoyle ; ou lorsque deux positions adjacentes sur le noyau phényle sont substituées par des groupes alcoxy, ces groupes peuvent être réunis pour former, avec les atomes de carbone auxquels ils sont tous deux attachés, un noyau hétérocyclique dioxolano ou dioxano à 5 ou 6 chaînons

6. Procédé de dissémination ou de préparation selon l'une quelconque des revendications 1 précédentes dans lequel $R^1$ ne contient pas plus de 10 atomes de carbone.

7. Procédé de dissémination ou de préparation selon l'une quelconque des revendications 1 à 4 et 6, dans lequel : X et X' représentent O ou S ; $R^1$ est un alkyle en $C_{3-8}$ ramifié non-substitué ou un alkyle à chaîne droite en $C_{1-4}$ substitué par un ou deux cycloalkyles en $C_{3-4}$ identiques ou différents ; A et B identiques ou différents représentent des naphtyles non-substitués ; ou des phényles non-substitués ou substitués où les substituants peuvent être de 1 à 3 des groupes identiques ou différents parmi halo ; nitro ; cyano ; alkyle en $C_{1-4}$ ; halo-alkyle en $C_{1-4}$ ; cyanoalkyle en $C_{1-4}$ ; alcoxy en $C_{1-4}$ ; alcoxy-alkyle ayant indépendamment de 1 à 4 atomes de carbone dans chaque groupe alkyle ; $-COD^4$ ; carboxy ; alcoxy en $C_{1-4}$-carbonyle ; alcanoyle en $C_{1-4}$-oxy ; alcényle en $C_{2-6}$ ; alcynyle en $C_{2-6}$ ; $-ND^4D^5$ ; thiocyanato ; alkylthio en $C_{1-4}$ ; $-CSD^4$ ; phényle non-substitué ou substitué ayant un ou deux groupes identiques ou différents parmi halo, nitro, alkyle en $C_{1-4}$, alcoxy en $C_{1-4}$, carboxy, $-ND^4D^5$ ; phénoxy, où le noyau phényle est non-substitué ou substitué par 1 ou 2 groupes identiques ou différents parmi halo, nitro, alkyle en $C_{1-4}$, alcoxy en $C_{1-4}$, carboxy, $-ND^4D^5$ ; ou lorsque deux positions adjacentes sur le noyau phényle sont substituées par des groupes alcoxy, ces groupes peuvent être réunis, avec les atomes de carbone auxquels ils sont attachés, pour former un noyau hétérocyclique dioxolano ou dioxano à 5 ou 6 chaînons ; où $D^4$ et $D^5$ représentent un hydrogène ou un

alkyle en $C_{1-4}$ .

8. Procédé de dissémination ou de préparation selon l'une quelconque des revendications 1 à 4 et 6, dans lequel : X et X' représentent O ou S ; $R_1$ est un alkyle en $C_{3-8}$ ramifié ; A et B identiques ou différents représentent des naphtyles non-substitués des phényles non-substitués ou substitués ayant de 1 à 3 groupes identiques ou différents parmi halo ; nitro ; cyano ; alkyle en $C_{1-4}$ ; halo-alkyle en $C_{1-4}$ ; cyano-alkyle en $C_{1-4}$ ; alcoxy en $C_{1-4}$ , alcoxy-alkyle ayant indépendamment de 1 à 4 atomes de carbone dans chaque groupe alkyle ; carbonyle (-$COD^4$) ; alcoxy en $C_{1-4}$-carbonyle ; alcanoyloxy en $C_{1-4}$ ; thiocyanato ; phényle non-substitué ou substitué ayant un ou deux groupes identiques ou différents parmi halo, nitro, alkyle en $C_{1-4}$, alcoxy en $C_{1-4}$, carboxy, -$NH_2$, -NHZ, -NZZ' ou phénoxy où le noyau phényle est non-substitué ou substitué par un ou deux groupes identiques ou différents parmi halo, nitro, alkyle en $C_{1-4}$, alcoxy en $C_{1-4}$, carboxy, -$NH_2$, -NHZ ou -NZZ' ; où $D^4$, Z et Z' sont tels que définis dans les revendications 5 et 3, respectivement.

9. Procédé de dissémination ou de préparation selon l'une quelconque des revendications 1 à 4 et 6, dans lequel : X et X' représentent O ; $R^1$ est un alcoyle en $C_{4-7}$ ramifié ; et A et B sont des groupes phényle ou phényle substitués identiques ou différents où les substituants peuvent être de 1 à 3 groupes identiques ou différents parmi halo, nitro, alkyle en $C_{1-4}$, alcoxy en $C_{1-4}$ ou halo-alkyle en $C_{1-4}$.

10. Procédé de dissémination ou de préparation selon l'une quelconque des revendications 1 à 4 et 6, dans lequel : X et X' représentent O ; $R^1$ est un t -butyle, néopentyl(2,2-diméthylpropyle) ou 1,2,2-triméthylpropyle ; A et B représentent des phényles ou phényles substitués identiques ou différents où les substituants peuvent être 1, 2 ou 3 groupes identiques ou différents parmi chloro, fluoro, bromo, iodo, méthyle, éthyle, méthoxy ou trifluorométhyle.

11. Procédé de dissémination ou de préparation selon l'une quelconque des revendications 1 à 4 et 6 dans lequel : X et X' représentent O ; $R^1$ est un t -butyle ; et A et B se conforment à l'une des définitions suivantes ; A est un 4-méthylphényle et B est un 3,5-diméthylphényle ; A est un phényle et B est un phényle ; A est un 4-méthylphényle et B est un 3-méthylphényle ; A est un phényle et B est un 4-chloro-phényle ; A est un 4-méthylphényle et B est un 3,5-diméthylphényle ; A est un 2,3-diméthylphényle et B est un 3-méthylphényle ; A est un 2,3-diméthylphényle et B est un 3,5-diméthylphényle ; A est un 2,3-diméthylphényle et B est un 2-bromophényle ; A est un 2,3-diméthylphényle et B est un 2,4-dichlorophényle ; A est un 2,3-diméthylphényle et B est un 2-chloro-5-méthylphényle ; A est un phényle et B est un 2-bromophényle ; A est un phényle et B est un 3,4-dichloro-phényle ; A est un 2-méthyl-3-chlorophényle et B est un phényle ; A est un 2-méthyl-3-chlorophényle et B est un 2,4-dichlorophényle ; A est un 2-méthyl-3-bromophényle et B est un 2,4-dichlorophényle ; A est un 2-dichloro-3-méthylphényle et B est un 2,4-dichlorophényle ; A est un 2-chloro-3-méthylphényle et B est un 3,5-diméthylphényle ; A est un 2,6-difluorophényle et B est un 3,4-dichlorophényle ; A est un 2,6-difluorophényle et B est un 2,4-dichlorophényle ; A est un 2,6-difluorophényle et B est un 3,5-dichlorophényle ; A est un 2-fluoro-6-chlorophényle et B est un 2,4-dichlorophényle ; A est un 2-chlorophényle et B est un 2,4-dichlorophényle ; A est un phényle et B est un 2,4-dichlorphényle; A est un 2-méthyl-3-chlorophényle et B est un 4-fluorophényle ; A est un 2-méthyl-3-chlorophényle et B est un 2-bromophényle ; A est un 2-méthyl-3-bromophényle et B est un 3-méthylphényle ; A est un phényle et B est un 3-chloro-4-fluorophényle ; A est un 2-méthyl-3-bromophényle et B est un 4-chlorophényle ; ou bien où : X et X' représentent O ; $R^1$ est un 1,2,2-triméthylpropyle ; A est un 4-éthylphényle ou 2,3-diméthylphényle ; et B est un 3,5-diméthylphényle.

12. Procédé de dissémination selon l'une quelconque des revendications 1, 2 et 5 à 11, dans lequel l'ingrédient actif est présent dans la composition à 0,01 jusqu'à 75 % en poids de la composition.

13. Procédé de dissémination selon l'une quelconque des revendications 1, 2 et 5 à 12, dans lequel la composition est utilisée sous forme d'un concentré émulsifiable, d'une poudre mouillable, d'un fluide, d'une poussière, de granulés ou d'un appât.

14. Procédé de dissémination selon l'une quelconque des revendications 1, 2 et 5 à 13, dans lequel ledit composé à action insecticide est appliqué dans l'étape 2 à des plantes qui croissent ou à une zone dans laquelle on doit cultiver des plantes et dans l'étape 3 la posologie est réglée à l0 g jusqu'à 10

kg/hectare.

**15.** Procédé de dissémination selon l'une quelconque des revendications 1, 2 et 5 à 14 dans lequel, dans l'étape 3, la dose est réglée à un un taux d'application allant de 100 g à 5 kg/hectare.

**16.** Procédé de dissémination selon l'une quelconque des revendications 1,2 et 5 à 15, dans lequel les insectes sont de l'ordre des lépidoptères ou des coléoptères.

Revendications pour l'Etat contractant suivant : AT

**1.** Composition insecticide comprenant comme ingrédient actif à action insecticide, un composé de formule :

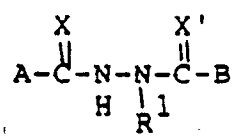

où X et X', identiques ou différents, représentent O, S ou NR ; $R^1$ est un alkyle ramifié en $C_{3-10}$ non-substitué ou un alkyle à chaîne droite en $C_{1-4}$ substitué par un ou deux radicaux cycloalkyle en $C_{3-6}$ identiques ou différents ; A et B identiques ou différents représentent des raradicaux naphtyle non-substitués ou substitués où les substituants peuvent être de un à trois radicaux halo ; nitro ; alcoxy en $C_1$ à $C_4$ ; alkyle en $C_1$ à $C_4$ ou amino ; identiques ou différents ; phényle non-substitué ou substitué où les substituants peuvent être de un à cinq, identiques ou différents, parmi les radicaux halo ; nitro ; cyano ; hydroxy ; alkyle en $C_{1-6}$ ; halo(alkyle en $C_{1-6}$) ; cyano(alkyle en $C_{1-6}$) ; hydroxy(alkyle en $C_{1-6}$) ; alcoxy en $C_{1-6}$ ; halo(alcoxy en $C_{1-6}$) ; alcoxyalkyle ayant indépendamment de 1 à 6 atomes de carbone dans chaque groupe alkyle ; alcoxyalcoxy ayant indépendamment de 1 à 6 atomes de carbone dans chaque groupe alkyle ; un groupe -ORSR' ; un groupe $-OCO_2R$ ; un alcanoyloxyalkyle ayant idépendamment de 1 à 6 atomes de carbone dans chaque groupe alkyle ; alcényle en $C_{2-6}$, éventuellement substitué par un halo, cyano, alkyle en $C_{1-4}$ ou alcoxy en $C_{1-4}$ ; alcényloxy en $C_{2-6}$ ; alcényle en $C_{2-6}$-carbonyle ; alcényle en $C_{2-6}$-oxycarbonyle ; alcynyle en $C_{2-6}$ éventuellement substitué par un halo ou un alkyle en $C_{1-4}$ ; un groupe $-RCO_2R'$ ; un groupe COR ; un halo-alkyle en $C_{1-6}$-carbonyle ; un groupe $-CO_2R$ ; un halo-alcoxy en $C_{1-6}$-carbonyle ; un groupe -OCOR ; un groupe $-ORCO_2R'$ ; un groupe NRR' ; un groupe -CONRR' ; un alcényle en $C_{2-6}$-carbonylamino ; hydroxyalkyle en $C_{1-6}$-aminocarbonyle ; un groupe -OCONRR' ; un groupe -NRCOR' ; un groupe $-NRCO_2R'$ ; un thiocyanato ; isothiocyanato ; thiocyanato-alkyle en $C_{1-6}$ ; alkyle en $C_{1-6}$-thio ; un groupe -S(O)R ; un groupe $-SO_2R$ ; un groupe $-OSO_2R$ ; un groupe $-SO_2NRR'$ ; un groupe -CSR ; un groupe -NRCSR' ; un phényle non-substitué ou substitué ayant de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; phénoxy où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; benzoyle où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; benzyloxy-alkyle en $C_{1-6}$, phénylthio-alkyle en $C_{1-6}$ où le noyau phényle est non-substitué ou substitué par de 1 à 3 groupes identiques ou différents parmi halo, cyano, nitro, alkyle en $C_{1-4}$, halo-alkyle en $C_{1-4}$, alcoxy en $C_{1-4}$, carboxy, $-NH_2$, -NHZ ou -NZZ' ; $-CR=N-R^2$ où $R^2$ est un hydroxy, alkyle en $C_{1-4}$, alcoxy en $C_{1-4}$, -NRR', phénylamino, -COR, ou benzoyle ; oxiranyle en $C_{2-6}$ ; acétylthiosemicarbazone ; pyrrolyle ; oxazolyle, non substitués ou substitués par un ou deux groupes méthyle ; ou lorsque deux positions adjacentes sur le noyau phényle sont substituées par des groupes alcoxy, ces groupes peuvent être réunis pour former, avec les atomes d'azote auxquels ils sont attachés, un noyau hétérocyclique dioxolano ou dioxano à 5 ou 6 chaînons ; où R et R' représentent hydrogène ou alkyle en $C_{1-6}$ , Z et z' représentent alkyle en $C_{1-4}$ et "amino" signifie NRR' ; et leurs sels agronomiquement acceptables ; et un diluant ou support agronomiquement acceptable.

**2.** Composition insecticide selon la revendication 1 où dans l'ingrédient à action insecticide, lorsque X et X' représentent O et A et B représentent un phényle non-substitué, $R^1$ n'est pas un isopropyle (-CH-

$(CH_3)_2)$ ; un 2-méthylpropyle ($-CH_2CH(CH_3)_2$) ; un 3-méthylbutyle ($-CH_2CH_2CH(CH_3)_2$) ; un néopentyle (2,2-diméthylpropyle : $-CH_2C(CH_3)_3$) ; ou un cyclohexylméthyle ($-CH_2C_6H_{11}$) et à condition en outre que lorsque X et X' représentent O et $R^1$ est un t -butyle ($-C(CH_3)_3$) et A est un phényle non-substitué, B n'est pas un 4-nitrophényle.

3. Composition selon la revendication 1 ou 2, où X, X' et R sont tels que définis et A et B identiques ou différents sont des groupes naphtyle non-substitués ou substitués ; où les substituants peuvent être de 1 à 3 groupes identiques ou différents parmi halo ; nitro ; alcoxy en $C_{1-4}$ ; alkyle en $C_{1-4}$ ; ou amino ; phényle non-substitué ou substitué où les substituants peuvent être de 1 à 5 groupes identiques ou différents parmi halo ; nitro ; cyano , hydroxy ; alkyle en $C_{1-6}$ ; halo-alkyle en $C_{1-6}$ ; cyano-alkyle en $C_{1-6}$ ; alcoxy en $C_{1-6}$ ; halo-alcoxy en $C_{1-6}$ ; alcoxy-alkyle ayant, indépendamment, de 1 à 6 atomes de carbone dans chaque groupe alkyle ; alcoxy-alcoxy ayant indépendamment de 1 à 6 atomes de carbone dans chaque groupe alkyle ; $-OCO_2R$ ; alcényle en $C_{2-6}$, éventuellement substitué par un halo, cyano ; alkyle en $C_{1-4}$ ; ou alcoxy en $C_{1-4}$ ; alcényle en $C_{2-6}$-carbonyle ; alcynyle en $C_{2-6}$ ; éventuellement substitué par un halo ou un alkyle en $C_{1-4}$ ; $-RCO_2R'$ ; -COR ; halo-alkyle en $C_{1-6}$-carbonyle ; $-CO_2R$ ; halo-alcoxy en $C_{1-6}$-carbonyle ; -OCOR ; -NRR' ; -CONRR' ; -OCONRR' ; -NRCOR' ; $-NRCO_2R'$ ; thiocyanato ; alkyle en $C_{1-6}$-thio ; -S(O)R ; $-SO_2R$ ; $-OSO_2R$ ; $-SO_2NRR'$ ; -CSR ; -NRCSR' ; phényle non-substitué ou substitué où les substituants peuvent être de 1 à 3 groupes identiques ou différents parmi halo, cyano, nitro, alkyle en $C_{1-4}$, alcoxy en $C_{1-4}$, carboxy, $-NH_2$, -NHZ ou -NZZ' ; phénoxy où le noyau phényle est non-substitué ou substitué, où les substituants peuvent être 1 à 3 des groupes identiques ou différents parmi halo, cyano, nitro, alkyle en $C_{1-4}$, alcoxy en $C_{1-4}$, carboxy, $-NH_2$, -NHZ ou -NZZ' ; benzoyle ou le noyau phenyle est non-substititué ou substitué ; où les substituants peuvent être de 1 à 3 des groupes identiques ou différents parmi halo, cyano, nitro, alcoyle en $C_{1-4}$, alcoxy en $C_{1-4}$, carboxy, $NH_2$, -NHZ ou -NZZ' ; $-CR=N-R^2$ où $R^2$ est un hydroxy, alkyle en $C_{1-4}$, alcoxy en $C_{1-4}$, amino, phénylamino, -COR, ou benzoyle ; ou lorsque deux positions adjacentes sur le noyau phényle sont substituées par des groupes alcoxy, ces groupes peuvent être réunis pour former, avec les atomes de carbone auxquels ils sont tous deux attachés, un noyau hétérocyclique dioxolano ou dioxano à 5 ou 6 chaînons

4. Composition ou composé selon l'une quelconque des revendications précédentes, où $R^1$ ne contient pas plus de 10 atomes de carbone.

5. Composition ou composé selon la revendication 1, 2 ou 4 où : X et X' représentent O ou S ; $R^1$ est un alkyle en $C_{3-8}$ ramifié non-substitué ou un alkyle à chaîne droite en $C_{1-4}$ substitué par un ou deux cycloalkyles en $C_{3-4}$ identiques ou différents ; A et B identiques ou différents représentent des naphtyles non-substitués ; ou des phényles non-substitués ou substitués où les substituants peuvent être de 1 à 3 des groupes identiques ou différents parmi halo ; nitro ; cyano ; alkyle en $C_{1-4}$ ; halo-alkyle en $C_{1-4}$ ; cyano-alkyle en $C_{1-4}$ ; alcoxy en $C_{1-4}$ ; alcoxyalkyle ayant indépendamment de 1 à 4 atomes de carbone dans chaque groupe alkyle ; $-COD^4$ ; carboxy ; alcoxy en $C_{1-4}$-carbonyle ; alcanoyle en $C_{1-4}$-oxy ; alcényle en $C_{2-6}$ ; alcynyle en $C_{2-6}$ ; $-ND^4D^5$ ; thiocyanato ; alkylthio en $C_{1-4}$ ; $-CSD^4$ ; phényle non-substitué ou substitué ayant un ou deux groupes identiques ou différents parmi halo, nitro, alkyle en $C_{1-4}$, alcoxy en $C_{1-4}$, carboxy, $-ND^4D^5$ ; phénoxy, où le noyau phényle est non-substitué ou substitué par 1 ou 2 groupes identiques ou différents parmi halo, nitro, alkyle en $C_{1-4}$, alcoxy en $C_{1-4}$, carboxy, $-ND^4D^5$ ; ou lorsque deux positions adjacentes sur le noyau phényle sont substituées par des groupes alcoxy, ces groupes peuvent être réunis, avec les atomes de carbone auxquels ils sont attachés, pour former un noyau hétérocyclique dioxolano ou dioxano à 5 ou 6 chaînons ; où $D^4$ et $D^5$ représentent un hydrogène ou un alkyle en $C_{1-4}$ .

6. Composition ou composé selon la revendication 1, 2 ou 4 où : X et X' représentent O ou S ; $R_1$ est un alkyle en $C_{3-8}$ ramifié ; A et B identiques ou différents représentent des naphtyles non-substitués ; des phényles non-substitués ou substitués ayant de 1 à 3 groupes identiques ou différents parmi halo ; nitro ; cyano ; alkyle en $C_{1-4}$ ; halo-alkyle en $C_{1-4}$ ; cyano-alkyle en $C_{1-4}$ ; alcoxy en $C_{1-4}$ , alcoxy-alkyle ayant indépendamment de 1 à 4 atomes de carbone dans chaque groupe alkyle ; carbonyle ($-COD^4$) ; alcoxy en $C_{1-4}$-carbonyle ; alcanoyloxy en $C_{1-4}$ ; thiocyanato ; phényle non-substitué ou substitué ayant un ou deux groupes identiques ou différents parmi halo, nitro, alkyle en $C_{1-4}$, alcoxy en $C_{1-4}$, carboxy, $-NH_2$, -NHZ, -NZZ' ou phénoxy où le noyau phényle est non-substitué ou substitué par un ou deux groupes identiques ou différents parmi halo, nitro, alkyle en $C_{1-4}$, alcoxy en $C_{1-4}$, carboxy, $-NH_2$, -NHZ ou -NZZ' ; où $D^4$, Z et Z' sont tels que définis dans les revendications 5 et 3,

respectivement.

7. Composition ou composé selon la revendication 1, 2 ou 4, où : X et X' représentent O ; $R^1$ est un alkyle en $C_{4-7}$ ramifié ; et A et B sont des groupes phényle ou phényle substitués identiques ou différents où les substituants peuvent être de 1 à 3 groupes identiques ou différents parmi halo, nitro, alkyle en $C_{1-4}$, alcoxy en $C_{1-4}$ ou halo-alkyle en $C_{1-4}$.

8. Composition ou composé selon la revendication 1, 2 ou 4, où X et X' représentent O ; $R^1$ est un t-butyle, néopentyl-(2,2-diméthylpropyle) ou 1,2,2-triméthylpropyle ; A et B représentent des phényles ou phényles substitués identiques ou différents où les substituants peuvent être 1, 2 ou 3 groupes identiques ou différents parmi chloro, fluoro, bromo, iodo, méthyle, éthyle, méthoxy ou trifluorométhyle.

9. Composition ou composé selon la revendication 1, 2 ou 4, où : X et X' représentent O ; $R^1$ est un t-butyle ; et A et B se conforment à l'une des définitions suivantes ;
A est un 4-méthylphényle et B est un 3,5-diméthylphényle ;
A est un phényle et B est un phényle ;
A est un 4-méthylphényle et B est un 3-méthylphényle ;
A est un phényle et B est un 4-chloro-phényle ;
A est un 4-méthylphényle et B est un 3,5-diméthylphényle ;
A est un 2,3-diméthylphényle et B est un 3-méthylphényle ;
A est un 2,3-diméthylphényle et B est un 3,5-diméthylphényle ;
A est un 2,3-diméthylphényle et B est un 2-bromophényle ;
A est un 2,3-diméthylphényle et B est un 2,4(dichlorophényle ;
A est un 2,3-diméthylphényle et B est un 2-chloro-5-méthylphényle ;
A est un phényle et B est un 2-bromophényle ;
A est un phényle et B est un 3,4-dichlorophényle ;

A est un 2-méthyl-3-chlorophényle et B est un phényle ;
A est un 2-méthyl-3-chlorophényle et B est un 2,4-dichlorophényle ;
A est un 2-méthyl-3-bromophényle et B est un 2,4-dichlorophényle ;
A est un 2-dichloro-3-méthylphényle et B est un 2,4-dichlorophényle ;
A est un 2-chloro-3-méthylphényle et B est un 3,5-diméthylphényle ;
A est un 2,6-difluorophényle et B est un 3,4-dichlorophényle ;
A est un 2,6-difluorophényle et B est un 2,4-dichlorophényle ;
A est un 2,6-difluorophényle et B est un 3,5-dichlorophényle ;
A est un 2-fluoro-6-chlorophényle et B est un 2,4-dichlorophényle ;
A est un 2-chlorophényle et B est un 2,4-dichlorophényle ;
A est un phényle et B est un 2,4-dichlorphényle;
A est un 2-méthyl-3-chlorophényle et B est un 4-fluorophényle ;
A est un 2-méthyl-3-chlorophényle et B est un 2-bromophényle ;
A est un 2-méthyl-3-bromophényle et B est un 3-méthylphényle ;
A est un phényle et B est un 3-chloro-4-fluorophényle ;
A est un 2-méthyl-3-bromophényle et B est un 4-chlorophényle ;
ou bien où : X et X' représentent O ; $R^1$ est un 1,2,2-triméthylpropyle ; A est un 4-éthylphényle ou 2,3-diméthylphényle ; et B est un 3,5-diméthylphényle.

10. Composition selon l'une quelconque des revendications précédentes, dans laquelle l'ingrédient actif est présent à 0,01 jusqu'à 75 % en poids de la composition.

11. Composition insecticide selon l'une quelconque des revendications précédentes, sous la forme d'un concentré émulsifiable, d'une poudre mouillable, d'un fluide, d'une poussière, de granulés ou d'un appât.

12. Procédé de lutte contre les insectes dans lequel on met en contact lesdits insectes avec une quantité efficace comme insecticide d'un composé actif comme insecticide tel que défini dans la revendication 1, éventuellement dans une composition selon l'une quelconque des revendications précédentes.

13. Procédé selon la revendication 12, dans lequel ledit composé insecticide actif est appliqué à des

plantes en croissance ou à une zone où l'on doit cultiver les plantes à une dose allant de 10 g à 10 kg/hectare.

**14.** Procédé selon la revendication 13, dans lequel le taux d'application est de 100 g à 5 kg/hectare.

**15.** Procédé selon l'une quelconque des revendications 12 à 14, de lutte contre les insectes des ordres des lépidoptères ou coléoptères.

**16.** Procédé selon l'une quelconque des revendications 12 à 15, dans lequel l'application est effectuée de manière à permettre l'absorption par les racines et le transport par les plantes.

## Ansprüche

**1.** Insektizide Zusammensetzung, umfassend als insektizid-wirksamen Bestandteil von 0,0001 bis 99 Gew.-% der Zusammensetzung eine Verbindung der Formel

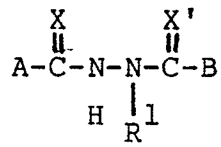

in der X und X' gleich oder verschieden voneinander O, S oder NR sind, $R^1$ ein unsubstituiertes verzweigtes ($C_3$-$C_{10}$)-Alkyl oder ein geradkettiges ($C_1$-$C_4$)-Alkyl ist, das mit einem oder zwei des gleichen bzw. eines unterschiedlichen ($C_3$-$C_6$)-Cycloalkyls substituiert ist, A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)-alkyl, Hydroxy($C_1$-$C_6$)-alkyl, ($C_1$-$C_6$)-Alkoxy, Halogen($C_1$-$C_6$)-alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein -ORSR'-Rest, ein $-OCO_2R'$-Rest, Alkanoyl-oxyalkyl, das unabhängig 1 bis 6 C-Atome in jeder Alkylgruppe aufweist, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyloxy, ($C_2$-$C_6$)-Alkenyl-carbonyl, ($C_2$-$C_6$)-Alkenyl-oxycarbonyl, ($C_2$-$C_6$)-Alkynyl, das gegebenenfalls mit Halogen oder ($C_1$-$C_4$)-Alkyl substituiert ist, ein $-RCO_2R'$-Rest, ein -COR-Rest, Halogen($C_1$-$C_6$)-alkyl-carbonyl, ein $-CO_2R$-Rest, Halogen($C_1$-$C_6$)-alkoxy-carbonyl, ein -OCOR-Rest, ein $-ORCO_2R'$-Rest, ein -NRR'-Rest, ein -CONRR'-Rest, ($C_2$-$C_6$)-Alkenyl-carbonylamino, Hydroxy($C_1$-$C_6$)-alkyl-aminocarbonyl, ein -OCONRR'-Rest, ein -NRCOR'-Rest, ein $-NRCO_2R'$-Rest, Thiocyanato, Isothiocyanato, Thiocyanato($C_1$-$C_6$)-alkyl, ($C_1$-$C_6$)-Alkyl-thio, ein -S(O)R-Rest, ein $-SO_2R$-Rest, ein $-OSO_2R$-Rest, ein $-SO_2NRR'$-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyloxy($C_1$-$C_6$)-alkyl, Phenylthio($C_1$-$C_6$)-alkyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, ein $-CR=N-R^2$-Rest, bei dem $R^2$ Hydroxy, ($C_1$-$C_4$)-Alkyl, ($C_1$-$C_4$)-Alkoxy, -NRR', Phenylamino, -COR oder Benzoyl ist, ($C_2$-$C_6$)-Oxiranyl, Acethylthiosemicarbazon, Pyrrolyl, ein unsubstituiertes oder mit ein oder zwei Methyl-Resten substituiertes Oxazolyl sein können, oder wenn zwei benachbarte Positionen auf dem Phenylring mit Alkoxy-Resten substituiert sind, diese Reste verbunden sein können, um zusammen mit den C-Atomen, mit denen sie

verknüpft sind, einen 5- oder 6-gliedrigen dioxolano- bzw. dioxano-heterocyclischen Ring auszubilden, wobei R und R' Wasserstoff oder ($C_1$-$C_6$)-Alkyl sind, und Z' ($C_1$-$C_4$)-Alkyl sind und "Amino" NRR' bedeutet, und ihre landwirtschaftlich verträglichen Salze, mit der Maßgabe, daß wenn X und X' 0 und A und B ein unsubstituiertes Phenyl sind, $R^1$ nicht Isopropyl ($-CH(CH_3)_2$), 2-Methylpropyl ($-CH_2CH(CH_3)_2$), 3-Methylbutyl ($-CH_2CH_2CH(CH_3)_2$), Neopentyl (2,2-dimethylpropyl: $-CH_2C(CH_3)_3$) oder Cyclohexylmethyl ($-CH_2C_6H_{11}$) ist und ferner mit der Maßgabe, daß wenn X und X' 0 sind und $R^1$ t-Butyl ($-C(CH_3)_3$) und A ein unsubstituiertes Phenyl ist, B nicht 4-Nitrophenyl ist, sowie ein landwirtschaftlich verträgliches Verdünnungsmittel bzw. ein Träger.

2. Insektizide Verbindung der Formel

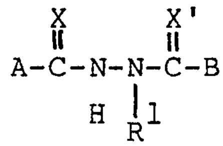

in der X und X' gleich oder verschieden voneinander O, S oder NR sind, $R^1$ ein unsubstituiertes verzweigtes ($C_3$-$C_{10}$)-Alkyl oder ein geradkettiges ($C_1$-$C_4$)-Alkyl ist, das mit einem oder zwei des gleichen bzw. eines unterschiedlichen ($C_3$-$C_6$)-Cycloalkyls substituiert ist, A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, ($C_1$-$C_6$)-Alkoxy, Halogen($C_1$-$C_6$)alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein -ORSR'-Rest, ein $-OCO_2R'$-Rest, Alkanoyl-oxyalkyl, das unabhängig 1 bis 6 C-Atome in jeder Alkylgruppe aufweist, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyloxy, ($C_2$-$C_6$)-Alkenyl-carbonyl, ($C_2$-$C_6$)-Alkenyl-oxycarbonyl, ($C_2$-$C_6$)-Alkynyl, das gegebenenfalls mit Halogen oder ($C_1$-$C_4$)-Alkyl substituiert ist, ein $-RCO_2R'$-Rest, ein -COR-Rest, Halogen($C_1$-$C_6$)-alkyl-carbonyl, ein $-CO_2R$-Rest, Halogen($C_1$-$C_6$)-alkoxy-carbonyl, ein -OCOR-Rest, ein $-ORCO_2R'$-Rest, ein -NRR'-Rest, ein -CONRR'-Rest, ($C_2$-$C_6$)-Alkenyl-carbonylamino, Hydroxy($C_1$-$C_6$)-alkyl-aminocarbonyl, ein -OCONRR'-Rest, ein -NRCOR'-Rest, ein $-NRCO_2R'$-Rest, Thiocyanato, Isothiocyanato, Thiocyanato($C_1$-$C_6$)-alkyl, ($C_1$-$C_6$)-Alkyl-thio, ein -S(O)R-Rest, ein $-SO_2R$-Rest, ein $-OSO_2R$-Rest, ein $-SO_2NRR'$-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyloxy($C_1$-$C_6$)-alkyl, Phenylthio($C_1$-$C_6$)-alkyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, ein $-CR=N-R^2$-Rest, bei dem $R^2$ Hydroxy, ($C_1$-$C_4$)-Alkyl, ($C_1$-$C_4$)-Alkoxy, -NRR', Phenylamino, -COR oder Benzoyl ist, ($C_2$-$C_6$)-Oxiranyl, Acethylthiosemicarbazon, Pyrrolyl, ein unsubstituiertes oder mit ein oder zwei Methyl-Resten substituiertes Oxazolyl sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden, wobei R und R' Wasserstoff oder ($C_1$-$C_6$)-Alkyl sind, und Z' ($C_1$-$C_4$)-Alkyl sind und "Amino" NRR' bedeutet, und ihre landwirtschaftlich verträglichen Salze mit der Maßgabe, daß wenn X und X' 0 und A und B ein unsubstituiertes Phenyl sind, $R^1$ nicht Isopropyl ($-CH(CH_3)_2$), 2-Methylpropyl ($-CH_2CH(CH_3)_2$), 3-Methylbutyl ($-CH_2CH_2CH(CH_3)_2$), Neopentyl (2,2-dimethylpropyl: $-CH_2C(CH_3)_3$) oder Cyclohexylmethyl ($-CH_2C_6H_{11}$) ist und ferner mit der Maßgabe, daß wenn X und X' 0 sind und $R^1$ t-Butyl ($-C(CH_3)_3$) und A ein unsubstituiertes Phenyl ist, B nicht 4-Nitrophenyl ist.

3. Zusammensetzung oder Verbindung nach Anspruch 1 oder 2, bei der X, X' und R wie angegeben sind und A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)alkyl, ($C_1$-$C_6$)-Alkoxy, Halogen($C_1$-$C_6$)alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein -$OCO_2$R-Rest, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyl-carbonyl, ($C_2$-$C_6$)-Alkynyl, das gegebenenfalls mit Halogen oder ($C_1$-$C_4$)-Alkyl substituiert ist, ein -$RCO_2$R'Rest, ein -COR-Rest, Halogen($C_1$-$C_6$)-Alkyl-carbonyl, ein -$CO_2$R-Rest, Halogen($C_1$-$C_6$)-Alkoxy-carbonyl, ein -OCOR-Rest, ein -NRR'-Rest, ein -CONRR'-Rest, ein -OCONRR'-Rest, ein -NRCOR'-Rest, ein -$NRCO_2$R'-Rest, Thiocyanato, ($C_1$-$C_6$)-Alkyl-thio, ein -S(O)R-Rest, ein -$SO_2$R-Rest, ein -$OSO_2$R-Rest, ein -$SO_2$NRR'-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl,($C_1$-$C_4$)-Alkoxy, Carboxy, ein -$NH_2$-Rest, ein -NHZ-Rest oder ein -NZZ'-Rest sein können, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein -$NH_2$-Rest, ein -NHZ-Rest oder ein -NZZ'-Rest sein können, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein -$NH_2$-Rest, ein -NHZ-Rest oder ein -NZZ'-Rest sein können, -CR=N-$R^2$, bei dem $R^2$ Hydroxy, ($C_1$-$C_4$)-Alkyl, ($C_1$-$C_4$)-Alkoxy, Amino, Phenylamino, -COR oder Benzoyl ist, sein können, oder wenn zwei benachbarte Positionen auf dem Phenylring mit Alkoxy-Resten substituiert sind, diese Gruppen verbunden sein können, um zusammen mit den C-Atomen, mit denen sie verknüpft sind, einen 5- oder 6-gliedrigen dioxolano- bzw. dioxano-heterocyclischen Ring auszubilden.

4. Zusammensetzung oder Verbindung nach einem der vorhergehenden Ansprüche, bei der $R^1$ nicht mehr als 10 C-Atome enthält.

5. Zusammensetzung oder Verbindung nach Anspruch 1, 2 oder 4, bei der X und X' O oder S sind, $R^1$ ein unsubstituiertes verzweigtes ($C_3$-$C_8$)-Alkyl oder ein geradkettiges ($C_1$-$C_4$)-Alkyl ist, das mit einem oder zwei des gleichen bzw. eines unterschiedlichen ($C_3$-$C_4$)-Cycloalkyls substituiert ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen($C_1$-$C_4$)-alkyl, Cyano($C_1$-$C_4$)-alkyl, ($C_1$-$C_4$)-Alkoxy, Alkoxyalkyl, das unabhängig 1 bis 4 C-Atome in jedem Alkyl-Rest enthält, -$COD^4$, Carboxy, ($C_1$-$C_4$)-Alkoxy-carbonyl, ($C_1$-$C_4$)-Alkanoyloxy, ($C_2$-$C_6$)-Alkenyl, ($C_2$-$C_6$)-Alkynyl, -$ND^4D^5$, Thiocyanato, ($C_1$-$C_4$)-Alkylthio, -$CSD^4$, ein unsustituiertes oder substituiertes Phenyl, bei dem ein oder zwei Substituenten gleich oder verschieden voneinander Halogen, Nitro, ($C_1$-$C_4$)-Alkyl, ($C_1$-$C_4$)-Alkoxy, Carboxy, -$ND^4D^5$ sind, Phenoxy, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, -$ND^4D^5$ sind, sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden, wobei $D^4$ und $D^5$ Wasserstoff oder ($C_1$-$C_4$)-Alkyl sind.

6. Zusammensetzung nach Anspruch 1, 2 oder 4, bei der X und X' O oder S sind, $R^1$ ein verzweigtes ($C_3$-$C_8$)-Alkyl ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen($C_1$-$C_4$)-alkyl, Cyano-($C_1$-$C_4$)-alkyl, ($C_1$-$C_4$)-Alkoxy, Alkoxyalkyl, das unabhängig 1 bis 4 C-Atome in jedem Alkyl-Rest enthält, Carbonyl (-$COD^4$), ($C_1$-$C_4$)-Alkoxy-carbonyl, ($C_1$-$C_4$)-Alkanoyloxy, Thiocyanato, ein unsubstituiertes oder substituiertes Phenyl, bei dem ein oder zwei Substituenten gleich oder verschieden voneinander Halogen, Nitro, ($C_1$-$C_4$)-Alkyl, ($C_1$-$C_4$-Alkoxy, Carboxy, -$NH_2$, -NHZ, -NZZ' sind, oder Phenoxy sind, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, -$NH_2$, -NHZ oder -NZZ' sind, wobei $D^4$, Z und Z' entsprechend ihrer Bedeutung in den Ansprüchen 5 und 3 sind.

7. Zusammensetzung oder Verbindung nach Anspruch 1, 2 oder 4, bei der X und X' 0 sind, $R^1$ ein verzweigtes ($C_4$-$C_7$)-Alkyl ist, und A und B gleich oder verschieden voneinander Phenyl oder ein substituiertes Phenyl sind, bei dem ein bis drei Substituenten gleich oder verschieden voneinander Halogen, Nitro, ($C_1$-$C_4$)-Alkyl, ($C_1$-$C_4$)-Alkoxy oder Halogen($C_1$-$C_4$)alkyl sein können.

8. Zusammensetzung oder Verbindung nach Anspruch 1, 2 oder 4, bei der X und X' O sind, $R^1$ t-Butyl, Neopentyl (2,2-dimethylpropyl) oder 1,2,2-Trimethylpropyl ist, A und B gleich oder verschieden voneinander Phenyl oder ein substituiertes Phenyl sind, bei dem ein, zwei oder drei Substituenten gleich oder verschieden voneinander Chlor, Fluor, Brom, Jod, Methyl, Ethyl, Methoxy oder Trifluormethyl sein können.

9. Zusammensetzung oder Verbindung nach Anspruch 1, 2 oder 4, bei der X und X' 0 sind, $R^1$ t-Butyl ist, und A und B eine der folgenden Bedeutungen haben: A ist 4-Methylphenyl und B 3,5-Dimethylphenyl;
A ist Phenyl und B Phenyl; A ist 4-Methylphenyl und B 3-Methylphenyl;
A ist Phenyl und B 4-Chlorphenyl;
A ist 4-Methylphenyl und B 3,5-Dimethylphenyl;
A ist 2,3-Dimethylphenyl und B 3-Methylphenyl;
A ist 2,3-Dimethylphenyl und B 3,5-Dimethylphenyl;
A ist 2,3-Dimethylphenyl und B 2-Bromphenyl;
A ist 2,3-Dimethylphenyl und B 2,4-Dichlorphenyl;
A ist 2,3-Dimethylphenyl und B 2-Chlor-5-methylphenyl;
A ist Phenyl und B 2-Bromphenyl;
A ist Phenyl und B 3,4-Dichlorphenyl;
A ist 2-Methyl-3-chlorphenyl und B Phenyl;
A ist 2-Methyl-3-chlorphenyl und B 2,4-Dichlorphenyl;
A ist 2-Methyl-3-bromphenyl und B 2,4-Dichlorphenyl;
A ist 2-Chlor-3-methylphenyl und B 2,4-Dichlorphenyl;
A ist 2-Chlor-3-methylphenyl und B 3,5-Dimethylphenyl;
A ist 2,6-Difluorphenyl und B 3,4-Dichlorphenyl;
A ist 2,6-Difluorphenyl und B 2,4-Dichlorphenyl;
A ist 2,6-Difluorphenyl und B 3,5-Dichlorphenyl;
A ist 2-Fluor-6-chlorphenyl und B 2,4-Dichlorphenyl;
A ist 2-Chlorphenyl und B 2,4-Dichlorphenyl;
A ist Phenyl und B 2,4-Dichlorphenyl;
A ist 2-Methyl-3-chlorphenyl und B 3-Fluorphenyl;
A ist 2-Methyl-3-chlorphenyl und B 2-Bromphenyl;
A ist 2-Methyl-3-Bromphenyl und B 3-Methylphenyl;
A ist Phenyl und B 3-Chlor-4-fluorphenyl;
A ist 2-Methyl-3-Bromphenyl und B 4-Chlorphenyl;
oder bei der:
X und X' 0 sind,
$R^1$ 1,2,2-Trimethylpropyl ist,
A 4-Ethylphenyl oder 2,3-Dimethylphenyl ist, und
B 3,5-Dimethylphenyl ist.

10. Zusammensetzung nach einem der Ansprüche 1 und 3 bis 9, bei der der wirksame Bestandteil 0,01 bis 75 Gew.-% der Zusammensetzung beträgt.

11. Insektizide Zusammensetzung nach einem der Ansprüche 1 und 3 bis 10 in Form eines emulgierbaren Konzentrats, eines benetzbaren Pulvers, eines fließbaren Stoffes, eines Staubes, eines Granulats oder eines Köders.

12. Verfahren zur Überwachung von Insekten, umfassend das Inkontaktbringen der Insekten mit einer insektizid-wirksamen Menge einer wie in Anspruch 1 angegebenen insektizid-wirksamen Verbindung, gegebenenfalls in einer Zusammensetzung nach einem der Ansprüche 1 und 3 bis 11.

13. Verfahren nach Anspruch 12, bei dem die insektizid-wirksame Verbindung wachsenden Pflanzen oder

einem Bereich, wo Pflanzen wachsen sollen, in einer Dosierungsrate von 10 g bis 10 kg pro ha zugegeben wird.

14. Verfahren nach Anspruch 13, bei dem die Applikationsrate von 100 g bis 5 kg pro ha reicht.

15. Verfahren nach einem der Ansprüche 12 bis 14, bei dem die zu überwachenden Insekten der Ordnung Lepidoptera oder Coleoptera angehören.

16. Verfahren nach einem der Ansprüche 12 bis 15, bei dem die Applikation so ausgeführt wird, daß Wurzel-Absorption und Transport durch Pflanzen ermöglicht ist.

Patentansprüche für folgenden Vertragsstaat : AT

1. Insektizide Zusammensetzung, umfassend als insektizid-wirksamen Bestandteil eine Verbindung der Formel

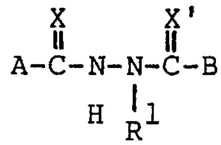

in der X und X' gleich oder verschieden voneinander O, S oder NR sind, $R^1$ ein unsubstituiertes verzweigtes ($C_3$-$C_{10}$)-Alkyl oder ein geradkettiges ($C_1$-$C_4$)-Alkyl ist, das mit einem oder zwei des gleichen bzw. eines unterschiedlichen ($C_3$-$C_6$)-Cycloalkyls substituiert ist, A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)-alkyl, Hydroxy($C_1$-$C_6$)-alkyl, ($C_1$-$C_6$)-Alkoxy, Halogen($C_1$-$C_6$)-alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein -ORSR'-Rest, ein $-OCO_2R'$-Rest, Alkanoyl-oxyalkyl, das unabhängig 1 bis 6 C-Atome in jeder Alkylgruppe aufweist, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyloxy, ($C_2$-$C_6$)-Alkenylcarbonyl, ($C_2$-$C_6$)-Alkenyl-oxycarbonyl, ($C_2$-$C_6$)-Alkynyl, das gegebenenfalls mit Halogen oder ($C_1$-$C_4$)-Alkyl substituiert ist, ein $-RCO_2R'$-Rest, ein -COR-Rest, Halogen($C_1$-$C_6$)-alkyl-carbonyl, ein $-CO_2R$-Rest, Halogen($C_1$-$C_6$)-alkoxy-carbonyl, ein -OCOR-Rest, ein $-ORCO_2R'$-Rest, ein -NRR'-Rest, ein -CONRR'-Rest, ($C_2$-$C_6$)-Alkenyl-carbonylamino, Hydroxy($C_1$-$C_6$)-alkyl-aminocarbonyl, ein -OCONRR'-Rest, ein -NRCOR'-Rest, ein $-NRCO_2R'$-Rest, Thiocyanato, Isothiocyanato, Thiocyanato($C_1$-$C_6$)-alkyl, ($C_1$-$C_6$)-Alkyl-thio, ein -S(O)R-Rest, ein $-SO_2R$-Rest, ein $-OSO_2R$-Rest, ein $-SO_2NRR'$-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, Benzoyloxy($C_1$-$C_6$)-alkyl, Phenylthio($C_1$-$C_6$)-alkyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein $-NH_2$-Rest, ein -NHZ-Rest oder ein -NZZ'-Rest sind, ein $-CR=N-R^2$-Rest, bei dem $R^2$ Hydroxy, ($C_1$-$C_4$)-Alkyl, ($C_1$-$C_4$)-Alkoxy, -NRR', Phenylamino, -COR oder Benzoyl ist, ($C_2$-$C_6$)-Oxiranyl, Acethylthiosemicarbazon, Pyrrolyl, ein unsubstituiertes oder mit ein oder zwei Methyl-Resten substituiertes Oxazolyl sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden,

wobei R und R' Wasserstoff oder ($C_1$-$C_6$)-Alkyl sind, Z und Z' ($C_1$-$C_4$)-Alkyl sind und "Amino" NRR' bedeutet, und ihre landwirtschaftlich verträglichen Salze sowie ein landwirtschaftlich verträgliches Verdünnungsmittel bzw. ein Träger.

**2.** Insektizide Zusammensetzung nach Anspruch 1, bei der beim insektizid-wirksamen Bestandteil, wenn X und X' 0 und A und B ein unsubstituiertes Phenyl sind, $R^1$ nicht Isopropyl ($-CH(CH_3)_2$), 2-Methylpropyl ($-CH_2CH(CH_3)_2$), 3-Methylbutyl ($-CH_2CH_2CH(CH_3)_2$), Neopentyl (2,2-dimethylpropyl: $-CH_2C(CH_3)_3$) oder Cyclohexylmethyl ($-CH_2C_6H_{11}$) ist und ferner mit der Maßgabe, daß wenn X und $X^1$ 0 sind und $R^1$ t-Butyl ($-C(CH_3)_3$) und A ein unsubstituiertes Phenyl ist, B nicht 4-Nitrophenyl ist.

**3.** Zusammensetzung nach Anspruch 1 oder 2, bei der X, X' und R wie angegeben sind und A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)-alkyl, ($C_1$-$C_6$)-Alkoxy, Halogen($C_1$-$C_6$)alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein $-OCO_2R$-Rest, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyl-carbonyl, ($C_2$-$C_6$)-Alkynyl, das gegebenenfalls mit Halogen oder ($C_1$-$C_4$)-Alkyl substituiert ist, ein $-RCO_2R'$Rest, ein -COR-Rest, Halogen($C_1$-$C_6$)-Alkyl-carbonyl, ein $-CO_2R$-Rest, Halogen($C_1$-$C_6$)-Alkoxy-carbonyl, ein -OCOR-Rest, ein -NRR'-Rest, ein -CONRR'-Rest, ein -OCONRR'-Rest, ein -NRCOR'-Rest, ein $-NRCO_2R'$-Rest, Thiocyanato, ($C_1$-$C_6$)-Alkyl-thio, ein -S(O)R-Rest, ein $-SO_2R$-Rest, ein $-OSO_2R$-Rest, ein $-SO_2NRR'$-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl,($C_1$-$C_4$)-Alkoxy, Carboxy, ein $-NH_2$-Rest, ein -NHZ-Rest oder ein -NZZ'-Rest sein können, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein $-NH_2$-Rest, ein -NHZ-Rest oder ein -NZZ'-Rest sein können, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein $-NH_2$-Rest, ein -NHZ-Rest oder ein -NZZ'-Rest sein können, $-CR=N-R^2$, bei dem $R^2$ Hydroxy, ($C_1$-$C_4$)-Alkyl, ($C_1$-$C_4$)-Alkoxy, Amino, Phenylamino, -COR oder Benzoyl ist, sein können, oder wenn zwei benachbarte Positionen auf dem Phenylring mit Alkoxy-Resten substituiert sind, diese Gruppen verbunden sein können, um zusammen mit den C-Atomen, mit denen sie verknüpft sind, einen 5- oder 6-gliedrigen dioxolano- bzw. dioxano-heterocyclischen Ring auszubilden.

**4.** Zusammensetzung nach einem der vorhergehenden Ansprüche, bei der $R^1$ nicht mehr als 10 C-Atome enthält.

**5.** Zusammensetzung nach Anspruch 1, 2 oder 4, bei der X und X' O oder S sind, $R^1$ ein unsubstituiertes verzweigtes ($C_3$-$C_8$)-Alkyl oder ein geradkettiges ($C_1$-$C_4$)-Alkyl ist, das mit einem oder zwei des gleichen bzw. eines unterschiedlichen ($C_3$-$C_4$)-Cycloalkyls substituiert ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen($C_1$-$C_4$)-alkyl, Cyano($C_1$-$C_4$)-alkyl, ($C_1$-$C_4$)-Alkoxy, Alkoxyalkyl, das unabhängig 1 bis 4 C-Atome in jedem Alkyl-Rest enthält, $-COD^4$, Carboxy, ($C_1$-$C_4$)-Alkoxy-carbonyl, ($C_1$-$C_4$)-Alkanoyl-oxy, ($C_2$-$C_6$)-Alkenyl, ($C_2$-$C_6$)-Alkynyl, $-ND^4 D^5$, Thiocyanato, ($C_1$-$C_4$)-Alkylthio, $-CSD^4$, ein unsubstituiertes oder substituiertes Phenyl, bei dem ein oder zwei Substituenten gleich oder verschieden voneinander Halogen, Nitro, ($C_1$-$C_4$)-Alkyl, ($C_1$-$C_4$)-Alkoxy, Carboxy, $-ND^4D^5$ sind, Phenoxy, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, $-ND^4D^5$ sind, sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden, wobei $D^4$ und $D^5$ Wasserstoff oder ($C_1$-$C_4$)-Alkyl sind.

**6.** Zusammensetzung nach Anspruch 1, 2 oder 4, bei der X und X' O oder S sind, $R^1$ ein verzweigtes ($C_3$-

$C_8$)-Alkyl ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen($C_1$-$C_4$)-alkyl, Cyano-($C_1$-$C_4$)-alkyl, ($C_1$-$C_4$)-Alkoxy, Alkoxyalkyl, das unabhängig 1 bis 4 C-Atome in jedem Alkyl-Rest enthält, Carbonyl (-$COD^4$), ($C_1$-$C_4$)-Alkoxy-carbonyl, ($C_1$-$C_4$)-Alkanoyloxy, Thiocyanato, ein unsubstituiertes oder substituiertes Phenyl, bei dem ein oder zwei Substituenten gleich oder verschieden voneinander Halogen, Nitro, ($C_1$-$C_4$)-Alkyl, ($C_1$-$C_4$)-Alkoxy, Carboxy, -$NH_2$, -NHZ, -NZZ' sind, oder Phenoxy sind, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, -$NH_2$, -NHZ oder -NZZ' sind, wobei $D^4$, Z und Z' entsprechend ihrer Bedeutung in den Ansprüchen 5 und 3 sind.

7. Zusammensetzung nach Anspruch 1, 2 oder 4, bei der X und X' 0 sind, $R^1$ ein verzweigtes ($C_4$-$C_7$)-Alkyl ist, und A und B gleich oder verschieden voneinander Phenyl oder ein substituiertes Phenyl sind, bei dem ein bis drei Substituenten gleich oder verschieden voneinander Halogen, Nitro, ($C_1$-$C_4$)-Alkyl, ($C_1$-$C_4$)-Alkoxy oder Halogen($C_1$-$C_4$)-alkyl sein können.

8. Zusammensetzung nach Anspruch 1, 2 oder 4, bei der X und X' O sind, $R^1$ t-Butyl, Neopentyl (2,2-dimethylpropyl) oder 1,2,2-Trimethylpropyl ist, A und B gleich oder verschieden voneinander Phenyl oder ein substituiertes Phenyl sind, bei dem ein, zwei oder drei Substituenten gleich oder verschieden voneinander Chlor, Fluor, Brom, Jod, Methyl, Ethyl, Methoxy oder Trifluormethyl sein können.

9. Zusammensetzung nach Anspruch 1, 2 oder 4, bei der X und X' 0 sind, $R^1$ t-Butyl ist, und A und B eine der folgenden Bedeutungen haben:
A ist 4-Methylphenyl und B 3,5-Dimethylphenyl;
A ist Phenyl und B Phenyl;
A ist 4-Methylphenyl und B 3-Methylphenyl;
A ist Phenyl und B 4-Chlorphenyl;
A ist 4-Methylphenyl und B 3,5-Dimethylphenyl;
A ist 2,3-Dimethylphenyl und B 3-Methylphenyl;
A ist 2,3-Dimethylphenyl und B 3,5-Dimethylphenyl;
A ist 2,3-Dimethylphenyl und B 2-Bromphenyl;
A ist 2,3-Dimethylphenyl und B 2,4-Dichlorphenyl;
A ist 2,3-Dimethylphenyl und B 2-Chlor-5-methylphenyl;
A ist Phenyl und B 2-Bromphenyl;
A ist Phenyl und B 3,4-Dichlorphenyl;
A ist 2-Methyl-3-chlorphenyl und B Phenyl;
A ist 2-Methyl-3-chlorphenyl und B 2,4-Dichlorphenyl;
A ist 2-Methyl-3-bromphenyl und B 2,4-Dichlorphenyl;
A ist 2-Chlor-3-methylphenyl und B 2,4-Dichlorphenyl;
A ist 2-Chlor-3-methylphenyl und B 3,5-Dimethylphenyl;
A ist 2,6-Difluorphenyl und B 3,4-Dichlorphenyl;
A ist 2,6-Difluorphenyl und B 2,4-Dichlorphenyl;
A ist 2,6-Difluorphenyl und B 3,5-Dichlorphenyl;
A ist 2-Fluor-6-chlorphenyl und B 2,4-Dichlorphenyl;
A ist 2-Chlorphenyl und B 2,4-Dichlorphenyl;
A ist Phenyl und B 2,4-Dichlorphenyl;
A ist 2-Methyl-3-chlorphenyl und B 3-Fluorphenyl;
A ist 2-Methyl-3-chlorphenyl und B 2-Bromphenyl;
A ist 2-Methyl-3-Bromphenyl und B 3-Methylphenyl;
A ist Phenyl und B 3-Chlor-4-fluorphenyl;
A ist 2-Methyl-3-Bromphenyl und B 4-Chlorphenyl;
oder bei der:
X und X' 0 sind,
$R^1$ 1,2,2-Trimethylpropyl ist,
A 4-Ethylphenyl oder 2,3-Dimethylphenyl ist, und
B 3,5-Dimethylphenyl ist.

10. Zusammensetzung nach einem der vorhergehenden Ansprüche, bei der der wirksame Bestandteil 0,01

bis 75 Gew.-% der Zusammensetzung beträgt.

**11.** Insektizide Zusammensetzung nach einem der vorhergenden Ansprüche in Form eines emulgierbaren Konzentrats, eines benetzbaren Pulvers, eines fließbaren Stoffes, eines Staubes, eines Granulats oder eines Köders.

**12.** Verfahren zur Überwachung von Insekten, umfassend das Inkontaktbringen der Insekten mit einer insektizid-wirksamen Menge einer wie in Anspruch 1 angegebenen insektizid-wirksamen Verbindung, gegebenenfalls in einer Zusammensetzung nach einem der vorhergehenden Ansprüche.

**13.** Verfahren nach Anspruch 12, bei dem die insektizid-wirksame Verbindung wachsenden Pflanzen oder einem Bereich, wo Pflanzen wachsen sollen, in einer Dosierungsrate von 10 g bis 10 kg pro ha zugegeben wird.

**14.** Verfahren nach Anspruch 13, bei dem die Applikationsrate von 100 g bis 5 kg pro ha reicht.

**15.** Verfahren nach einem der Ansprüche 12 bis 14, bei dem die zu überwachenden Insekten der Ordnung Lepidoptera oder Coleoptera angehören.

**16.** Verfahren nach einem der Ansprüche 12 bis 15, bei dem die Applikation so ausgeführt wird, daß Wurzel-Absorption und Transport durch Pflanzen ermöglicht ist.

Patentansprüche für folgenden Vertragsstaat : ES

**1.** Verfahren zur Verbesserung des wirtschaftlichen Werts und/oder der Rentabilität von verkäuflicher Ernte von Pflanzen, deren Wachstum durch Insekten beeinträchtigt ist oder wahrscheinlich beeinträchtigt wird und/oder zur Verbesserung dieses Wachstums, umfassend (1) die Beschickung eines Behälters, einer Räuchervorrichtung oder einer mechanischen Verbreitungsvorrichtung mit einer insektiziden Zusammensetzung, umfassend als insektizid-wirksamen Bestandteil von 0,0001 bis 99 Gew.-% der Zusammensetzung eine Verbindung der Formel:

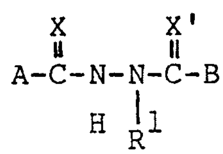

in der X und X' gleich oder verschieden voneinander O, S oder NR sind, $R^1$ ein unsubstituiertes verzweigtes ($C_3$-$C_{10}$)-Alkyl oder ein geradkettiges ($C_1$-$C_4$)-Alkyl ist, das mit einem oder zwei des gleichen bzw. eines unterschiedlichen ($C_3$-$C_6$)-Cycloalkyls substituiert ist, A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)-alkyl, Hydroxy($C_1$-$C_6$)alkyl, ($C_1$-$C_6$)-Alkoxy, Halogen($C_1$-$C_6$)-alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein -ORSR'-Rest, ein -$OCO_2R'$-Rest, Alkanoyl-oxyalkyl, das unabhängig 1 bis 6 C-Atome in jeder Alkylgruppe aufweist, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyloxy, ($C_2$-$C_6$)-Alkenyl-carbonyl, ($C_2$-$C_6$)-Alkenyl-oxycarbonyl, ($C_2$-$C_6$)-Alkynyl, das gegebenenfalls mit Halogen oder ($C_1$-$C_4$)-Alkyl substituiert ist, ein -$RCO_2R'$-Rest, ein -COR-Rest, Halogen($C_1$-$C_6$)-alkyl-carbonyl, ein -$CO_2R$-Rest, Halogen($C_1$-$C_6$)-alkoxy-carbonyl, ein -OCOR-Rest, ein -$ORCO_2R'$-Rest, ein -NRR'-Rest, ein -CONRR'-Rest, ($C_2$-$C_6$)-Alkenyl-carbonylamino, Hydroxy($C_1$-$C_6$)-alkyl-aminocarbonyl, ein -OCONRR'-Rest, ein -NRCOR'-Rest, ein -$NRCO_2R'$-Rest, Thiocyanato, Isothiocyanato, Thiocyanato($C_1$-$C_6$)-alkyl, ($C_1$-$C_6$)-Alkyl-thio, ein -S(O)R-Rest, ein -$SO_2R$-Rest, ein -$OSO_2R$-Rest, ein -$SO_2NRR'$-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl, Halogen($C_1$-$C_4$)-alkyl, ($C_1$-

$C_4$)-Alkoxy, Carboxy, ein -$NH_2$-Rest, ein -NHZ-Rest oder ein -NZZ'-Rest sind, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein -$NH_2$-Rest, ein -NHZ-Rest oder ein -NZZ'-Rest sind, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein -$NH_2$-Rest, ein -NHZ-Rest oder ein -NZZ'-Rest sind, Benzoyloxy($C_1$-$C_6$)-alkyl, Phenylthio($C_1$-$C_6$)-alkyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, Halogen($C_1$-$C_4$)-alkyl, ($C_1$-$C_4$)-Alkoxy, Carboxy, ein -$NH_2$-Rest, ein -NHZ-Rest oder ein -NZZ'-Rest sind, ein -CR=N-$R^2$-Rest, bei dem $R^2$ Hydroxy, ($C_1$-$C_4$)-Alkyl, ($C_1$-$C_4$)-Alkoxy, -NRR', Phenylamino, -COR oder Benzoyl ist, ($C_2$-$C_6$)-Oxiranyl, Acethylthiosemicarbazon, Pyrrolyl, ein unsubstituiertes oder mit ein oder zwei Methyl-Resten substituiertes Oxazolyl sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden, wobei R und R' Wasserstoff oder ($C_1$-$C_6$)-Alkyl sind, Z und Z' ($C_1$-$C_4$)-Alkyl sind und "Amino" NRR' bedeutet, und ihre landwirtschaftlich verträglichen Salze sowie ein landwirtschaftlich verträgliches Verdünnungsmittel bzw. ein Träger, (2) die Verwendung des Behälters, der Räuchervorrichtung oder der mechanischen Verbreitungsvorrichtung zur Verabreichung der insektiziden Zusammensetzung in Form von Granulat, Staub, Rauch, Dampf oder einer ein Tensid enthaltenden Flüssigkeits-Zubereitung wachsenden Pflanzen oder einem Wachstumsmedium, in dem Pflanzen wachsen oder wachsen sollen, oder Insekten selbst, und (3) die Überwachung der Dosis des wirksamen Bestandteils während dieser Applikationsstufe, so daß die Applikationsrate der insektizid-wirksamen Verbindung ausreichend ist, die Insekten zu bekämpfen, nicht aber genügend ist, einen unannehmbar nachteiligen Effekt auf die Ernte-Pflanzen, die wachsen oder in dem behandelten Bereich wachsen sollen, zu verursachen.

2. Verfahren zur Verbreitung nach Anspruch 1, bei dem in der insektiziden Verbindung, wenn X und X' 0 und A und B ein unsubstituiertes Phenyl sind, $R^1$ nicht Isopropyl (-$CH(CH_3)_2$), 2-Methylpropyl (-$CH_2CH(CH_3)_2$), 3-Methylbutyl (-$CH_2CH_2CH(CH_3)_2$), Neopentyl (2,2-dimethylpropyl: -$CH_2C(CH_3)_3$) oder Cyclohexylmethyl (-$CH_2C_6H_{11}$) ist und ferner mit der Maßgabe, daß wenn X und X' 0 sind und $R^1$ t-Butyl (-$C(CH_3)_3$) und A ein unsubstituiertes Phenyl ist, B nicht 4-Nitrophenyl ist.

3. Verfahren zur Herstellung einer insektizid-wirksamen Verbindung der Formel

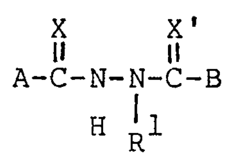

in der X, X', $R^1$, A und B wie in Anspruch 1 angegeben und wie in Anspruch 2 beschränkt sind, umfassend die Umsetzung eines ersten Reaktionspartners (I), der den Substituenten A enthält, mit einem zweiten Reaktionspartner (II), der den Substituenten B enthält, in Gegenwart einer Base und eines Lösungsmittels, wobei, wenn X und X' 0 sind

(a) der Reaktionspartner I die Formel 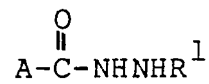 und

der Reaktionspartner II die Formel 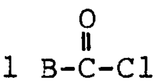 hat, oder

(b) der Reaktionspartner I die Formel 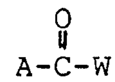 und

der Reaktionspartner II die Formel 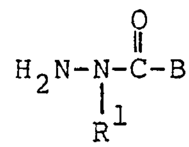 hat,

wobei W ein leicht abspaltbarer Rest ist, oder wenn zumindest einer von X und X' S ist,

(c) der Reaktionspartner I die Formel 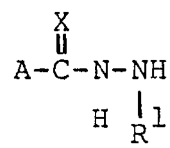 und

der Reaktionspartner II die Formel 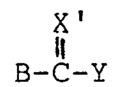 hat, wobei Y ein leicht abspaltbarer Rest ist.

4. Verfahren zur Herstellung nach Anspruch 3, bei dem in der Umsetzung (a) $R^1$ ein Rest der Formel

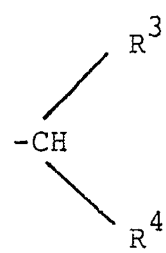

ist, in der $R^3$ und $R^4$ gleich oder verschieden voneinander Wasserstoff oder ein unsubstituiertes verzweigtes ($C_3$-$C_9$)-Alkyl oder ein unsubstituiertes geradkettiges ($C_1$-$C_8$)-Alkyl oder ($C_3$-$C_6$)-Cycloalkyl oder ein geradkettiges ($C_1$-$C_3$)-Alkyl sind, das mit ein oder zwei eines ($C_3$-$C_6$)-Cycloalkyls substituiert ist, oder in der Umsetzung (b) W-Cl ist.

5. Verfahren zur Verbreitung oder Herstellung nach einem der vorhergehenden Ansprüche, bei dem X, X' und R wie angegeben sind und A und B gleich oder verschieden voneinander ein unsubstituiertes oder substituiertes Naphthyl, bei dem ein bis drei Substituenten gleich oder verschieden voneinander Halogen, Nitro, ($C_1$-$C_4$)-Alkoxy, ($C_1$-$C_4$)-Alkyl oder Amino sein können, oder ein unsubstituiertes oder substituiertes Phenyl sind, bei dem ein bis fünf Substituenten gleich oder verschieden voneinander Halogen, Nitro, Cyano, Hydroxy, ($C_1$-$C_6$)-Alkyl, Halogen($C_1$-$C_6$)-alkyl, Cyano($C_1$-$C_6$)-alkyl, ($C_1$-$C_6$)-Alkoxy, Halogen($C_1$-$C_6$)-alkoxy, Alkoxyalkyl, das unabhängig 1 bis 6 C-Atome in jeder Alkylgruppe aufweist, Alkoxyalkoxy, das unabhängig 1 bis 6 C-Atome in jeder Alkylgruppe aufweist, ein $-OCO_2R$-Rest, ($C_2$-$C_6$)-Alkenyl, das gegebenenfalls mit Halogen, Cyano, ($C_1$-$C_4$)-Alkyl oder ($C_1$-$C_4$)-Alkoxy substituiert ist, ($C_2$-$C_6$)-Alkenyl-carbonyl, ($C_2$-$C_6$)-Alkynyl, das gegebenenfalls mit Halogen oder ($C_1$-

$C_4$)-Alkyl substituiert ist, ein $-RCO_2R$'Rest, ein -COR-Rest, Halogen($C_1$-$C_6$)-Alkyl-carbonyl, ein $-CO_2R$-Rest, Halogen($C_1$-$C_6$)-Alkoxy-carbonyl, ein -OCOR-Rest, ein -NRR'-Rest, ein -CONRR'-Rest, ein -OCONRR'-Rest, ein -NRCOR'-Rest, ein $-NRCO_2R$'-Rest, Thiocyanato, ($C_1$-$C_6$)-Alkyl-thio, ein -S(O)R-Rest, ein $-SO_2R$-Rest, ein $-OSO_2R$-Rest, ein $-SO_2NRR$'-Rest, ein -CSR-Rest, ein -NRCSR'-Rest, ein unsubstituiertes oder substituiertes Phenyl, bei dem ein bis drei Substituenten gleich oder verschieden voneinander Halogen, Cyano, Nitro, ($C_1$-$C_4$)-Alkyl,($C_1$-$C_4$)Alkoxy, Carboxy, ein $-NH_2$-Rest, ein -NHZ-Rest oder ein -NZZ'-Rest sein können, Phenoxy, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein $-NH_2$-Rest, ein -NHZ-Rest oder ein -NZZ'-Rest sein können, Benzoyl, bei dem der Phenylring unsubstituiert oder substituiert ist, wobei ein bis drei Substituenten gleich oder verschieden voneinander Halogen, Cyano, Nitro, ($C_1$-$C_4$)-Alkyl, ($C_1$-$C_4$)-Alkoxy, Carboxy, ein $-NH_2$-Rest, ein -NHZ-Rest oder ein -NZZ'-Rest sein können, $-CR=N-R^2$, bei dem $R^2$ Hydroxy, ($C_1$-$C_4$)-Alkyl, ($C_1$-$C_4$)-Alkoxy, Amino, Phenylamino, -COR oder Benzoyl ist, sein können, oder wenn zwei benachbarte Positionen auf dem Phenylring mit Alkoxy-Resten substituiert sind, diese Gruppen verbunden sein können, um zusammen mit den C-Atomen, mit denen sie verknüpft sind, einen 5- oder 6-gliedrigen dioxolano- bzw. dioxano-heterocyclischen Ring auszubilden.

**6.** Verfahren zur Verbreitung oder Herstellung nach einem der vorhergehenden Ansprüche, bei dem $R^1$ nicht mehr als 10 C-Atome enthält.

**7.** Verfahren zur Verbreitung oder Herstellung nach einem der Ansprüche 1 bis 4 und 6, bei dem X und X' O oder S sind, $R^1$ ein unsubstituiertes verzweigtes ($C_3$-$C_8$)-Alkyl oder ein geradkettiges ($C_1$-$C_4$)-Alkyl ist, das mit einem oder zwei des gleichen bzw. eines unterschiedlichen ($C_3$-$C_4$)-Cycloalkyls substituiert ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen($C_1$-$C_4$)-alkyl, Cyano($C_1$-$C_4$)alkyl, ($C_1$-$C_4$)-Alkoxy, Alkoxyalkyl, das unabhängig 1 bis 4 C-Atome in jedem Alkyl-Rest enthält, $-COD^4$, Carboxy, ($C_1$-$C_4$)-Alkoxy-carbonyl, ($C_1$-$C_4$)-Alkanoyl-oxy, ($C_2$-$C_6$)-Alkenyl, ($C_2$-$C_6$)-Alkynyl, $-ND^4D^5$, Thiocyanato, ($C_1$-$C_4$)-Alkylthio, $-CSD^4$, ein unsubstituiertes oder substituiertes Phenyl, bei dem ein oder zwei Substituenten gleich oder verschieden voneinander Halogen, Nitro, ($C_1$-$C_4$)-Alkyl, ($C_1$-$C_4$)-Alkoxy, Carboxy, $-ND^4D^5$ sind, Phenoxy, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, $-ND^4D^5$ sind, sein können, oder wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen dioxolano- bzw. dioxano-heterocyclischen Ring auszubilden, wobei $D^4$ und $D^5$ Wasserstoff oder ($C_1$-$C_4$)-Alkyl sind.

**8.** Verfahren zur Verbreitung oder Herstellung nach einem der Ansprüche 1 bis 4 und 6, bei dem X und X' O oder S sind, $R^1$ ein verzweigtes ($C_3$-$C_8$)-Alkyl ist, A und B gleich oder verschieden voneinander ein unsubstituiertes Naphthyl, oder ein unsubstituiertes oder substituiertes Phenyl sind, bei dem ein bis drei Substituenten gleich oder verschieden voneinander Halogen, Nitro, Cyano, ($C_1$-$C_4$)-Alkyl, Halogen-($C_1$-$C_4$)-alkyl, Cyano-($C_1$-$C_4$)-alkyl, ($C_1$-$C_4$)-Alkoxy, Alkoxyalkyl, das unabhängig 1 bis 4 C-Atome in jedem Alkyl-Rest enthält, Carbonyl ($-COD^4$), ($C_1$-$C_4$)-Alkoxy-carbonyl, ($C_1$-$C_4$)-Alkanoyloxy, Thiocyanato, ein unsubstituiertes oder substituiertes Phenyl, bei dem ein oder zwei Substituenten gleich oder verschieden voneinander Halogen, Nitro, ($C_1$-$C_4$)-Alkyl, ($C_1$-$C_4$)-Alkoxy, Carboxy, $-NH_2$, -NHZ, -NZZ' sind, oder Phenoxy sind, bei dem der Phenylring unsubstituiert oder substituiert ist, wobei ein oder zwei Substituenten gleich oder verschieden voneinander Halogen, Nitro, ($C_1$-$C_4$)-Alkyl, ($C_1$-$C_4$)-Alkoxy, Carboxy, $-NH_2$, -NHZ oder -NZZ' sind, wobei $D^4$, Z und Z' entsprechend ihrer Bedeutung in den Ansprüchen 5 und 3 sind.

**9.** Verfahren zur Verbreitung oder Herstellung nach einem der Ansprüche 1 bis 4 und 6, bei dem X und X' 0 sind, $R^1$ ein verzweigtes ($C_4$-$C_7$)-Alkyl ist, und A und B gleich oder verschieden voneinander Phenyl oder ein substituiertes Phenyl sind, bei dem ein bis drei Substituenten gleich oder verschieden voneinander Halogen, Nitro, ($C_1$-$C_4$)-Alkyl, ($C_1$-$C_4$)-Alkoxy oder Halogen($C_1$-$C_4$)-alkyl sein können.

**10.** Verfahren zur Verbreitung oder Herstellung nach einem der Ansprüche 1 bis 4 und 6, bei dem X und X' O sind, $R^1$ t-Butyl, Neopentyl (2,2-dimethylpropyl) oder 1,2,2-Trimethylpropyl ist, A und B gleich oder verschieden voneinander Phenyl oder ein substituiertes Phenyl sind, bei dem ein, zwei oder drei

Substituenten gleich oder verschieden voneinander Chlor, Fluor, Brom, Jod, Methyl, Ethyl, Methoxy oder Trifluormethyl sein können.

**11.** Verfahren zur Verbreitung oder Herstellung nach einem der Ansprüche 1 bis 4 und 6, bei dem X und X' 0 sind, $R^1$ t-Butyl ist, und A und B eine der folgenden Bedeutungen haben:
A ist 4-Methylphenyl und B 3,5-Dimethylphenyl;
A ist Phenyl und B Phenyl;
A ist 4-Methylphenyl und B 3-Methylphenyl;
A ist Phenyl und B 4-Chlorphenyl;
A ist 4-Methylphenyl und B 3,5-Dimethylphenyl;
A ist 2,3-Dimethylphenyl und B 3-Methylphenyl;
A ist 2,3-Dimethylphenyl und B 3,5-Dimethylphenyl;
A ist 2,3-Dimethylphenyl und B 2-Bromphenyl;
A ist 2,3-Dimethylphenyl und B 2,4-Dichlorphenyl;
A ist 2,3-Dimethylphenyl und B 2-Chlor-5-methylphenyl;
A ist Phenyl und B 2-Bromphenyl;
A ist Phenyl und B 3,4-Dichlorphenyl;
A ist 2-Methyl-3-chlorphenyl und B Phenyl;
A ist 2-Methyl-3-chlorphenyl und B 2,4-Dichlorphenyl;
A ist 2-Methyl-3-bromphenyl und B 2,4-Dichlorphenyl;
A ist 2-Chlor-3-methylphenyl und B 2,4-Dichlorphenyl;
A ist 2-Chlor-3-methylphenyl und B 3,5-Dimethylphenyl;
A ist 2,6-Difluorphenyl und B 3,4-Dichlorphenyl;
A ist 2,6-Difluorphenyl und B 2,4-Dichlorphenyl;
A ist 2,6-Difluorphenyl und B 3,5-Dichlorphenyl;
A ist 2-Fluor-6-chlorphenyl und B 2,4-Dichlorphenyl;
A ist 2-Chlorphenyl und B 2,4-Dichlorphenyl;
A ist Phenyl und B 2,4-Dichlorphenyl;
A ist 2-Methyl-3-chlorphenyl und B 3-Fluorphenyl;
A ist 2-Methyl-3-chlorphenyl und B 2-Bromphenyl;
A ist 2-Methyl-3-Bromphenyl und B 3-Methylphenyl;
A ist Phenyl und B 3-Chlor-4-fluorphenyl;
A ist 2-Methyl-3-Bromphenyl und B 4-Chlorphenyl;
oder bei der:
X und X' 0 sind,
$R^1$ 1,2,2-Trimethylpropyl ist,
A 4-Ethylphenyl oder 2,3-Dimethylphenyl ist, und
B 3,5-Dimethylphenyl ist.

**12.** Verfahren zur Verbreitung nach einem der Ansprüche 1, 2 und 5 bis 11, bei dem der wirksame Bestandteil 0,01 bis 75 Gew.-% der Zusammensetzung beträgt.

**13.** Verfahren zur Verbreitung nach einem der Ansprüche 1, 2 und 5 bis 12, bei dem die Zusammensetzung in Form eines emulgierbaren Konzentrats, eines benetzbaren Pulvers, eines fließbaren Stoffes, eines Staubes, eines Granulats oder eines Köders verwendet wird.

**14.** Verfahren zur Verbreitung nach einem der Ansprüche 1, 2 und 5 bis 13, bei dem die insektizidwirksame Verbindung in Stufe (2) wachsenden Pflanzen oder einem Bereich zugegeben wird, in dem Pflanzen wachsen sollen, und in Stufe (3) die Dosierungsrate von 10 g bis 10 kg pro ha eingehalten wird.

**15.** Verfahren zur Verbreitung nach einem der Ansprüche 1, 2 und 5 bis 14, bei dem in Stufe (3) die Dosierung mit einer Applikationsrate von 100 g bis 5 kg pro ha eingehalten wird.

**16.** Verfahren zur Verbreitung nach einem der Ansprüche 1, 2 und 5 bis 15, bei dem die Insekten der Ordnung Lepidoptera oder Coleoptera angehören.